# EUROPEAN PATENT APPLICATION

(11) **EP 1 535 610 A1**
(43) Date of publication of application: **01.06.2005**
(21) Application number: 03730832.7
(22) Date of filing: 05.06.2003
(51) Int. Cl.: A61K 31/167, A61K 31/18, A61K 31/381, A61K 31/40, A61K 31/404, A61K 31/4164, A61K 31/421, A61K 31/422, A61K 31/426, A61K 31/437, A61K 31/4402, A61K 31/445, A61K 31/451, A61K 31/255, A61K 31/47, A61K 31/505, A61K 31/498, A61K 31/5375, A61K 31/616, A61P 35/00, A61P 35/02

(54) **THERAPEUTIC AGENT FOR CANCER**

(30) Priority: 10.06.2002 JP 2002168332
(71) Applicant: Institute of Medicinal Molecular Design, Inc., Tokyo 113-0033 (JP)
(72) Inventor: MUTO, Susumu, Koganei-shi, Tokyo 184-0003 (JP); ITAI,A., INSTITUTE OF MEDICINAL MOLECULAR DESIGN, Hongo 5-chome, Bunkyo-ku, Tokyo 113-0 (JP)
(74) Representative: Sternagel, Fleischer, Godemeyer & Partner
(86) International application number: PCT/JP2003/007121
(87) International publication number: WO 2003/103655

(57) **Abstract**

A medicament for the prevention and/or treatment of cancers and the like which comprises as an active ingredient a substance selected from the group consisting of a compound represented by the following general formula (I) and a pharmacologically acceptable salt thereof, and a hydrate thereof and a solvate thereof: 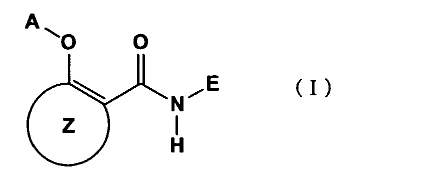 wherein A represents hydrogen atom or acetyl group,
E represents a 2,5-di-substituted or a 3,5-di-substituted phenyl group, or a monocyclic or a fused polycyclic heteroaryl group which may be substituted, provided that the compound wherein said heteroaryl group is ① a fused polycyclic heteroaryl group wherein the ring which binds directly to ―CONH― group in the formula (I) is a benzene ring, ② unsubstituted thiazol-2-yl group, or ③ unsubstituted benzothiazol-2-yl group is excluded,
ring Z represents an arene which may have one or more substituents in addition to the group represented by formula ―O―A wherein A has the same meaning as that defined above and the group represented by formula ―CONH―E wherein E has the same meaning as that defined above, or a heteroarene which may have one or more substituents in addition to the group represented by formula ―O―A wherein A has the same meaning as that defined above and the group represented by formula ― CONH―E wherein E has the same meaning as that defined above.

## Description

### Field of Invention

The present invention relates to a medicament which can terminate proliferation of cancer cells which proliferate randomly, and enables preventive and/or therapeutic treatment of cancers by inducing apoptosis of immortalized cancer cells.

### Background Art

N-Phenylsalicylamide derivatives are disclosed as a plant growth inhibitor in the specification of U.S. Patent No.4,358,443. As medicaments, said derivatives are disclosed as anti-inflammatory agents in the specification of European Patent No.0,221,211, Japanese Patent Unexamined Publication (KOKAI) No.(Sho)62-99329, and the specification of U.S. Patent No.6,117,859. Furthermore, they are disclosed as NF-κB inhibitors in the pamphlets of International Publication WO99/65499, International Publication WO02/49632, and International Publication WO02/076918. N-Phenylsalicylamide derivatives are suggested as an anticancer agent in the pamphlets of International Publication WO99/65499, International Publication WO02/49632, and International Publication WO02/076918. However, absolutely no data that directly indicate usefulness of those derivatives as anticancer agents is disclosed. Moreover, in the pamphlet of International Publication WO99/65449, only a small number of compounds were tested for inhibitory activity against NF-κB, and as for a position of a substituent on the aniline moiety, studies were made on very limited compounds. N-Phenylsalicylamide derivatives are disclosed as an inhibitor against production of cytokines in the pamphlet of International Publication WO02/051397.

### Disclosure of the Invention

An object of the present invention is to provide anticancer agents having superior effectiveness and reduced side effects. The inventors of the present invention conducted various studies on anticancer actions of salicylamide derivatives which are generally considered to have low toxicity. As a result, they found that N-substituted salicylamide derivatives, particularly, N-arylsalicylamide derivatives, have superior activity to have cancer cells trigger apoptosis, and that, even within an effective dose ranges, said derivatives have no actions that relate to side effects observed with available anticancer agents such as hepatic disorder, renal disorder, or myerosuppression. The inventors further conducted similar studies on hydroxyaryl derivatives which are analogous compounds thereof. The present invention was achieved on the basis of these findings.

The present invention thus provides:
(1) A medicament for the preventive and/or therapeutic treatment of a cancer which comprises as an active ingredient a substance selected from the group consisting of a compound represented by the following general formula (I) and a pharmacologically acceptable salt thereof, and a hydrate thereof and a solvate thereof: 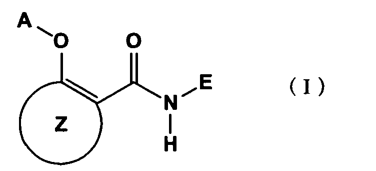 wherein A represents hydrogen atom or acetyl group,
   E represents a 2,5-di-substituted or a 3,5-di-substituted phenyl group, or a monocyclic or a fused polycyclic heteroaryl group which may be substituted, provided that the compound wherein said heteroaryl group is ① a fused polycyclic heteroaryl group wherein the ring which binds directly to ―CONH― group in the formula (I) is a benzene ring, ② unsubstituted thiazol-2-yl group, or ③ unsubstituted benzothiazol-2-yl group is excluded,
   ring Z represents an arene which may have one or more substituents in addition to the group represented by formula ―O―A wherein A has the same meaning as that defined above and the group represented by formula ―CONH―E wherein E has the same meaning as that defined above, or a heteroarene which may have one or more substituents in addition to the group represented by formula ―O―A wherein A has the same meaning as that defined above and the group represented by formula ― CONH―E wherein E has the same meaning as that defined above.
   Examples of preferred medicaments of the present invention include:
(2) the aforementioned medicament which comprises as an active ingredient a substance selected from the group consisting of the compound and a pharmacologically acceptable salt thereof, and a hydrate thereof and a solvate thereof, wherein A is a hydrogen atom;
(3) the aforementioned medicament which comprises as an active ingredient a substance selected from the group consisting of the compound and a pharmacologically acceptable salt thereof, and a hydrate thereof and a solvate thereof, wherein ring Z is a C₆ to C₁₀ arene which may have one or more substituents in addition to the group represented by formula ―O―A wherein A has the same meaning as that defined in the general formula (I) and the group represented by formula ―CONH―E wherein E has the same meaning as that defined in the general formula (I), or a 5 to 10-membered heteroarene which may have one or more substituents in addition to the group represented by formula ―O―A wherein A has the same meaning as that defined in the general formula (I) and the group represented by formula ―CONH―E wherein E has the same meaning as that defined in the general formula (I);
(4) the aforementioned medicament which comprises as an active ingredient a substance selected from the group consisting of the compound and a pharmacologically acceptable salt thereof, and a hydrate thereof and a solvate thereof, wherein ring Z is a benzene ring which may have one or more substituents in addition to the group represented by formula ―O―A wherein A has the same meaning as that defined in the general formula (I) and the group represented by formula ―CONH―E wherein E has the same meaning as that defined in the general formula (I), or a naphthalene ring which may have one or more substituents in addition to the group represented by formula ―O―A wherein A has the same meaning as that defined in the general formula (I) and the group represented by formula ―CONH―E wherein E has the same meaning as that defined in the general formula (I);
(5) the aforementioned medicament which comprises as an active ingredient a substance selected from the group consisting of a compound represented by the following general formula (I) and a pharmacologically acceptable salt thereof, and a hydrate thereof and a solvate thereof, wherein ring Z is a benzene ring which is substituted with halogen atom(s) in addition to the group represented by formula ― O ―A wherein A has the same meaning as that defined in the general formula (I) and the group represented by formula ―CONH―E wherein E has the same meaning as that defined in the general formula (I);
(6) the aforementioned medicament which comprises as an active ingredient a substance selected from the group consisting of the compound and a pharmacologically acceptable salt thereof, and a hydrate thereof and a solvate thereof, wherein ring Z is a naphthalene ring which may have one or more substituents in addition to the group represented by formula ―O―A wherein A has the same meaning as that defined in the general formula (I) and the group represented by formula ―CONH―E wherein E has the same meaning as that defined in the general formula (I);
(7) a medicament having inhibitory activity against NF-κB activation which comprises as an active ingredient a substance selected from the group consisting of a compound represented by the following general formula (I) and a pharmacologically acceptable salt thereof, and a hydrate thereof and a solvate thereof, wherein E is a 2,5-di-substituted phenyl group or a 3,5-di-substituted phenyl group;
(8) the aforementioned medicament which comprises as an active ingredient a substance selected from the group consisting of the compound and a pharmacologically acceptable salt thereof, and a hydrate thereof and a solvate thereof, wherein E is a 2,5-di-substituted phenyl group wherein at least one of said substituents is trifluoromethyl group, or a 3,5-di-substituted phenyl group wherein at least one of said substituents is trifluoromethyl group;
(9) the aforementioned medicament which comprises as an active ingredient a substance selected from the group consisting of the compound and a pharmacologically acceptable salt thereof, and a hydrate thereof and a solvate thereof, wherein E is 3,5-bis(trifluoromethyl)phenyl group;
(10) the aforementioned medicament which comprises as an active ingredient a substance selected from the group consisting of the compound and a pharmacologically acceptable salt thereof, and a hydrate thereof and a solvate thereof, wherein E is a monocyclic or a fused polycyclic heteroaryl group which may be substituted, provided that the compound wherein said heteroaryl group is ① a fused polycyclic heteroaryl group wherein the ring which binds directly to ―CONH― group in the formula (I) is a benzene ring, ② unsubstituted thiazol-2-yl group, or ③ unsubstituted benzothiazol-2-yl group is excluded;
(11) the aforementioned medicament which comprises as an active ingredient a substance selected from the group consisting of the compound and a pharmacologically acceptable salt thereof, and a hydrate thereof and a solvate thereof, wherein E is a 5-membered monocyclic heteroaryl group which may be substituted, provided that the compounds wherein said heteroaryl group is unsubstituted thiazol-2-yl group are excluded.

From another aspect, the present invention provides use of each of the substances for manufacture of the medicament according to the aforementioned (1) to (11). The present invention further provides a method for preventive and/or therapeutic treatment of cancers in a mammal including a human, which comprises the step of administering a preventively and/or therapeutically effective amount of each of the aforementioned substances to a mammal including a human.

### Brief Explanation of the Drawings

Fig. 1 shows an inhibitory activity of the medicament of the present invention (Compound No.4) against the proliferation of cancer cells (B16 melanoma).

Fig. 2 shows an inhibitory activity of the medicament of the present invention (Compound No.4) against the proliferation of cancer cells (HT-1080 fibrosarcoma).

Fig. 3 shows an inhibitory activity of the medicament of the present invention (Compound No.4) against the proliferation of cancer cells (NB-1 neuroblastoma).

Fig. 4 shows an inhibitory activity of the medicament of the present invention (Compound No.4) against the proliferation of cancer cells (HMC-1-8 breast cancer).

Fig. 5 shows an anticancer activity of the medicament of the present invention (Compound No.4) against tumors.

### Best Mode for Carrying out the Invention

Reference to the disclosure of the pamphlet of International Publication WO02/49632 is useful for better understanding of the present invention. The entire disclosure of the aforementioned pamphlet of International Publication WO02/49632 is incorporated by reference in the disclosures of the present specification.

The terms used in the present specification have the following meanings.

As the halogen atom, any of fluorine atom, chlorine atom, bromine atom, or iodine atom may be used unless otherwise specifically referred to.

Examples of the hydrocarbon group include, for example, an aliphatic hydrocarbon group, an aryl group, an arylene group, an aralkyl group, a bridged cyclic hydrocarbon group, a spiro cyclic hydrocarbon group, and a terpene hydrocarbon.

Examples of the aliphatic hydrocarbon group include, for example, alkyl group, alkenyl group, alkynyl group, alkylene group, alkenylene group, alkylidene group and the like which are straight chain or branched chain monovalent or bivalent acyclic hydrocarbon groups; cycloalkyl group, cycloalkenyl group, cycloalkanedienyl group, cycloalkyl-alkyl group, cycloalkylene group, and cycloalkenylene group, which are saturated or unsaturated monovalent or bivalent alicyclic hydrocarbon groups.

Examples of the alkyl group include, for example, methyl, ethyl, n-propyl, isopropyl, n-butyl, isobutyl, sec-butyl, tert-butyl, n-pentyl, isopentyl, 2-methylbutyl, 1-methylbutyl, neopentyl, 1,2-dimethylpropyl, 1-ethylpropyl, n-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-ethylbutyl, 1-ethyl-1-methylpropyl, n-heptyl, n-octyl, n-nonyl, n-decyl, n-undecyl, n-dodecyl, n-tridecyl, n-tetradecyl, and n-pentadecyl, which are C₁ to C₁₅ straight chain or branched chain alkyl groups.

Examples of the alkenyl group include, for example, vinyl, prop-1-en-1-yl, allyl, isopropenyl, but-1-en-1-yl, but-2-en-1-yl, but-3-en-1-yl, 2-methylprop-2-en-1-yl, 1-methylprop-2-en-1-yl, pent-1-en-1-yl, pent-2-en-1-yl, pent-3-en-1-yl, pent-4-en-1-yl, 3-methylbut-2-en-1-yl, 3-methylbut-3-en-1-yl, hex-1-en-1-yl, hex-2-en-1-yl, hex-3-en-1-yl, hex-4-en-1-yl, hex-5-en-1-yl, 4-methylpent-3-en-1-yl, 4-methylpent-3-en-1-yl, hept-1-en-1-yl, hept-6-en-1-yl, oct-1-en-1-yl, oct-7-en-1-yl, non-1-en-1-yl, non-8-en-1-yl, dec-1-en-1-yl, dec-9-en-1-yl, undec-1-en-1-yl, undec-10-en-1-yl, dodec-1-en-1-yl, dodec-11-en-1-yl, tridec-1-en-1-yl, tridec-12-en-1-yl, tetradec-1-en-1-yl, tetradec-13-en-1-yl, pentadec-1-en-1-yl, and pentadec-14-en-1-yl, which are C₂ to C₁₅ straight chain or branched chain alkenyl groups.

Examples of the alkynyl group include, for example, ethynyl, prop-1-yn-1-yl, prop-2-yn-1-yl, but-1-yn-1-yl, but-3-yn-1-yl, 1-methylprop-2-yn-1-yl, pent-1-yn-1-yl, pent-4-yn-1-yl, hex-1-yn-1-yl, hex-5-yn-1-yl, hept-1-yn-1-yl, hept-6-yn-1-yl, oct-1-yn-1-yl, oct-7-yn-1-yl, non-1-yn-1-yl, non-8-yn-1-yl, dec-1-yn-1-yl, dec-9-yn-1-yl, undec-1-yn-1-yl, undec-10-yn-1-yl, dodec-1-yn-1-yl, dodec-11-yn-1-yl, tridec-1-yn-1-yl, tridec-12-yn-1-yl, tetradec-1-yn-1-yl, tetradec-13-yn-1-yl, pentadec-1-yn-1-yl, and pentadec-14-yn-1-yl, which are C₂ to C₁₅ straight chain or branched chain alkynyl groups.

Examples of the alkylene group include, for example, methylene, ethylene, ethane-1,1-diyl, propane-1,3-diyl, propane-1,2-diyl, propane-2,2-diyl, butane-1,4-diyl, pentane-1,5-diyl, hexane-1,6-diyl, and 1,1,4,4-tetramethylbutane-1,4-diyl group, which are C₁ to C₈ straight chain or branched chain alkylene groups.

Examples of the alkenylene group include, for example, ethene-1,2-diyl, propene-1,3-diyl, but-1-ene-1,4-diyl, but-2-ene-1,4-diyl, 2-methylpropene-1,3-diyl, pent-2-ene-1,5-diyl, and hex-3-ene-1,6-diyl, which are C₁ to C₆ straight chain or branched chain alkylene groups.

Examples of the alkylidene group include, for example, methylidene, ethylidene, propylidene, isopropylidene, butylidene, pentylidene, and hexylidene, which are C₁ to C₆ straight chain or branched chain alkylidene groups.

Examples of the cycloalkyl group include, for example, cyclopropyl, cyclobutyl, cyclopentyl, cyclohexyl, cycloheptyl, and cyclooctyl, which are C₃ to C₈ cycloalkyl groups.

The aforementioned cycloalkyl group may be fused with benzene ring, naphthalene ring and the like, and examples include, for example, 1-indanyl, 2-indanyl, 1,2,3,4-tetrahydronaphthalen-1-yl, and 1,2,3,4-tetrahydronaphthalen-2-yl.

Examples of the cycloalkenyl group include, for example, 2-cyclopropen-1-yl, 2-cyclobuten-1-yl, 2-cyclopenten-1-yl, 3-cyclopenten-1-yl, 2-cyclohexen-1-yl, 3-cyclohexen-1-yl, 1-cyclobuten-1-yl, and 1-cyclopenten-1-yl, which are C₃ to C₆ cycloalkenyl groups.

The aforementioned cycloalkenyl group may be fused with benzene ring, naphthalene ring and the like, and examples include, for example, 1-indanyl, 2-indanyl, 1,2,3,4-tetrahydronaphthalen-1-yl, 1,2,3,4-tetrahydronaphthalen-2-yl, 1-indenyl, and 2-indenyl.

Examples of the cycloalkanedienyl group include, for example, 2,4-cyclopentadien-1-yl, 2,4-cyclohexanedien-1-yl, and 2,5-cyclohexanedien-1-yl, which are C₅ to C₆ cycloalkanedienyl groups.

The aforementioned cycloalkanedienyl group may be fused with benzene ring, naphthalene ring and the like, and examples include, for example, 1-indenyl and 2-indenyl.

Examples of the cycloalkyl-alkyl group include the groups in which one hydrogen atom of the alkyl group is substituted with a cycloalkyl group, and include, for example, cyclopropylmethyl, 1-cyclopropylethyl, 2-cyclopropylethyl, 3-cyclopropylpropyl, 4-cyclopropylbutyl, 5-cyclopropylpentyl, 6-cyclopropylhexyl, cyclobutylmethyl, cyclopentylmethyl, cyclobutylmethyl, cyclopentylmethyl, cyclohexylmethyl, cyclohexylpropyl, cyclohexylbutyl, cycloheptylmethyl, cyclooctylmethyl, and 6-cyclooctylhexyl, which are C₄ to C₁₄ cycloalkyl-alkyl groups.

Examples of the cycloalkylene group include, for example, cyclopropane-1,1-diyl, cyclopropane-1,2-diyl, cyclobutane-1,1-diyl, cyclobutane-1,2-diyl, cyclobutane-1,3-diyl, cyclopentane-1,1-diyl, cyclopentane-1,2-diyl, cyclopentane-1,3-diyl, cyclohexane-1,1-diyl, cyclohexane-1,2-diyl, cyclohexane-1,3-diyl, cyclohexane-1,4-diyl, cycloheptane-1,1-diyl, cycloheptane-1,2-diyl, cyclooctane-1,1-diyl, and cyclooctane-1,2-diyl, which are C₃ to C₈ cycloalkylene groups.

Examples of the cycloalkenylene group include, for example, 2-cyclopropene-1,1-diyl, 2-cyclobutene-1,1-diyl, 2-cyclopentene-1,1-diyl, 3-cyclopentene-1,1-diyl, 2-cyclohexene-1,1-diyl, 2-cyclohexene-1,2-diyl, 2-cyclohexene-1,4-diyl, 3-cyclohexene-1,1-diyl, 1-cyclobutene-1,2-diyl, 1-cyclopentene-1,2-diyl, and 1-cyclohexene-1,2-diyl, which are C₃ to C₆ cycloalkenylene groups.

Examples of the aryl group include a monocyclic or a fused polycyclic aromatic hydrocarbon group, and include, for example, phenyl, 1-naphthyl, 2-naphthyl, anthryl, phenanthryl, and acenaphthylenyl, which are C₆ to C₁₄ aryl groups.

The aforementioned aryl group may be fused with the aforementioned C₃ to C₈ cycloalkyl group, C₃ to C₆ cycloalkenyl group, C₅ to C₆ cycloalkanedienyl group or the like, and examples include, for example, 4-indanyl, 5-indanyl, 1,2,3,4-tetrahydronaphthalen-5-yl, 1,2,3,4-tetrahydronaphthalen-6-yl, 3-acenaphthenyl, 4-acenaphthenyl, inden-4-yl, inden-5-yl, inden-6-yl, inden-7-yl, 4-phenalenyl, 5-phenalenyl, 6-phenalenyl, 7-phenalenyl, 8-phenalenyl, and 9-phenalenyl.

Examples of the arylene group include, for example, 1,2-phenylene, 1,3-phenylene, 1,4-phenylene, naphthalene-1,2-diyl, naphthalene-1,3-diyl, naphthalene-1,4-diyl, naphthalene-1,5-diyl, naphthalene-1,6-diyl, , naphthalene-1,7-diyl, naphthalene-1,8-diyl, naphthalene-2,3-diyl, naphthalene-2,4-diyl, naphthalene-2,5-diyl, naphthalene-2,6-diyl, naphthalene-2,7-diyl, naphthalene-2,8-diyl, and anthracene-1,4-diyl, which are C₆ to C₁₄ arylene groups.

Examples of the aralkyl group include the groups in which one hydrogen atom of the alkyl group is substituted with an aryl group, and include, for example, benzyl, 1-naphthylmethyl, 2-naphthylmethyl, anthracenylmethyl, phenanthrenylmethyl, acenaphthylenylmethyl, diphenylmethyl, 1-phenethyl, 2-phenethyl, 1-(1-naphthyl)ethyl, 1-(2-naphthyl)ethyl, 2-(1-naphthyl)ethyl, 2-(2-naphthyl)ethyl, 3-phenylpropyl, 3-(1-naphthyl)propyl, 3-(2-naphthyl)propyl, 4-phenylbutyl, 4-(1-naphthyl)butyl, 4-(2-naphthyl)butyl, 5-phenylpentyl, 5-(1-naphthyl)pentyl, 5-(2-naphthyl)pentyl, 6-phenylhexyl, 6-(1-naphthyl)hexyl, and 6-(2-naphthyl)hexyl, which are C₇ to C₁₆ aralkyl groups.

Examples of the bridged cyclic hydrocarbon group include, for example, bicyclo[2.1.0]pentyl, bicyclo[2.2.1]heptyl, bicyclo[2.2.1]octyl, and adamantyl.

Examples of the spiro cyclic hydrocarbon group include, for example, spiro[3.4]octyl, and spiro[4.5]deca-1,6-dienyl.

Examples of the terpene hydrocarbon include, for example, geranyl, neryl, linalyl, phytyl, menthyl, and bornyl.

Examples of the halogenated alkyl group include the groups in which one hydrogen atom of the alkyl group is substituted with a halogen atom, and include, for example, fluoromethyl, difluoromethyl, trifluoromethyl, chloromethyl, dichloromethyl, trichloromethyl, bromomethyl, dibromomethyl, tribromomethyl, iodomethyl, diiodomethyl, triiodomethyl, 2,2,2-trifluoroethyl, pentafluoroethyl, 3,3,3-trifluoropropyl, heptafluoropropyl, heptafluoroisopropyl, nonafluorobutyl, and perfluorohexyl, which are C₁ to C₆ straight chain or branched chain halogenated alkyl groups substituted with 1 to 13 halogen atoms.

Examples of the heterocyclic group include, for example, a monocyclic or a fused polycyclic hetero aryl group which comprises at least one atom of 1 to 3 kinds of hetero atoms selected from oxygen atom, sulfur atom, nitrogen atom and the like as ring-constituting atoms (ring forming atoms), and a monocyclic or a fused polycyclic non-aromatic heterocyclic group which comprises at least one atom of 1 to 3 kinds of hetero atoms selected from oxygen atom, sulfur atom, nitrogen atom and the like as ring-constituting atoms (ring forming atoms).

Examples of the monocyclic heteroaryl group include, for example, 2-furyl, 3-furyl, 2-thienyl, 3-thienyl, 1-pyrrolyl, 2-pyrrolyl, 3-pyrrolyl, 2-oxazolyl, 4-oxazolyl, 5-oxazolyl, 3-isoxazolyl, 4-isoxazolyl, 5-isoxazolyl, 2-thiazolyl, 4-thiazolyl, 5-thiazolyl, 3-isothiazolyl, 4-isothiazolyl, 5-isothiazolyl, 1-imidazolyl, 2-imidazolyl, 4-imidazolyl, 5-imidazolyl, 1-pyrazolyl, 3-pyrazolyl, 4-pyrazolyl, 5-pyrazolyl, (1,2,3-oxadiazol)-4-yl, (1,2,3-oxadiazol)-5-yl, (1,2,4-oxadiazol)-3-yl, (1,2,4-oxadiazol)-5-yl, (1,2,5-oxadiazol)-3-yl, (1,2,5-oxadiazol)-4-yl, (1,3,4-oxadiazol)-2-yl, (1,3,4-oxadiazol)-5-yl, furazanyl, (1,2,3-thiadiazol)-4-yl, (1,2,3-thiadiazol)-5-yl, (1,2,4-thiadiazol)-3-yl, (1,2,4-thiadiazol)-5-yl, (1,2,5-thiadiazol)-3-yl, (1,2,5-thiadiazol)-4-yl, (1,3,4-thiadiazolyl)-2-yl, (1,3,4-thiadiazolyl)-5-yl, (1H-1,2,3-triazol)-1-yl, (1H-1,2,3-triazol)-4-yl, (1H-1,2,3-triazol)-5-yl, (2H-1,2,3-triazol)-2-yl, (2H-1,2,3-triazol)-4-yl, (1H-1,2,4-triazol)-1-yl, (1H-1,2,4-triazol)-3-yl, (1H-1,2,4-triazol)-5-yl, (4H-1,2,4-triazol)-3-yl, (4H-1,2,4-triazol)-4-yl, (1H-tetrazol)-1-yl, (1H-tetrazol)-5-yl, (2H-tetrazol)-2-yl, (2H-tetrazol)-5-yl, 2-pyridyl, 3-pyridyl, 4-pyridyl, 3-pyridazinyl, 4-pyridazinyl, 2-pyrimidinyl, 4-pyrimidinyl, 5-pyrimidinyl, 2-pyrazinyl, (1,2,3-triazin)-4-yl, (1,2,3-triazin)-5-yl, (1,2,4-triazin)-3-yl, (1,2,4-triazin)-5-yl, (1,2,4-triazin)-6-yl, (1,3,5-triazin)-2-yl, 1-azepinyl, 2-azepinyl, 3-azepinyl, 4-azepinyl, (1,4-oxazepin)-2-yl, (1,4-oxazepin)-3-yl, (1,4-oxazepin)-5-yl, (1,4-oxazepin)-6-yl, (1,4-oxazepin)-7-yl, (1,4-thiazepin)-2-yl, (1,4-thiazepin)-3-yl, (1,4-thiazepin)-5-yl, (1,4-thiazepin)-6-yl, and (1,4-thiazepin)-7-yl, which are 5 to 7-membered monocyclic heteroaryl groups.

Examples of the fused polycyclic heteroaryl group include, for example, 2-benzofuranyl, 3-benzofuranyl, 4-benzofuranyl, 5-benzofuranyl, 6-benzofuranyl, 7-benzofuranyl, 1-isobenzofuranyl, 4-isobenzofuranyl, 5-isobenzofuranyl, 2-benzo[b]thienyl, 3-benzo[b]thienyl, 4-benzo[b]thienyl, 5-benzo[b]thienyl, 6-benzo[b]thienyl, 7-benzo[b]thienyl, 1-benzo[c]thienyl, 4-benzo[c]thienyl, 5-benzo[c]thienyl, 1-indolyl, 1-indolyl, 2-indolyl, 3-indolyl, 4-indolyl, 5-indolyl, 6-indolyl, 7-indolyl, (2H-isoindol)-1-yl, (2H-isoindol)-2-yl, (2H-isoindol)-4-yl, (2H-isoindol)-5-yl, (1H-indazol)-1-yl, (1H-indazol)-3-yl, (1H-indazol)-4-yl, (1H-indazol)-5-yl, (1H-indazol)-6-yl, (1H-indazol)-7-yl, (2H-indazol)-1-yl, (2H-indazol)-2-yl, (2H-indazol)-4-yl, (2H-indazol)-5-yl, 2-benzoxazolyl, 2-benzoxazolyl, 4-benzoxazolyl, 5-benzoxazolyl, 6-benzoxazolyl, 7-benzoxazolyl, (1,2-benzisoxazol)-3-yl, (1,2-benzisoxazol)-4-yl, (1,2-benzisoxazol)-5-yl, (1,2-benzisoxazol)-6-yl, (1,2-benzisoxazol)-7-yl, (2,1-benzisoxazol)-3-yl, (2,1-benzisoxazol)-4-yl, (2,1-benzisoxazol)-5-yl, (2,1-benzisoxazol)-6-yl, (2,1-benzisoxazol)-7-yl, 2-benzothiazolyl, 4-benzothiazolyl, 5-benzothiazolyl, 6-benzothiazolyl, 7-benzothiazolyl, (1,2-benzisothiazol)-3-yl, (1,2-benzisothiazol)-4-yl, (1,2-benzisothiazol)-5-yl, (1,2-benzisothiazol)-6-yl, (1,2-benzisothiazol)-7-yl, (2,1-benzisothiazol)-3-yl, (2,1-benzisothiazol)-4-yl, (2,1-benzisothiazol)-5-yl, (2,1-benzisothiazol)-6-yl, (2,1-benzisothiazol)-7-yl, (1,2,3-benzoxadiazol)-4-yl, (1,2,3-benzoxadiazol)-5-yl, (1,2,3-benzoxadiazol)-6-yl, (1,2,3-benzoxadiazol)-7-yl, (2,1,3-benzoxadiazol)-4-yl, (2,1,3-benzoxadiazol)-5-yl, (1,2,3-benzothiadiazol)-4-yl, (1,2,3-benzothiadiazol)-5-yl, (1,2,3-benzothiadiazol)-6-yl, (1,2,3-benzothiadiazol)-7-yl, (2,1,3-benzothiadiazol)-4-yl, (2,1,3-benzothiadiazol)-5-yl, (1H-benzotriazol)-1-yl, (1H-benzotriazol)-4-yl, (1H-benzotriazol)-5-yl, (1H-benzotriazol)-6-yl, (1H-benzotriazol)-7-yl, (2H-benzotriazol)-2-yl, (2H-benzotriazol)-4-yl, (2H-benzotriazol)-5-yl, 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 3-cinnolinyl, 4-cinnolinyl, 5-cinnolinyl, 6-cinnolinyl, 7-cinnolinyl, 8-cinnolinyl, 2-quinazolinyl, 4-quinazolinyl, 5-quinazolinyl, 6-quinazolinyl, 7-quinazolinyl, 8-quinazolinyl, 2-quinoxalinyl, 5-quinoxalinyl, 6-quinoxalinyl, 1-phthalazinyl, 5-phthalazinyl, 6-phthalazinyl, 2-naphthyridinyl, 3-naphthyridinyl, 4-naphthyridinyl, 2-purinyl, 6-purinyl, 7-purinyl, 8-purinyl, 2-pteridinyl, 4-pteridinyl, 6-pteridinyl, 7-pteridinyl, 1-carbazolyl, 2-carbazolyl, 3-carbazolyl, 4-carbazolyl, 9-carbazolyl, 2-(α-carbolinyl), 3-(α-carbolinyl), 4-(α-carbolinyl), 5-(α-carbolinyl), 6-(α-carbolinyl), 7-(α-carbolinyl), 8-(α-carbolinyl), 9-(α-carbolinyl), 1-(β-carbolinyl), 3-(β-carbolinyl), 4-(β-carbolinyl), 5-(β-carbolinyl), 6-(β-carbolinyl), 7-(β-carbolinyl), 8-(β-carbolinyl), 9-(β-carbolinyl), 1-(γ-carbolinyl), 2-(γ-carbolinyl), 4-(γ-carbolinyl), 5-(γ-carbolinyl), 6-(γ-carbolinyl), 7-(γ-carbolinyl), 8-(γ-carbolinyl), 9-(γ-carbolinyl), 1-acridinyl, 2-acridinyl, 3-acridinyl, 4-acridinyl, 9-acridinyl, 1-phenoxazinyl, 2-phenoxazinyl, 3-phenoxazinyl, 4-phenoxazinyl, 10-phenoxazinyl, 1-phenothiazinyl, 2-phenothiazinyl, 3-phenothiazinyl, 4-phenothiazinyl, 10-phenothiazinyl, 1-phenazinyl, 2-phenazinyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthridinyl, 2-phenanthrolinyl, 3-phenanthrolinyl, 4-phenanthrolinyl, 5-phenanthrolinyl, 6-phenanthrolinyl, 7-phenanthrolinyl, 8-phenanthrolinyl, 9-phenanthrolinyl, 10-phenanthrolinyl, 1-thianthrenyl, 2-thianthrenyl, 1-indolizinyl, 2-indolizinyl, 3-indolizinyl, 5-indolizinyl, 6-indolizinyl, 7-indolizinyl, 8-indolizinyl, 1-phenoxathiinyl, 2-phenoxathiinyl, 3-phenoxathiinyl, 4-phenoxathiinyl, thieno[2,3-b]furyl, pyrrolo[1,2-b]pyridazinyl, pyrazolo[1,5-a]pyridyl, imidazo[11,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, and 1,2,4-triazolo[4,3-a]pyridazinyl, which are 8 to 14-membered fused polycyclic heteroaryl groups.

Examples of the monocyclic non-aromatic heterocyclic group include, for example, 1-aziridinyl, 1-azetidinyl, 1-pyrrolidinyl, 2-pyrrolidinyl, 3-pyrrolidinyl, 2-tetrahydrofuryl, 3-tetrahydrofuryl, thiolanyl, 1-imidazolidinyl, 2-imidazolidinyl, 4-imidazolidinyl, 1-pyrazolidinyl, 3-pyrazolidinyl, 4-pyrazolidinyl, 1-(2-pyrrolinyl), 1-(2-imidazolinyl), 2-(2-imidazolinyl), 1-(2-pyrazolinyl), 3-(2-pyrazolinyl), piperidino, 2-piperidinyl, 3-piperidinyl, 4-piperidinyl, 1-homopiperidinyl, 2-tetrahydropyranyl, morpholino, (thiomorpholin)-4-yl, 1-piperazinyl, and 1-homopiperazinyl, which are 3 to 7-membered saturated or unsaturated monocyclic non-aromatic heterocyclic groups.

Examples of the fused polycyclic non-aromatic heterocyclic group include, for example, 2-quinuclidinyl, 2-chromanyl, 3-chromanyl, 4-chromanyl, 5-chromanyl, 6-chromanyl, 7-chromanyl, 8-chromanyl, 1-isochromanyl, 3-isochromanyl, 4-isochromanyl, 5-isochromanyl, 6-isochromanyl, 7-isochromanyl, 8-isochromanyl, 2-thiochromanyl, 3-thiochromanyl, 4-thiochromanyl, 5-thiochromanyl, 6-thiochromanyl, 7-thiochromanyl, 8-thiochromanyl, 1-isothiochromanyl, 3-isothiochromanyl, 4-isothiochromanyl, 5-isothiochromanyl, 6-isothiochromanyl, 7-isothiochromanyl, 8-isothiochromanyl, 1-indolinyl, 2-indolinyl, 3-indolinyl, 4-indolinyl, 5-indolinyl, 6-indolinyl, 7-indolinyl, 1-isoindolinyl, 2-isoindolinyl, 4-isoindolinyl, 5-isoindolinyl, 2-(4H-chromenyl), 3-(4H-chromenyl), 4-(4H-chromenyl), 5-(4H-chromenyl), 6-(4H-chromenyl), 7-(4H-chromenyl), 8-(4H-chromenyl), 1-isochromenyl, 3-isochromenyl, 4-isochromenyl, 5-isochromenyl, 6-isochromenyl, 7-isochromenyl, 8-isochromenyl, 1-(1H-pyrrolidinyl), 2-(1H-pyrrolidinyl), 3-(1H-pyrrolidinyl), 5-(1H-pyrrolidinyl), 6-(1H-pyrrolidinyl), and 7-(1H-pyrrolidinyl), which are 8 to 10-membered saturated or unsaturated fused polycyclic non-aromatic heterocyclic groups.

Among the aforementioned heterocyclic groups, a monocyclic or a fused polycyclic hetero aryl groups which may have 1 to 3 kinds of hetero atoms selected from oxygen atom, sulfur atom, nitrogen atom and the like, in addition to the nitrogen atom that has the bond, as ring-constituting atoms (ring forming atoms), and a monocyclic or a fused polycyclic non-aromatic heterocyclic groups which may have 1 to 3 kinds of hetero atoms selected from oxygen atom, sulfur atom, nitrogen atom and the like, in addition to the nitrogen atom that has the bond, as ring-constituting atoms (ring forming atoms) are referred to as "cyclic amino group." Examples include, for example, 1-pyrrolidinyl, 1-imidazolidinyl, 1-pyrazolidinyl, 1-oxazolidinyl, 1-thiazolidinyl, piperidino, morpholino, 1-piperazinyl, thiomorpholin-4-yl, 1-homopiperidinyl, 1-homopiperazinyl, 2-pyrolin-1-yl, 2-imidazolin-1-yl, 2-pyrazolin-1-yl, 1-indolinyl, 2-isoindolinyl, 1,2,3,4-tetrahydroquinolin-1-yl, 1,2,3,4-tetrahydroisoquinolin-2-yl, 1-pyrrolyl, 1-imidazolyl, 1-pyrazolyl, 1-indolyl, 1-indazolyl, and 2-isoindolyl.

The aforementioned cycloalkyl group, cycloalkenyl group, cycloalkanedienyl group, aryl group, cycloalkylene group, cycloalkenylene group, arylene group, bridged cyclic hydrocarbon group, spiro cyclic hydrocarbon group, and heterocyclic group are generically referred to as "cyclic group." Furthermore, among said cyclic groups, particularly, aryl group, arylene group, monocyclic heteroaryl group, and fused polycyclic heteroaryl group are generically referred to as "aromatic ring group."

Examples of the hydrocarbon-oxy group include the groups in which a hydrogen atom of the hydroxy group is substituted with a hydrocarbon group, and examples of the hydrocarbon include similar groups to the aforementioned hydrocarbon groups. Examples of the hydrocarbon-oxy group include, for example, alkoxy group (alkyl-oxy group), alkenyl-oxy group, alkynyl-oxy group, cycloalkyl-oxy group, cycloalkyl-alkyl-oxy group and the like, which are aliphatic hydrocarbon-oxy groups; aryl-oxy group; aralkyl-oxy group; and alkylene-dioxy group.

Examples of the alkoxy (alkyl-oxy group) include, for example, methoxy, ethoxy, n-propoxy, isopropoxy, n-butoxy, isobutoxy, sec-butoxy, tert-butoxy, n-pentyloxy, isopentyloxy, 2-methylbutoxy, 1-methylbutoxy, neopentyloxy, 1,2-dimethylpropoxy, 1-ethylpropoxy, n-hexyloxy, 4-methylpentyloxy, 3-methylpentyloxy, 2-methylpentyloxy, 1-methylpentyloxy, 3,3-dimethylbutoxy, 2,2-dimethybutoxy, 1,1-dimethylbutoxy, 1,2-dimethylbutoxy, 1,3-dimethylbutoxy, 2,3-dimethylbutoxy, 2-ethylbutoxy, 1-ethylbutoxy, 1-ethyl-1-methylpropoxy, n-heptyloxy, n-octyloxy, n-nonyloxy, n-decyloxy, n-undecyloxy, n-dodecyloxy, n-tridecyloxy, n-tetradecyloxy, and n-pentadecyloxy, which are C₁ to C₁₅ straight chain or branched chain alkoxy groups.

Examples of the alkenyl-oxy group include, for example, vinyloxy, (prop-1-en-1-yl)oxy, allyloxy, isopropenyloxy, (but-1-en-1-yl)oxy, (but-2-en-1-yl)oxy, (but-3-en-1-yl)oxy, (2-methylprop-2-en-1-yl)oxy, (1-methylprop-2-en-1-yl)oxy, (pent-1-en-1-yl)oxy, (pent-2-en-1-yl)oxy, (pent-3-en-1-yl)oxy, (pent-4-en-1-yl)oxy, (3-methylbut-2-en-1-yl)oxy, (3-methylbut-3-en-1-yl)oxy, (hex-1-en-1-yl)oxy, (hex-2-en-1-yl)oxy, (hex-3-en-1-yl)oxy, (hex-4-en-1-yl)oxy, (hex-5-en-1-yl)oxy, (4-methylpent-3-en-1-yl)oxy, (4-methylpent-3-en-1-yl)oxy, (hept-1-en-1-yl)oxy, (hept-6-en-1-yl)oxy, (oct-1-en-1-yl)oxy, (oct-7-en-1-yl)oxy, (non-1-en-1-yl)oxy, (non-8-en-1-yl)oxy, (dec-1-en-1-yl)oxy, (dec-9-en-1-yl)oxy, (undec-1-en-1-yl)oxy, (undec-10-en-1-yl)oxy, (dodec-1-en-1-yl)oxy, (dodec-11-en-1-yl)oxy, (tridec-1-en-1-yl)oxy, (tridec-12-en-1-yl)oxy, (tetradec-1-en-1-yl)oxy, (tetradec-13-en-1-yl)oxy, (pentadec-1-en-1-yl)oxy, and (pentadec-14-en-1-yl)oxy, which are C₂ to C₁₅ straight chain or branched chain alkenyl-oxy groups.

Examples of the alkynyl-oxy group include, for example, ethynyloxy, (prop-1-yn-1-yl)oxy, (prop-2-yn-1-yl)oxy, (but-1-yn-1-yl)oxy, (but-3-yn-1-yl)oxy, (1-methylprop-2-yn-1-yl)oxy, (pent-1-yn-1-yl)oxy, (pent-4-yn-1-yl)oxy, (hex-1-yn-1-yl)oxy, (hex-5-yn-1-yl)oxy, (hept-1-yn-1-yl)oxy, (hept-6-yn-1-yl)oxy, (oct-1-yn-1-yl)oxy, (oct-7-yn-1-yl)oxy, (non-1-yn-1-yl)oxy, (non-8-yn-1-yl)oxy, (dec-1-yn-1-yl)oxy, (dec-9-yn-1-yl)oxy, (undec-1-yn-1-yl)oxy, (undec-10-yn-1-yl)oxy, (dodec-1-yn-1-yl)oxy, (dodec-11-yn-1-yl)oxy, (tridec-1-yn-1-yl)oxy, (tridec-12-yn-1-yl)oxy, (tetradec-1-yn-1-yl)oxy, (tetradec-13-yn-1-yl)oxy, (pentadec-1-yn-1-yl)oxy, and (pentadec-14-yn-1-yl)oxy, which are C₂ to C₁₅ straight chain or branched chain alkynyl-oxy groups.

Examples of the cycloalkyl-oxy group include, for example, cyclopropoxy, cyclobutoxy, cyclopentyloxy, cyclohexyloxy, cycloheptyloxy, and cyclooctyloxy, which are C₃ to C₈ cycloalkyl-oxy groups.

Examples of the cycloalkyl-alkyl-oxy group include, for example, cyclopropylmethoxy, 1-cyclopropylethoxy, 2-cyclopropylethoxy, 3-cyclopropylpropoxy, 4-cyclopropylbutoxy, 5-cyclopropylpentyloxy, 6-cyclopropylhexyloxy, cyclobutylmethoxy, cyclopentylmethoxy, cyclobutylmethoxy, cyclopentylmethoxy, cyclohexylmethoxy, 2-cyclohexylethoxy, 3-cyclohexylpropoxy, 4-cyclohexylbutoxy, cycloheptylmethoxy, cyclooctylmethoxy, and 6-cyclooctylhexyloxy, which are C₄ to C₁₄ cycloalkyl-alkyl-oxy groups.

Examples of the aryl-oxy group include, for example, phenoxy, 1-naphthyloxy, 2-naphthyloxy, anthryloxy, phenanthryloxy, and acenaphthylenyloxy, which are C₆ to C₁₄ aryl-oxy groups.

Examples of the aralkyl-oxy group include, for example, benzyloxy, 1-naphthylmethoxy, 2-naphthylmethoxy, anthracenylmethoxy, phenanthrenylmethoxy, acenaphthylenylmethoxy, diphenylmethoxy, 1-phenethyloxy, 2-phenethyloxy, 1-(1-naphthyl)ethoxy, 1-(2-naphthyl)ethoxy, 2-(1-naphthyl)ethoxy, 2-(2-naphthyl)ethoxy, 3-phenylpropoxy, 3-(1-naphthyl)propoxy, 3-(2-naphthyl)propoxy, 4-phenylbutoxy, 4-(1-naphthyl)butoxy, 4-(2-naphthyl)butoxy, 5-phenylpentyloxy, 5-(1-naphthyl)pentyloxy, 5-(2-naphthyl)pentyloxy, 6-phenylhexyloxy, 6-(1-naphthyl)hexyloxy, and 6-(2-naphthyl)hexyloxy, which are C₇ to C₁₆ aralkyl-oxy groups.

Examples of the alkylenedioxy group include, for example, methylenedioxy, ethylenedioxy, 1-methylmethylenedioxy, and 1,1-dimethylmethylenedioxy.

Examples of the halogenated alkoxy group (halogenated alkyl-oxy group) include the groups in which a hydrogen atom of the hydroxy group is substituted with a halogenated alkyl group, and include, for example, fluoromethoxy, difluoromethoxy, chloromethoxy, bromomethoxy, iodomethoxy, trifluoromethoxy, trichloromethoxy, 2,2,2-trifluoroethoxy, pentafluoroethoxy, 3,3,3-trifluoropropoxy, heptafluoropropoxy, heptafluoroisopropoxy, nonafluorobutoxy, and perfluorohexyloxy, which are C₁ to C₆ straight chain or branched chain halogenated alkoxy groups substituted with 1 to 13 halogen atoms.

Examples of the heterocyclic-oxy group include the groups in which a hydrogen atom of the hydroxy group is substituted with a heterocyclic group, and examples of the heterocyclic ring include similar groups to the aforementioned heterocyclic groups. Examples of the heterocyclic-oxy group include, for example, a monocyclic heteroaryl-oxy group, a fused polycyclic heteroaryl-oxy group, a monocyclic non-aromatic heterocyclic-oxy group, and a fused polycyclic non-aromatic heterocyclic-oxy group.

Examples of the monocyclic heteroaryl-oxy group include, for example, 3-thienyloxy, (isoxazol-3-yl)oxy, (thiazol-4-yl)oxy, 2-pyridyloxy, 3-pyridyloxy, 4-pyridyloxy, and (pyrimidin-4-yl)oxy.

Examples of the fused polycyclic heteroaryl-oxy group include, for example, 5-indolyloxy, (benzimidazol-2-yl)oxy, 2-quinolyloxy, 3-quinolyloxy, and 4-quinolyloxy.

Examples of the monocyclic non-aromatic heterocyclic-oxy group include, for example, 3-pyrrolidinyloxy, and 4-piperidinyloxy.

Examples of the fused polycyclic non-aromatic heterocyclic-oxy group include, for example, 3-indolynyloxy, and 4-chromanyloxy.

Examples of the hydrocarbon-sulfanyl group include the groups in which a hydrogen atom of the sulfanyl group is substituted with a hydrocarbon group, and examples of the hydrocarbon include similar groups to the aforementioned hydrocarbon groups. Examples of the hydrocarbon-sulfanyl groups include, for example, alkyl-sulfanyl group, alkenyl-sulfanyl group, alkynyl-sulfanyl group, cycloalkyl-sulfanyl group, cycloalkyl-alkyl-sulfanyl group and the like, which are aliphatic hydrocarbon-sulfanyl groups; aryl-sulfanyl group, and aralkyl-sulfanyl group.

Examples of the alkyl-sulfanyl group include, for example, methylsulfanyl, ethylsulfanyl, n-propylsulfanyl, isopropylsulfanyl, n-butylsulfanyl, isobutylsulfanyl, sec-butylsulfanyl, tert-butylsulfanyl, n-pentylsulfanyl, isopentylsulfanyl, (2-methylbutyl)sulfanyl, (1-methylbutyl)sulfanyl, neopentylsulfanyl, (1,2-dimethylpropyl)sulfanyl, (1-ethylpropyl)sulfanyl, n-hexylsulfanyl, (4-methylpentyl)sulfanyl, (3-methylpentyl)sulfanyl, (2-methylpentyl)sulfanyl, (1-methylpentyl)sulfanyl, (3,3-dimethylbutyl)sulfanyl, (2,2-dimethylbutyl)sulfanyl, (1,1-dimethylbutyl)sulfanyl, (1,2-dimethylbutyl)sulfanyl, (1,3-dimethylbutyl)sulfanyl, (2,3-dimethylbutyl)sulfanyl, (2-ethylbutyl)sulfanyl, (1-ethylbutyl)sulfanyl, (1-ethyl-1-methylpropyl)sulfanyl, n-heptylsulfanyl, n-octylsulfanyl, n-nonylsulfanyl, n-decylsulfanyl, n-undecylsulfanyl, n-dodecylsulfanyl, n-tridecylsulfanyl, n-tetradecylsulfanyl, and n-pentadecylsulfanyl, which are C₁ to C₁₅ straight chain or branched chain alkyl-sulfanyl groups.

Examples of the alkenyl-sulfanyl group include, for example, vinylsulfanyl, (prop-1-en-1-yl)sulfanyl, allylsulfanyl, isopropenylsulfanyl, (but-1-en-1-yl)sulfanyl, (but-2-en-1-yl)sulfanyl, (but-3-en-1-yl)sulfanyl, (2-methylprop-2-en-1-yl)sulfanyl, (1-methylprop-2-en-1-yl)sulfanyl, (pent-1-en-1-yl)sulfanyl, (pent-2-en-1-yl)sulfanyl, (pent-3-en-1-yl)sulfanyl, (pent-4-en-1-yl)sulfanyl, (3-methylbut-2-en-1-yl)sulfanyl, (3-methylbut-3-en-1-yl)sulfanyl, (hex-1-en-1-yl)sulfanyl, (hex-2-en-1-yl)sulfanyl, (hex-3-en-1-yl)sulfanyl, (hex-4-en-1-yl)sulfanyl, (hex-5-en-1-yl)sulfanyl, (4-methylpent-3-en-1-yl)sulfanyl, (4-methylpent-3-en-1-yl)sulfanyl, (hept-1-en-1-yl)sulfanyl, (hept-6-en-1-yl)sulfanyl, (oct-1-en-1-yl)sulfanyl, (oct-7-en-1-yl)sulfanyl, (non-1-en-1-yl)sulfanyl, (non-8-en-1-yl)sulfanyl, (dec-1-en-1-yl)sulfanyl, (dec-9-en-1-yl)sulfanyl, (undec-1-en-1-yl)sulfanyl, (undec-10-en-1-yl)sulfanyl, (dodec-1-en-1-yl)sulfanyl, (dodec-11-en-1-yl)sulfanyl, (tridec-1-en-1-yl)sulfanyl, (tridec-12-en-1-yl)sulfanyl, (tetradec-1-en-1-yl)sulfanyl, (tetradec-13-en-1-yl)sulfanyl, (pentadec-1-en-1-yl)sulfanyl, and (pentadec-14-en-1-yl)sulfanyl, which are C₂ to C₁₅ straight chain or branched chain alkenyl-sulfanyl groups.

Examples of the alkynyl-sulfanyl group include, for example, ethynylsulfanyl, (prop-1-yn-1-yl)sulfanyl, (prop-2-yn-1-yl)sulfanyl, (but-1-yn-1-yl)sulfanyl, (but-3-yn-1-yl)sulfanyl, (1-methylprop-2-yn-1-yl)sulfanyl, (pent-1-yn-1-yl)sulfanyl, (pent-4-yn-1-yl)sulfanyl, (hex-1-yn-1-yl)sulfanyl, (hex-5-yn-1-yl)sulfanyl, (hept-1-yn-1-yl)sulfanyl, (hept-6-yn-1-yl)sulfanyl, (oct-1-yn-1-yl)sulfanyl, (oct-7-yn-1-yl)sulfanyl, (non-1-yn-1-yl)sulfanyl, (non-8-yn-1-yl)sulfanyl, (dec-1-yn-1-yl)sulfanyl, (dec-9-yn-1-yl)sulfanyl, (undec-1-yn-1-yl)sulfanyl, (undec-10-yn-1-yl)sulfanyl, (dodec-1-yn-1-yl)sulfanyl, (dodec-11-yn-1-yl)sulfanyl, (tridec-1-yn-1-yl)sulfanyl, (tridec-12-yn-1-yl)sulfanyl, (tetradec-1-yn-1-yl)sulfanyl, (tetradec-13-yn-1-yl)sulfanyl, (pentadec-1-yn-1-yl)sulfanyl, and (pentadec-14-yn-1-yl)sulfanyl, which are C₂ to C₁₅ straight chain or branched chain alkynyl-sulfanyl groups.

Examples of the cycloalkyl-sulfanyl group include, for example, cyclopropylsulfanyl, cyclobutylsulfanyl, cyclopentylsulfanyl, cyclohexylsulfanyl, cycloheptylsulfanyl, and cyclooctylsulfanyl, which are C₃ to C₈ cycloalkyl-sulfanyl groups.

Examples of the cycloalkyl-alkyl-sulfanyl group include, for example, (cyclopropylmethyl)sulfanyl, (1-cyclopropylethyl)sulfanyl, (2-cyclopropylethyl)sulfanyl, (3-cyclopropylpropyl)sulfanyl, (4-cyclopropylbutyl)sulfanyl, (5-cyclopropylpentyl)sulfanyl, (6-cyclopropylhexyl)sulfanyl, (cyclobutylmethyl)sulfanyl, (cyclopentylmethyl)sulfanyl, (cyclobutylmethyl)sulfanyl, (cyclopentylmethyl)sulfanyl, (cyclohexylmethyl)sulfanyl, (2-cyclohexylethyl)sulfanyl, (3-cyclohexylpropyl)sulfanyl, (4-cyclohexylbutyl)sulfanyl, (cycloheptylmethyl)sulfanyl, (cyclooctylmethyl)sulfanyl, and (6-cyclooctylhexyl)sulfanyl, which are C₄ to C₁₄ cycloalkyl-alkyl-sulfanyl groups.

Examples of the aryl-sulfanyl group include, for example, phenylsulfanyl, 1-naphthylsulfanyl, 2-naphthylsulfanyl, anthrylsulfanyl, fenanthrylsulfanyl, and acenaphthylenylsulfanyl, which are C₆ to C₁₄ aryl-sulfanyl groups.

Examples of the aralkyl-sulfanyl group include, for example, benzylsulfanyl, (1-naphthylmethyl)sulfanyl, (2-naphthylmethyl)sulfanyl, (anthracenylmethyl)sulfanyl, (phenanthrenylmethyl)sulfanyl, (acenaphthylenylmethyl)sulfanyl, (diphenylmethyl)sulfanyl, (1-phenethyl)sulfanyl, (2-phenethyl)sulfanyl, (1-(1-naphthyl)ethyl)sulfanyl, (1-(2-naphthyl)ethyl)sulfanyl, (2-(1-naphthyl)ehyl)sulfanyl, (2-(2-naphthyl)ethyl)sulfanyl, (3-phenylpropyl)sulfanyl, (3-(1-naphthyl)propyl)sulfanyl, (3-(2-naphthyl)propyl)sulfanyl, (4-phenylbutyl)sulfanyl, (4-(1-naphthyl)butyl)sulfanyl, (4-(2-naphthyl)butyl)sulfanyl, (5-phenylpentyl)sulfanyl, (5-(1-naphthyl)pentyl)sulfanyl, (5-(2-naphthyl)pentyl)sulfanyl, (6-phenylhexyl)sulfanyl, (6-(1-naphthyl)hexyl)sulfanyl, and (6-(2-naphthyl)hexyl)sulfanyl, which are C₇ to C₁₆ aralkyl-sulfanyl groups.

Examples of the halogenated alkyl-sulfanyl group include the groups in which a hydrogen atom of the sulfanyl group is substituted with a halogenated alkyl group, and include, for example, (fluoromethyl)sulfanyl, (chloromethyl)sulfanyl, (bromomethyl)sulfanyl, (iodomethyl)sulfanyl, (difluoromethyl)sulfanyl, (trifluoromethyl)sulfanyl, (trichloromethyl)sulfanyl, (2,2,2-trifluoroethyl)sulfanyl, (pentafluoroethyl)sulfanyl, (3,3,3-trifluoropropyl)sulfanyl, (heptafluoropropyl)sulfanyl, (heptafluoroisopropyl)sulfanyl, (nonafluorobutyl)sulfanyl, and (perfluorohexyl)sulfanyl, which are C₁ to C₆ straight chain or branched chain halogenated alkyl-sulfanyl groups substituted with 1 to 13 halogen atoms.

Examples of the heterocyclic-sulfanyl group include the groups in which a hydrogen atom of the sulfanyl group is substituted with a heterocyclic group, and examples of the heterocyclic ring include similar groups to the aforementioned heterocyclic groups. Examples of the heterocyclic-sulfanyl group include, for example, a monocyclic heteroaryl-sulfanyl group, a fused polycyclic heteroaryl-sulfanyl group, a monocyclic non-aromatic heterocyclic-sulfanyl group, and a fused polycyclic non-aromatic heterocyclic-sulfanyl group.

Examples of the monocyclic heteroaryl-sulfanyl group include, for example, (imidazol-2-yl)sulfanyl, (1,2,4-triazol-2-yl)sulfanyl, (pyridin-2-yl)sulfanyl, (pyridin-4-yl)sulfanyl, and (pyrimidin-2-yl)sulfanyl.

Examples of the fused polycyclic heteroaryl-sulfanyl group include, for example, (benzimidazol-2-yl)sulfanyl, (quinolin-2-yl)sulfanyl, and (quinolin-4-yl)sulfanyl.

Examples of the monocyclic non-aromatic heterocyclic-sulfanyl groups include, for example, (3-pyrrolidinyl)sulfanyl, and (4-piperidinyl)sulfanyl.

Examples of the fused polycyclic non-aromatic heterocyclic-sulfanyl group include, for example, (3-indolinyl)sulfanyl, and (4-chromanyl)sulfanyl.

Examples of the acyl group include, for example, formyl group, glyoxyloyl group, thioformyl group, carbamoyl group, thiocarbamoyl group, sulfamoyl group, sulfinamoyl group, carboxy group, sulfo group, phosphono group, and groups represented by the following formulas: 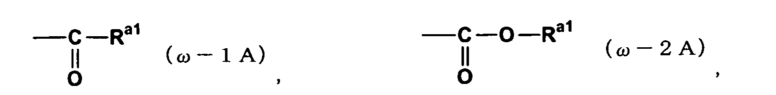 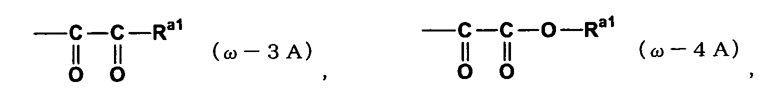 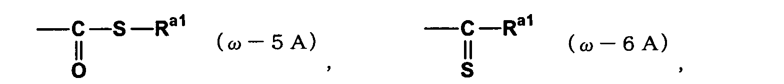 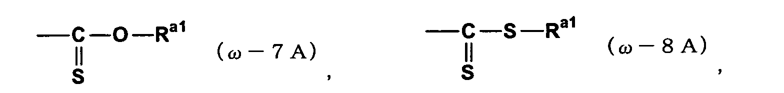 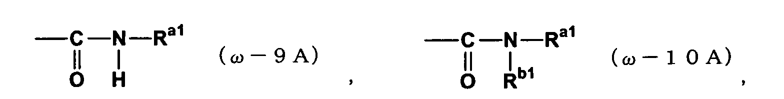 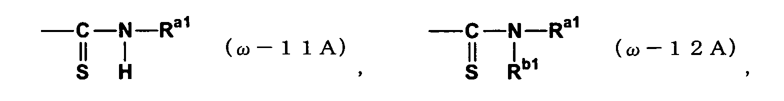 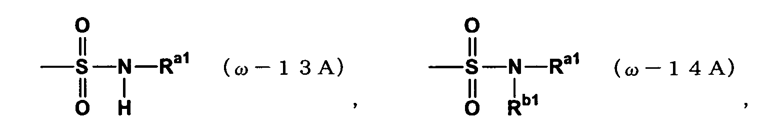 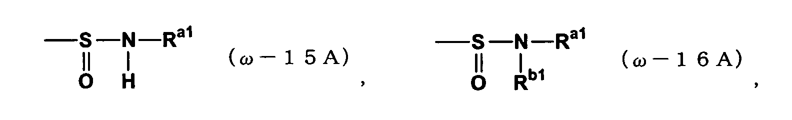 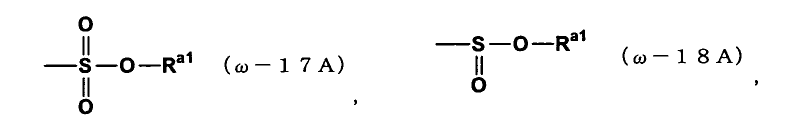 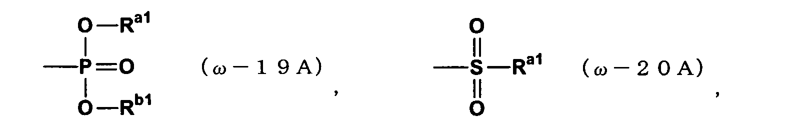 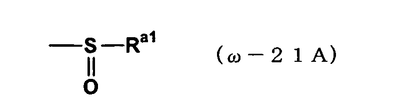 wherein R^{a1} and R^{b1} may be the same or different and represent a hydrocarbon group or a heterocyclic group, or R^{a1} and R^{b1} combine to each other, together with the nitrogen atom to which they bind, to form a cyclic amino group.

In the definition of the aforementioned acyl group, among the groups represented by the formula (ω-1A), those groups in which R^{a1} is a hydrocarbon group are referred to as "hydrocarbon-carbonyl group" whose examples include, for example, acetyl, propionyl, butyryl, isobutyryl, valeryl, isovaleryl, pivaloyl, lauroyl, myristoryl, palmitoyl, acryloyl, propioloyl, methacryloyl, crotonoyl, isocrotonoyl, cyclohexylcarbonyl, cyclohexylmethylcarbonyl, benzoyl, 1-naphthoyl, 2-naphthoyl, and phenylacetyl, and those groups in which R^{a1} is a heterocyclic group are referred to as "heterocyclic ring-carbonyl group" whose examples include, for example, 2-thenoyl, 3-furoyl, nicotinoyl, and isonicotinoyl.

Among the groups represented by the formula (ω-2A), those groups in which R^{a1} is a hydrocarbon group are referred to as "hydrocarbon-oxy-carbonyl group" whose examples include, for example, methoxycarbonyl, ethoxycarbonyl, phenoxycarbonyl, and benzyloxycarbonyl, and those groups in which R^{a1} is a heterocyclic group are referred to as "heterocyclic ring-oxy-carbonyl group" whose examples include, for example, 3-pyridyloxycarbonyl.

Among the groups represented by the formula (ω-3A), those groups in which R^{a1} is a hydrocarbon group are referred to as "hydrocarbon-carbonyl-carbonyl group" whose examples include, for example, pyruvoyl, and those groups in which R^{a1} is a heterocyclic group are referred to as "heterocyclic ring-carbonyl-carbonyl group."

Among the groups represented by the formula (ω-4A), those groups in which R^{a1} is a hydrocarbon group are referred to as "hydrocarbon-oxy-carbonyl-carbonyl group" whose examples include, for example, methoxalyl and ethoxalyl groups, and those groups in which R^{a1} is a heterocyclic group are referred to as "heterocyclic ring-oxy-carbonyl-carbonyl group."

Among the groups represented by the formula (ω-5A), those groups in which R^{a1} is a hydrocarbon group are referred to as "hydrocarbon-sulfanyl-carbonyl group," and those groups in which R^{a1} is a heterocyclic group are referred to as "heterocyclic ring-sulfanyl-carbonyl group."

Among the groups represented by the formula (ω-6A), those groups in which R^{a1} is a hydrocarbon group are referred to as "hydrocarbon-thiocarbonyl group," and those groups in which R^{a1} is a heterocyclic group are referred to as "heterocyclic ring-thiocarbonyl group."

Among the groups represented by the formula (ω-7A), those groups in which R^{a1} is a hydrocarbon group are referred to as "hydrocarbon-oxy-thiocarbonyl group," and those groups in which R^{a1} is a heterocyclic group are referred to as "heterocyclic ring-oxy-thiocarbonyl group."

Among the groups represented by the formula (ω-8A), those groups in which R^{a1} is a hydrocarbon group are referred to as "hydrocarbon-sulfanyl-thiocarbonyl group," and those groups in which R^{a1} is a heterocyclic group are referred to as "heterocyclic ring-sulfanyl-thiocarbonyl group."

Among the groups represented by the formula (ω-9A), those groups in which R^{a1} is a hydrocarbon group are referred to as referred to as "N-hydrocarbon-carbamoyl group" whose examples include, for example, N-methylcarbamoyl group, and those groups in which R^{a1} is a heterocyclic group are referred to as "N-heterocyclic ring-carbamoyl group."

Among the groups represented by the formula (ω-10A), those groups in which both R^{a1} and R^{b1} are hydrocarbon groups are referred to as "N,N-di(hydrocarbon)-carbamoyl group" whose examples include, for example, N,N-dimethylcarbamoyl group, those groups in which both R^{a1} and R^{b1} are heterocyclic groups are referred to as "N,N-di(heterocyclic ring)-carbamoyl group," those groups in which R^{a1} is a hydrocarbon group and R^{b1} is a heterocyclic group are referred to as "N-hydrocarbon-N-heterocyclic ring-substituted carbamoyl group," and those groups in which R^{a1} and R^{b1} combine to each other, together with the nitrogen atom to which they bind, to form a cyclic amino group are referred to as "cyclic amino-carbonyl group" whose examples include, for example, morpholino-carbonyl.

Among the groups represented by the formula (ω-11A), those groups in which R^{a1} is a hydrocarbon group are referred to as "N-hydrocarbon-thiocarbamoyl group," and those groups in which R^{a1} is a heterocyclic group are referred to as "N-heterocyclic ring-thiocarbamoyl group."

Among the groups represented by the formula (ω-12A), those groups in which both R^{a1} and R^{b1} are hydrocarbon groups are referred to as "N,N-di(hydrocarbon)-thiocarbamoyl group," those groups in which both R^{a1} and R^{b1} are heterocyclic groups are referred to as "N,N-di(heterocyclic ring)-thiocarbamoyl group," those groups in which R^{a1} is a hydrocarbon group and R^{b1} is a heterocyclic group are referred to as "N-hydrocarbon-N-heterocyclic ring-thiocarbamoyl group," and those groups in which R^{a1} and R^{b1} combine to each other, together with the nitrogen atom to which they bind, to form a cyclic amino group are referred to as "cyclic amino-thiocarbonyl group."

Among the groups represented by the formula (ω-13A), those groups in which R^{a1} is a hydrocarbon group are referred to as "N-hydrocarbon-sulfamoyl group," and those groups in which R^{a1} is a heterocyclic group are referred to as "N-heterocyclic ring-sulfamoyl group."

Among the groups represented by the formula (ω-14A), those groups in which both R^{a1} and R^{b1} are hydrocarbon groups are referred to as "N,N-di(hydrocarbon)-sulfamoyl group" whose examples include, for example, N,N-dimethylsulfamoyl group, those groups in which both R^{a1} and R^{b1} are heterocyclic groups are referred to as "N,N-di(heterocyclic ring)-sulfamoyl group," those groups in which R^{a1} is a hydrocarbon group and R^{b1} is a heterocyclic group are referred to as "N-hydrocarbon-N-heterocyclic ring-sulfamoyl group," and those groups in which R^{a1} and R^{b1} combine to each other, together with the nitrogen atom to which they bind, to form a cyclic amino group are referred to as "cyclic amino-sulfonyl group" whose examples include, for example 1-pyrrolylsulfonyl.

Among the groups represented by the formula (ω-15A), those groups in which R^{a1} is a hydrocarbon group are referred to as "N-hydrocarbon-sulfinamoyl group," and those groups in which R^{a1} is a heterocyclic group are referred to as "N-heterocyclic ring-sulfinamoyl group." Among the groups represented by the formula (ω-16A), those groups in which both R^{a1} and R^{b1} are hydrocarbon groups are referred to as "N,N-di(hydrocarbon)-sulfinamoyl group," those groups in which both R^{a1} and R^{b1} are heterocyclic groups are referred to as "N,N-di(heterocyclic ring)-sulfinamoyl group," those groups in which R^{a1} is a hydrocarbon group and R^{b1} is a heterocyclic group are referred to as "N-hydrocarbon-N-heterocyclic ring-sulfinamoyl group," and those groups in which R^{a1} and R^{b1} combine to each other, together with the nitrogen atom to which they bind, to form a cyclic amino group are referred to as "cyclic amino-sulfinyl group."

Among the groups represented by the formula (ω-17A), those groups in which R^{a1} is a hydrocarbon group are referred to as "hydrocarbon-oxy-sulfonyl group," and those groups in which R^{a1} is a heterocyclic group are referred to as "heterocyclic ring-oxy-sulfonyl group."

Among the groups represented by the formula (ω-18A), those groups in which R^{a1} is a hydrocarbon group are referred to as "hydrocarbon-oxy-sulfinyl group," and those groups in which R^{a1} is a heterocyclic group are referred to as "heterocyclic ring-oxy-sulfinyl group."

Among the groups represented by the formula (ω-19A), those groups in which both R^{a1} and R^{b1} are hydrocarbon groups are referred to as "O,O'-di(hydrocarbon)-phosphono group," those groups in which both R^{a1} and R^{b1} are heterocyclic groups are referred to as "O,O'-di(heterocyclic ring)-phosphono group," and those groups in which R^{a1} is a hydrocarbon group and R^{b1} is a heterocyclic group are referred to as "O-hydrocarbon-O'-heterocyclic ring-phosphono group."

Among the groups represented by the formula (ω-20A), those groups in which R^{a1} is a hydrocarbon group are referred to as "hydrocarbon-sulfonyl group" whose examples include, for example, methanesulfonyl and benzenesulfonyl, and those groups in which R^{a1} is a heterocyclic group are referred to as "heterocyclic ring-sulfonyl group."

Among the groups represented by the formula (ω-21A), those groups in which R^{a1} is a hydrocarbon group are referred to as "hydrocarbon-sulfinyl group" whose examples include, for example, methylsulfinyl and benzenesulfinyl, and those groups in which R^{a1} is a heterocyclic group are referred to as "heterocyclic ring-sulfinyl group."

Examples of the hydrocarbon in the groups represented by the aforementioned formulas (ω-1A) through (ω-21A) include the similar groups to the aforementioned hydrocarbon group. Examples of the hydrocarbon-carbonyl group represented by the formula (ω-1A) include, for example, an alkyl-carbonyl group, an alkenyl-carbonyl group, an alkynyl-carbonyl group, a cycloalkyl-carbonyl group, a cycloalkenyl-carbonyl group, a cycloalkanedienyl-carbonyl group, a cycloalkyl-alkyl-carbonyl group, which are aliphatic hydrocarbon-carbonyl groups; an aryl-carbonyl group; an aralkyl-carbonyl group; a bridged cyclic hydrocarbon-carbonyl group; a spirocyclic hydrocarbon-carbonyl group; and a terpene family hydrocarbon-carbonyl group. In the following, groups represented by the formulas (ω-2A) through (ω-21A) are similar to those explained above.

Examples of the heterocyclic ring in the groups represented by the aforementioned formulas (ω-1A) through (ω-21A) include similar groups to the aforementioned heterocyclic group. Examples of the heterocyclic ring-carbonyl group represented by the formula (ω-1A) include, for example, a monocyclic heteroaryl-carbonyl group, a fused polycyclic heteroaryl-carbonyl group, a monocyclic non-aromatic heterocyclic ring-carbonyl group, and a fused polycyclic non-aromatic heterocyclic ring-carbonyl group. In the following, groups represented by the formulas (ω-2A) through (ω-21A) are similar to those explained above.

Examples of the cyclic amino in the groups represented by the aforementioned formulas (ω-10A) through (ω-16A) include similar groups to the aforementioned cyclic amino group.

In the present specification, when a certain functional group is defined as "which may be substituted," the definition means that the functional group may sometimes have one or more substituents at chemically substitutable positions, unless otherwise specifically mentioned. Kind of substituents, number of substituents, and the position of substituents existing in the functional groups are not particularly limited, and when two or more substituents exist, they may be the same or different. Examples of the substituent existing in the functional group include, for example, halogen atoms, oxo group, thioxo group, nitro group, nitroso group, cyano group, isocyano group, cyanato group, thiocyanato group, isocyanato group, isothiocyanato group, hydroxy group, sulfanyl group, carboxy group, sulfanylcarbonyl group, oxalo group, methooxalo group, thiocarboxy group, dithiocarboxy group, carbamoyl group, thiocarbamoyl group, sulfo group, sulfamoyl group, sulfino group, sulfinamoyl group, sulfeno group, sulfenamoyl group, phosphono group, hydroxyphosphonyl group, hydrocarbon group, heterocyclic group, hydrocarbon-oxy group, heterocyclic ring-oxy group, hydrocarbon-sulfanyl group, heterocyclic ring-sulfanyl group, acyl group, amino group, hydrazino group, hydrazono group, diazenyl group, ureido group, thioureido group, guanidino group, carbamoimidoyl group (amidino group), azido group, imino group, hydroxyamino group, hydroxyimino group, aminooxy group, diazo group, semicarbazino group, semicarbazono group, allophanyl group, hydantoyl group, phosphano group, phosphoroso group, phospho group, boryl group, silyl group, stannyl group, selanyl group, oxido group and the like.

When two or more substituents exist according to the aforementioned definition of "which may be substituted," said two or more substituents may combine to each other, together with atom(s) to which they bind, to form a ring. For these cyclic groups, as ring-constituting atoms (ring forming atoms), one to three kinds of one or more hetero atoms selected from oxygen atom, sulfur atom, nitrogen atom and the like may be included, and one or more substituents may exist on the ring. The ring may be monocyclic or fused polycyclic, and aromatic or non-aromatic.

The above substituents according to the aforementioned definition of "which may be substituted" may further be substituted with the aforementioned substituents at the chemically substitutable positions on the substituent. Kind of substituents, number of substituents, and positions of substituents are not particularly limited, and when the substituents are substituted with two or more substituents, they may be the same or different. Examples of the substituent include, for example, a halogenated alkyl-carbonyl group whose examples include, for example, trifluoroacetyl, a halogenated alkyl-sulfonyl group whose examples include, for example, trifluoromethanesulfonyl, an acyl-oxy group, an acyl-sulfanyl group, an N-hydrocarbon-amino group, an N,N-di(hydrocarbon)-amino group, an N-heterocyclic ring-amino group, an N-hydrocarbon-N-heterocyclic ring-amino group, an acyl-amino group, and a di(acyl)-amino group. Moreover, substitution on the aforementioned substituents may be repeated multiple orders.

Examples of the acyl-oxy group include the groups in which hydrogen atom of hydroxy group is substituted with acyl group, and include, for example, formyloxy group, glyoxyloyloxy group, thioformyloxy group, carbamoloxy group, thiocarbamoyloxy group, sulfamoyloxy group, sulfinamoloxy group, carboxyoxy group, sulphooxy group, phosphonooxy group, and groups represented by the following formulas: 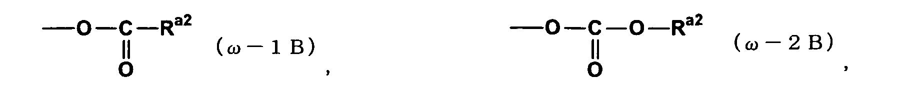 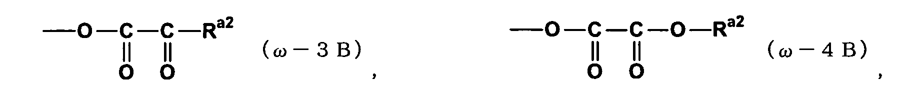 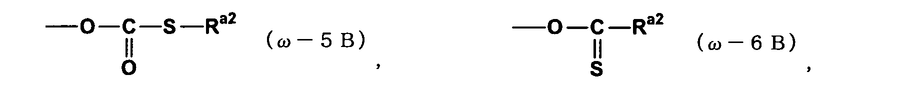 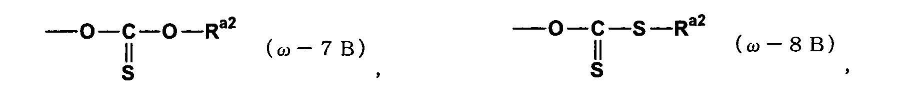 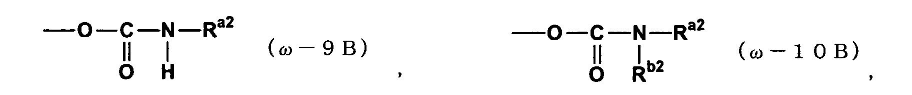 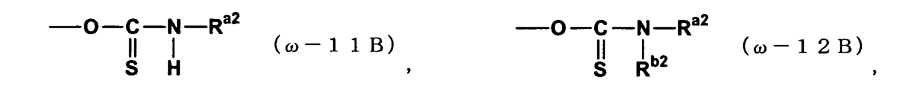 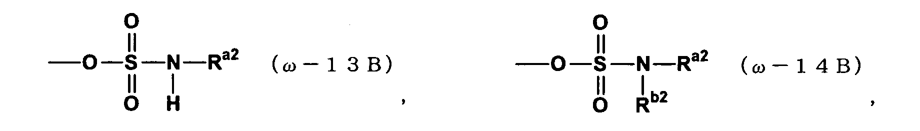 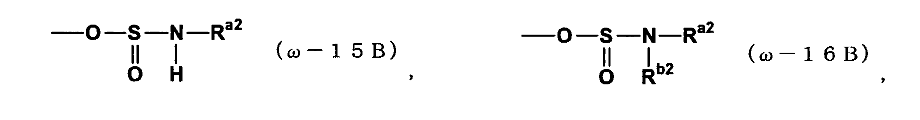 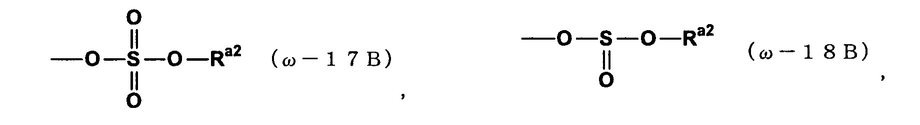 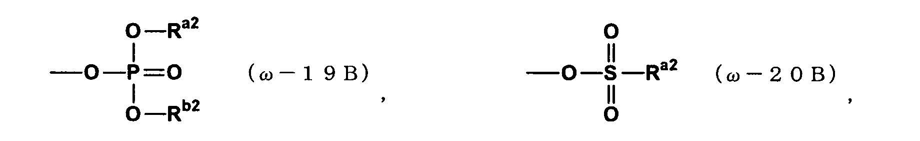 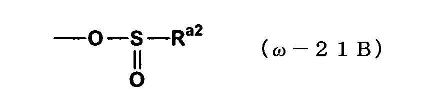 wherein R^{a2} and R^{b2} may be the same or different and represent a hydrocarbon group or a heterocyclic group, or R^{a2} and R^{b2} combine to each other, together with the nitrogen atom to which they bind, to form a cyclic amino group.

In the definition of the aforementioned acyl-oxy group, among the groups represented by the formula (ω-1B), those groups in which R^{a2} is a hydrocarbon group are referred to as "hydrocarbon-carbonyl-oxy group" whose examples include, for example, acetoxy and benzoyloxy, and those groups in which R^{a2} is a heterocyclic group are referred to as "heterocyclic ring-carbonyl-oxy group."

Among the groups represented by the formula (ω-2B), those groups in which R^{a2} is a hydrocarbon group are referred to as "hydrocarbon-oxy-carbonyl-oxy group," and those groups in which R^{a2} is a heterocyclic group are referred to as "heterocyclic ring-oxy-carbonyl-oxy group."

Among the groups represented by the formula (ω-3B), those groups in which R^{a2} is a hydrocarbon group are referred to as "hydrocarbon-carbonyl-carbonyl-oxy group," and those groups in which R^{a2} is a heterocyclic group are referred to as "heterocyclic ring-carbonyl-carbonyl-oxy group."

Among the groups represented by the formula (ω-4B), those groups in which R^{a2} is a hydrocarbon group are referred to as "hydrocarbon-oxy-carbonyl-carbonyl-oxy group," and those groups in which R^{a2} is a heterocyclic group are referred to as "heterocyclic ring-oxy-carbonyl-carbonyl-oxy group."

Among the groups represented by the formula (ω-5B), those groups in which R^{a2} is a hydrocarbon group are referred to as "hydrocarbon-sulfanyl-carbonyl-oxy group," and those groups where R^{a2} is a heterocyclic group are referred to as "heterocyclic ring-sulfanyl-carbonyl-oxy group."

Among the groups represented by the formula (ω-6B), those groups in which R^{a2} is a hydrocarbon group are referred to as "hydrocarbon-thiocarbonyl-oxy group," and those groups where R^{a2} is a heterocyclic group are referred to as "heterocyclic ring-thiocarbonyl-oxy group."

Among the groups represented by the formula (ω-7B), those groups in which R^{a2} is a hydrocarbon group are referred to as "hydrocarbon-oxy-thiocarbonyl-oxy group," and those groups in which R^{a2} is a heterocyclic group are referred to as "heterocyclic ring-oxy-thiocarbonyl-oxy group."

Among the groups represented by the formula (ω-8B), those groups in which R^{a2} is a hydrocarbon group are referred to as "hydrocarbon-sulfanyl-thiocarbonyl-oxy group," and those groups wherein R^{a2} is a heterocyclic group are referred to as "heterocyclic ring-sulfanyl-thiocarbonyl-oxy group."

Among the groups represented by the formula (ω-9B), those groups in which R^{a2} is a hydrocarbon group are referred to as "N-hydrocarbon-carbamoyl-oxy group," and those groups in which R^{a2} is a heterocyclic group are referred to as "N-heterocyclic ring-carbamoyl-oxy group."

Among the groups represented by the formula (ω-10B), those groups in which both R^{a2} and R^{b2} are hydrocarbon groups are referred to as "N,N-di(hydrocarbon)-carbamoyl-oxy group," those groups in which both R^{a2} and R^{b2} are heterocyclic groups are referred to as "N,N-di(heterocyclic ring)-carbamoyl-oxy group," those groups in which R^{a2} is a hydrocarbon group and R^{b2} is a heterocyclic group are referred to as "N-hydrocarbon-N-heterocyclic ring-carbamoyl-oxy group," and those groups in which R^{a2} and R^{b2} combine to each other, together with the nitrogen atom to which they bind, to form a cyclicic amino group are referred to as "cyclicamino-carbonyl-oxy group."

Among the groups represented by the formula (ω-11B), those groups in which R^{a2} is a hydrocarbon group are referred to as "N-hydrocarbon-thiocarbamoyl-oxy group," and those groups in which R^{a2} is a heterocyclic group are referred to as "N-heterocyclic ring-thiocarbamoyl-oxy group."

Among the groups represented by the formula (ω-12B), those groups in which both R^{a2} and R^{b2} are hydrocarbon groups are referred to as "N,N-di(hydrocarbon)-thiocarbamoyl-oxy group," those groups in which both R^{a2} and R^{b2} are heterocyclic groups are referred to as "N,N-di(heterocyclic ring)-thiocarbamoyl-oxy group," those groups in which R^{a2} is a hydrocarbon group and R^{b2} is a heterocyclic group are referred to as "N-hydrocarbon-N-heterocyclic ring-thiocarbamoyl-oxy group," and those groups in which R^{a2} and R^{b2} combine to each other, together with the nitrogen atom to which they bind, to form a cyclic amino group are referred to as "cyclicamino-thiocarbonyl-oxy group."

Among the groups represented by the formula (ω-13B), those groups in which R^{a2} is a hydrocarbon group are referred to as "N-hydrocarbon-sulfamoyl-oxy group," and those groups in which R^{a2} is a heterocyclic group are referred to as "N-heterocyclic ring-sulfamoyl-oxy group."

Among the groups represented by the formula (ω-14B), those groups in which both R^{a2} and R^{b2} are hydrocarbon groups are referred to as "N,N-di(hydrocarbon)-sulfamoyl-oxy group," those groups in which both R^{a2} and Rb² are heterocyclic groups are referred to as "N,N-di(heterocyclic ring)-sulfamoyl-oxy group," those groups in which R^{a2} is a hydrocarbon group and R^{b2} is a heterocyclic group are referred to as "N-hydrocarbon-N-heterocyclic ring-sulfamoyl-oxy group," and those groups in which R^{a2} and R^{b2} combine to each other, together with the nitrogen atom to which they bind, to form a cyclic amino group are referred to as "cyclic amino-sulfonyl-oxy group."

Among the groups represented by the formula (ω-15B), those groups in which R^{a2} is a hydrocarbon group are referred to as "N-hydrocarbon-sulfinamoyl-oxy group," and those groups where R^{a2} is a heterocyclic group are referred to as "N-heterocyclic ring-sulfinamoyl-oxy group."

Among the groups represented by the formula (ω-16B), those groups in which both R^{a2} and R^{b2} are hydrocarbon groups are referred to as "N,N-di(hydrocarbon)-sulfinamoyl-oxy group," those groups in which both R^{a2} and R^{b2} are heterocyclic groups are referred to as "N,N-di(heterocyclic ring)-sulfinamoyl-oxy group," those groups in which R^{a2} is a hydrocarbon group and R^{b2} is a heterocyclic group are referred to as "N-hydrocarbon-N-heterocyclic ring-sulfinamoyl-oxy group," and those groups in which R^{a2} and R^{b2} combine to each other, together with the nitrogen atom to which they bind, to form a cyclic amino group are referred to as "cyclic amino-sulfinyl-oxy group."

Among the groups represented by the formula (ω-17B), those groups in which R^{a2} is a hydrocarbon group are referred to as "hydrocarbon-oxy-sulfonyl-oxy group," and those groups in which R^{a2} is a heterocyclic group are referred to as "heterocyclic ring-oxy-sulfonyl-oxy group."

Among the groups represented by the formula (ω-18B), those groups in which R^{a2} is a hydrocarbon group are referred to as "hydrocarbon-oxy-sulfinyl-oxy group," those groups in which R^{a2} is a heterocyclic group are referred to as "heterocyclic ring-oxy-sulfinyl-oxy group."

Among the groups represented by the formula (ω-19B), those groups in which both R^{a2} and R^{b2} are hydrocarbon groups are referred to as "O,O'-di(hydrocarbon)-phosphono-oxy group," those groups in which both R^{a2} and R^{b2} are heterocyclic groups are referred to as "O,O'-di(heterocyclic ring)-phosphono-oxy group," and those groups in which R^{a2} is a hydrocarbon group and R^{b2} is a heterocyclic group are referred to as "O-hydrocarbon substituted-O'-heterocyclic ring substituted phophono-oxy group."

Among the groups represented by the formula (ω-20B), those groups in which R^{a2} is a hydrocarbon group are referred to as "hydrocarbon-sulfonyl-oxy group," and those groups in which R^{a2} is a heterocyclic group referred to as "heterocyclic ring-sulfonyl-oxy group."

Among the groups represented by the formula (ω-21B), those groups in which R^{a2} is a hydrocarbon group are referred to as "hydrocarbon-sulfinyl-oxy group," and those groups in which R^{a2} is a heterocyclic group are referred to as "heterocyclic ring-sulfinyl-oxy group."

Examples of the hydrocarbon in the groups represented by the aforementioned formulas (ω-1B) through (ω-21B) include the similar groups to the aforementioned hydrocarbon group. Examples of the hydrocarbon-carbonyl-oxy group represented by the formula (ω-1B) include, for example, an alkyl-carbonyl-oxy group, an alkenyl-carbonyl-oxy group, an alkynyl-carbonyl-oxy group, a cycloalkyl-carbonyl-oxy group, a cycloalkenyl-carbonyl-oxy group, a cycloalkanedienyl-carbonyl-oxy group, and a cycloalkyl-alkyl-carbonyl-oxy group, which are aliphatic hydrocarbon-carbonyl-oxy groups; an aryl-carbonyl-oxy group; an aralkyl-carbonyl-oxy group; a bridged cyclic hydrocarbon-carbonyl-oxy group; a spirocyclic hydrocarbon-carbonyl-oxy group; and a terpene family hydrocarbon-carbonyl-oxy group. In the following, groups represented by the formulas (ω-2B) through (ω-21B) are similar to those explained above.

Examples of the heterocyclic ring in the groups represented by the aforementioned formulas (ω-1B) through (ω-21B) include similar groups to the aforementioned heterocyclic group. Examples of the heterocyclic ring-carbonyl group represented by the formula (ω-1B) include, for example, a monocyclic heteroaryl-carbonyl group, a fused polycyclic heteroaryl-carbonyl group, a monocyclic non-aromatic heterocyclic ring-carbonyl group, and a fused polycyclic non-aromatic heterocyclic ring-carbonyl group. In the following, groups represented by the formulas (ω-2B) through (ω-21B) are similar to those groups explained above.

Examples of the cyclic amino in the groups represented by the aforementioned formulas (ω-10B) through (ω-16B) include similar groups to the aforementioned cyclic amino group.

The aforementioned acyl-oxy group, hydrocarbon-oxy group, and heterocyclic-oxy group are generically referred to as "substituted oxy group." Moreover, these substituted oxy group and hydroxy group are generically referred to as "hydroxy group which may be substituted."

Examples of the acyl-sulfanyl group include the groups in which hydrogen atom of sulfanyl group is substituted with acyl group, and include, for example, formylsulfanyl group, glyoxyloylsulfanyl group, thioformylsulfanyl group, carbamoyloxy group, thicarbamoyloxy group, sulfamoyloxy group, sulfinamoyloxy group, carboxyoxy group, sulphooxy group, phosphonooxy group, and groups represented by the following formulas: 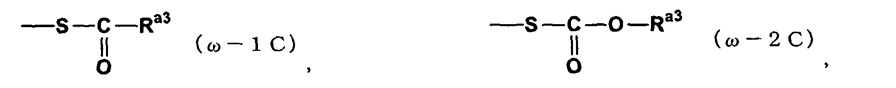 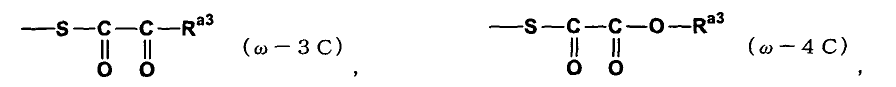 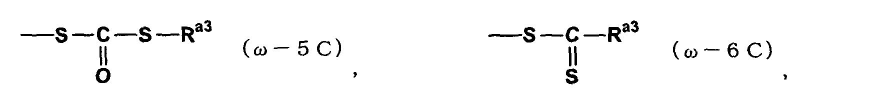 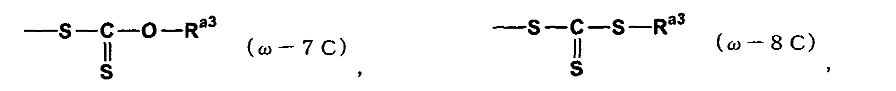 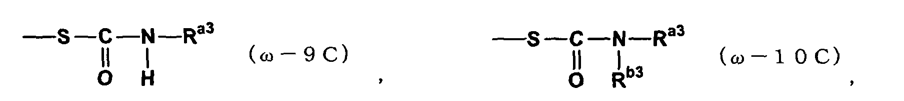 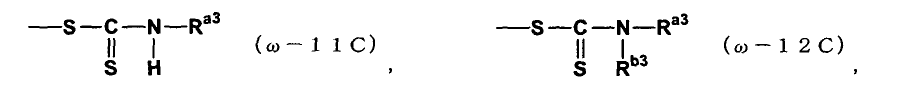 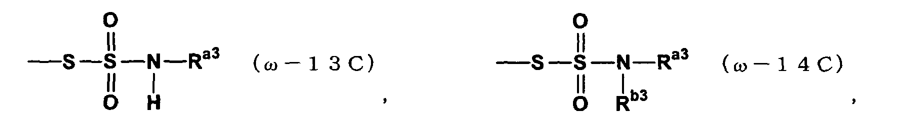 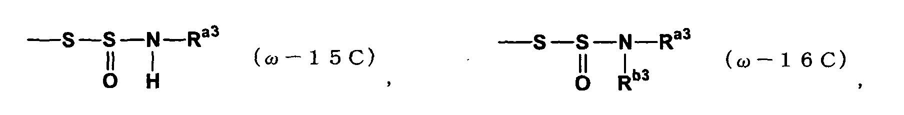 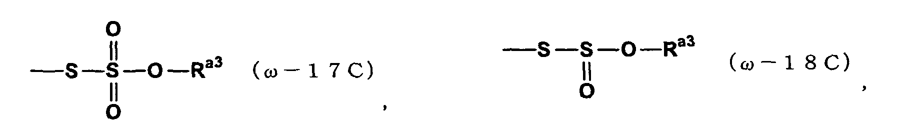 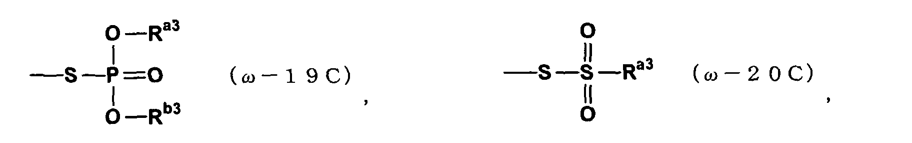 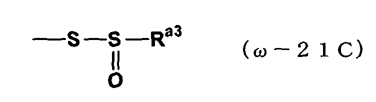 wherein R^{a3} and R^{b3} may be the same or different and represent a hydrocarbon group which may be substituted or a heterocyclic group which may be substituted, or R^{a3} and R^{b3} combine to each other, together with the nitrogen atom to which they bind, to form a cyclic amino group which may be substituted.

In the definition of the aforementioned acyl-sulfanyl group, among the groups represented by the formula (ω-1C), those groups in which R^{a3} is a hydrocarbon group are referred to as "hydrocarbon-carbonyl-sulfanyl group," and those groups in which R^{a3} is a heterocyclic group are referred to as "heterocyclic ring-carbonyl-sulfanyl group."

Among the groups represented by the formula (ω-2C), those groups in which R^{a3} is a hydrocarbon group are referred to as "hydrocarbon-oxy-carbonyl-sulfanyl group," and those groups in which R^{a3} is a heterocyclic group are referred to as "heterocyclic ring-oxy-carbonyl-sulfanyl group."

Among the groups represented by the formula (ω-3C), those groups in which R^{a3} is a hydrocarbon group are referred to as "hydrocarbon-carbonyl-carbonyl-sulfanyl group," and those groups in which R^{a3} is a heterocyclic group are referred to as "heterocyclic ring-carbonyl-carbonyl-sulfanyl group."

Among the groups represented by the formula (ω-4C), those groups in which R^{a3} is a hydrocarbon group are referred to as "hydrocarbon-oxy-carbonyl-carbonyl-sulfanyl group," and those groups in which R^{a3} is a heterocyclic group are referred to as "heterocyclic ring-oxy-carbonyl-carbonyl-sulfanyl group."

Among the groups represented by the formula (ω-5C), those groups in which R^{a3} is a hydrocarbon group are referred to as "hydrocarbon-sulfanyl-carbonyl-sulfanyl group," and those groups in which R^{a3} is a heterocyclic group are referred to as "heterocyclic ring-sulfanyl-carbonyl-sulfanyl group."

Among the groups represented by the formula (ω-6C), those groups in which R^{a3} is a hydrocarbon group are referred to as "hydrocarbon-thiocarbonyl-sulfanyl group," and those groups in which R^{a3} is a heterocyclic group are referred to as "heterocyclic ring-thiocarbonyl-sulfanyl group."

Among the groups represented by the formula (ω-7C), those groups in which R^{a3} is a hydrocarbon group are referred to as "hydrocarbon-oxy-thiocarbonyl-sulfanyl group," and those groups in which R^{a3} is a heterocyclic group are referred to as "heterocyclic ring-oxy-thiocarbonyl-sulfanyl group."

Among the groups represented by the formula (ω-8C), those groups in which R^{a3} is a hydrocarbon group are referred to as "hydrocarbon-sulfanyl-thiocarbonyl-sulfanyl group," and those groups in which R^{a3} is a heterocyclic group are referred to as "heterocyclic ring-sulfanyl-thiocarbonyl-sulfanyl group."

Among the groups represented by the formula (ω-9C), those groups in which R^{a3} is a hydrocarbon group are referred to as "N-hydrocarbon-carbamoyl-sulfanyl group," and those groups in which R^{a3} is a heterocyclic group are referred to as "N-heterocyclic ring-carbamoyl-sulfanyl group."

Among the groups represented by the formula (ω-10C), those groups in which both R^{a3} and R^{b3} are a hydrocarbon groups are referred to as "N,N-di(hydrocarbon)-carbamoyl-sulfanyl group," those groups in which both R^{a3} and R^{b3} are heterocyclic groups are referred to as "N,N-di(heterocyclic ring)-carbamoyl-sulfanyl group," those groups in which R^{a3} is a hydrocarbon group and R^{b3} is a heterocyclic group are referred to as "N-hydrocarbon-N-heterocyclic ring-carbamoyl-sulfanyl group," and those groups in which R^{a3} and R^{b3} combine to each other, together with the nitrogen atom to which they bind, to form a cyclic amino group are referred to as "cyclicamino-carbonyl-sulfamoyl group."

Among the groups represented by the formula (ω-11C), those groups in which R^{a3} is a hydrocarbon group are referred to as "N-hydrocarbon-thiocarbamoyl-sulfanyl group," and those groups in which R^{a3} is a heterocyclic group are referred to as "N-heterocyclic ring-thiocarbamoyl-sulfanyl group."

Among the groups represented by the formula (ω-12C), those groups in which both R^{a3} and R^{b3} are hydrocarbon groups are referred to as "N,N-di(hydrocarbon)-thiocarbamoyl-sulfanyl group," those groups in which and R^{a3} and R^{b3} are heterocyclic groups are referred to as "N,N-di(heterocyclic ring)-thiocarbamoyl-sulfanyl group," those groups in which R^{a3} is a hydrocarbon group and R^{b3} is a heterocyclic group are referred to as "N-hydrocarbon-N-heterocyclic ring-thiocarbamoyl-sulfanyl group," and those groups in which R^{a3} and R^{b3} combine to each other, together with the nitrogen atom to which they bind, to form a cyclic amino group are referred to as "cyclicamino-thiocarbonyl-sulfamoyl group."

Among the groups represented by the formula (ω-13C), those groups in which R^{a3} is a hydrocarbon group are referred to as "N-hydrocarbon-sulfamoyl-sulfanyl group," and those groups in which R^{a3} is a heterocyclic group are referred to as "N-heterocyclic ring-sulfamoyl-sulfanyl group."

Among the groups represented by the formula (ω-14C), those groups in which both R^{a3} and R^{b3} are hydrocarbon groups are referred to as "N,N-di(hydrocarbon)-sulfamoyl-sulfanyl group," those groups in which both R^{a3} and Rb3 are heterocyclic groups are referred to as "N,N-di(heterocyclic ring)-sulfamoyl-sulfinyl group," those groups in which R^{a3} is a hydrocarbon group and R^{b3} is a heterocyclic group are referred to as "N-hydrocarbon-N-heterocyclic ring-sulfamoyl-sulfanyl group," and those groups in which R^{a3} and R^{b3} combine to each other, together with the nitrogen atom to which they bind, to form a cyclic amino group are referred to as "cyclicamino-sulfonyl-sulfanyl group."

Among the groups represented by the formula (ω-15C), those groups in which R^{a3} is a hydrocarbon group are referred to as "N-hydrocarbon-sulfinamoyl-sulfanyl group," and those groups in which R^{a3} is a heterocyclic group are referred to as "N-heterocyclic ring-sulfinamoyl-sulfanyl group."

Among the groups represented by the formula (ω-16C), those groups in which both R^{a3} and R^{b3} are hydrocarbon groups are referred to as "N,N-di(hydrocarbon)-sulfinamoyl-sulfanyl group," those groups in which both R^{a3} and R^{b3} are heterocyclic groups are referred to as "N,N-di(heterocyclic ring)-sulfinamoyl-sulfanyl group," those groups in which R^{a3} is a hydrocarbon group and R^{b3} is a heterocyclic group are referred to as "N-hydrocarbon-N-heterocyclic ring-sulfinamoyl-sulfanyl group," and those groups in which R^{a3} and R^{b3} combine to each other, together with the nitrogen atom to which they bind, to form a cyclic amino group are referred to as "cyclicamino-sulfanyl-sulfanyl group."

Among the groups represented by the formula (ω-17C), those groups in which R^{a3} is a hydrocarbon group are referred to as "hydrocarbon-oxy-sulfonyl-sulfanyl group," and those groups in which R^{a3} is a heterocyclic group are referred to as "heterocyclic ring-oxy-sulfonyl-sulfanyl group."

Among the groups represented by the formula (ω-18C), those groups in which R^{a3} is a hydrocarbon group are referred to as "hydrocarbon-oxy-sulfinyl-sulfanyl group," and those groups in which R^{a3} is a heterocyclic group are referred to as "heterocyclic ring-oxy-sulfinyl-sulfanyl group."

Among the groups represented by the formula (ω-19C), those groups in which both R^{a3} and R^{b3} are hydrocarbon groups are referred to as "O,O'-di(hydrocarbon)-phosphono-sulfanyl group," those groups in which both R^{a3} and R^{b3} are heterocyclic groups are referred to as "O,O'-di(heterocyclic ring)-phosphono-sulfanyl group," and those groups in which R^{a3} is a hydrocarbon group and R^{b3} is a heterocyclic group are referred to as "O-hydrocarbon-O'-heterocyclic ring-phosphono-sulfanyl group."

Among the groups represented by the formula (ω-20C), those groups in which R^{a3} is a hydrocarbon group are referred to as "hydrocarbon-sulfonyl-sulfanyl group," and those groups in which R^{a3} is a heterocyclic group are referred to as "heterocyclic ring-sulfonyl-sulfanyl group."

Among the groups represented by the formula (ω-21C), those groups in which R^{a3} is a hydrocarbon group are referred to as "hydrocarbon-sulfinyl-sulfanyl group," and those groups in which R^{a3} is a heterocyclic group are referred to as "heterocyclic ring-sulfinyl-sulfanyl group."

Examples of the hydrocarbon in the groups represented by the aforementioned formulas (ω-1C) through (ω-21C) include similar groups to the aforementioned hydrocarbon group. Examples of the hydrocarbon-carbonyl-sulfanyl group represented by the formula (ω-1C) include, for example, an alkyl-carbonyl-sulfanyl group, an alkenyl-carbonyl-sulfanyl group, an alkynyl-carbonyl-sulfanyl group, a cycloalkyl-carbonyl-sulfanyl group, a cycloalkenyl-carbonyl-sulfanyl group, a cycloalkanedienyl-carbonyl-sulfanyl group, a cycloalkyl-alkyl-carbonyl-sulfanyl group which are aliphatic hydrocarbon-carbonyl-sulfanyl groups; an aryl-carbonyl-sulfanyl group; an aralkyl-carbonyl-sulfanyl group; a bridged cyclic hydrocarbon-carbonyl-sulfanyl group; a spiro cyclic hydrocarbon-carbonyl-sulfanyl group; and a terpene family hydrocarbon-carbonyl-sulfanyl group. In the following, groups represented by the formulas (ω-2C) through (ω-21C) are similar to those explained above.

Examples of the heterocyclic ring in the groups represented by the aforementioned formulas (ω-1C) through (ω-21C) include similar groups to the aforementioned heterocyclic group. Examples of the heterocyclic ring-carbonyl-sulfanyl group represented by the formula (ω-1C) include, for example, a monocyclic heteroaryl-carbonyl-sulfanyl group, a fused polycyclic heteroaryl-carbonyl-sulfanyl group, a monocyclic non-aromatic heterocyclic ring-carbonyl-sulfanyl group, and a fused polycyclic non-aromatic heterocyclic ring-carbonyl-sulfanyl group. In the following, groups represented by the formula (ω -2C) through (ω-21C) are similar to those groups explained above.

Examples of the cyclic amino in the groups represented by the aforementioned formulas (ω-10C) through (ω-16C) include similar groups to the aforementioned cyclic amino group.

The aforementioned acyl-sulfanyl group, hydrocarbon-sulfanyl group, and heterocyclic-sulfanyl group are generically referred to as "substituted sulfanyl group." Moreover, these substituted sulfanyl group and sulfanyl group are generically referred to as "sulfanyl group which may be substituted."

Examples of the N-hydrocarbon-amino group include the groups in which one hydrogen atom of amino group is substituted with a hydrocarbon group, and include, for example, an N-alkyl-amino group, an N-alkenyl-amino group, an N-alkynyl-amino group, an N-cycloalkyl-amino group, an N-cycloalkyl-alkyl-amino group, an N-aryl-amino group, and an N-aralkyl-amino group.

Examples of the N-alkyl-amino group include, for example, methylamino, ethylamino, n-propylamino, isopropylamino, n-butylamino, isobutylamino, sec-butylamino, tert-butylamino, n-pentylamino, isopentylamino, (2-methylbutyl)amino, (1-methylbutyl)amino, neopentylamino, (1,2-dimethylpropyl)amino, (1-ethylpropyl)amino, n-hexylamino, (4-methylpentyl)amino, (3-methylpentyl)amino, (2-methylpentyl)amino, (1-methylpentyl)amino, (3,3-dimethylbutyl)amino, (2,2-dimethylbutyl)amino, (1,1-dimethylbutyl)amino, (1,2-dimethylbutyl)amino, (1,3-dimethylbutyl)amino, (2,3-dimethylbutyl)amino, (2-ethylbutyl)amino, (1-ethylbutyl)amino, (1-ethyl-1-methylpropyl)amino, n-heptylamino, n-octylamino, n-nonylamino, n-decylamino, n-undecylamino, n-dodecylamino, n-tridecylamino, n-tetradecylamino, and n-pentadecylamino, which are C₁ to C₁₅ straight chain or branched chain N-alkyl amino groups.

Examples of the N-alkenyl-amino group include, for example, vinyl amino, (prop-1-en-1-yl)amino, allylamino, isopropenylamino, (but-1-en-1-yl)amino, (but-2-en-1-yl)amino, (but-3-en-1-yl)amino, (2-methylprop-2-en-1-yl)amino, (1-methylprop-2-en-1-yl)amino, (pent-1-en-1-yl)amino, (pent-2-en-1-yl)amino, (pent-3-en-1-yl)amino, (pent-4-en-1-yl)amino, (3-methylbut-2-en-1-yl)amino, (3-methylbut-3-en-1-yl)amino, (hex-1-en-1-yl)amino, (hex-2-en-1-yl)amino, (hex-3-en-1-yl)amino, (hex-4-en-1-yl)amino, (hex-5-en-1-yl)amino, (4-methylpent-3-en-1-yl)amino, (4-methylpent-3-en-1-yl)amino, (hept-1-en-1-yl)amino, (hept-6-en-1-yl)amino, (oct-1-en-1-yl)amino, (oct-7-en-1-yl)amino, (non-1-en-1-yl)amino, (non-8-en-1-yl)amino, (dec-1-en-1-yl)amino, (dec-9-en-1-yl)amino, (undec-1-en-1-yl)amino, (undec-10-en-1-yl)amino, (dodec-1-en-1-yl)amino, (dodec-11-en-1-yl)amino, (tridec-1-en-1-yl)amino, (tridec-12-en-1-yl)amino, (tetradec-1-en-1-yl)amino, (tetradec-13-en-1-yl)amino, (pentadec-1-en-1-yl)amino, and (pentadec-14-en-1-yl)amino, which are C₂ to C₁₅ straight chain or branched chain N-alkenyl amino groups.

Examples of the N-alkynyl-amino group include, for example, ethynylamino, (prop-1-yn-1-yl)amino, (prop-2-yn-1-yl)amino, (but-1-yn-1-yl)amino, (but-3-yn-1-yl)amino, (1-methylprop-2-yn-1-yl)amino, (pent-1-yn-1-yl)amino, (pent-4-yn-1-yl)amino, (hex-1-yn-1-yl)amino, (hex-5-yn-1-yl)amino, (hept-1-yn-1-yl)amino, (hept-6-yn-1-yl)amino, (oct-1-yn-1-yl)amino, (oct-7-yn-1-yl)amino, (non-1-yn-1-yl)amino, (non-8-yn-1-yl)amino, (dec-1-yn-1-yl)amino, (dec-9-yn-1-yl)amino, (undec-1-yn-1-yl)amino, (undec-10-yn-1-yl)amino, (dodec-1-yn-1-yl)amino, (dodec-11-yn-1-yl)amino, (tridec-1-yn-1-yl)amino, (tridec-12-yn-1-yl)amino, (tetradec-1-yn-1-yl)amino, (tetradec-13-yn-1-yl)amino, (pentadec-1-yn-1-yl)amino, and (pentadec-14-yn-1-yl)amino, which are C₂ to C₁₅ straight chain or branched chain N-alkynyl-amino groups.

Examples of the N-cycloalkyl-amino group include, for example, cyclopropylamino, cyclobutylamino, cyclopentylamino, cyclohexylamino, cycloheptylamino, and cyclooctylamino, which are C₃ to C₈ N-cycloalkyl-amino groups.

Examples of the N-cycloalkyl-alkyl-amino group include, for example, (cyclopropylmethyl)amino, (1-cyclopropylethyl)amino, (2-cyclopropylethyl)amino, (3-cyclopropylpropyl)amino, (4-cyclopropylbutyl)amino, (5-cyclopropylpentyl)amino, (6-cyclopropylhexyl)amino, (cyclobutylmethyl)amino, (cyclopentylmethyl)amino, (cyclobutylmethyl)amino, (cyclopentylmethyl)amino, (cyclohexylmethyl)amino, (2-cyclohexylethyl)amino, (3-cyclohexylpropyl)amino, (4-cyclohexylbutyl)amino, (cycloheptylmethyl)amino, (cyclooctylmethyl)amino, and (6-cyclooctylhexyl)amino, which are C₄ to C₁₄ N-cycloalkyl-alkyl-amino groups.

Examples of the N-aryl-amino group include, for example, phenylamino, 1-naphthylamino, 2-naphtylamino, anthrylamino, phenanthrylamino, and acenaphthylenylamino, which are C₆ to C₁₄ N-mono-arylamino groups.

Examples of the N-aralkyl-amino group include, for example, benzylamino, (1-naphthylmethyl)amino, (2-naphthylmethyl)amino, (anthracenylmethyl)amino, (phenanthrenylmethyl)amino, (acenaphthylenylmethyl)amino, (diphenylmethyl)amino, (1-phenethyl)amino, (2-phenethyl)amino, (1-(1-naphthyl)ethyl)amino, (1-(2-naphthyl)ethyl)amino, (2-(1-naphthyl)ethyl)amino, (2-(2-naphthyl)ethyl)amino, (3-phenylpropyl)amino, (3-(1-naphthyl)propyl)amino, (3-(2-naphthyl)propyl)amino, (4-phenylbutyl)amino, (4-(1-naphthyl)butyl)amino, (4-(2-naphthyl)butyl)amino, (5-phenylpentyl)amino, (5-(1-naphthyl)pentyl)amino, (5-(2-naphthyl)pentyl)amino, (6-phenylhexyl)amino, (6-(1-naphthyl)hexyl)amino, and (6-(2-naphthyl)hexyl)amino, which are C₇ to C₁₆ N-aralkyl-amino groups.

Examples of the N,N-di(hydrocarbon)-amino group include the groups in which two hydrogen atoms of amino group are substituted with hydrocarbon groups, and include, for example, N,N-dimethylamino, N,N-diethylamino, N-ethyl-N-methylamino, N,N-di-n-propylamino, N,N-diisopropylamino, N-allyl-N-methylamino, N-(prop-2-yn-1-yl)-N-methylamino, N,N-dicyclohexylamino, N-cyclohexyl-N-methylamino, N-cyclohexylmethylamino-N-methylamino, N,N-diphenylamino, N-methyl-N-phenylamino, N,N-dibenzylamino, and N-benzyl-N-methylamino.

Examples of the N-heterocyclic ring-amino group include the groups in which one hydrogen atom of amino group is substituted with a heterocyclic group, and include, for example, (3-pyrrolizinyl)amino, (4-piperidinyl)amino, (2-tetrahydropyranyl)amino, (3-indolinyl)amino, (4-chromanyl)amino, (3-thienyl)amino, (3-pyridyl)amino, (3-quinolyl)amino, and (5-indolyl)amino.

Examples of the N-hydrocarbon-N-heterocyclic ring-amino group include the groups in which two hydrogen atoms of amino group are substituted with hydrocarbon group and heterocyclic group respectively, and include, for example, N-methyl-N-(4-piperidinyl)amino, N-(4-chromanyl)-N-methylamino, N-methyl-N-(3-thienyl)amino, N-methyl-N-(3-pyridyl)amino, N-methyl-N-(3-quinolyl)amino.

Examples of the acyl-amino group include the groups in which one hydrogen atom of the amino group is substituted with an acyl group, and include, for example, formylamino group, glyoxyloylamino group, thioformylamino group, carbamoylamino group, thiocarbamoylamino group, sulfamoylamino group, sulfinamoylamino group, carboxyamino group, sulphoamino group, phosphonoamino group, and groups represented by the following formulas : 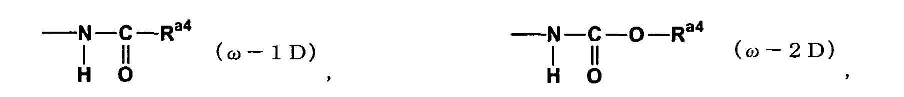 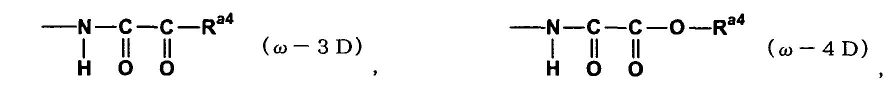 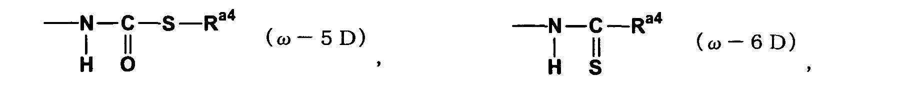 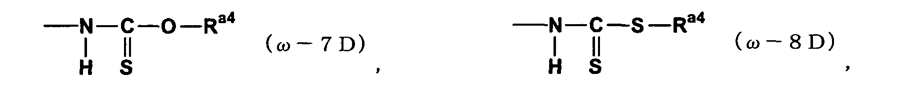 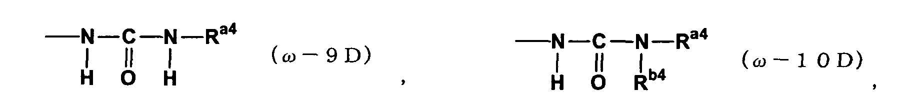 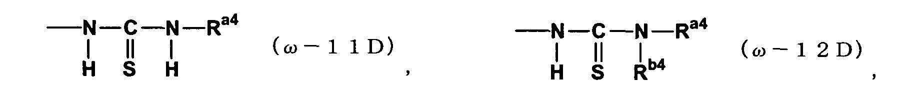 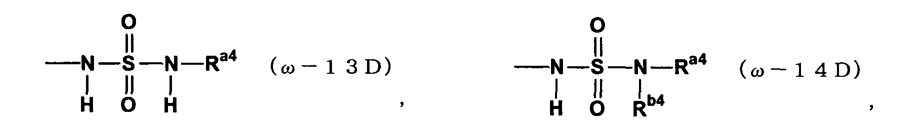 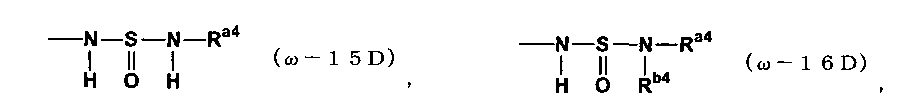 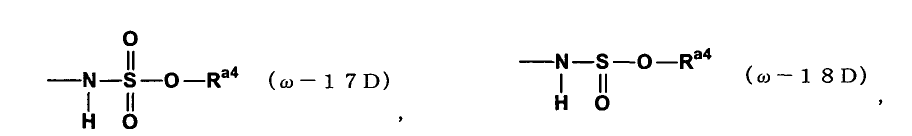 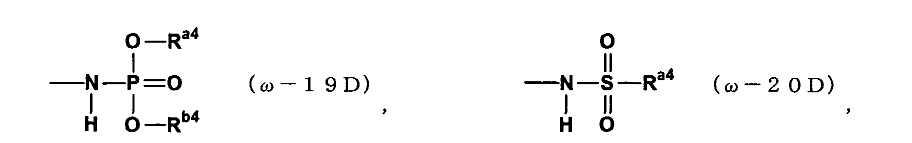 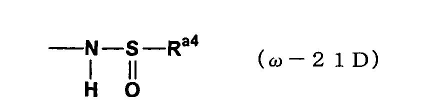 wherein R^{a4} and R^{b4} may be the same or different and represent a hydrocarbon group which may be substituted or a heterocyclic group which may be substituted, or R^{a4} and R^{b4} combine to each other, together with the nitrogen atom to which they bind, to form a cyclic amino group which may be substituted.

In the definition of the aforementioned acyl-amino group, among the groups represented by the formula (ω-1D), those groups in which R^{a4} is a hydrocarbon group are referred to as "hydrocarbon-carbonyl-amino group," and those groups in which R^{a4} is a heterocyclic group are referred to as "heterocyclic ring-carbonyl-amino group."

Among the groups represented by the formula (ω-2D), those groups in which R^{a4} is a hydrocarbon group are referred to as "hydrocarbon-oxy-carbonyl-amino group," and those groups in which R^{a4} is a heterocyclic group are referred to as "heterocyclic ring-oxy-carbonyl-amino group."

Among the groups represented by the formula (ω-3D), those groups in which R^{a4} is a hydrocarbon group are referred to as "hydrocarbon-carbonyl-carbonyl-amino group," and those groups in which R^{a4} is a heterocyclic group are referred to as "heterocyclic ring-carbonyl-carbonyl-amino group."

Among the groups represented by the formula (ω-4D), those groups in which R^{a4} is a hydrocarbon group are referred to as "hydrocarbon-oxy-carbonyl-carbonyl-amino group," and those groups in which R^{a4} is a heterocyclic group are referred to as "heterocyclic ring-oxy-carbonyl-carbonyl-amino group."

Among the groups represented by the formula (ω-5D), those groups in which R^{a4} is a hydrocarbon group are referred to as "hydrocarbon-sulfanyl-carbonyl-amino group," and those groups in which R^{a4} is a heterocyclic group are referred to as "heterocyclic ring-sulfanyl-carbonyl-amino group."

Among the groups represented by the formula (ω-6D), those groups in which R^{a4} is a hydrocarbon group are referred to as "hydrocarbon-thiocarbonyl-amino group," and those groups in which R^{a4} is a heterocyclic group are referred to as "heterocyclic ring-thiocarbonyl-amino group."

Among the groups represented by the formula (ω-7D), those groups in which R^{a4} is a hydrocarbon group are referred to as "hydrocarbon-oxy-thiocarbonyl-amino group," and those groups in which R^{a4} is a heterocyclic group are referred to as "heterocyclic ring-oxy-thiocarbonyl-amino group."

Among the groups represented by the formula (ω-8D), those groups in which R^{a4} is a hydrocarbon group are referred to as "hydrocarbon-sulfanyl-thiocarbonyl-amino group," and those groups in which R^{a4} is a heterocyclic group are referred to as "heterocyclic ring-sulfanyl-thiocarbonyl-amino group."

Among the groups represented by the formula (ω-9D), those groups in which R^{a4} is a hydrocarbon group are referred to as "N-hydrocarbon-carbamoyl group," and those groups in which R^{a4} is a heterocyclic group are referred to as "N-heterocyclic ring-carbamoyl-amino group."

Among the groups represented by the formula (ω-10D), those groups in which both R^{a4} and R^{b4} are hydrocarbon groups are referred to as "N,N-di(hydrocarbon)-carbamoyl-amino group," those groups in which both R^{a4} and R^{b4} are heterocyclic groups are referred to as "N,N-di(heterocyclic ring)-carbamoyl-amino group," those groups in which R^{a4} is a hydrocarbon group and R^{b4} is a heterocyclic group are referred to as "N-hydrocarbon-N-heterocyclic ring-carbamoyl-amino group," and those groups in which R^{a4} and R^{b4} combine to each other, together with the nitrogen atom to which they bind, to form a cyclic amino group are referred to as "cyclic amino-carbonyl-amino group."

Among the groups represented by the formula (ω-11D), those groups in which R^{a4} is a hydrocarbon group are referred to as "N-hydrocarbon-thiocarbamoyl-amino group," and those groups in which R^{a4} is a heterocyclic ring group are referred to as "N-heterocyclic-thiocarbamoyl-amino group."

Among the groups represented by the formula (ω-12D), those groups in which both R^{a4} and R^{b4} are hydrocarbon groups are referred to as "N,N-di(hydrocarbon)-thiocarbamoyl-amino group," those groups in which both R^{a4} and R^{b4} are heterocyclic groups are referred to as "N,N-di(heterocyclic ring)-thiocarbamoyl-amino group," those groups in which R^{a4} is a hydrocarbon group and R^{b4} is a heterocyclic group are referred to as "N-hydrocarbon-N-heterocyclic ring-thiocarbamoyl-amino group," and those groups in which R^{a4} and R^{b4} combine to each other, together with the nitrogen atom to which they bind, to form a cyclic amino group are referred to as "cyclic amino-thiocarbonyl-amino group."

Among the groups represented by the formula (ω-13D), those groups in which R^{a4} is a hydrocarbon group are referred to as "N-hydrocarbon-sulfamoyl-amino group," and those groups in which R^{a4} is a heterocyclic group are referred to as "N-heterocyclic ring-sulfamoyl-amino group."

Among the groups represented by the formula (ω-14D), those groups in which both R^{a4} and R^{b4} are hydrocarbon groups are referred to as "di(hydrocarbon)-sulfamoyl-amino group," those groups in which both R^{a4} and R^{b4} are heterocyclic groups are referred to as "N,N-di(heterocyclic ring)-sulfamoyl-amino group," those groups in which R^{a4} is a hydrocarbon group and R^{b4} is a heterocyclic group are referred to as "N-hydrocarbon-N-heterocyclic ring-sulfamoyl-amino group," and those groups in which R^{a4} and R^{b4} combine to each other, together with the nitrogen atom to which they bind, to form a cyclic amino group are referred to as "cyclic amino-sulfonyl-amino group."

Among the groups represented by the formula (ω-15D), those groups in which R^{a4} is a hydrocarbon group are referred to as "N-hydrocarbon-sulfinamoyl-amino group," and those groups in which R^{a4} is a heterocyclic group are referred to as "N-heterocyclic ring-sulfinamoyl-amino group."

Among the groups represented by the formula (ω-16D), those groups in which both R^{a4} and R^{b4} are hydrocarbon groups are referred to as "N,N-di(hydrocarbon)-sulfinamoyl-amino group," those groups in which both R^{a4} and R^{b4} are heterocyclic groups are referred to as "N,N-di(heterocyclic ring)-sulfinamoyl-amino group," groups in which R^{a4} is a hydrocarbon group and R^{b4} is a heterocyclic group are referred to as "N-hydrocarbon-N-heterocyclic ring-sulfinamoyl-amino group," and those groups in which R^{a4} and R^{b4} combine to each other, together with the nitrogen atom to which they bind, to form a cyclic amino group are referred to as "cyclic amino-sulfinyl-amino group."

Among the groups represented by the formula (ω-17D), those groups in which R^{a4} is a hydrocarbon group are referred to as "hydrocarbon-oxy-sulfonyl-amino group," and those groups in which R^{a4} is a heterocyclic group are referred to as "heterocyclic ring-oxy-sulfoyl-amino group."

Among the groups represented by the formula (ω-18D), those groups in which R^{a4} is a hydrocarbon group are referred to as "hydrocarbon-oxy-sulfinyl-amino group," and those groups in which R^{a4} is a heterocyclic group are referred to as "heterocyclic ring-oxy-sulfinyl-amino group."

Among the groups represented by the formula (ω-19D), those groups in which both R^{a4} and R^{b4} are hydrocarbon groups are referred to as "O,O'-di(hydrocarbon)-phosphono-amino group," those groups in which both R^{a4} and R^{b4} are heterocyclic groups are referred to as "O,O'-di(heterocyclic ring)-phosphono-amino group," and those groups in which R^{a4} is a hydrocarbon group and R^{b4} is a heterocyclic group are referred to as "O-hydrocarbon-O'-heterocyclic ring-phosphono-amino group."

Among the groups represented by the formula (ω-20D), those groups in which R^{a4} is a hydrocarbon group are referred to as "hydrocarbon-sulfonyl-amino group," and those groups in which R^{a4} is a heterocyclic group are referred to as "heterocyclic ring-sulfonyl-amino group."

Among the groups represented by the formula (ω-21D), those groups in which R^{a4} is a hydrocarbon group are referred to as "hydrocarbon-sulfinyl-amino group," and those groups in which R^{a4} is a heterocyclic group are referred to as "heterocyclic ring-sulfinyl-amino group."

Examples of the hydrocarbon in the groups represented by the aforementioned formulas (ω-1D) through (ω-21D) include the similar groups to the aforementioned hydrocarbon group. Examples of the hydrocarbon-carbonyl-amino groups represented by the formula (ω-1D) include, for example, an alkyl-carbonyl-amino group, an alkenyl-carbonyl-amino group, an alkynyl-carbonyl-amino group, a cycloalkyl-carbonyl-amino group, a cycloalkenyl-carbonyl-amino group, a cycloalkanedienyl-carbonyl-amino group, a cycloalkyl-alkyl-carbonyl-amino group which are aliphatic hydrocarbon-carbonyl-amino groups; an aryl-carbonyl-amino group; an aralkyl-carbonyl-amino group; a bridged cyclic hydrocarbon-carbonyl-amino group; a spiro cyclic hydrocarbon-carbonyl-amino group; and a terpene family hydrocarbon-carbonyl-amino group. In the following, groups represented by the formulas (ω-2D) through (ω-21D) are similar to those explained above.

Examples of the heterocyclic ring in the groups represented by the aforementioned formulas (ω-1D) through (ω-21D) include similar groups to the aforementioned heterocyclic group. Examples of the heterocyclic ring-carbonyl-amino group represented by the formula (ω-1D) include, for example, a monocyclic heteroaryl-carbonyl-amino group, a fused polycyclic heteroaryl-carbonyl-amino group, a monocyclic non-aromatic heterocyclic-carbonyl-amino group, and a fused polycyclic non-aromatic heterocyclic-carbonyl-amino group. In the following, groups represented by the formulas (ω-2D) through (ω-21D) are similar to those groups explained above.

Examples of the cyclic amino in the groups represented by the aforementioned formulas (ω-10D) through (ω-16D) include similar groups to the aforementioned cyclic amino group.

Examples of the di(acyl)-amino group include the groups in which two hydrogen atoms of amino group are substituted with acyl groups in the definitions of the aforementioned substituents according to "which may be substituted." Examples include, for example, di(formyl)-amino group, di(glyoxyloyl)-amino group, di(thioformyl)-amino group, di(carbamoyl)-amino group, di(thiocarbamoyl)-amino group, di(sulfamoyl)-amino group, di(sulfinamoyl)-amino group, di(carboxy)-amino group, di(sulfo)-amino group, di(phosphono)-amino group, and groups represented by the following formulas : 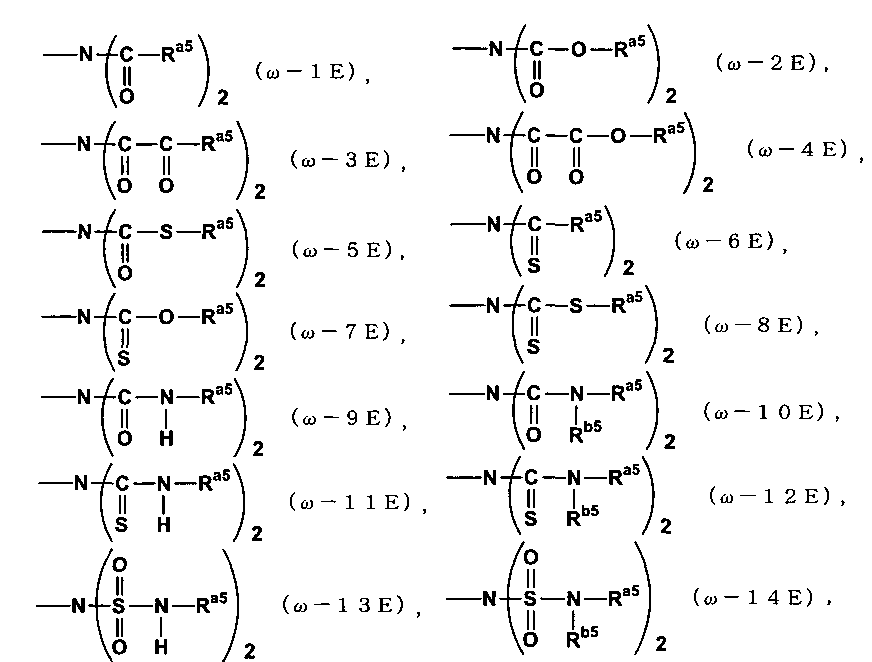 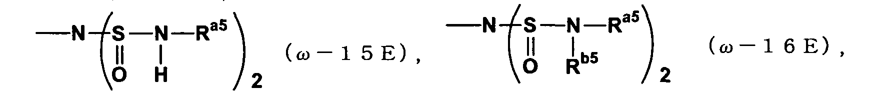 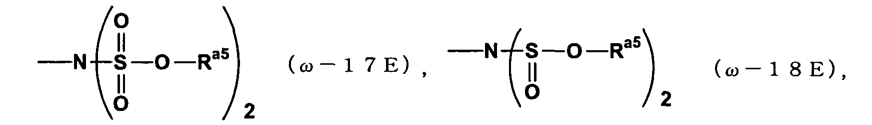 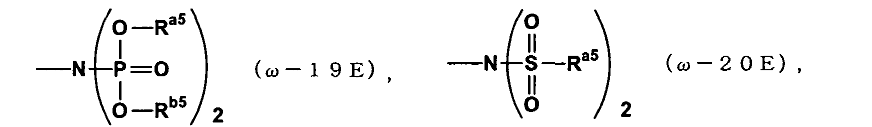 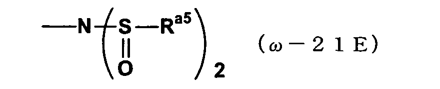 wherein R^{a5} and R^{b5} may be the same or different and represent hydrogen atom, a hydrocarbon group which may be substituted or a heterocyclic group which may be substituted, or R^{a5} and R^{b5} combine to each other, together with the nitrogen atom to which they bind, to form a cyclic amino group which may be substituted.

In the definition of aforementioned di(acyl)-amino group, among the groups represented by the formula (ω-1E), those groups in which R^{a5} is a hydrocarbon group are referred to as "bis(hydrocarbon-carbonyl)-amino group," and those groups in which R^{a5} is a heterocyclic group are referred to as "bis(heterocyclic ring-carbonyl)-amino group."

Among the groups represented by the formula (ω-2E), those groups in which R^{a5} is a hydrocarbon group are referred to as "bis(hydrocarbon-oxy-carbonyl)-amino group," and those groups in which R^{a5} is a heterocyclic group are referred to as "bis(heterocyclic ring-oxy-carbonyl)-amino group."

Among the groups represented by the formula (ω-3E), those groups in which R^{a5} is a hydrocarbon group are referred to as "bis(hydrocarbon-carbonyl-carbonyl)-amino group," and those groups in which R^{a5} is a heterocyclic group are referred to as "bis(heterocyclic ring-carbonyl-carbonyl)-amino group."

Among the groups represented by the formula (ω-4E), those groups in which R^{a5} is a hydrocarbon group are referred to as "bis(hydrocarbon-oxy-carbonyl-carbonyl)-amino group," and those groups in which R^{a5} is a heterocyclic group are referred to as "bis(heterocyclic ring-oxy-carbonyl-carbonyl)-amino group."

Among the groups represented by the formula (ω-5E), those groups in which R^{a5} is a hydrocarbon group are referred to as "bis(hydrocarbon-sulfanyl-carbonyl)-amino group," and those groups in which R^{a5} is a heterocyclic group are referred to as "bis(heterocyclic ring-sulfanyl-carbonyl)-amino group."

Among the groups represented by the formula (ω-6E), those groups in which R^{a5} is a hydrocarbon group are referred to as "bis(hydrocarbon-thiocarbonyl)-amino group," and those groups in which R^{a5} is a heterocyclic group are referred to as "bis(heterocyclic ring-thiocarbonyl)-amino group."

Among the groups represented by the formula (ω-7E), those groups in which R^{a5} is a hydrocarbon group are referred to as "bis(hydrocarbon-oxy-thiocarbonyl)-amino group," and those groups in which R^{a5} is a heterocyclic group are referred to as "bis(heterocyclic ring-oxy-thiocarbonyl)-amino group."

Among the groups represented by the formula (ω-8E), those groups in which R^{a5} is a hydrocarbon group are referred to as "bis(hydrocarbon-sulfanyl-thiocarbonyl)-amino group," and those groups in which R^{a5} is a heterocyclic group are referred to as "bis(heterocyclic ring-sulfanyl-thiocarbonyl)-amino group."

Among the groups represented by the formula (ω-9E), those groups in which R^{a5} is a hydrocarbon group are referred to as "bis(N-hydrocarbon-carbamoyl)-amino group," and those groups in which R^{a5} is a heterocyclic group are referred to as "bis(N-heterocyclic ring-carbamoyl)-amino group."

Among the groups represented by the formula (ω-10E), those groups in which both R^{a5} and R^{b5} are hydrocarbon groups are referred to as "bis[N,N-di(hydrocarbon)-carbamoyl]-amino group," those groups in which both R^{a5} and R^{b5} are heterocyclic groups are referred to as "bis[N,N-di(heterocyclic ring)-carbamoyl]-amino group," groups in which R^{a5} is a hydrocarbon group and R^{b5} is a heterocyclic group are referred to as "bis(N-hydrocarbon-N-heterocyclic ring-carbamoyl)-amino group," and those groups in which R^{a5} and R^{b5} combine to each other, together with the nitrogen atom to which they bind, to form a cyclic amino groups are referred to as "bis(cyclic amino-carbonyl)amino group."

Among the groups represented by the formula (ω-11E), those groups in which R^{a5} is a hydrocarbon group are referred to as "bis(N-hydrocarbon-thiocarbamoyl)-amino group," and those groups in which R^{a5} is a heterocyclic group are referred to as "bis(N-heterocyclic ring-thiocarbamoyl)-amino group."

Among the groups represented by the formula (ω-12E), those groups in which both R^{a5} and R^{b5} are hydrocarbon groups are referred to as "bis[N,N-di(hydrocarbon)-thiocarbamoyl]-amino group," those groups in which both R^{a5} and R^{b5} are heterocyclic groups are referred to as "bis[N,N-di(heterocyclic ring)-thiocarbamoyl]-amino group," those groups in which R^{a5} is a hydrocarbon group and R^{b5} is a heterocyclic group are referred to as "bis(N-hydrocarbon-N-heterocyclic ring-thiocarbamoyl)-amino group," and those groups in which R^{a5} and R^{b5} combine to each other, together with the nitrogen atom to which they bind, to form a cyclic amino group are referred to as "bis(cyclic amino-thiocarbonyl)-amino group."

Among the groups represented by the formula (ω-13E), those groups in which R^{a5} is a hydrocarbon group are referred to as "bis(N-hydrocarbon-sulfamoyl)-amino group," and those groups in which R^{a5} is a heterocyclic group are referred to as "bis(N-heterocyclic ring-sulfamoyl)-amino group."

Among the groups represented by the formula (ω-14E), those groups in which both R^{a5} and R^{b5} are hydrocarbon groups are referred to as "bis[N,N-di(hydrocarbon)-sulfamoyl]-amino group," those groups in which both R^{a5} and R^{b5} are heterocyclic groups are referred to as "bis[N,N-di(heterocyclic ring)-sulfamoyl]-amino group," those groups in which R^{a5} is a hydrocarbon group and R^{b5} is a heterocyclic group are referred to as "bis(N-hydrocarbon-N-heterocyclic ring-sulfamoyl)-amino group," and those groups in which R^{a5} and R^{b5} combine to each other, together with the nitrogen atom to which they bind, to form a cyclic amino group are referred to as "bis(cyclic amino-sulfonyl)amino group."

Among the groups represented by the formula (ω-15E), those groups in which R^{a5} is a hydrocarbon group are referred to as "bis(N-hydrocarbon-sulfinamoyl)-amino group," and those groups in which R^{a5} is a heterocyclic group are referred to as "bis(N-heterocyclic ring-sulfinamoyl)-amino group."

Among the groups represented by the formula (ω-16E), those groups in which R^{a5} and R^{b5} are hydrocarbon groups are referred to as "bis[N,N-di(hydrocarbon)-sulfinamoyl]-amino group," those groups in which R^{a5} and R^{b5} are heterocyclic groups are referred to as "bis[N,N-di(heterocyclic ring)-sulfinamoyl]-amino group," those groups in which R^{a5} is a hydrocarbon group and R^{b5} is a heterocyclic group are referred to as "bis(N-hydrocarbon-N-heterocyclic ring-sulfinamoyl)-amino group," and those groups in which R^{a5} and R^{b5} combine to each other, together with the nitrogen atom to which they bind, to form a cyclic amino group are referred to as "bis(cyclic amino-sulfinyl)amino group."

Among the groups represented by the formula (ω-17E), those groups in which R^{a5} is a hydrocarbon group are referred to as "bis(hydrocarbon-oxy-sulfonyl)-amino group," and those groups in which R^{a5} is a heterocyclic group are referred to as "bis(heterocyclic ring-oxy-sulfonyl)-amino group."

Among the groups represented by the formula (ω-18E), those groups in which R^{a5} is a hydrocarbon group are referred to as "bis(hydrocarbon-oxy-sulfinyl)-amino group," and those groups in which R^{a5} is a heterocyclic group are referred to as "bis(heterocyclic ring-oxy-sulfinyl)-amino group."

Among the groups represented by the formula (ω-19E), those groups in which both R^{a5} and R^{b5} are hydrocarbon groups are referred to as "bis[O,O'-di(hydrocarbon)-phosphono]-amino group," those groups in which both R^{a5} and R^{b5} are heterocyclic groups are referred to as "bis[O,O'-di(heterocyclic ring)-phosphono]-amino group," and those groups in which R^{a5} is a hydrocarbon group and R^{b5} is a heterocyclic group are referred to as "bis(O-hydrocarbon-O'-heterocyclic ring-phosphono)-amino group."

Among the groups represented by the formula (ω-20E), those groups in which R^{a5} is a hydrocarbon group are referred to as "bis(hydrocarbon-sulfonyl)-amino group," and those groups in which R^{a5} is a heterocyclic group are referred to as "bis(heterocyclic ring-sulfonyl)-amino group."

Among the groups represented by the formula (ω-21E), those groups in which R^{a5} is a hydrocarbon group are referred to as "bis(hydrocarbon-sulfinyl)-amino group," and those groups in which R^{a5} is a heterocyclic group are referred to as "bis(heterocyclic ring-sulfinyl)-amino group."

Examples of the hydrocarbon in the groups represented by the aforementioned formulas (ω-1E) through (ω-21E) include the similar groups to the aforementioned hydrocarbon group. Examples of the bis(hydrocarbon-carbonyl)-amino groups represented by the formula (ω-1E) include, for example, a bis(alkyl-carbonyl)-amino group, a bis(alkenyl-carbonyl)-amino group, a bis(alkynyl-carbonyl)-amino group, a bis(cycloalkyl-carbonyl)-amino group, a bis(cycloalkenyl-carbonyl)-amino group, a bis(cycloalkanedienyl-carbonyl)-amino group, a bis(cycloalkyl-alkyl-carbonyl)-amino group which are bis(aliphatic hydrocarbon-carbonyl)-amino groups; a bis(aryl-carbonyl)-amino group; a bis(aralkyl-carbonyl)-amino group; a bis(bridged cyclic hydrocarbon-carbonyl)-amino group; a bis(spiro cyclic hydrocarbon-carbonyl)-amino group; and a bis(terpene family hydrocarbon-carbonyl)-amino group. In the following, groups represented by the formulas (ω-2E) through (ω-21E) are similar to those explained above.

Examples of the heterocyclic ring in the groups represented by the aforementioned formulas (ω-1E) through (ω-21E) include similar groups to the aforementioned heterocyclic group. Examples of the bis(heterocyclic ring-carbonyl)-amino group represented by the formula (ω-1E) include, for example, a bis(monocyclic heteroaryl-carbonyl)-amino group, a bis(fused polycyclic heteroaryl-carbonyl)-amino group, a bis(monocyclic non-aromatic heterocyclic-carbonyl)-amino group, and a bis(fused polycyclic non-aromatic heterocyclic-carbonyl)-amino group. In the following, groups represented by the formulas (ω-2E) through (ω-21E) are similar to those groups explained above.

Examples of the cyclic amino in the groups represented by the aforementioned formulas (ω-10E) through (ω-16E) include similar groups to the aforementioned cyclic amino group.

The aforementioned acyl-amino group and di(acyl)-amino group are generically referred to as "acyl substituted amino group." Furthermore, the aforementioned N-hydrocarbon-amino group, N,N-di(hydrocarbon)-amino group, N-heterocyclic-amino group, N-hydrocarbon-N-heterocyclic-amino group, cyclic amino group, acyl-amino group, and di(acyl)-amino group are generically referred to as "substituted amino group."

The compounds represented by the aforementioned general formula (I) are explained in details.

In the aforementioned general formula (I), examples of "A" include hydrogen atom or acetyl group, and hydrogen atom is preferred.

Examples of the "arene" in "an arene which may have one or more substituents in addition to the group represented by formula ―O―A wherein A has the same meaning as that defined above and the group represented by formula ―CONH―E wherein E has the same meaning as that defined above" in the definition of ring Z include a monocyclic or fused heterocyclic aromatic hydrocarbon, and include, for example, benzene ring, naphthalene ring, anthracene ring, phenanthrene ring, and acenaphylene ring. C₆ to C₁₀ arenes such as benzene ring, naphthalene ring and the like are preferred, benzene ring, and naphthalene ring are more preferred, and benzene ring is most preferred.

Examples of the substituent in the definition of "an arene which may have one or more substituents in addition to the group represented by formula ―O―A wherein A has the same meaning as that defined above and the group represented by formula ―CONH―E wherein E has the same meaning as that defined above" in the aforementioned definition of ring Z include similar groups to the substituent explained for the definition "which may be substituted." The position of substituents existing on the arene is not particularly limited, and when two or more substituents exist, they may be the same or different.

When "an arene which may have one or more substituents in addition to the group represented by formula ―O―A wherein A has the same meaning as that defined above and the group represented by formula ―CONH―E wherein E has the same meaning as that defined above" in the aforementioned definition of ring Z is "a benzene ring which may have one or more substituents in addition to the group represented by formula ―O―A wherein A has the same meaning as that defined above and the group represented by formula ―CONH―E wherein E has the same meaning as that defined above," "a benzene ring which has one to three substituents in addition to the group represented by formula ―O―A wherein A has the same meaning as that defined above and the group represented by formula ―CONH―E wherein E has the same meaning as that defined above" is preferred, and "a benzene ring which has one substituent in addition to the group represented by formula ―O―A wherein A has the same meaning as that defined above and the group represented by formula ―CONH―E wherein E has the same meaning as that defined above" is more preferred. Preferred examples of said substituents include groups selected from the following Substituent Group γ-1z. A halogen atom and tert-butyl group [(1,1-dimethyl)ethyl group] are more preferred, and a halogen atom is most preferred.
[Substituent Group γ-1z] a halogen atom, nitro group, cyano group, hydroxy group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group [thiophen-2-yl group], 3-thienyl group [thiophen-3-yl group], 1-pyrrolyl group [pyrrol-1-yl group], 2-methylthiazol-4-yl group, imidazo[1,2-a]pyridin-2-yl group, 2-pyridyl group [pyridin-2-yl group], acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, and {[4-(pyridin-2-yl)sulfamoyl]phenyl}diazenyl group

When "an arene which may have one or more substituents in addition to the group represented by formula ―O―A wherein A has the same meaning as that defined above and the group represented by formula ―CONH―E wherein E has the same meaning as that defined above" in the aforementioned definition of ring Z is "a benzene ring which may have one or more substituents in addition to the group represented by formula ―O―A wherein A has the same meaning as that defined above and the group represented by formula ―CONH―E wherein E has the same meaning as that defined above," it is most preferable that one substituent exists and locates on the position of R^{z} when the following partial formula (Iz-1) in the general formula containing ring Z 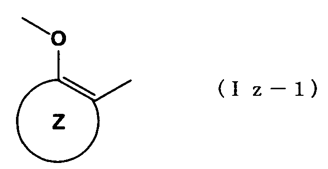 is represented by the following formula (Iz-2). 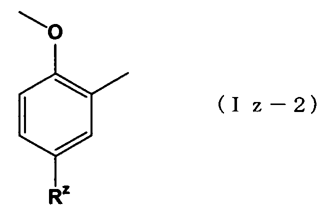 In this embodiment, said substituents can be defined as R^{z}. Preferred examples of R^{z} include a group selected from the following Substituent Group γ-2z. A halogen atom and tert-butyl group are more preferred, and a halogen atom is most preferred.
[Substituent Group γ-2z] a halogen atom, nitro group, cyano group, methoxy group, methyl group, isopropyl group, tert-butyl group, 1,1,3,3-tetramethylbutyl group, 2-phenylethen-1-yl group, 2,2-dicyanoethen-1-yl group, 2-cyano-2-(methoxycarbonyl)ethen-1-yl group, 2-carboxy-2-cyanoethen-1-yl group, ethynyl group, phenylethynyl group, (trimethylsilyl)ethynyl group, trifluoromethyl group, pentafluoroethyl group, phenyl group, 4-(trifluoromethyl)phenyl group, 4-fluorophenyl group, 2,4-difluorophenyl group, 2-phenethyl group, 1-hydroxyethyl group, 1-(methoxyimino)ethyl group, 1-[(benzyloxy)imino]ethyl group, 2-thienyl group, 3-thienyl group, 1-pyrrolyl group, 2-methylthiazol-4-yl group, imidazo[1,2-a]pyridin-2-yl group, 2-pyridyl group, acetyl group, isobutyryl group, piperidinocarbonyl group, 4-benzylpiperidinocarbonyl group, (pyrrol-1-yl)sulfonyl group, carboxy group, methoxycarbonyl group, N-[3,5-bis(trifluoromethyl)phenyl]carbamoyl group, N,N-dimethylcarbamoyl group, sulfamoyl group, N-[3,5-bis(trifluoromethyl)phenyl]sulfamoyl group, N,N-dimethylsulfamoyl group, amino group, N,N-dimethylamino group, acetylamino group, benzoylamino group, methanesulfonylamino group, benzenesulfonylamino group, 3-phenylureido group, (3-phenyl)thioureido group, (4-nitrophenyl)diazenyl group, and {[4-(pyridin-2-yl)sulfamoyl]phenyl}diazenyl group

When "an arene which may have one or more substituents in addition to the group represented by formula ―O―A wherein A has the same meaning as that defined above and the group represented by formula ―CONH―E wherein E has the same meaning as that defined above" in the aforementioned definition of ring Z is "a naphthalene ring which may have one or more substituents in addition to the group represented by formula ―O―A wherein A has the same meaning as that defined above and the group represented by formula ―CONH―E wherein E has the same meaning as that defined above," naphthalene ring is preferred.

Examples of the "hetero arene" in "a hetero arene which may have one or more substituents in addition to the group represented by formula ―0―A wherein A has the same meaning as that defined above and the group represented by formula ―CONH―E wherein E has the same meaning as that defined above" in the aforementioned definition of ring Z include a monocyclic or a fused polycyclic aromatic heterocyclic rings containing at least one of 1 to 3 kinds of heteroatoms selected from oxygen atom, sulfur atom and nitrogen atom and the like as ring-constituting atoms (ring forming atoms), and include, for example, furan ring, thiophene ring, pyrrole ring, oxazole ring, isoxazole ring, thiazole ring, isothiazole ring, imidazole ring, pyrazole ring, 1,2,3-oxadiazole ring, 1,2,3-thiadiazole ring, 1,2,3-triazole ring, pyridine ring, pyridazine ring, pyrimidine ring, pyrazine ring, 1,2,3-triazine ring, 1,2,4-triazine ring, 1H-azepine ring, 1,4-oxepine ring, 1,4-thiazepine ring, benzofuran ring, isobenzofuran ring, benzo[b]thiophene ring, benzo[c]thiophene ring, indole ring, 2H-isoindole ring, 1H-indazole ring, 2H-indazole ring, benzoxazole ring, 1,2-benzisoxazole ring, 2,1-benzisoxazole ring, benzothiazole ring, 1,2-benzisothiazole ring, 2,1-benzisothiazole ring, 1,2,3-benzoxadiazol ring, 2,1,3-benzoxadiazol ring, 1,2,3-benzothiadiazole ring, 2,1,3-benzothiadiazole ring, 1H-benzotriazole ring, 2H-benzotriazole ring, quinoline ring, isoquinoline ring, cinnoline ring, quinazoline ring, quinoxaline ring, phthalazine ring, naphthyridine ring, 1H-1,5-benzodiazepine ring, carbazole ring, α-carboline ring, β-carboline ring, γ-carboline ring, acridine ring, phenoxazine ring, phenothiazine ring, phenazine ring, phenanthridine ring, phenanthroline ring, thianthrene ring, indolizine ring, and phenoxathiine ring, which are 5 to 14-membered monocyclic or fused polycyclic aromatic heterocyclic rings. 5 to 10-membered monocyclic or fused polycyclic aromatic heterocyclic rings are preferred, and thiophene ring, pyridine ring, indole ring, and quinoxaline ring are more preferred.

Examples of the substituent in the definition of "a hetero arene which may have one or more substituents in addition to the group represented by formula ―0―A wherein A has the same meaning as that defined above and the group represented by formula ―CONH―E wherein E has the same meaning as that defined above" in the aforementioned definition of ring Z include similar groups to the substituent explained for the aforementioned definition "which may be substituted." The position of substituents existing on the hetero arene is not particularly limited, and when two or more substituents exist, they may be the same or different.

A halogen atom is preferred as the substituent in the definition of "a hetero arene which may have one or more substituents in addition to the group represented by formula ―O―A wherein A has the same meaning as that defined above and the group represented by formula ―CONH―E wherein E has the same meaning as that defined above" in the aforementioned definition of ring Z.

Examples of the substituent in the definition of "a 2,5-di-substituted phenyl group" in the definition of E include similar groups to the substituent explained for the definition "which may be substituted."

Preferred examples of the "2,5-di-substituted phenyl group" in the definition of E include groups represented by the following Substituent Group δ-1e.
[Substituent Group δ-1e] 2,5-dimethoxyphenyl group, 2-chloro-5-(trifluoromethyl)phenyl group, 2,5-bis(trifluoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2,5-dichlorophenyl group, 2,5-bis[(1,1-dimethyl)ethyl]phenyl group, 5-[(1,1-dimethyl)ethyl]-2-methoxyphenyl group, 4-methoxybiphenyl-3-yl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 5-isopropyl-2-methylphenyl group, 2,5-diethoxyphenyl group, 2,5-dimethylphenyl group, 5-chloro-2-cyano group, 5-diethylsulfamoyl-2-methoxyphenyl group, 2-chloro-5-nitrophenyl group, 2-methoxy-5-(phenylcarbamoyl)phenyl group, 5-acetylamino-2-methoxyphenyl group, 5-methoxy-2-methylphenyl group, 2,5-dibutoxyphenyl group, 2,5-diisopentyloxy group, 5-carbamoyl-2-methoxyphenyl group, 5-[(1,1-dimethyl)propyl]-2-phenoxyphenyl group, 2-hexyloxy-5-methanesulfonyl group, 5-(2,2-dimethylpropionyl)-2-methylphenyl group, 5-methoxy-2-(1-pyrrolyl)phenyl group, 5-chloro-2-(p-toluenesulfonyl)phenyl group, 2-chloro-5-(p-toluenesulfonyl)phenyl group, 2-fluoro-5-methanesulfonyl group, 2-methoxy-5-phenoxy group, 2-methoxy-5-(1-methyl-1-phenylethyl)phenyl group, 5-morpholino-2-nitrophenyl group, 5-fluoro-2-(1-imidazolyl)phenyl group, 2-butyl-5-nitrophenyl group, 5-[(1,1-dimethyl)propyl]-2-hydroxyphenyl group, 2-methoxy-5-methylphenyl group, 2,5-difluorophenyl group, 2-benzoyl-5-methylphenyl group, 2-(4-cyanophenoxy)-5-(trifluoromethyl)phenyl group, and 2-(4-methoxyphenoxy)-5-(trifluoromethyl)phenyl group

"A 2,5-di-substituted phenyl group wherein at least one of said substituents is trifluoromethyl group" is more preferred, a group selected from the following Substituent Group δ-2e is further preferred, and 2,5-bis(trifluoromethyl)phenyl group is most preferred.
[Substituent Group δ-2e] 2-chloro-5-(trifluoromethyl)phenyl group, 2,5-bis(trinuoromethyl)phenyl group, 2-fluoro-5-(trifluoromethyl)phenyl group, 2-nitro-5-(trifluoromethyl)phenyl group, 2-methyl-5-(trifluoromethyl)phenyl group, 2-methoxy-5-(trifluoromethyl)phenyl group, 2-methylsulfanyl-5-(trifluoromethyl)phenyl group, 2-(1-pyrrolidinyl)-5-(trifluoromethyl)phenyl group, 2-morpholino-5-(trifluoromethyl)phenyl group, 2-bromo-5-(trifluoromethyl)phenyl group, 2-(2-naphthyloxy)-5-(trifluoromethyl)phenyl group, 2-(2,4-dichlorophenoxy)-5-(trifluoromethyl)phenyl group, 2-[4-(trifluoromethyl)piperidin-1-yl]-5-(trifluoromethyl)phenyl group, 2-(2,2,2-trifluoroethoxy)-5-(trifluoromethyl)phenyl group, 2-(2-methoxyphenoxy)-5-(trifluoromethyl)phenyl group, 2-(4-chloro-3,5-dimethylphenoxy)-5-(trifluoromethyl)phenyl group, 2-piperidino-5-(trifluoromethyl)phenyl group, 2-(4-methylphenoxy)-5-(trifluoromethyl)phenyl group, 2-(4-chlorophenoxy)-5-(trifluoromethyl)phenyl group, 2-(4-cyanophenoxy)-5-(trifluoromethyl)phenyl group, and 2-(4-methoxyphenoxy)-5-(trifluoromethyl)phenyl group

Examples of the substituent in the definition of "a 3,5-di-substituted phenyl group" in the definition of E include similar groups to the substituent explained for the definition "which may be substituted."

Preferred examples of the "3,5-di-substituted phenyl group" in the definition of E include groups represented by the following Substituent Group δ-3e.
[Substituent Group δ-3e] 3,5-bis(trifluoromethyl)phenyl group, 3,5-dichlorophenyl group, 3,5-bis[(1,1-dimethyl)ethyl]phenyl group, 3-fluoro-5-(trifluoromethyl)phenyl group, 3-bromo-5-(trifluoromethyl)phenyl group, 3-methoxy-5-(trifluoromethyl)phenyl group, 3,5-difluorophenyl group, 3,5-dinitrophenyl group, 3,5-dimethylphenyl group, 3,5-dimethoxyphenyl group, 3,5-bis(methoxycarbonyl)phenyl group, 3-methoxycarbonyl-5-(trifluoromethyl)phenyl group, 3-carboxy-5-(trifluoromethyl)phenyl group, and 3,5-dicarboxyphenyl group

"A 3,5-di-substituted phenyl group wherein at least one of said substituents is trifluoromethyl group" is more preferred, a group selected from the following Substituent Group δ-4e is further preferred, and 3,5-bis(trifluoromethyl)phenyl group is most preferred.
[Substituent Group δ-4e] 3,5-bis(trifluoromethyl)phenyl group, 3-fluoro-5-(trifluoromethyl)phenyl group, 3-bromo-5-(trifluoromethyl)phenyl group, 3-methoxy-5-(trifluoromethyl)phenyl group, 3-methoxycarbonyl-5-(trifluoromethyl)phenyl group, and 3-carboxy-5-(trifluoromethyl)phenyl group

Examples of the substituent in the definition of "a monocyclic or a fused polycyclic heteroaryl group which may be substituted, provided that the compound wherein said heteroaryl group is ① a fused polycyclic heteroaryl group wherein the ring which binds directly to ―CONH― group is a benzene ring, ② unsubstituted thiazol-2-yl group, or ③ unsubstituted benzothiazol-2-yl group is excluded" in the aforementioned definition of E include similar groups to the substituent explained for the definition "which may be substituted." The position of substituents existing on the heteroaryl group is not particularly limited, and when two or more substituents exist, they may be the same or different.

Examples of the "monocyclic heteroaryl group" in "a monocyclic or a fused polycyclic heteroaryl group which may be substituted" in the aforementioned definition of E include similar groups to the "monocyclic heteroaryl group" in the definition of the aforementioned "heterocyclic group."

Examples of the "fused polycyclic heteroaryl group" in "a monocyclic or a fused polycyclic heteroaryl group which may be substituted" in the aforementioned definition of E include similar groups to the "fused polycyclic heteroaryl group" in the definition of the aforementioned "heterocyclic group."

As "a monocyclic or a fused polycyclic heteroaryl group which may be substituted" in the aforementioned definition of E, ① a fused polycyclic heteroaryl group wherein the ring which binds directly to ―CONH― group in the general formula (I) is a benzene ring, ② unsubstituted thiazol-2-yl group, and ③ unsubstituted benzothiazol-2-yl group are excluded.

A 5 to 10-membered monocyclic or fused polycyclic heteroaryl group is preferred as "a monocyclic or a fused polycyclic heteroaryl group" in "a monocyclic or a fused polycyclic heteroaryl group which may be substituted" in the aforementioned definition of E, and preferred examples of the group include thiazolyl group, thienyl group, pyrazolyl group, oxazolyl group, 1,3,4-thiadiazolyl group, pyridyl group, pyrimidinyl group, pyrazinyl group, and quinolyl group.

A 5-membered monocyclic heteroaryl group is more preferred as "a monocyclic or a fused polycyclic heteroaryl group" in "a monocyclic or a fused polycyclic heteroaryl group which may be substituted" in the aforementioned definition of E. Thiazolyl group, thienyl group, pyrazolyl group, oxazolyl group, and 1,3,4-thiadiazolyl group are further preferred, and thiazolyl group is most preferred.

A substituted thiazolyl group is most preferred as said "a monocyclic or a fused polycyclic heteroaryl group which may be substituted," because unsubstituted thiazol-2-yl group is excluded as "a monocyclic or a fused polycyclic heteroaryl group which may be substituted."

When "a monocyclic or a fused polycyclic heteroaryl group which may be substituted" in the aforementioned definition of E is "a substituted thiazolyl group," "a mono-substituted thiazol-2-yl group" and "a di-substituted thiazol-2-yl group" are preferred, and "a di-substituted thiazol-2-yl group" is further preferred.

When "a monocyclic or a fused polycyclic heteroaryl group which may be substituted" in the aforementioned definition of E is "a di-substituted thiazol-2-yl group," a group selected from the following Substituent Group δ-5e is further preferred, and 4-[(1,1-dimethyl)ethyl]-5-[(2,2-dimethyl)propionyl]thiazol-2-yl group is most preferred.
[Substituent Group δ-5e] 5-bromo-4-[(1,1-dimethyl)ethyl]thiazol-2-yl group, 5-bromo-4-(trifluoromethyl)thiazol-2-yl group, 5-cyano-4-[(1,1-dimethyl)ethyl]thiazol-2-yl group, 5-methylthiazol-2-yl group, 4,5-dimethylthiazol-2-yl group, 5-methyl-4-phenylthiazol-2-yl group, 5-(4-fluorophenyl)-4-methylthiazol-2-yl group, 4-methyl-5-[3-(trifluoromethyl)phenyl]thiazol-2-yl group, 4-[(1,1-dimethyl)ethyl]-5-ethylthiazol-2-yl group, 4-ethyl-5-phenylthiazol-2-yl group, 4-isopropyl-5-phenylthiazol-2-yl group, 4-butyl-5-phenylthiazol-2-yl group, 4-[(1,1-dimethyl)ethyl]-5-[(2,2-dimethyl)propionyl]thiazol-2-yl group, 4-[(1,1-dimethyl)ethyl]-5-(ethoxycarbonyl)thiazol-2-yl group, 4-[(1,1-dimethyl)ethyl]-5-piperidinothiazol-2-yl group, 4-[(1,1-dimethyl)ethyl]-5-morpholinothiazol-2-yl group, 4-[(1,1-dimethyl)ethyl]-5-(4-methylpiperazin-1-yl)thiazol-2-yl group, 4-[(1,1-dimethyl)ethyl]-5-(4-phenylpiperazin-1-yl)thiazol-2-yl group, 5-carboxymethyl-4-phenylthiazol-2-yl group, 4,5-diphenylthiazol-2-yl group, 4-benzyl-5-phenylthiazol-2-yl group, 5-phenyl-4-(trifluoromethyl)thiazol-2-yl group, 5-acetyl-4-phenylthiazol-2-yl group, 5-benzoyl-4-phenylthiazol-2-yl group, 5-ethoxycarbonyl-4-phenylthiazol-2-yl group, 5-ethoxycarbonyl-4-(pentafluorophenyl)thiazol-2-yl group, 5-methylcarbamoyl-4-phenylthiazol-2-yl group, 5-ethylcarbamoyl-4-phenylthiazol-2-yl group, 5-isopropylcarbamoyl-4-phenylthiazol-2-yl group, 5-(2-phenylethyl)carbamoyl-4-phenylthiazol-2-yl group, 5-ethoxycarbonyl-4-(trifluoromethyl)thiazol-2-yl group, 5-carboxy-4-[(1,1-dimethyl)ethyl]thiazol-2-yl group, 5-(ethoxycarbonyl)methyl-4-phenylthiazol-2-yl group, 5-carboxy-4-phenylthiazol-2-yl group, and 5-propylcarbamoyl-4-phenylthiazol-2-yl group.

When "a monocyclic or a fused polycyclic heteroaryl group which may be substituted" in the aforementioned definition of E is "a mono-substituted thiazol-2-yl group," preferred examples of the group include groups represented by the following Substituent Group δ-6e.
[Substituent Group δ-6e] 4-[(1,1-dimethyl)ethyl]thiazol-2-yl group, 4-phenylthiazol-2-yl group, 4-[3,5-bis(trifluoromethyl)phenyl]thiazol-2-yl group, 4-(2,4-dichlorophenyl)thiazol-2-yl group, 4-(3,4-dichlorophenyl)thiazol-2-yl group, 4-[4-(trifluoromethyl)phenyl]thiazol-2-yl group, 4-(2,5-difluorophenyl)thiazol-2-yl group, 4-(4-methoxyphenyl)thiazol-2-yl group, 4-[3-(trifluoromethyl)phenyl]thiazol-2-yl group, and 4-(pentafluorophenyl)thiazol-2-yl group

Compounds other than "substituted benzoic acid derivatives represented by the following general formula (X-1)" are preferred as the compound represented by the general formula (I). 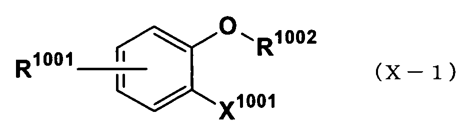 wherein R¹⁰⁰¹ represents the following general formula (X-2): 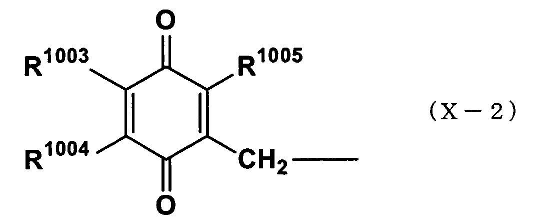 or the following general formula (X-3): 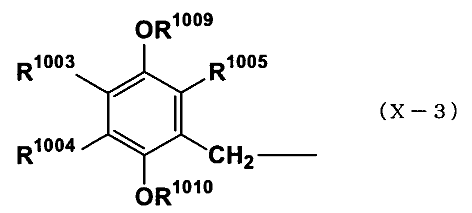 wherein each of R¹⁰⁰³, R¹⁰⁰⁴ and R¹⁰⁰⁵ independently represents hydrogen atom, an alkyl group having from 1 to 6 carbons or an alkoxy group having from 1 to 6 carbons, each of R¹⁰⁰⁹ and R¹⁰¹⁰ independently represents hydrogen atom, an alkyl group having from 1 to 6 carbons, or an acyl group having from 2 to 11 carbons;
R¹⁰⁰² represents hydrogen atom, a lower alkyl group having from 1 to 6 carbons, which may be substituted, an aryl group having from 6 to 12 carbons, which may be substituted, a heteroaryl group having from 4 to 11 carbons, which may be substituted, an aralkyl group having from 7 to 14 carbons, which may be substituted, a heteroarylalkyl group having from 5 to 13 carbons, which may be substituted, or an acyl group having from 2 to 11 carbons;
X¹⁰⁰¹ represents carboxy group which may be esterified or amidated.

The compounds represented by the aforementioned general formula (I) may form salts. Examples of pharmacologically acceptable salts include, when acidic groups exist, metal salts such as lithium salt, sodium salt, potassium salt, magnesium salt, calcium salts, or ammonium salts such as ammonium salt, methylammonium salt, dimethylammonium salt, trimethylammonium salt, dicyclohexylammonium salt, and when basic groups exist, mineral acid salts such as hydrochloride, oxalate, hydrosulfate, nitrate, phosphate, or organic acid salts such as methane sulfonate, benzene sulfonate, para-toluene sulfonate, acetate, propionate, tartrate, fumarate, maleate, malate, oxalate, succinate, citrate, benzoate, mandelate, cinnamate, lactate. Salts may sometimes be formed with amino acids such as glycine. As active ingredients of the medicament of the present invention, pharmacologically acceptable salts may also be suitably used.

The compounds or salts thereof represented by the aforementioned general formula (I) may exist as hydrates or solvates. As active ingredients of the medicament of the present invention, any of the aforementioned substances may be used. Furthermore, the compounds represented by the aforementioned general formula (I) may sometimes have one or more asymmetric carbons, and may exist as steric isomers such as optically active substance and diastereomer. As active ingredients of the medicament of the present invention, pure forms of stereoisomers, arbitrary mixture of enantiomers or diastereomers, and racemates may be used.

Furthermore, when the compounds represented by the general formula (I) has, for example, 2-hydroxypyridine form, the compounds may exist as 2-pyridone form which is a tautomer. As active ingredients of the medicament of the present invention, pure forms of tautomers or a mixture thereof may be used. When the compounds represented by the general formula (I) have olefinic double bonds, the configuration may be in either E or Z, and as active ingredients of the medicament of the present invention, geometrical isomer in either of the configurations or a mixture thereof may be used.

Examples of the compounds included in the general formula (I) as active ingredients of the medicaments of the present invention are shown below. However, the active ingredients of the medicaments of the present invention are not limited to the compound set out below.

The abbreviations used in the following tables have the following meanings.

Me: methyl group, Et: ethyl group.

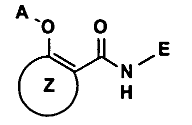

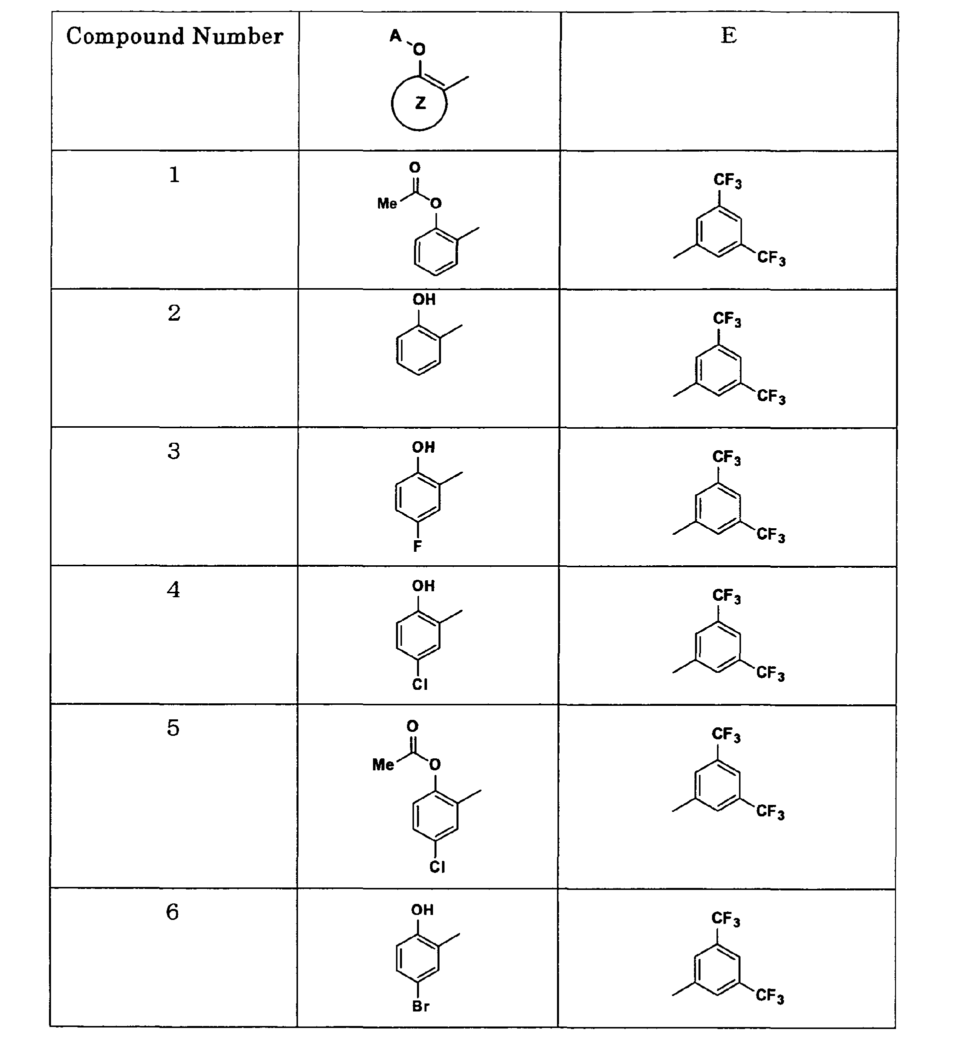

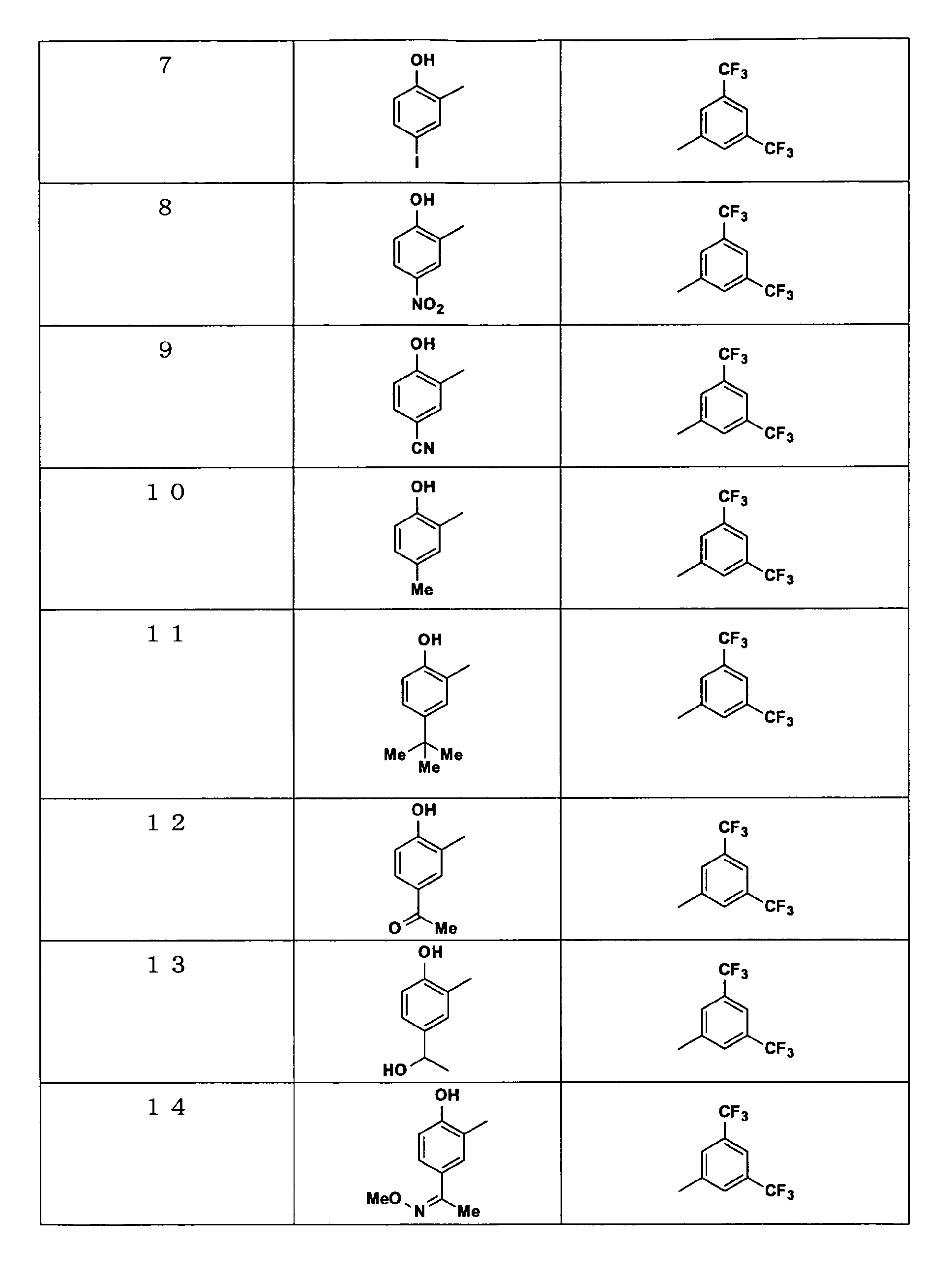

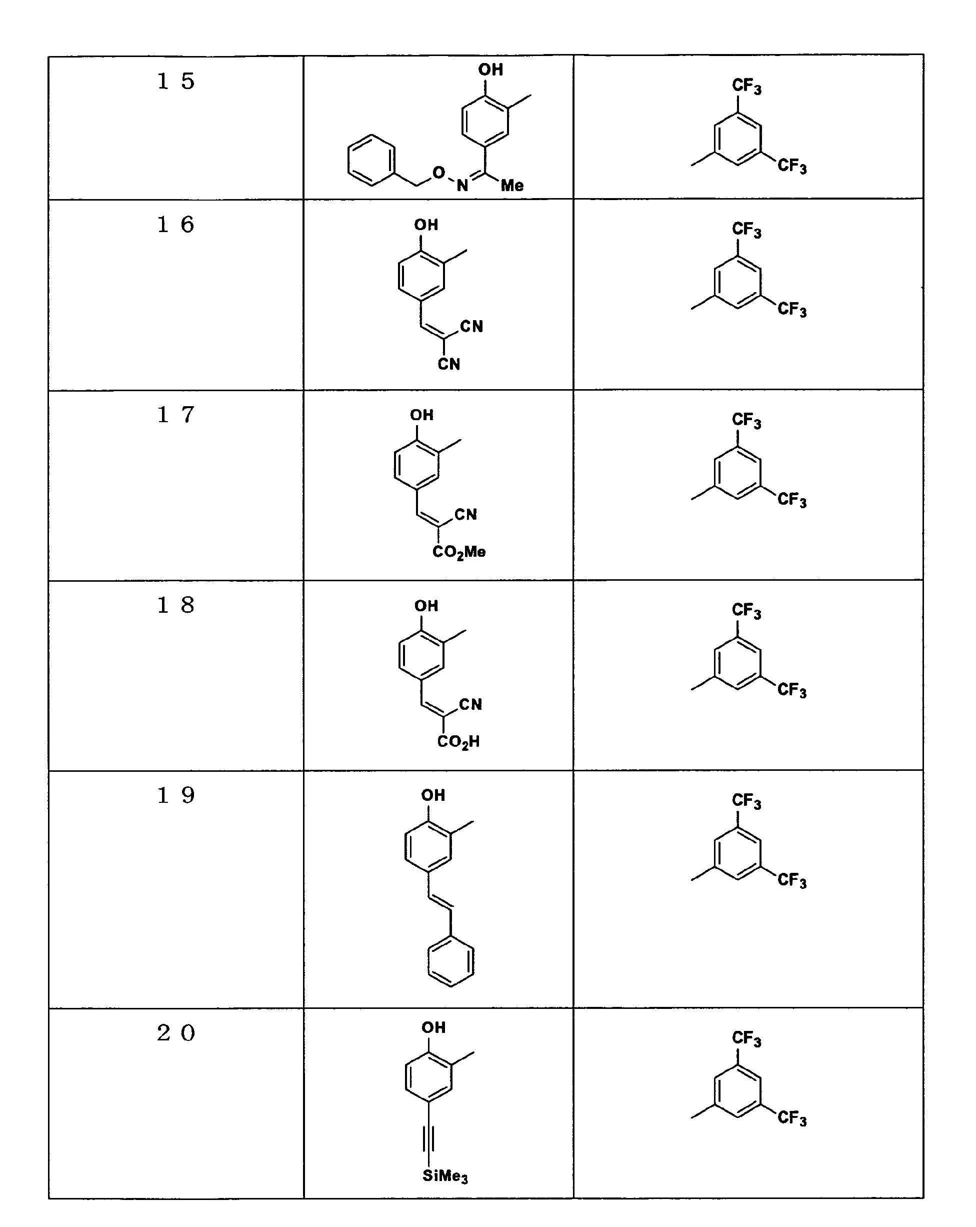

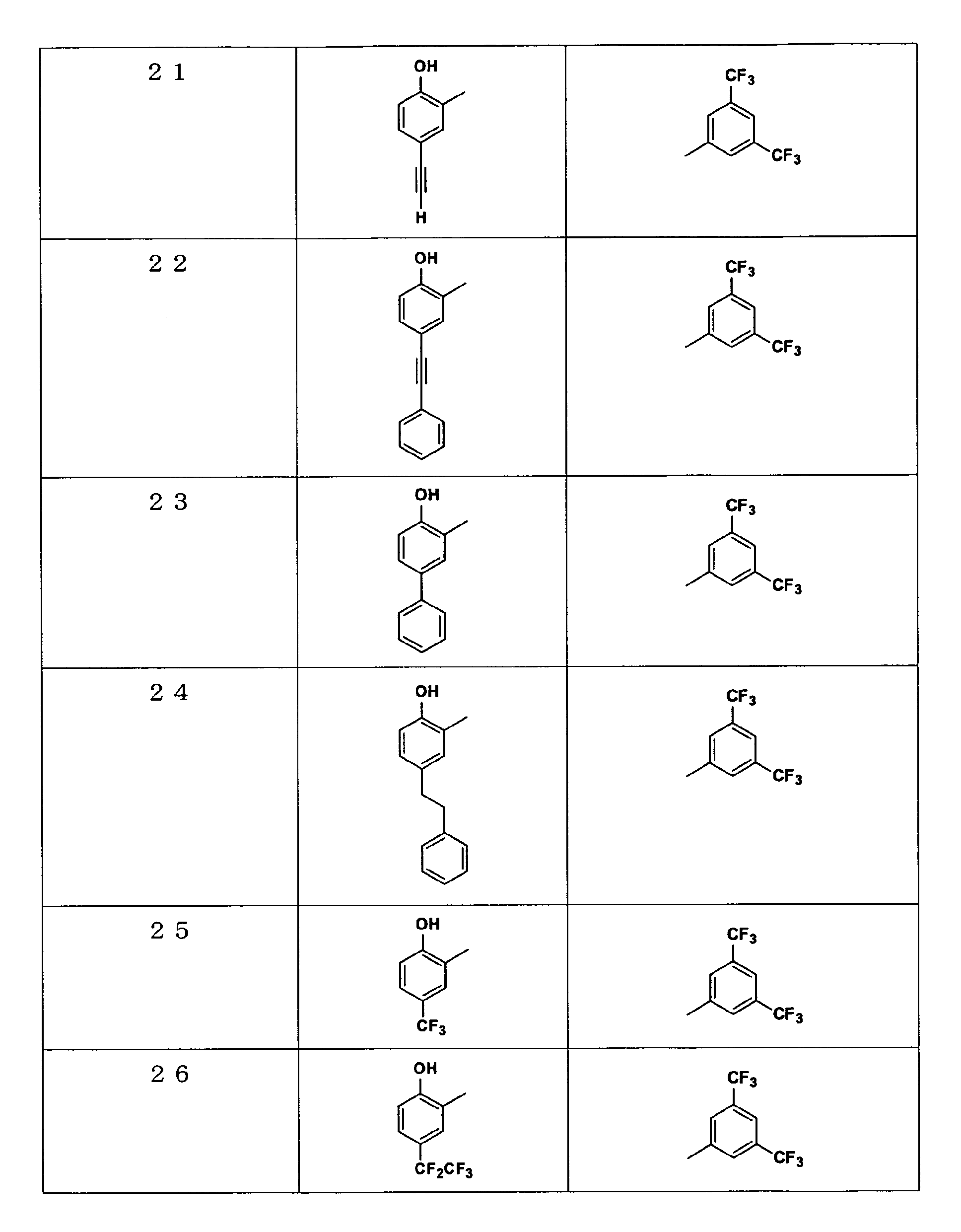

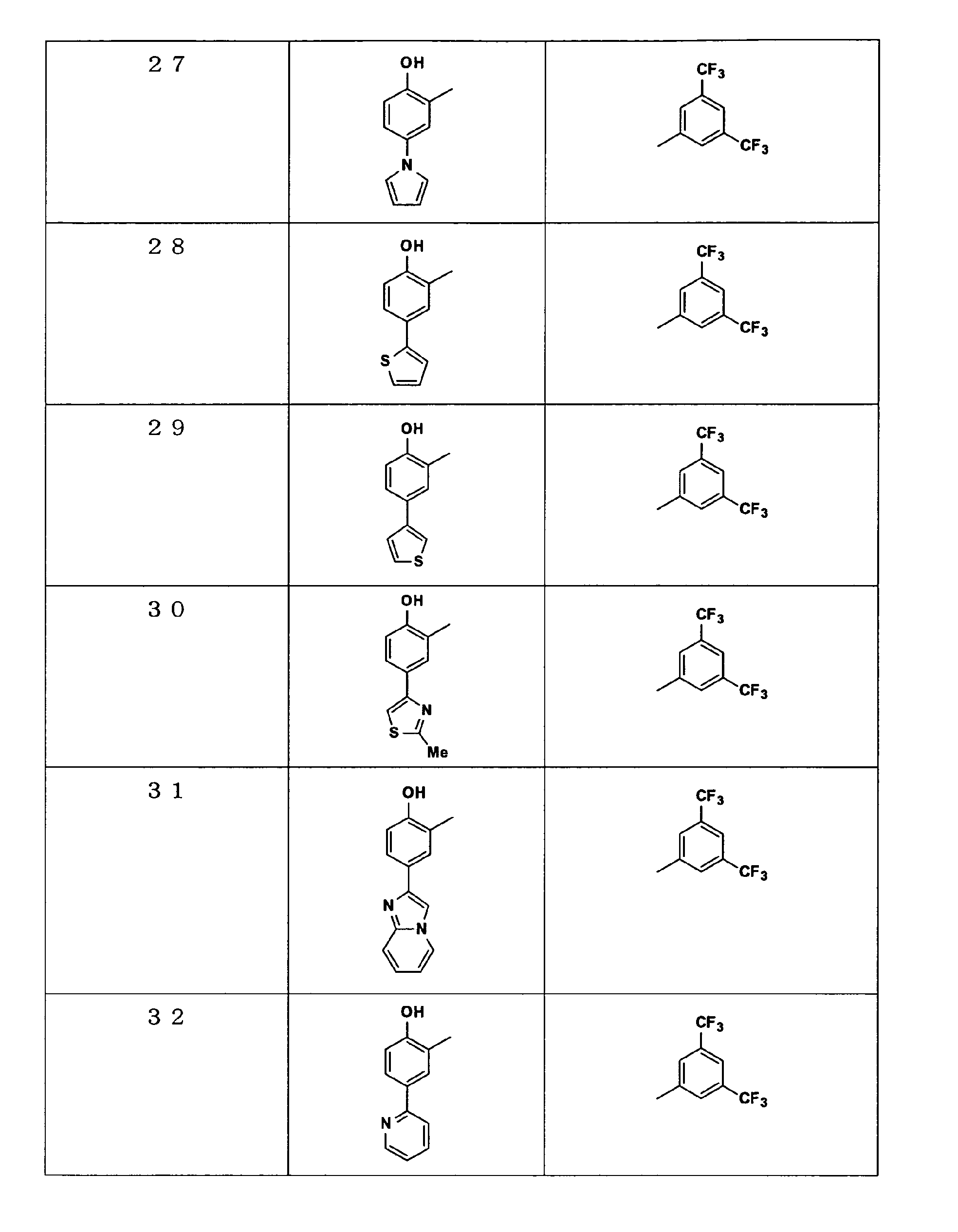

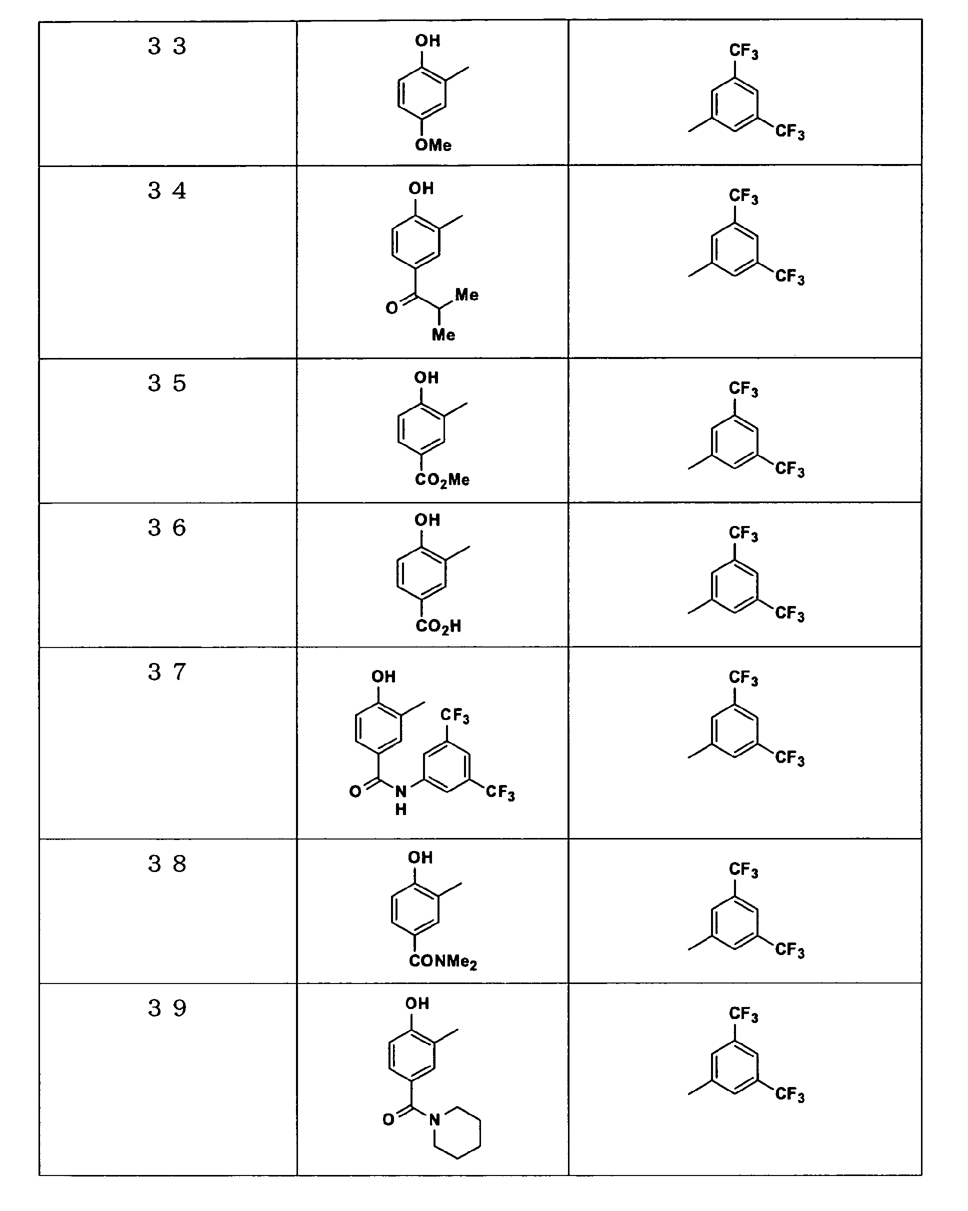

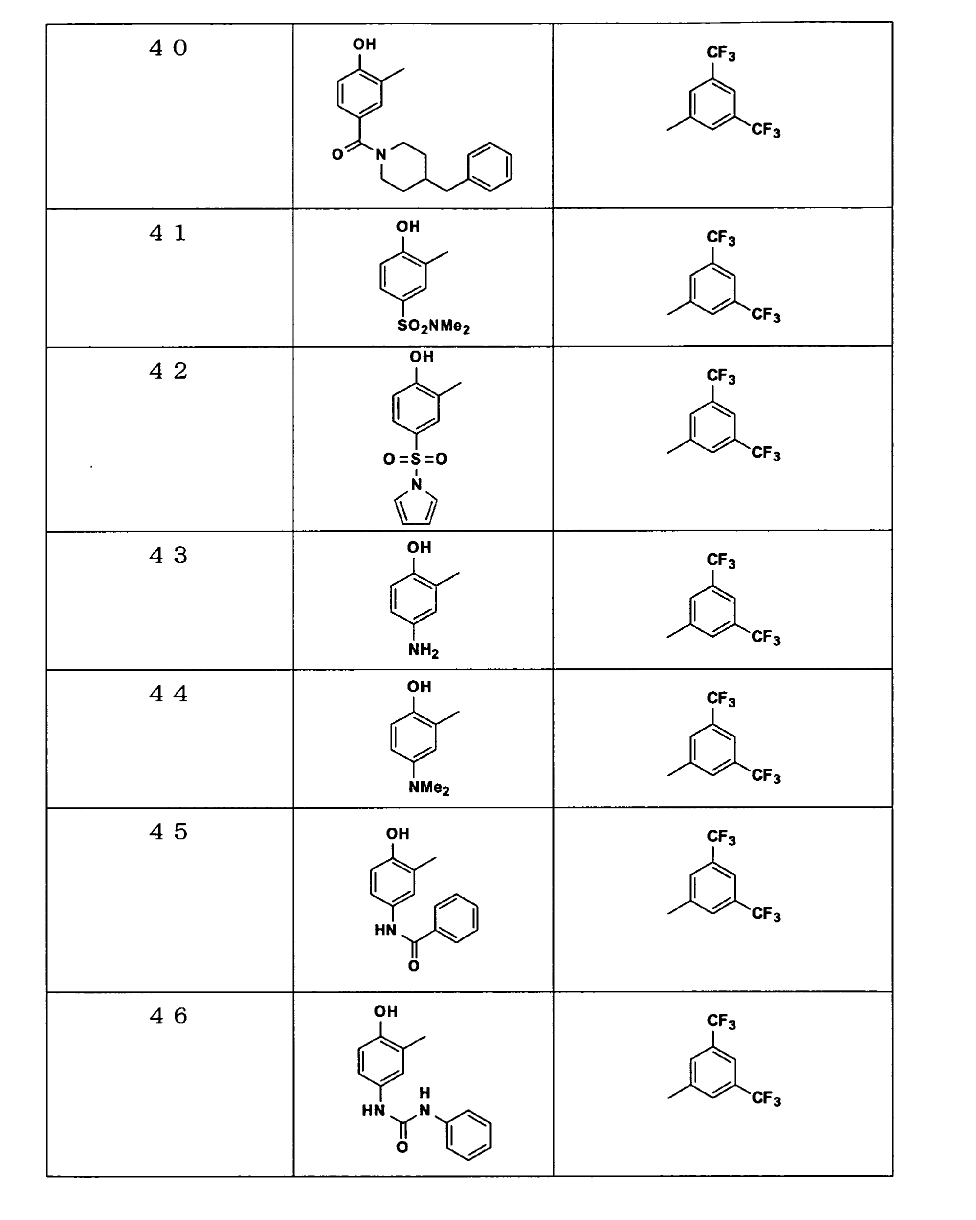

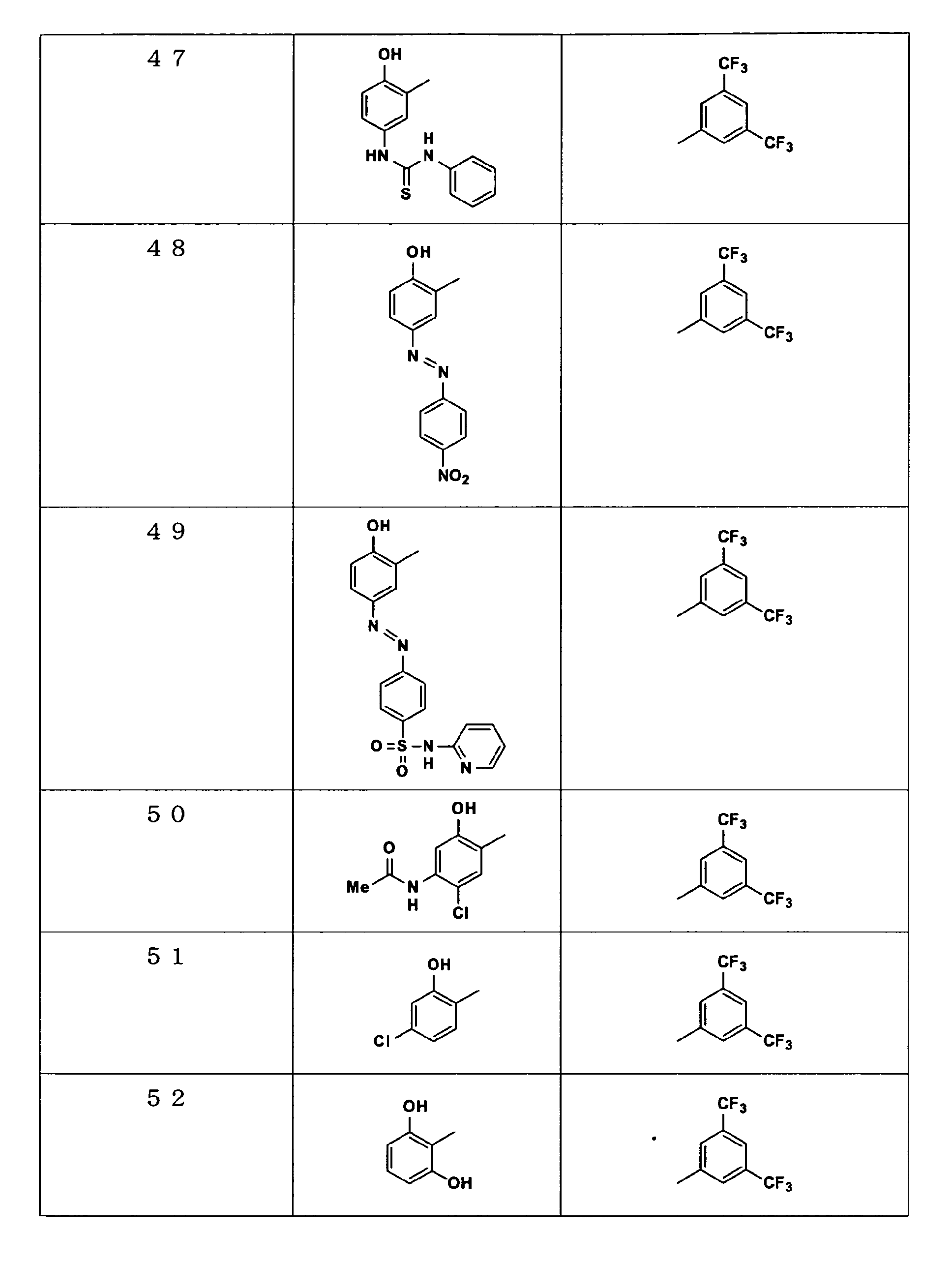

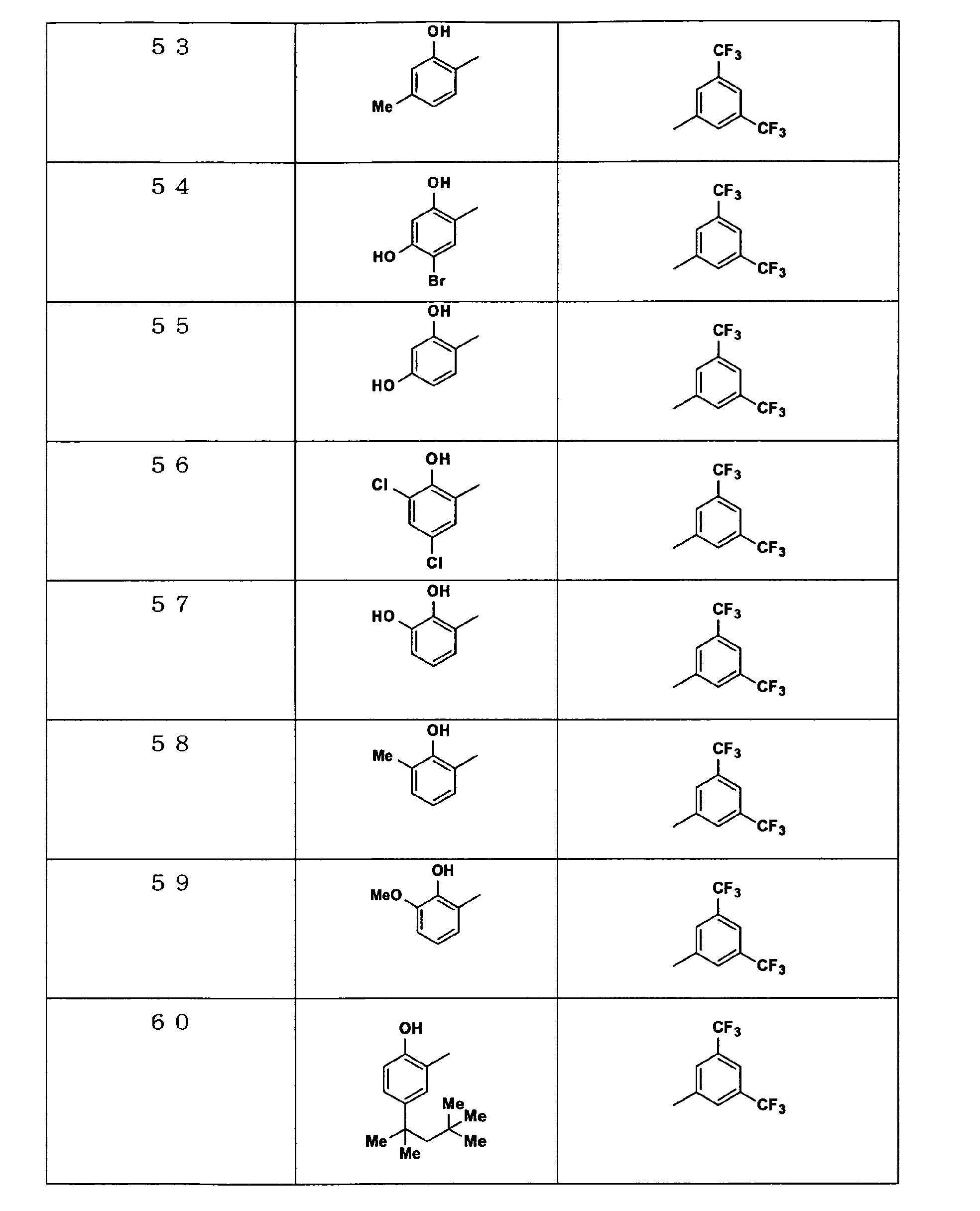

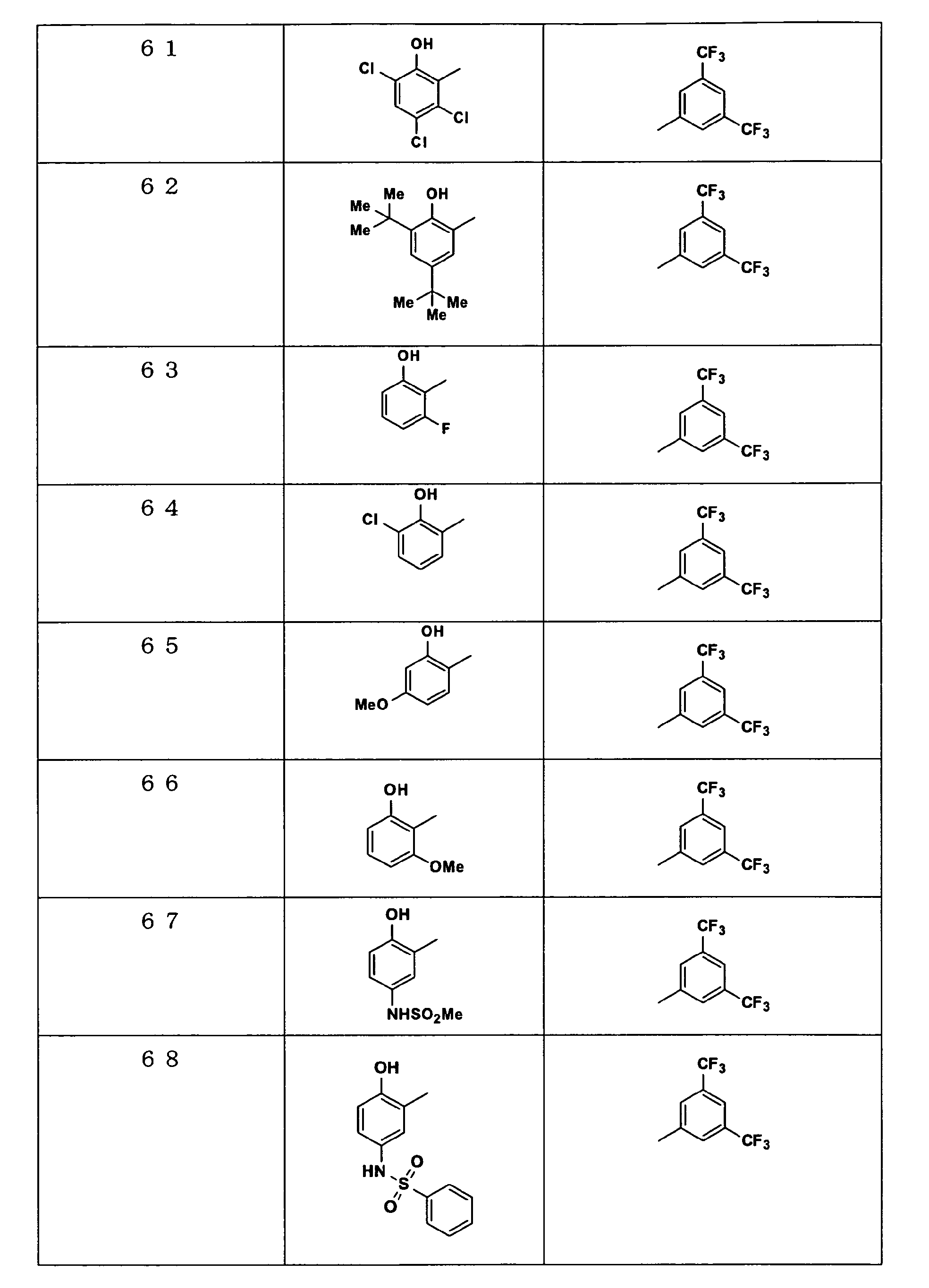

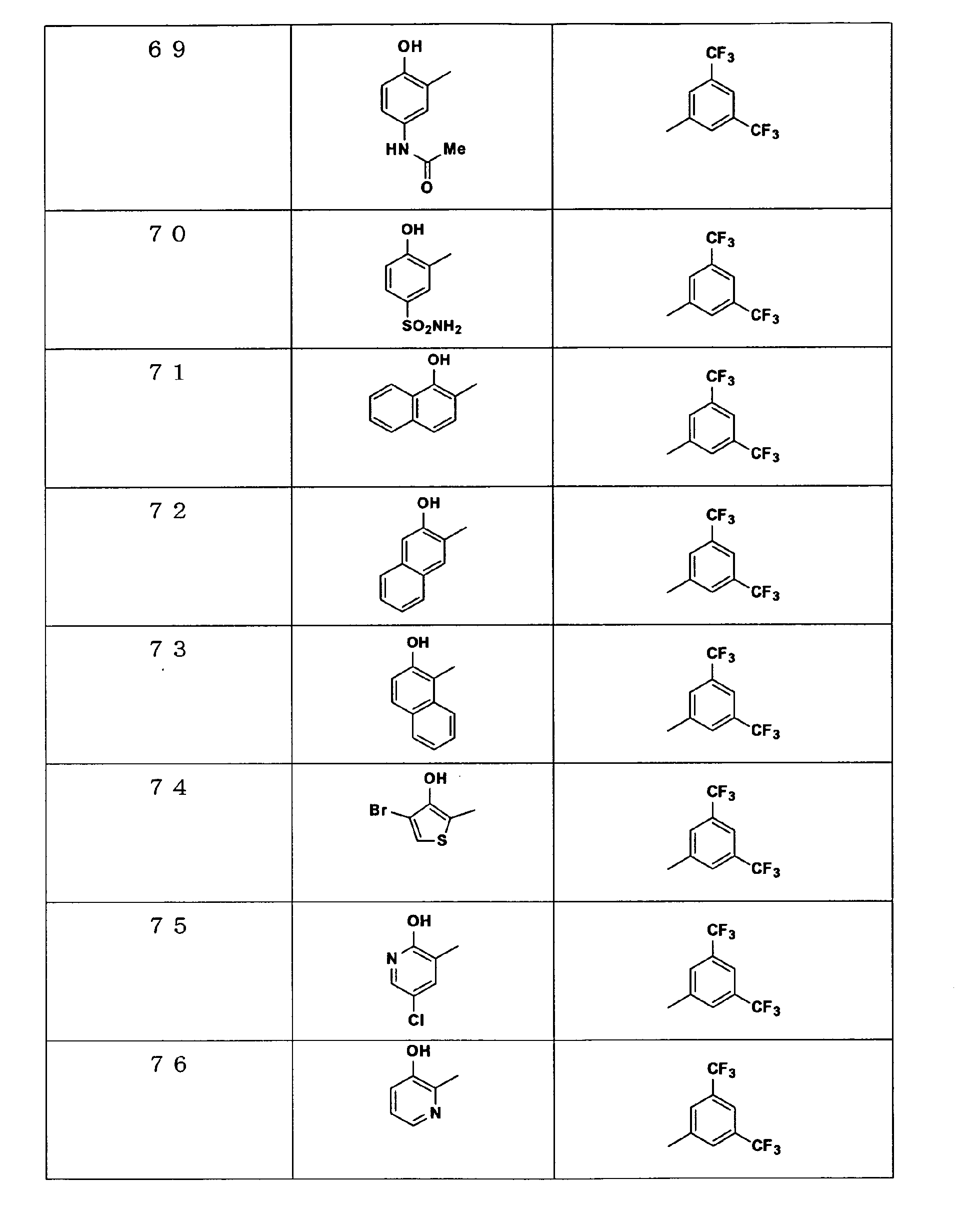

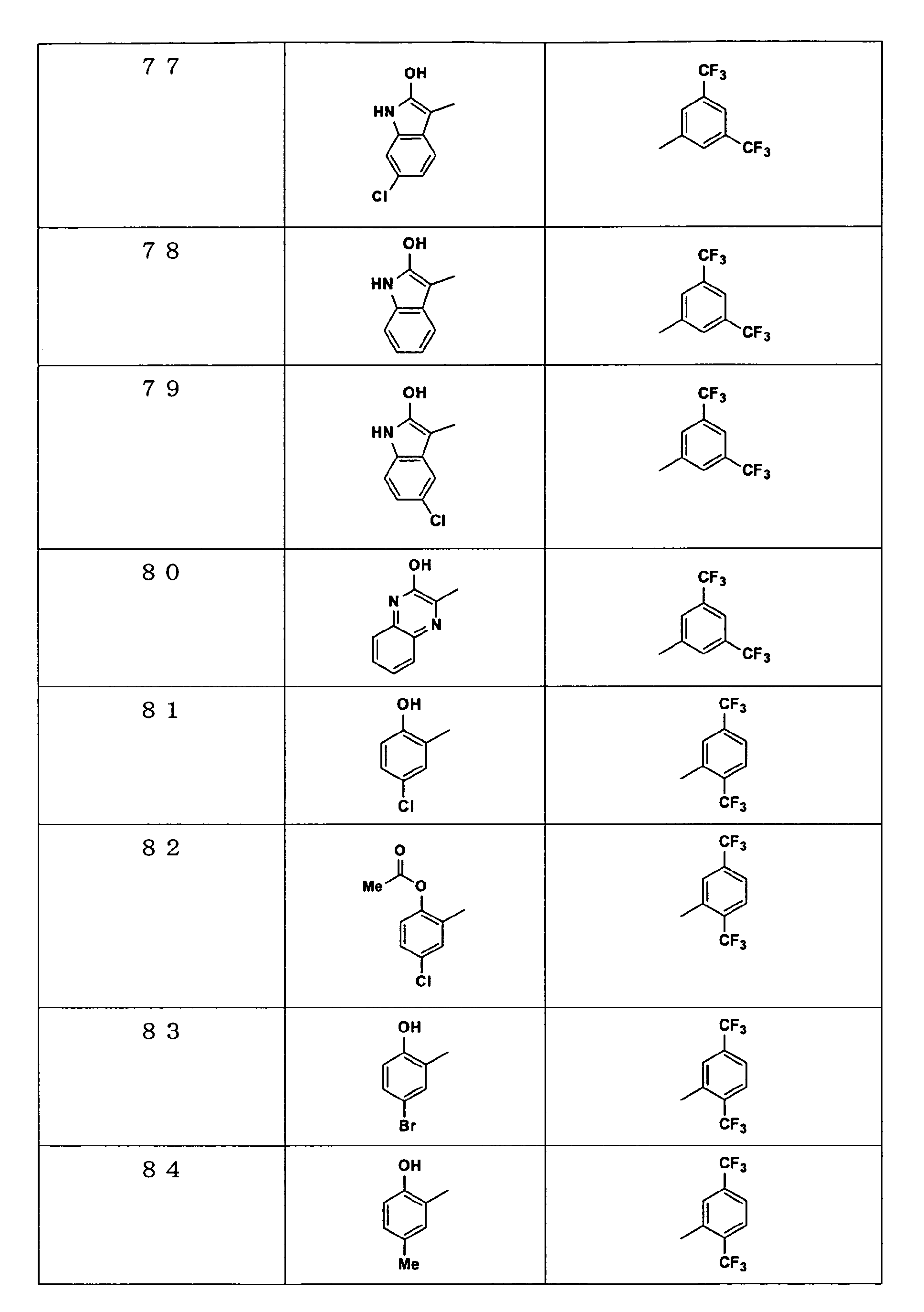

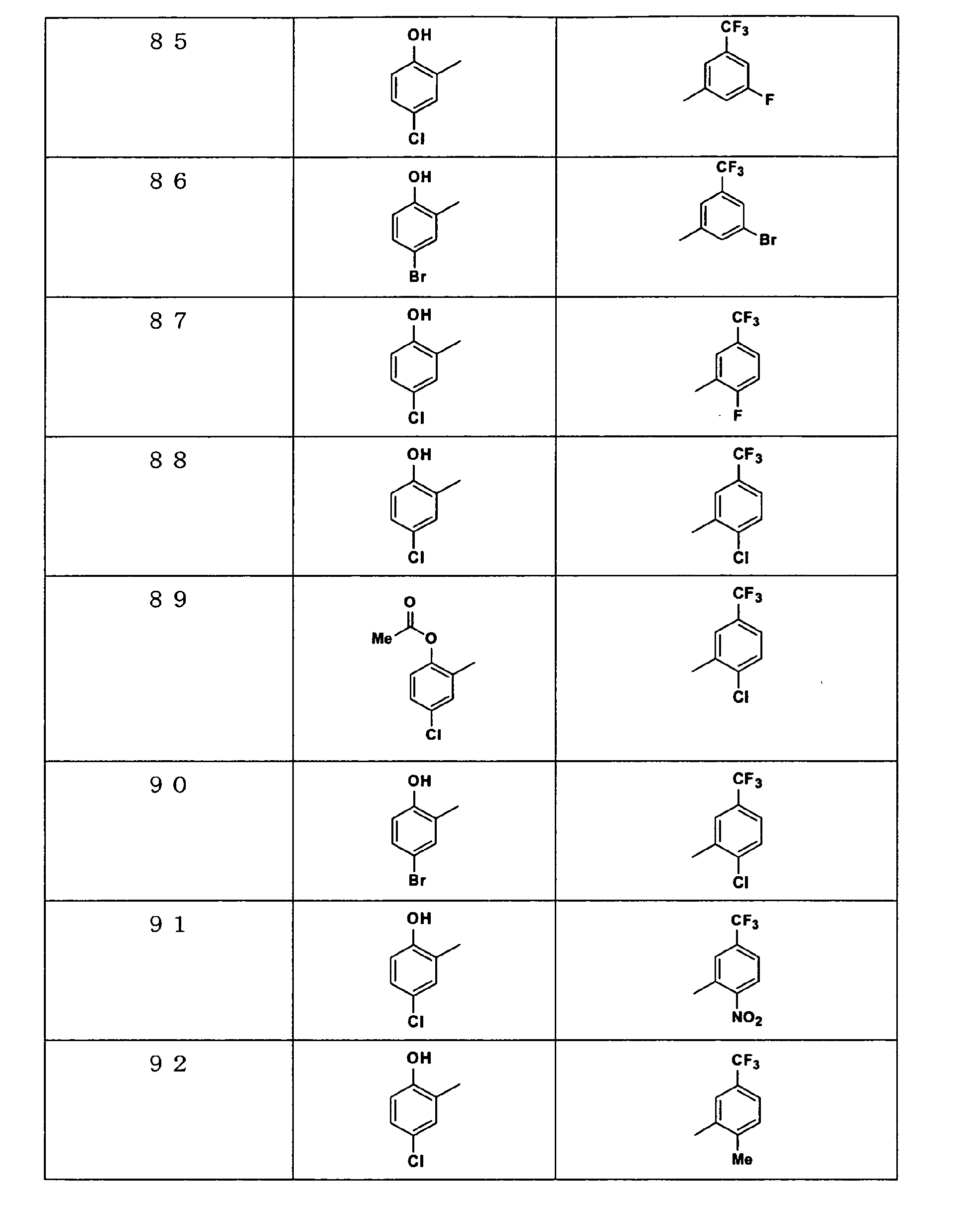

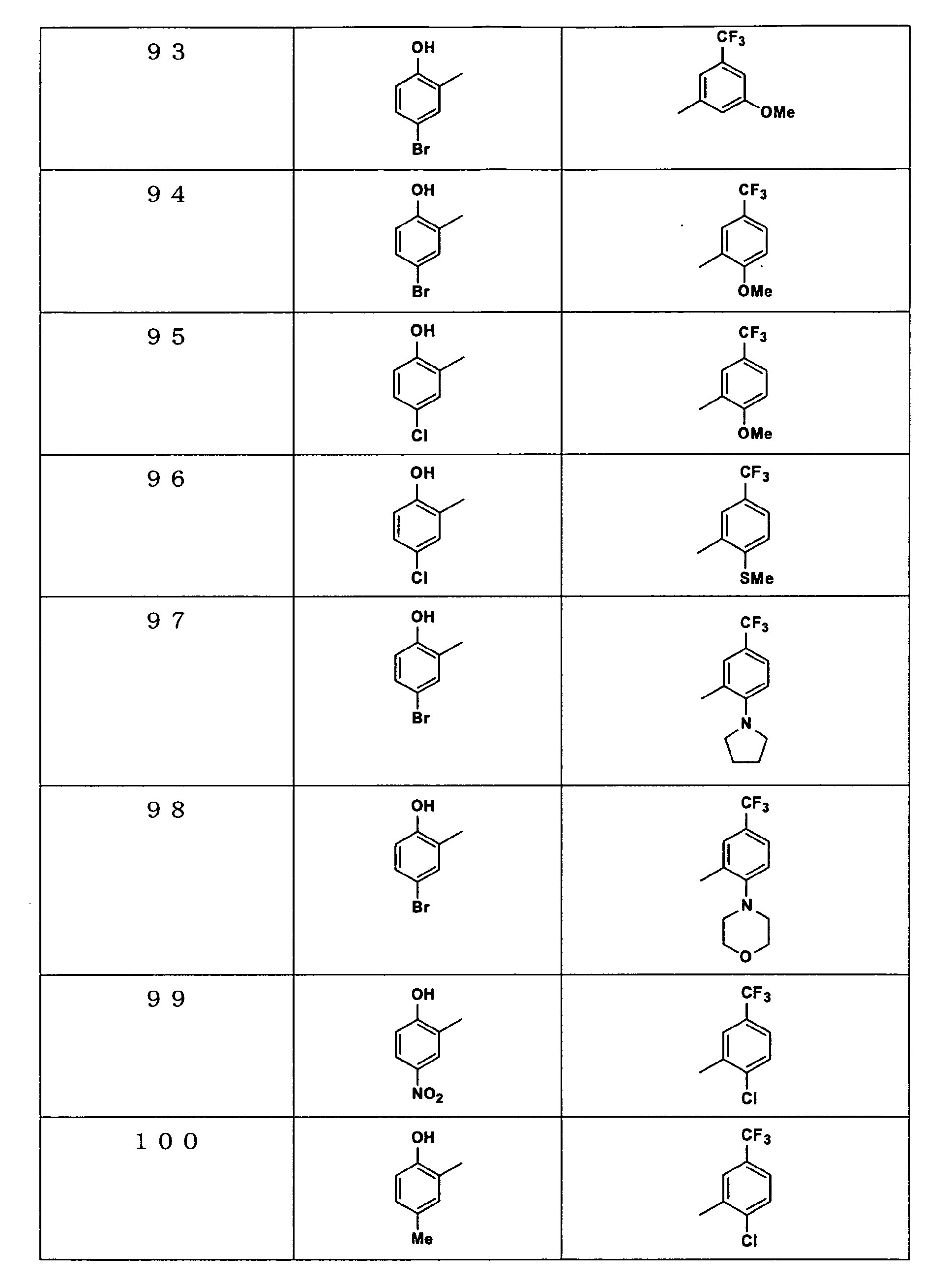

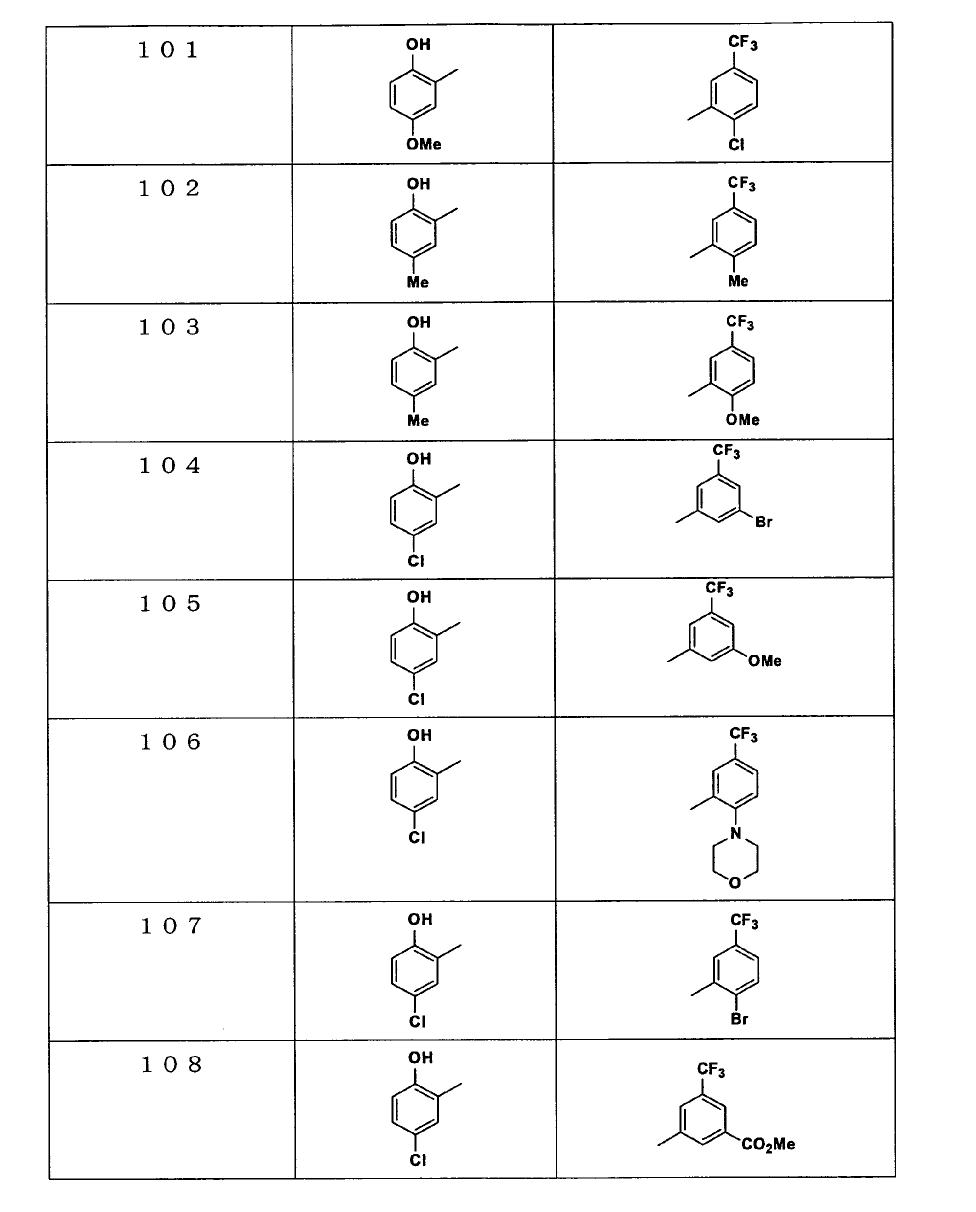

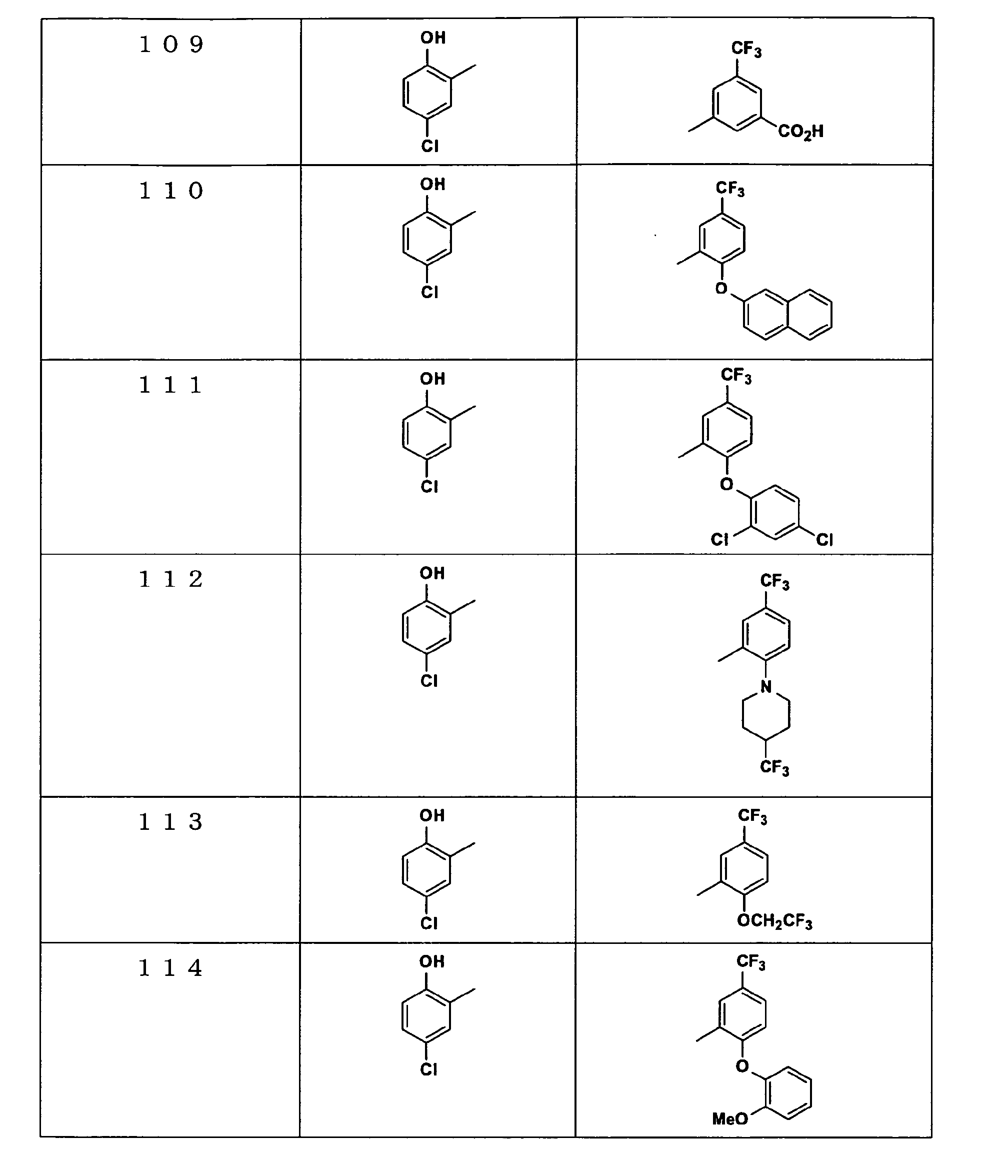

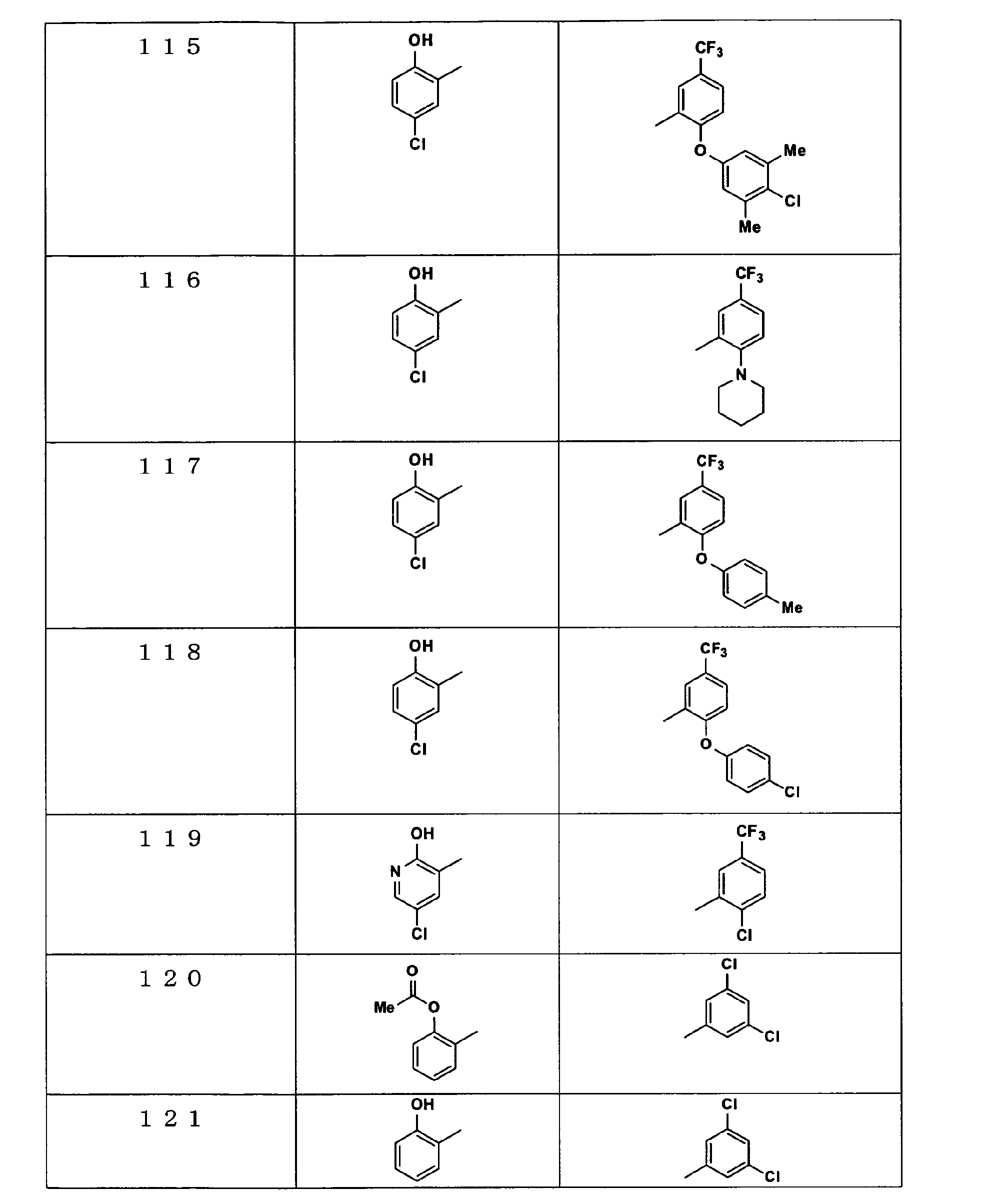

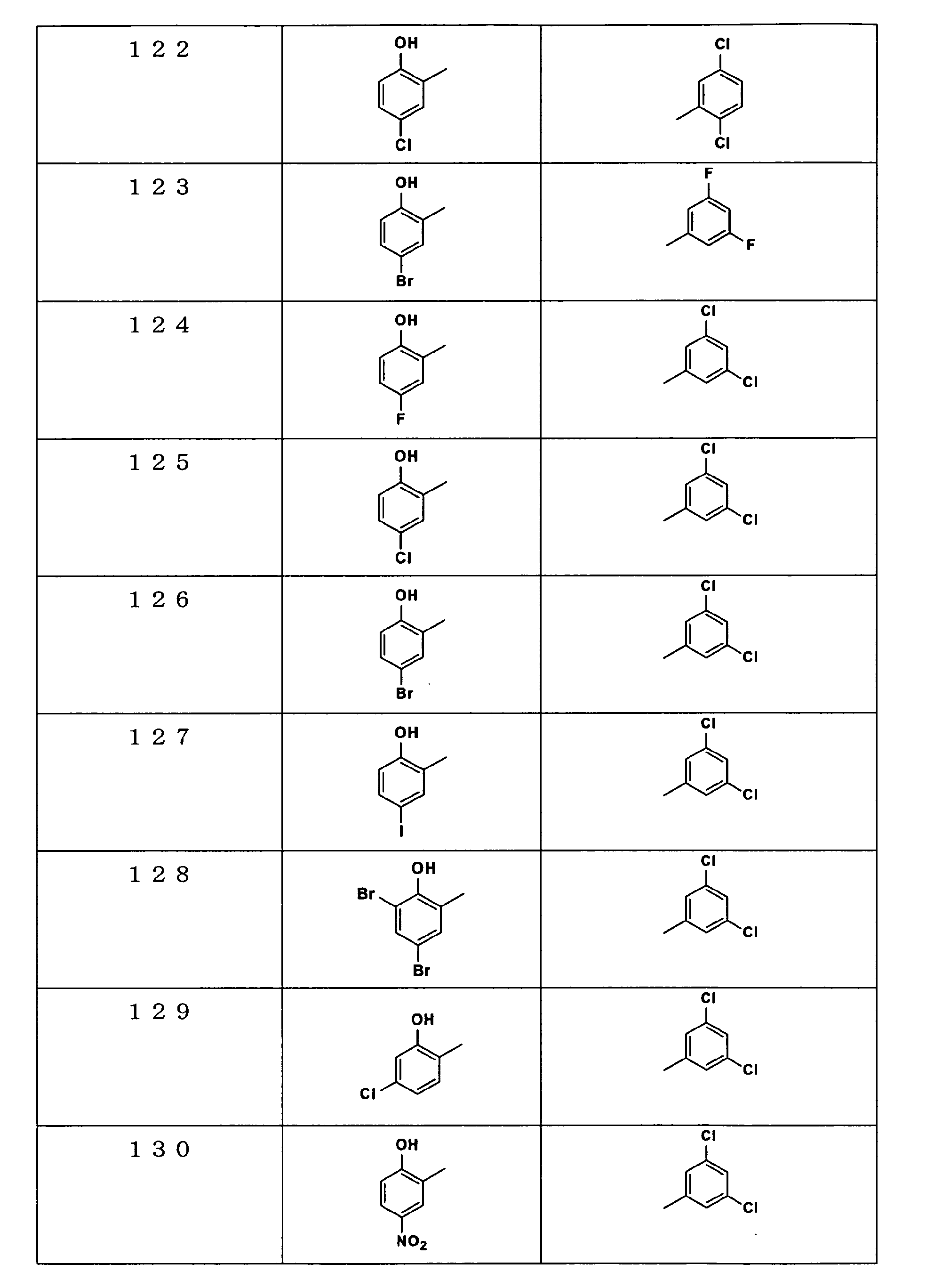

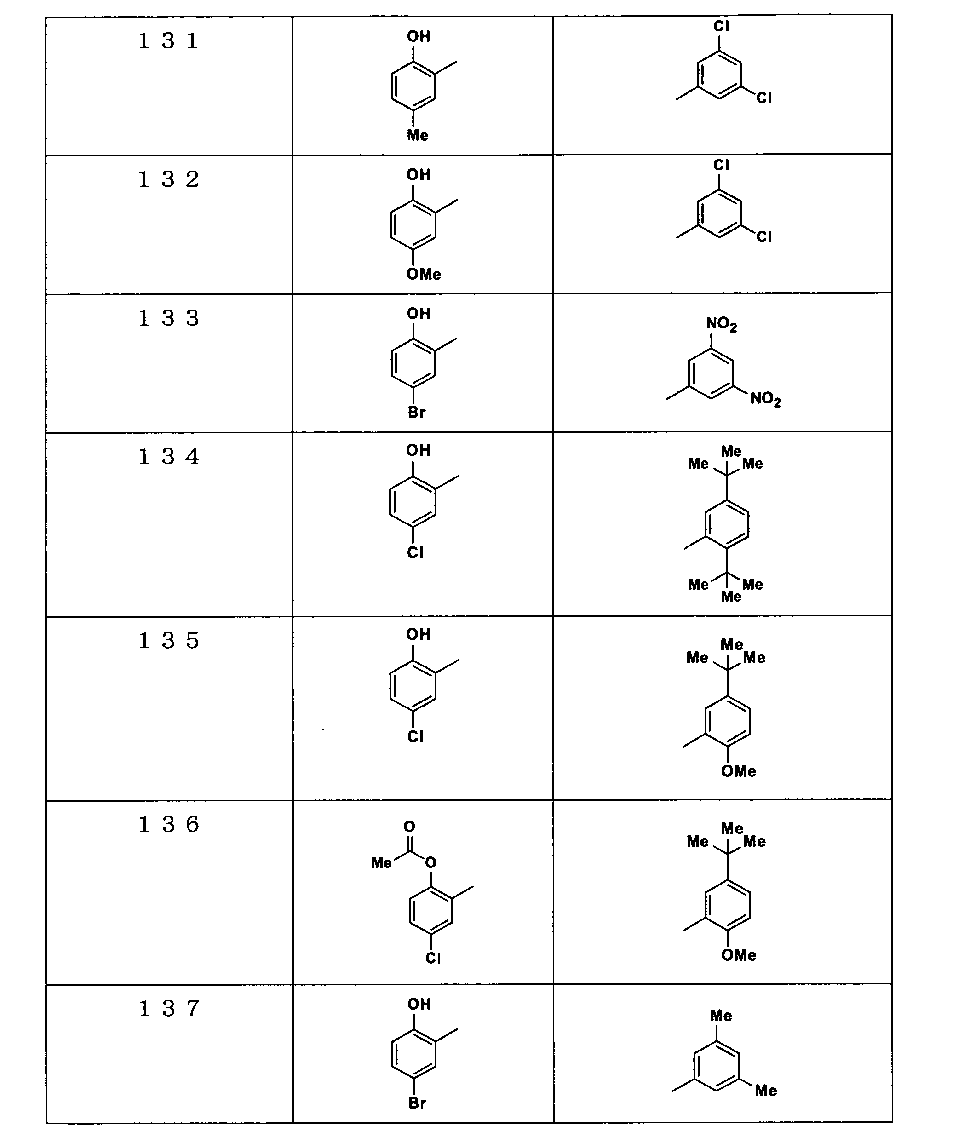

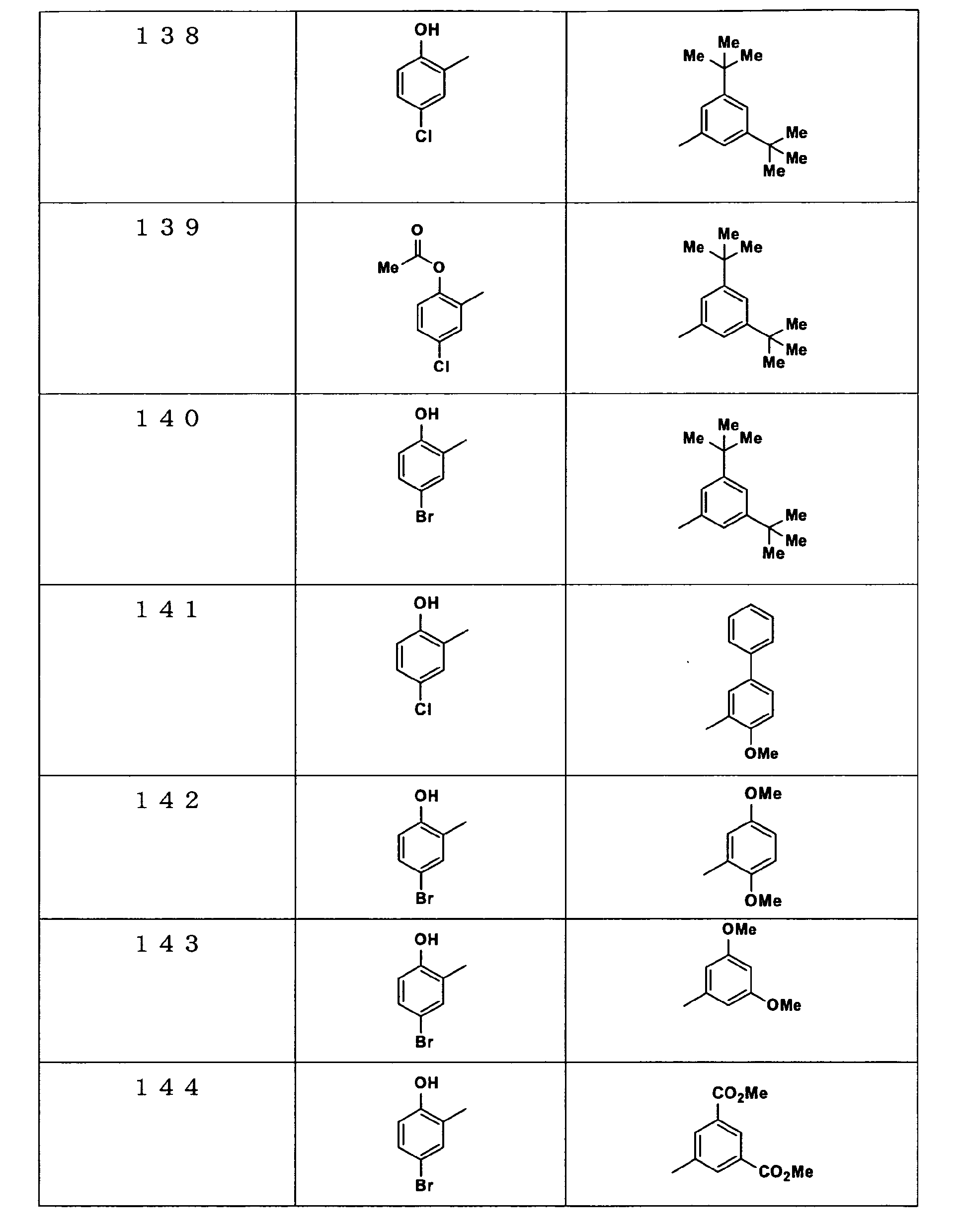

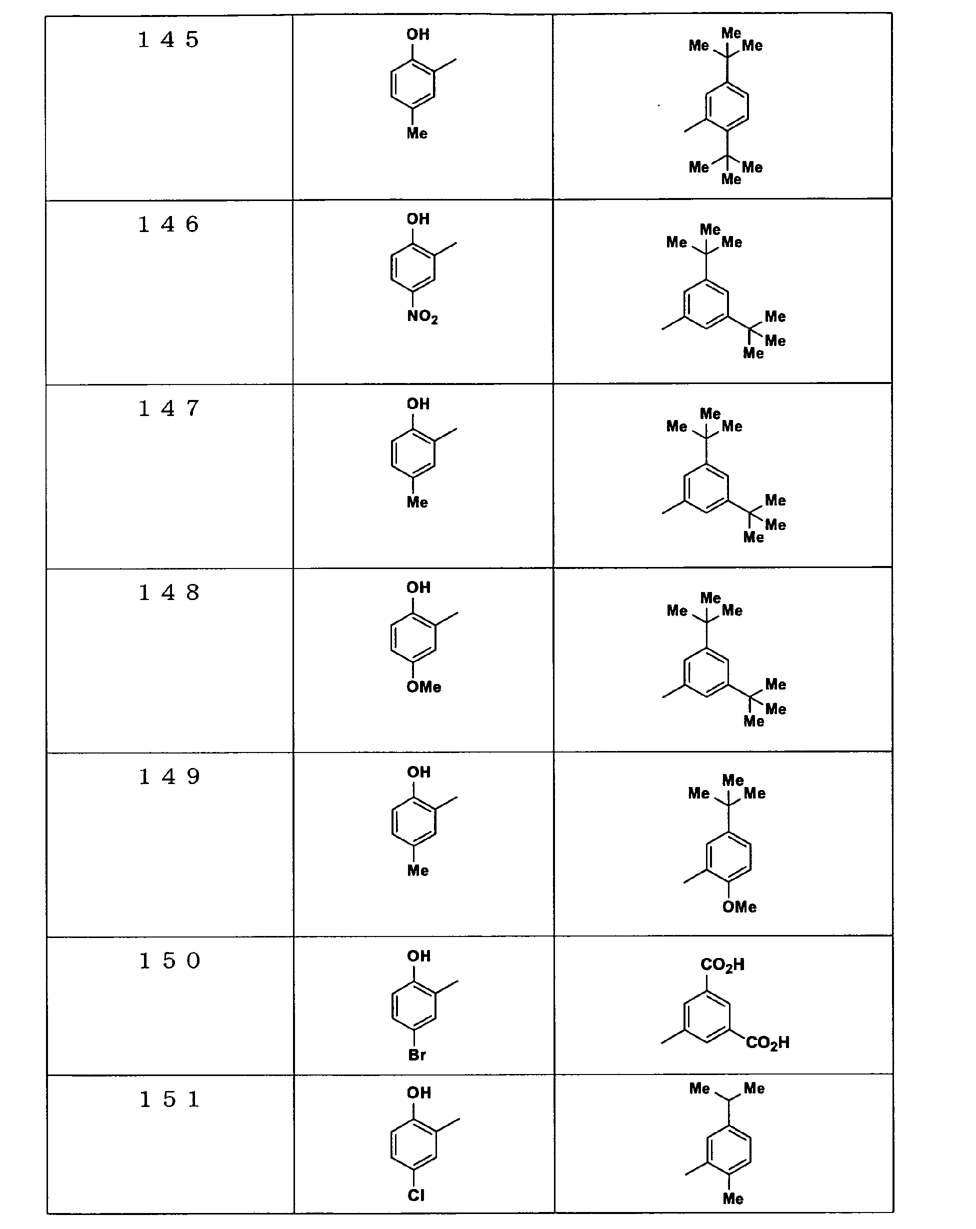

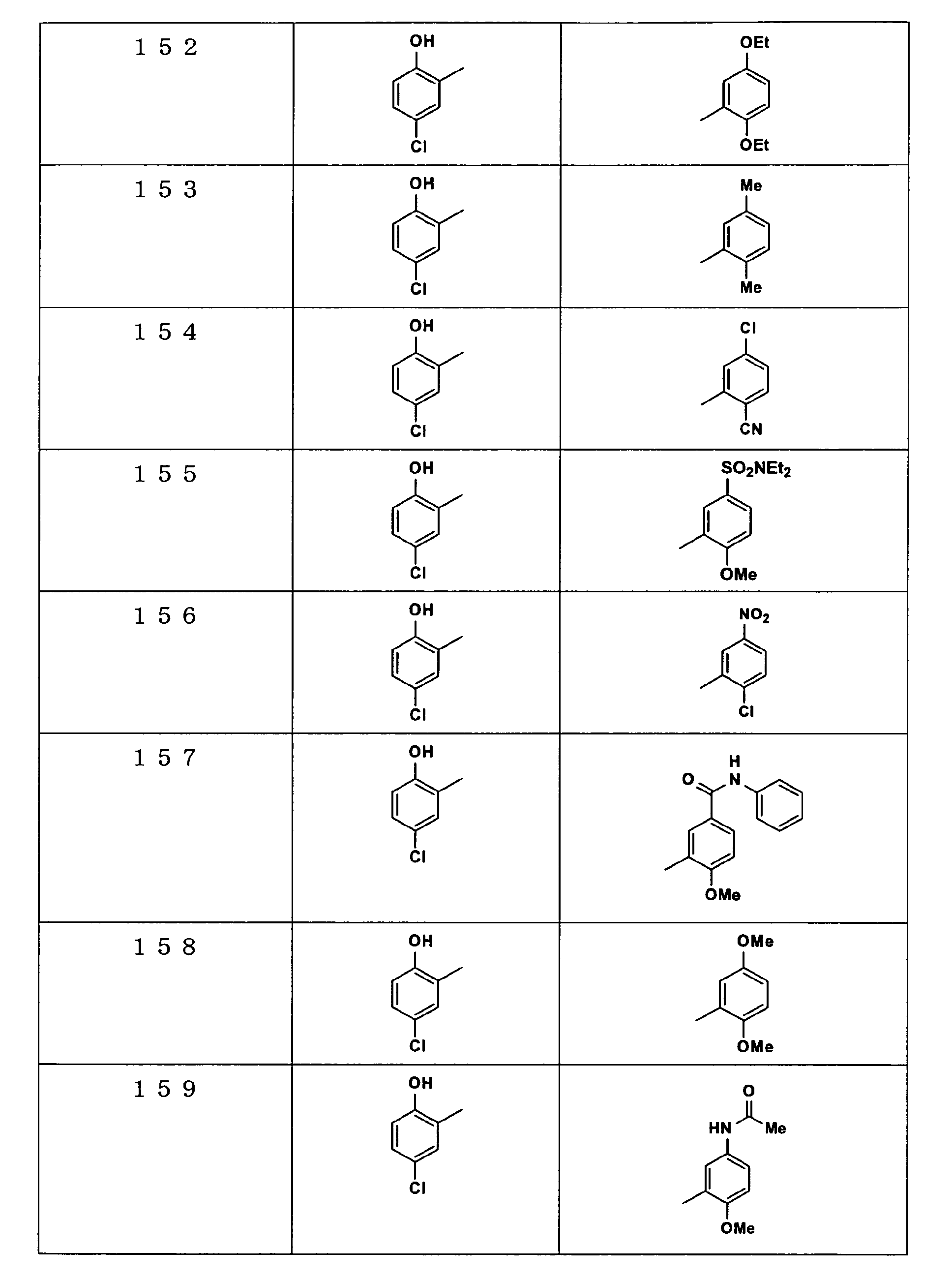

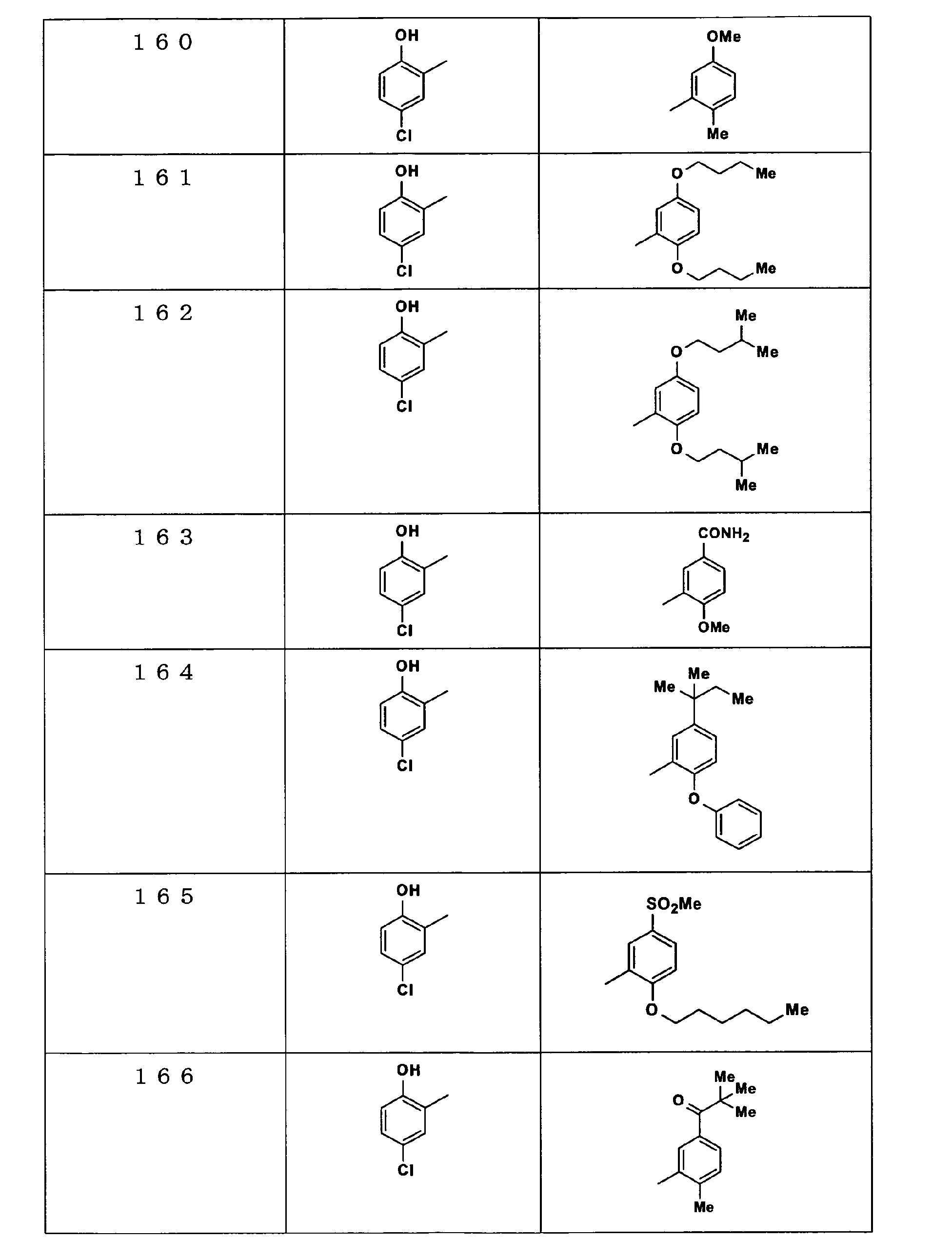

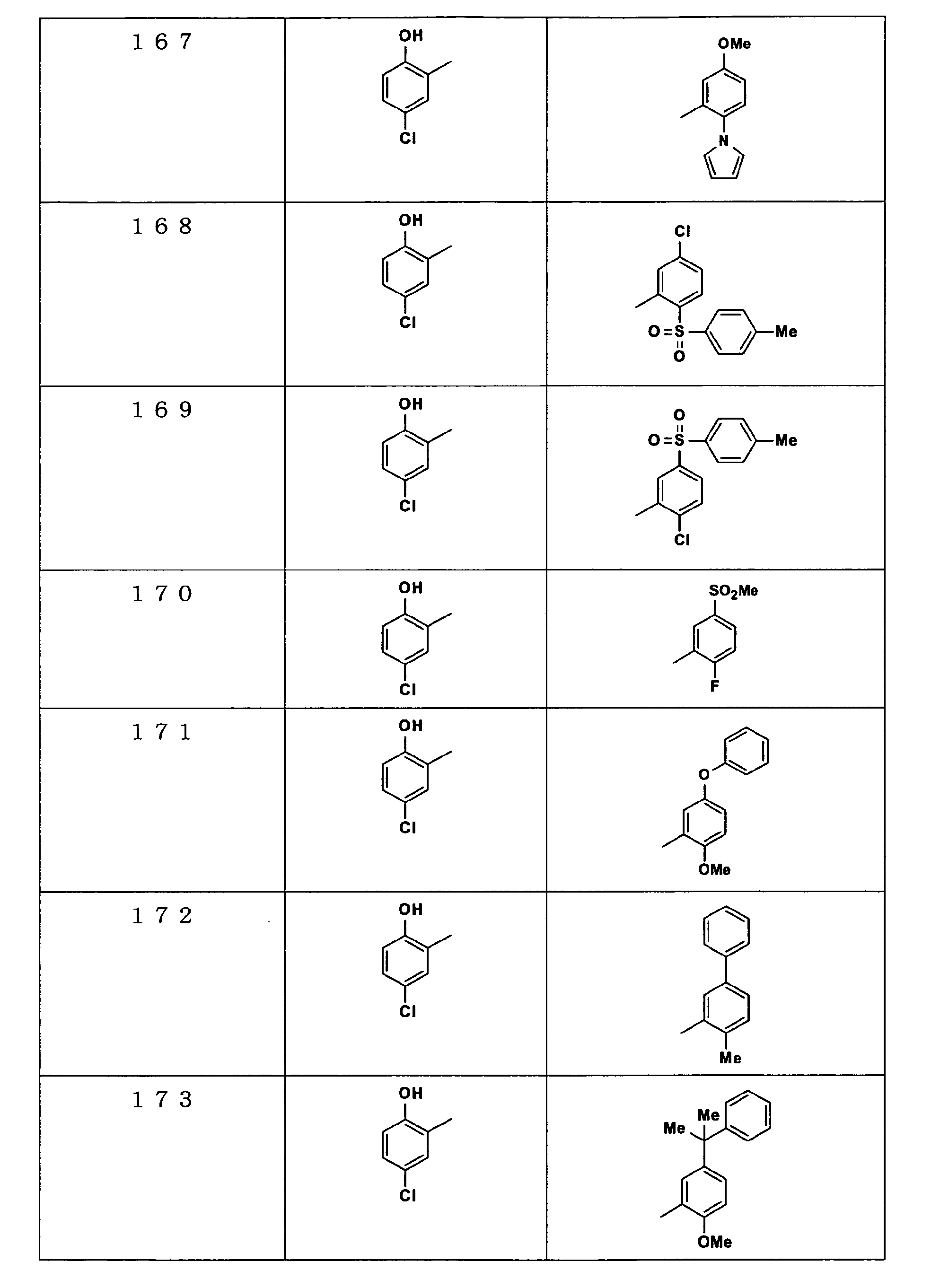

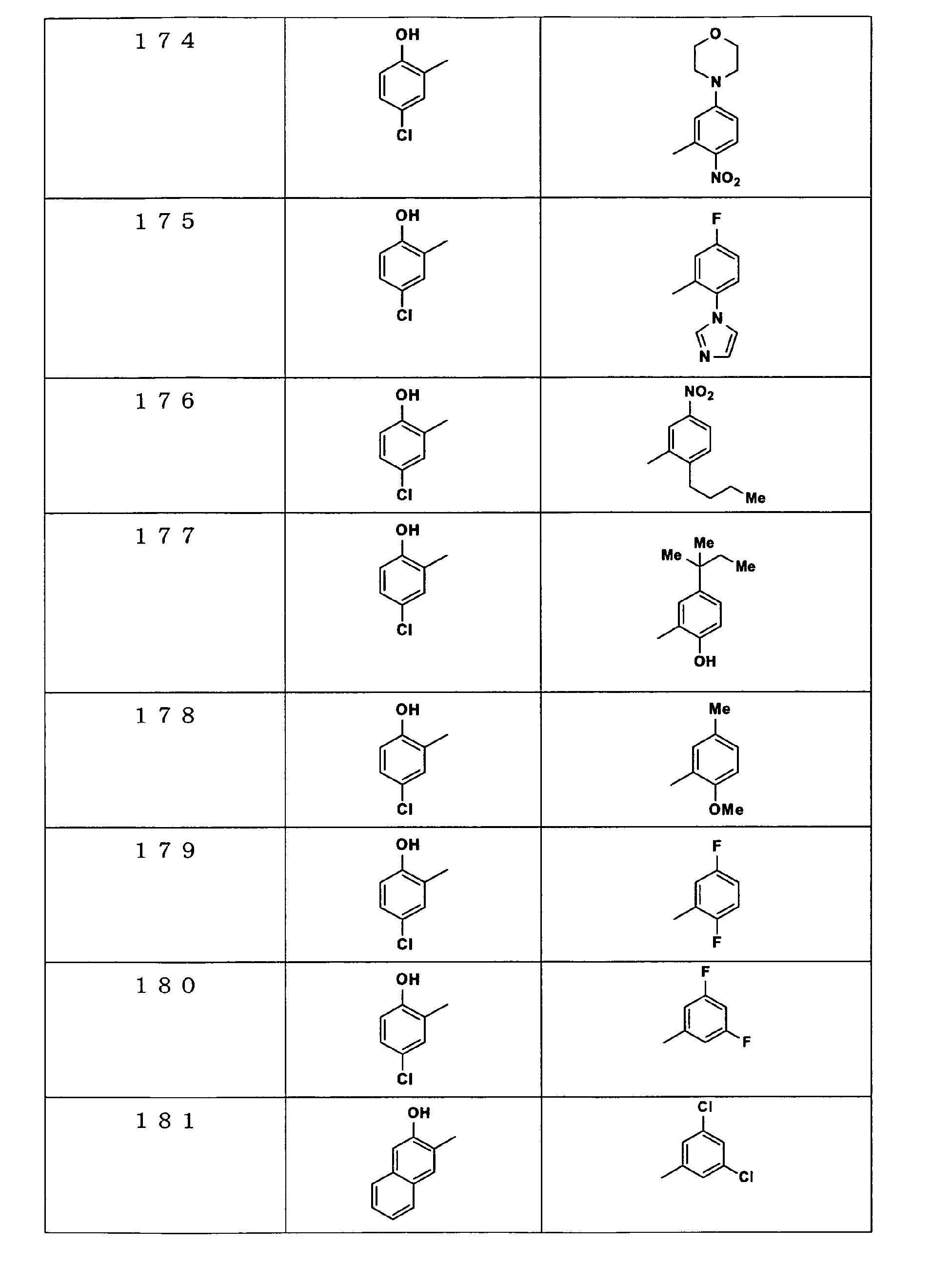

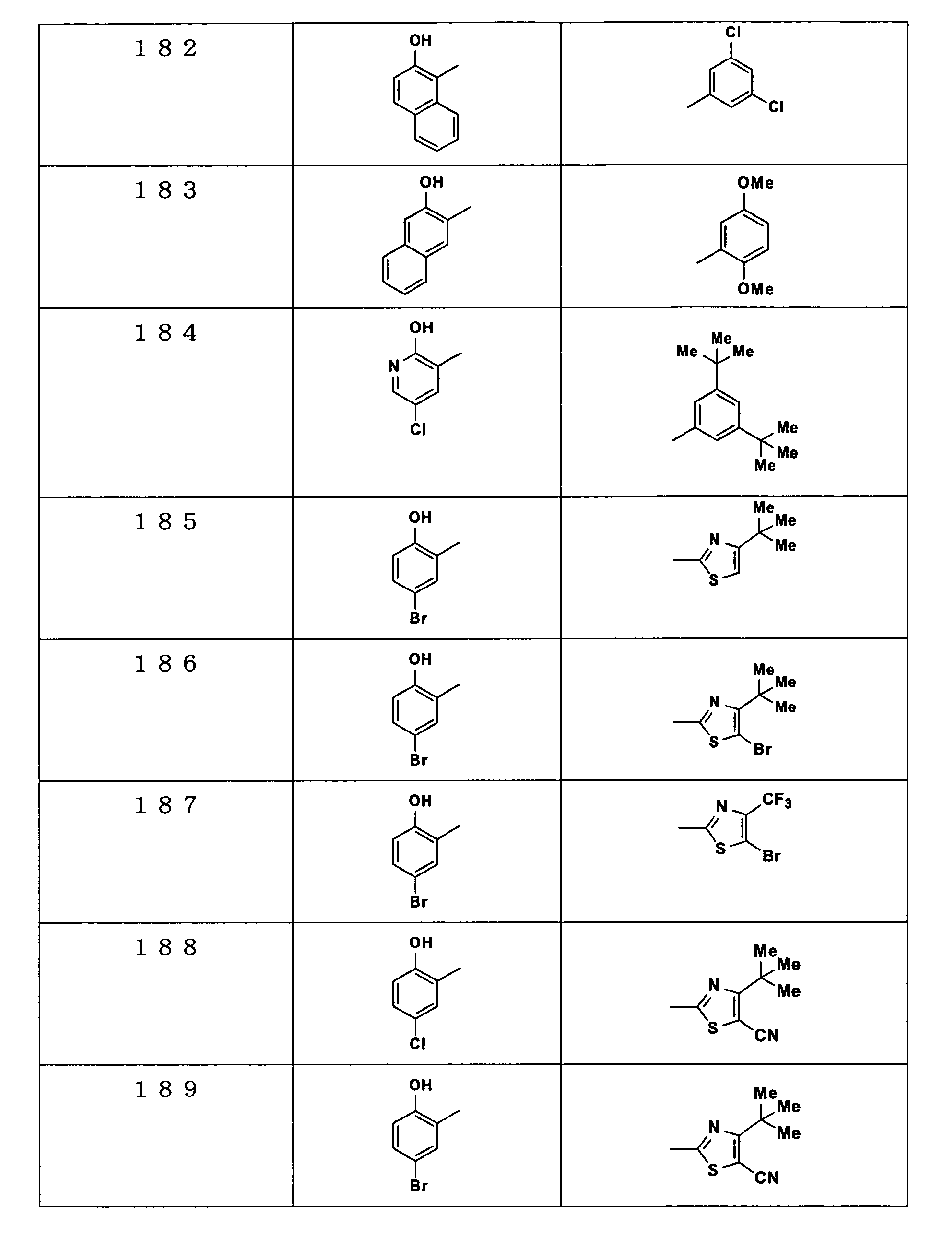

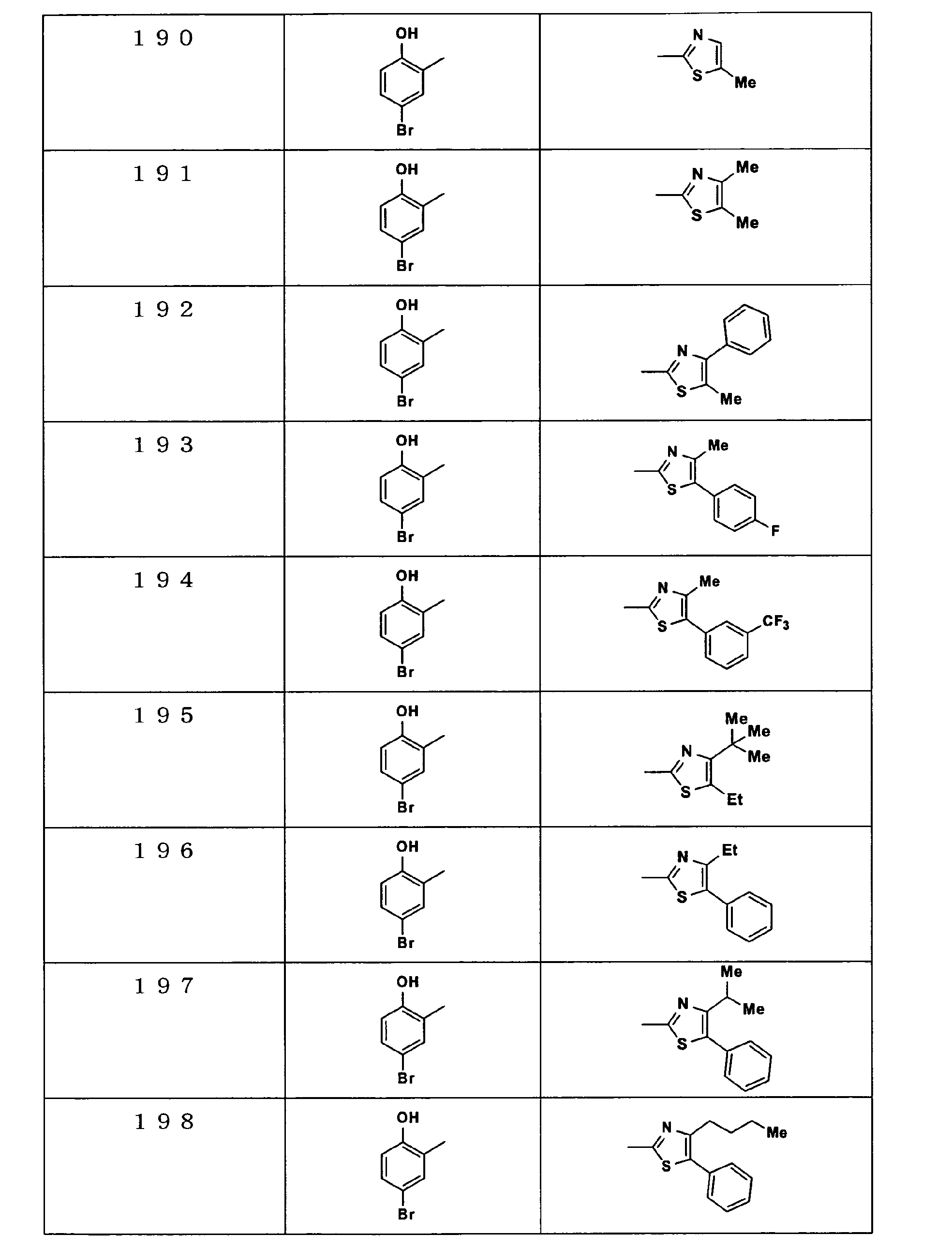

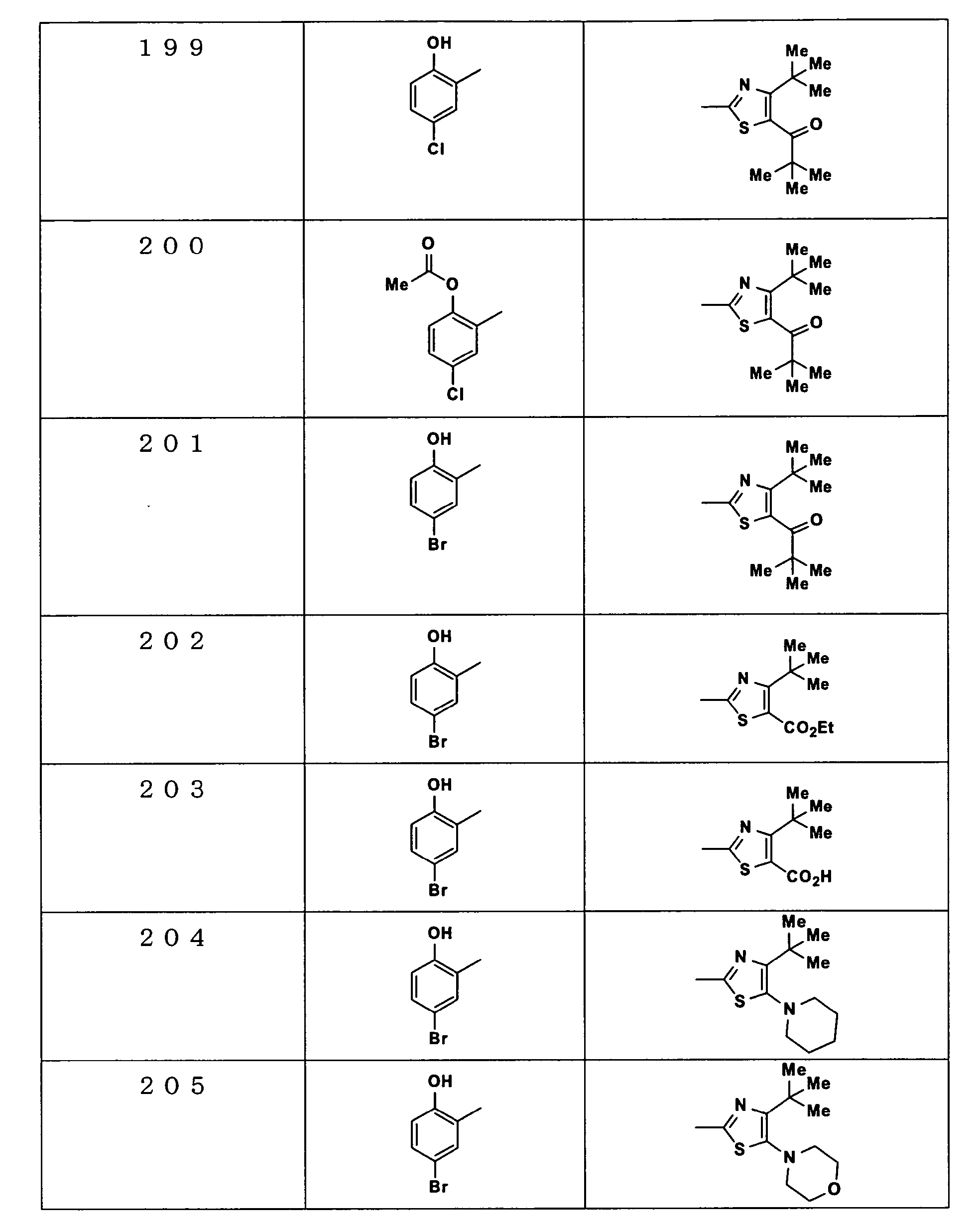

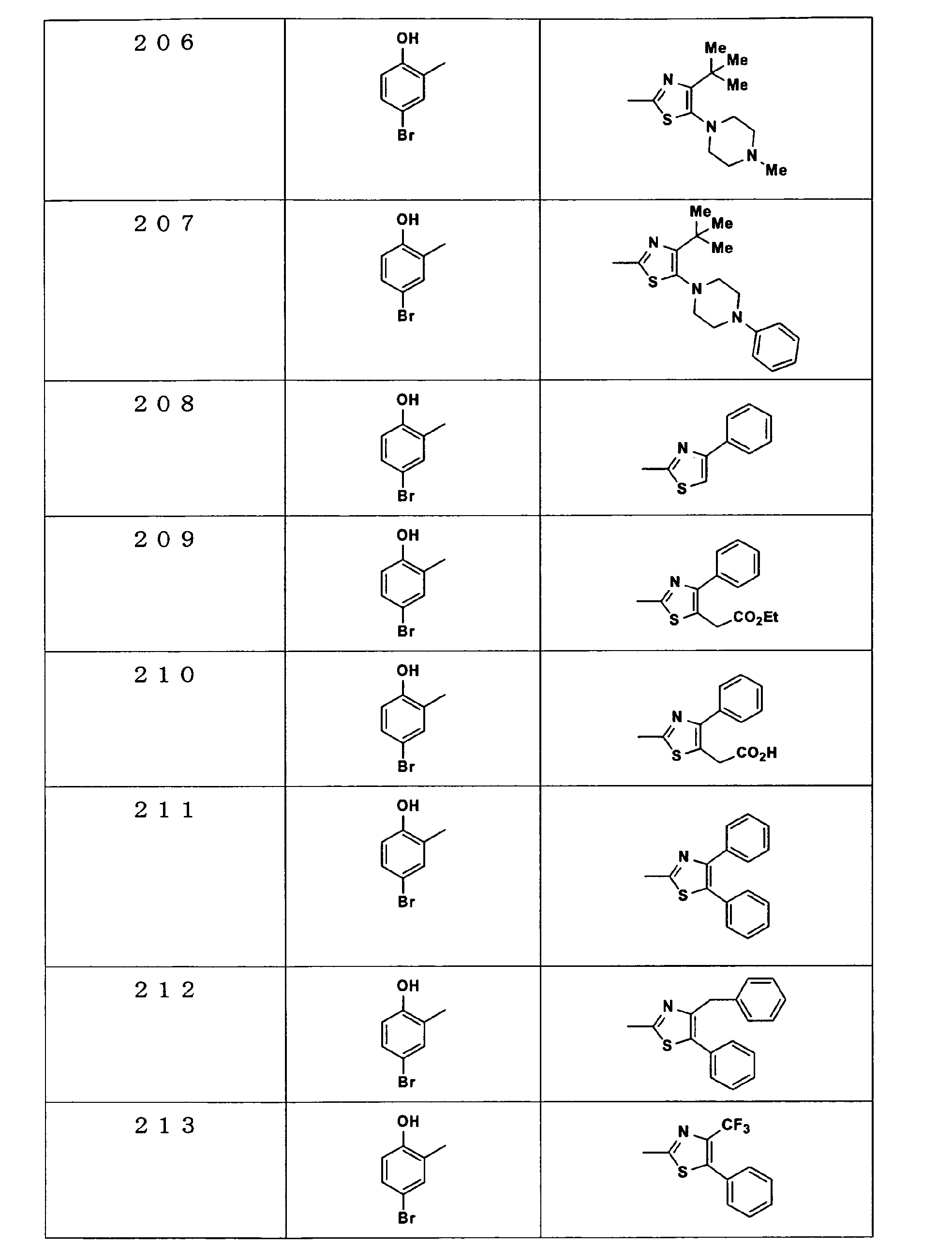

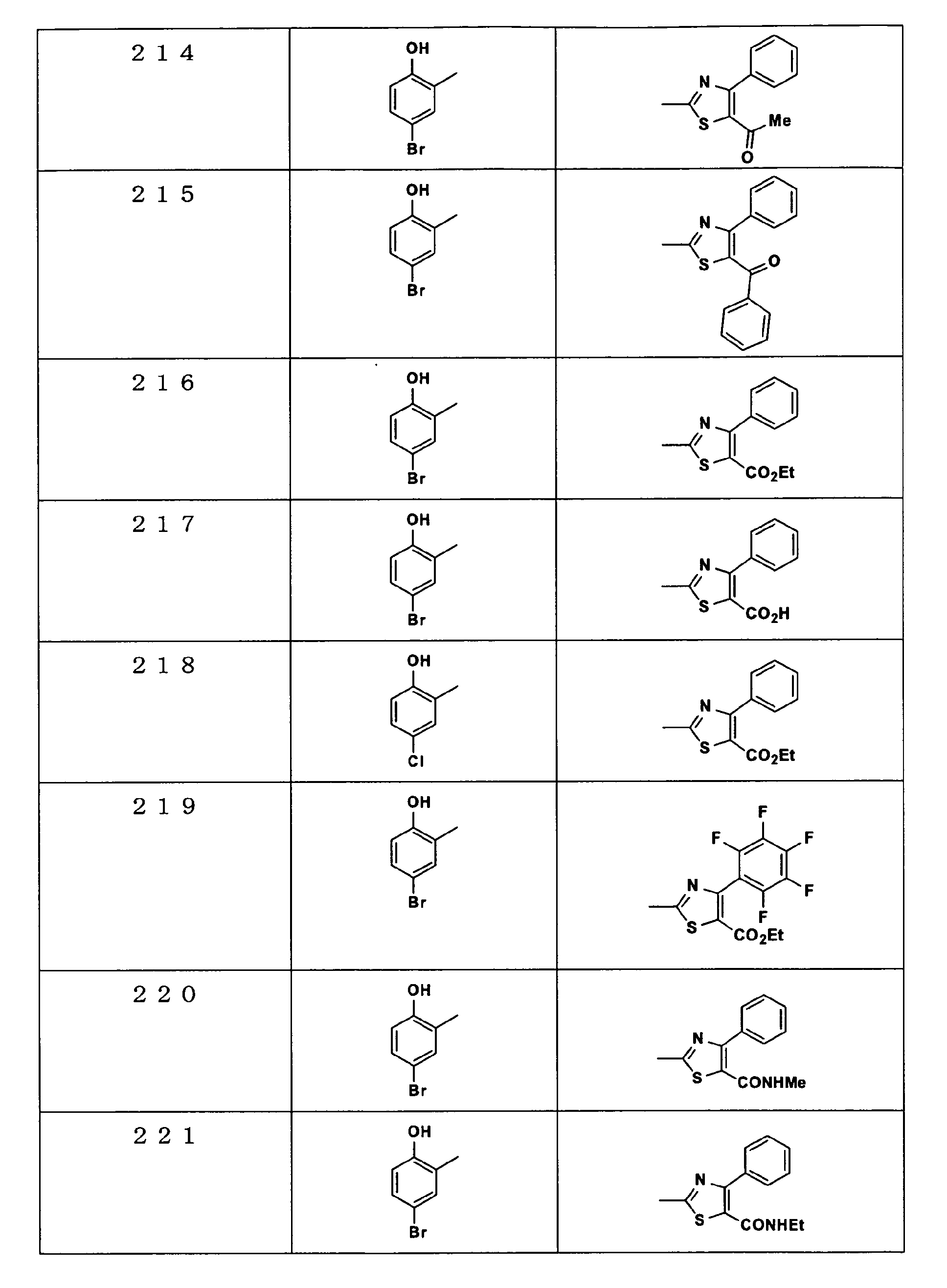

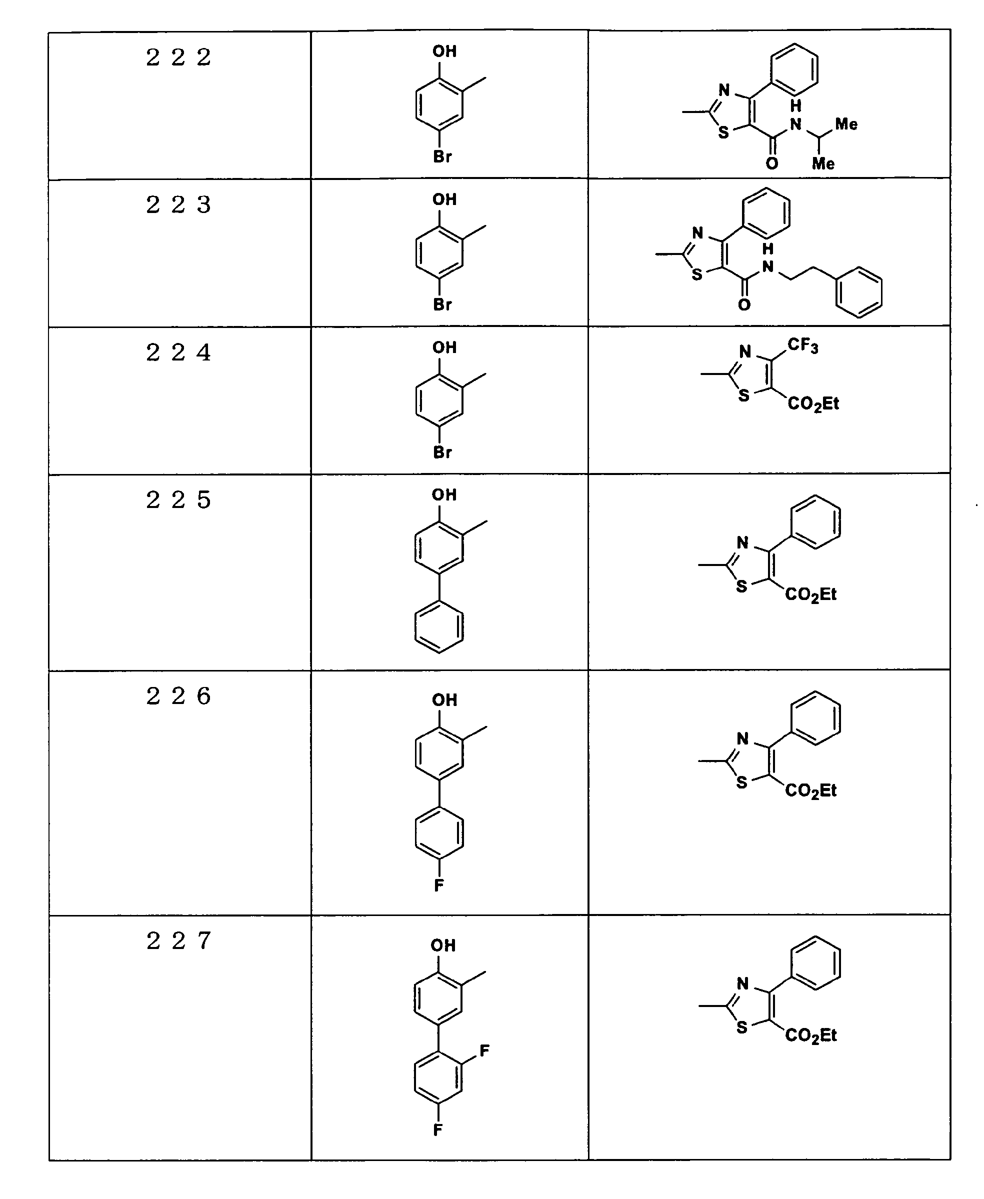

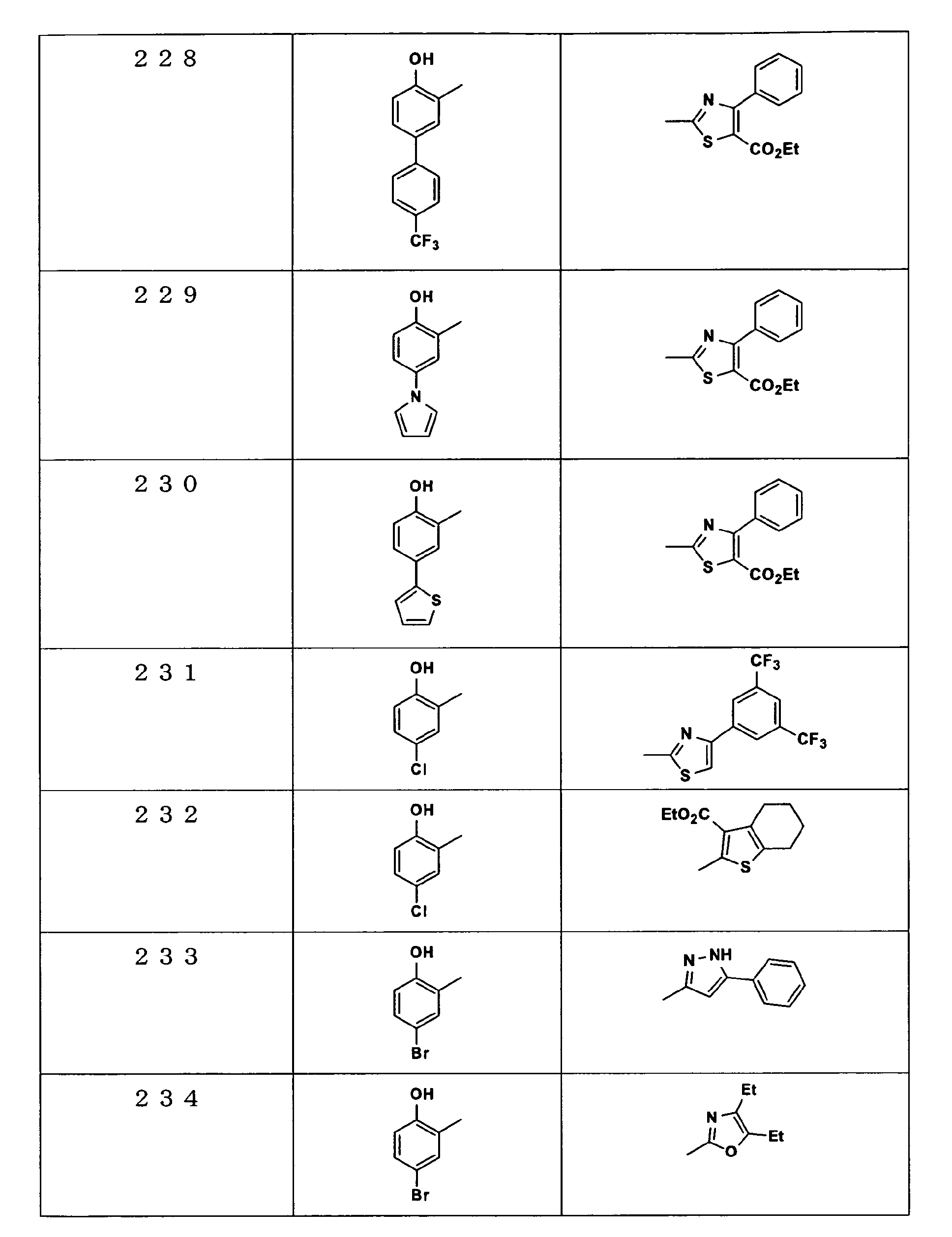

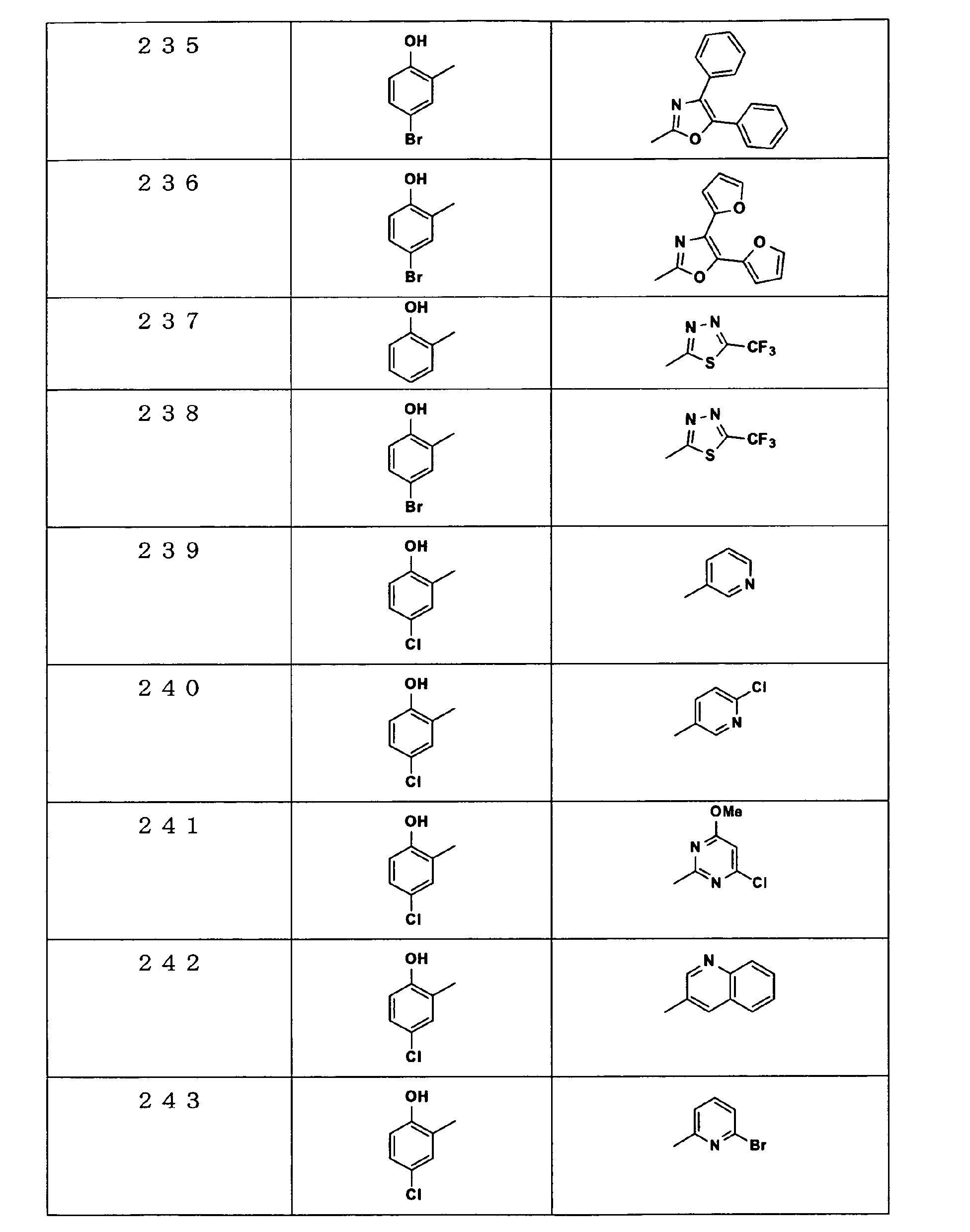

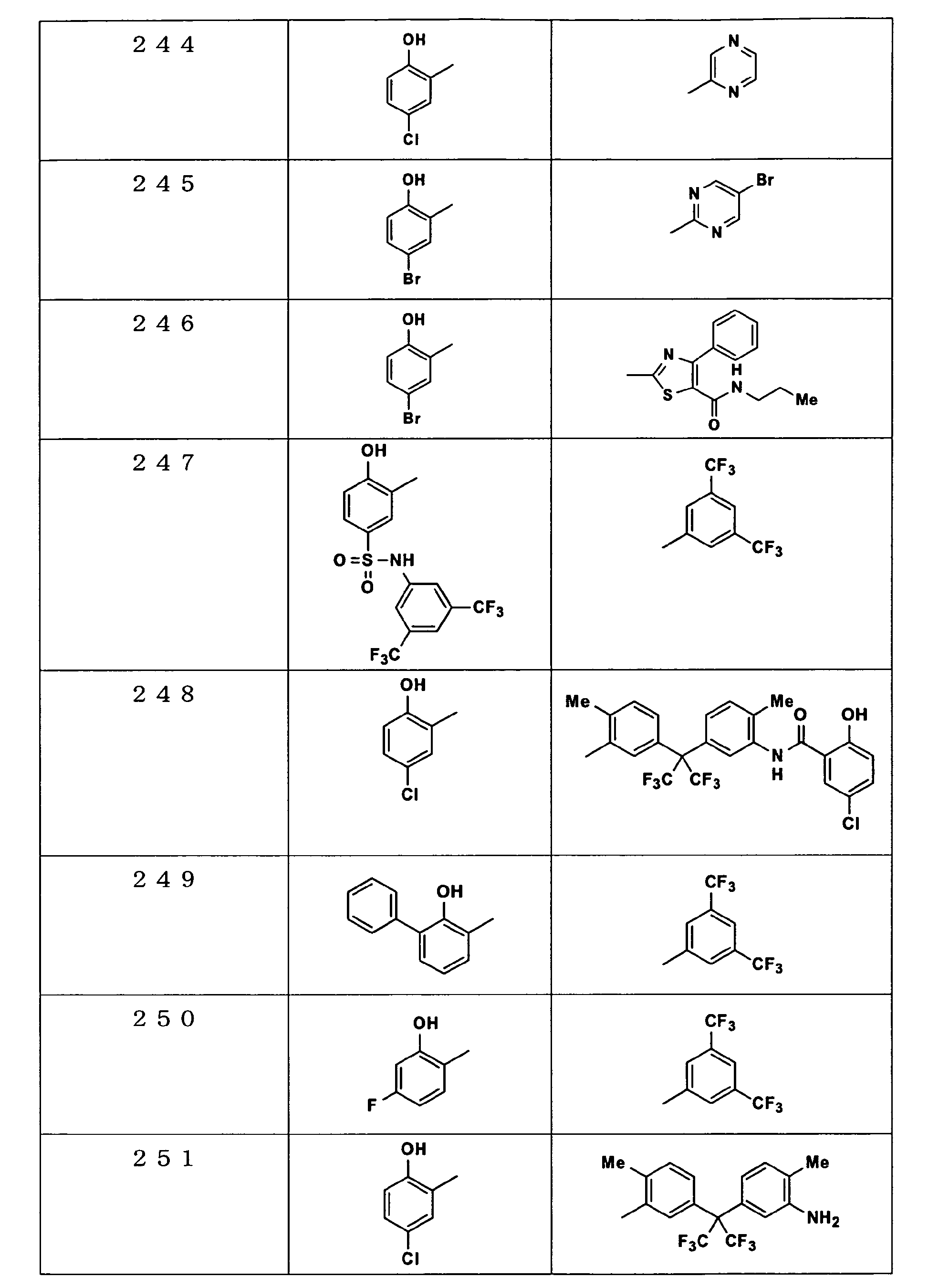

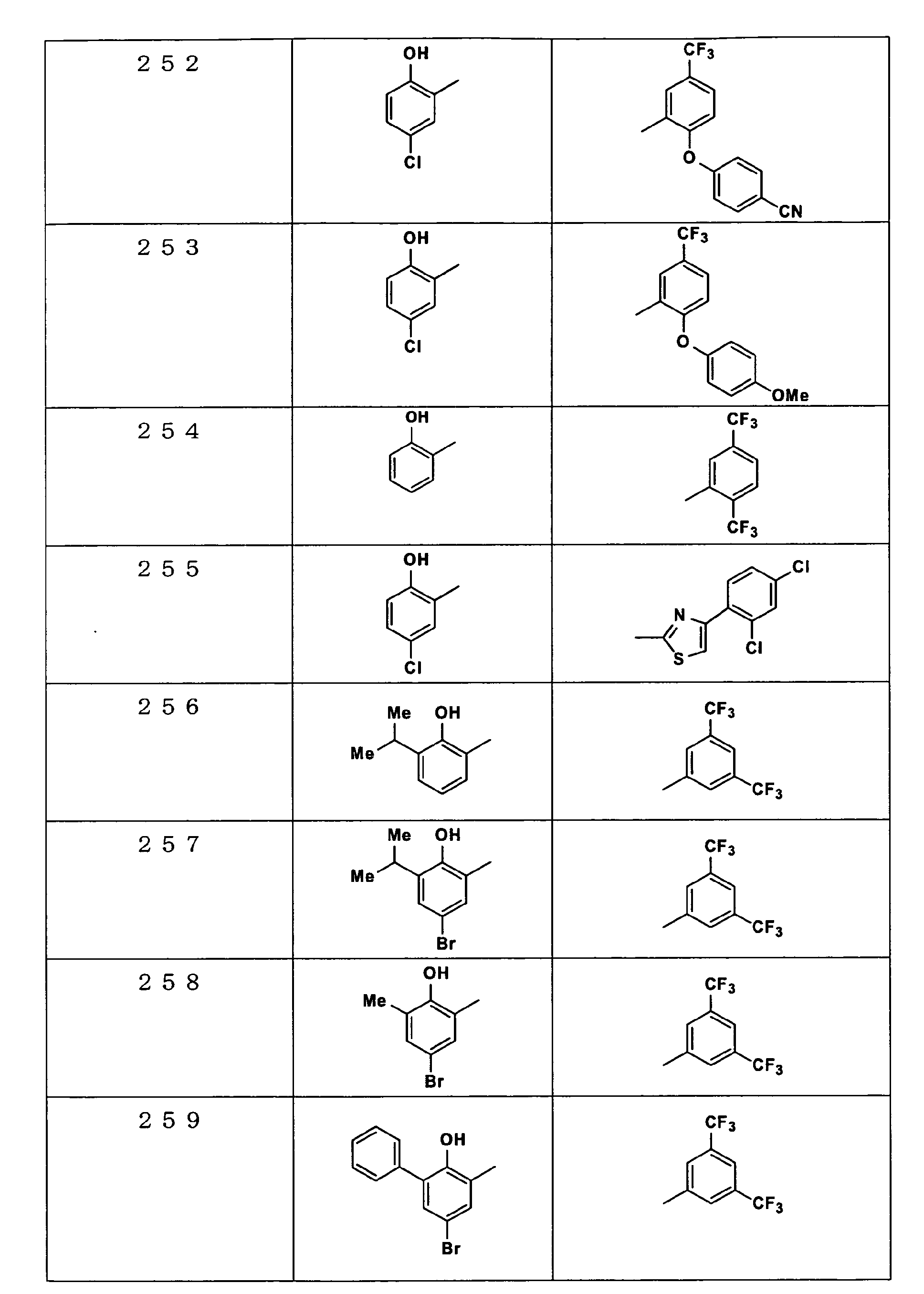

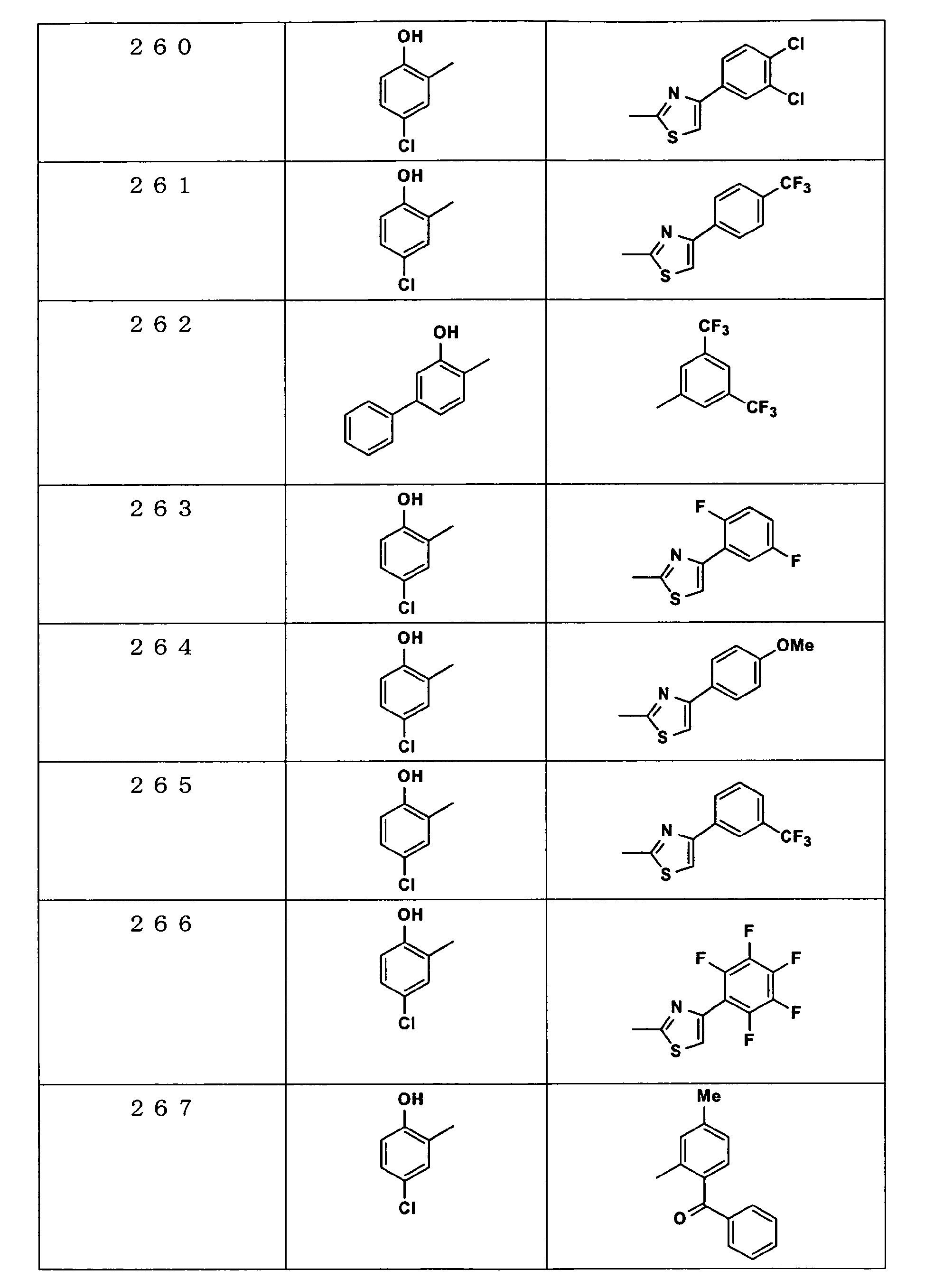

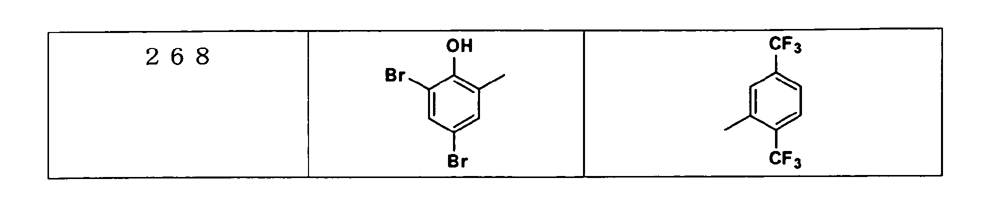

The compounds represented by the general formula (I) can be prepared, for example, by a method described in the following reaction scheme.

### Reaction Scheme

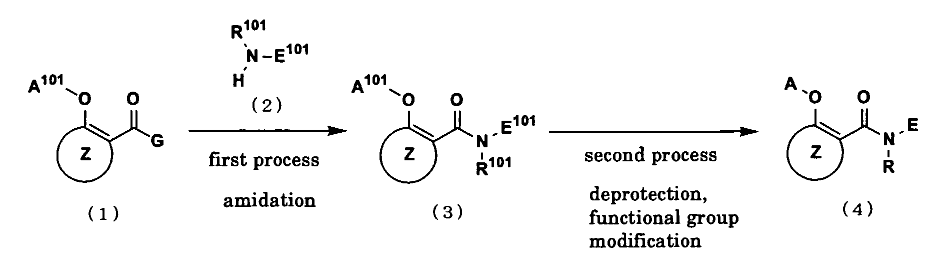

wherein each of A, ring Z, and E has the same meaning as that defined in the general formula (I), A¹⁰¹ represents a hydrogen atom or protecting groups of hydroxy group (preferably, an alkyl group such as methyl group and the like; an aralkyl group such as benzyl group and the like; an acetyl group, an alkoxyalkyl group such as methoxymethyl group and the like; a substituted silyl group such as trimethylsilyl group or the like), each of R and R¹⁰¹ represents a hydrogen atom, a C₁ to C₆ alkyl group or the like, E¹⁰¹ represents E or precursor of E in the definition of the general formula (I), G represents a hydroxy group, halogen atoms (preferably, a chlorine atom), a hydrocarbon-oxy group (preferably, an aryl-oxy group which may be substituted by halogen atom), an acyl-oxy group, an imido-oxy group or the like.

### (First Step)

The amide (3) can be prepared by dehydrocondensation of the carboxylic acid derivative (1) and the amine (2). This reaction is carried out at a reaction temperature of from 0°C to 180°C, without solvent or in an aprotic solvent, in the presence of an acid halogenating agent or a dehydrocondensing agent, and in the presence or absence of a base.

As the halogenating agent, examples include, for example, thionyl chloride, thionyl bromide, sulfuryl chloride, phosphorus oxychloride, phosphorus trichloride, phosphorus pentachloride or the like. When A¹⁰¹ is hydrogen atom, phosphorus trichloride is preferable, and when A¹⁰¹ is acetyl group or the like, phosphorus oxychloride is preferable. As the dehydrocondensing agent, examples include, for example, N,N'-dicyclohexylcarbodiimide, 1-ethyl-3-(3-dimethylaminopropyl)carbodiimide hydrochloride, diphenylphosphorylazide or the like. As the base, examples include inorganic bases such as sodium carbonate, potassium carbonate, sodium hydrogencarbonate or the like, or organic bases such as pyridine, triethylamine, N,N'-diethylaniline or the like. As the aprotic solvent, examples include dichloromethane, dichloroethane, chloroform, tetrahydrofuran, 1,4-dioxane, benzene, toluene, monochlorobenzene, o-dichlorobenzene, N,N'-dimethylformamide, N-methylpyrrolidone or the like, when the reaction is carried out in the presence of the acid halogenating agent, particularly, toluene, monochlorobenzene, o-dichlorobenzene are preferable.

A target compound can also be prepared, for example, by a method or similar method described in Journal of Medicinal Chemistry, (USA), 1998, Vol.41, No.16, p.2939-2945, in which the acid chloride is prepared and isolated beforehand from carboxylic acid, then the result is made to react with an amine having E¹⁰¹.

When G is hydroxy group, the reaction condition described in Archiv der Pharmazie, (Germany), 1998, Vol.331, No.1, p.3-6 can be used as a preferred reaction condition.

Kinds of carboxylic acid derivative (1) and amine (2) are not particularly limited, and new compounds synthesized by referring to well-known preparation method described in the literature or commercially available reagents can be used for the aforementioned reaction.

### (Second Step)

When the amide (3) has a protecting group and/or has a favorable substituent for functional group modification, for example, an amino group and a protected amino group or its precursor; a carboxy group and a protected carboxy group or its precursor; a hydroxy group and a protected hydroxy group or its precursor, the final target compound (4) can be prepared by a reaction for deprotection and/or functional group modification in this step. Various well-known methods can be used for the reaction. For the reaction of deprotection and functional group modification, for example, methods described in "Protective Groups in Organic Syntheses", (USA), Theodra W. Green, Peter G.M. Wuts, Eds., Third edition, Apr. in 1999, John Wiley & Sons, and "Handbook of Reagents for Organic Synthesis", (USA), 4 Volumes, Jun. in 1999, John Wiley & Sons can be used, and for the reaction of functional group modification, for example, methods described in "Palladium Reagents in Organic Syntheses", (USA), Richard F. Heck, 1985, Academic Press, and "Palladium Reagents and Catalysts: Innovations in Organic Synthesis", (USA), J. Tsuji, 1999, John Wiley & Sons, or the like can be used.

The compounds represented by the general formula (I) prepared by the aforementioned methods can be isolated and purified by methods widely known by those skilled in the art, for example, extraction, precipitation, fractional chromatography, fractional crystallization, suspension and washing, and recrystallization. Furthermore, each of the pharmaceutically acceptable salt of the compound of the present invention, the hydrate thereof and the solvate thereof can be prepared by methods widely known by those skilled in the art.

In the examples of the specification, preparation methods of typical compounds included in the general formula (I) are explained in details. Therefore, those skilled in the art can prepare any compound fall within the general formula (I) by referring to the explanations of the aforementioned general preparation methods and those of specific preparation methods of the examples, by choosing appropriate reaction raw materials, reaction reagents, and reaction conditions, and by adding appropriate modification and alteration of these methods, if necessary.

The compounds represented by the general formula (I) have anticancer action, therefore, the medicament comprising said compounds as active ingredients can be used for preventive and/or therapeutic treatment of cancers. In the present specification, "the prevention and/or treatment of cancers" or their synonyms should be interpreted in a broadest sense including inhibitory action against cancerous transformation of tissue or cells, inhibitory action against metastasis of cancer, enhancement of existing anticancer agents, action for overcoming drug tolerances of existing anticancer agents, action for improvement of cancerous cachexia, preventive action against recurrence, action for prolonging lifetime of cancer patients or the like, as well as actions of killing cancer cells or suppressing cancers, and should not be interpreted any limitative sense. The medicament of the present invention may be used for preventive and/or therapeutic treatment of skin cancer, melanoma, kidney cancer, lung cancer, liver cancer, breast cancer, uterine cancer, pancreatic cancer, other solid cancer, sarcoma, osteosarcoma, metastatic invasion of cancer, canceration of inflammatory focus, cancerous cachexia, metastasis of cancer, leukemia such as acute myeloblastic leukemia, multiple myeloma, Lennert's lymphoma, malignant lymphoma, development of carcinostatic resistance of cancer, canceration of foci such as viral hepatitis and cirrhosis, canceration from polyp of colon, brain tumor, nervous tumor, sarcoidosis or the like. However, disease to be applicable by the medicaments of the present invention are not limited to these cancers.

As the active ingredient of the medicament on the present invention, one or more kinds of substances selected from the group consisting of the compound represented by the general formula (I) and a pharmacologically acceptable salt thereof, and a hydrate thereof and a solvate thereof may be used. The aforementioned substance, per se, may be administered as the medicament of the present invention, however, preferably, the medicament of the present invention is provided in the form of a pharmaceutical composition comprising the aforementioned substance which is an active ingredient together with one or more pharmacologically acceptable pharmaceutical additives. In the aforementioned pharmaceutical compositions, a ratio of the active ingredient to the pharmaceutical additives is 1 weight % to 90 weight %.

The pharmaceutical compositions of the present invention may be administered as pharmaceutical compositions for oral administration, for example, granules, subtilized granules, powders, hard capsules, soft capsules, syrup, emulsion, suspension, or solution, or may be administered as pharmaceutical compositions for parenteral administration, for example, injections for intravenous administration, intramuscular administration, or subcutaneous administration, drip infusions, suppositories, percutaneous absorbent, transmucosal absorption preparations, nasal drops, ear drops, instillation, and inhalants. Preparations made as pharmaceutical compositions in a form of powder may be dissolved when necessary and used as injections or drip infusions.

For preparation of pharmaceutical compositions, solid or liquid pharmaceutical additives may be used. Pharmaceutical additives may either be organic or inorganic. When an oral solid preparation is prepared, an excipient is added to the active ingredient, and further binders, disintegrator, lubricant, colorant, corrigent are added, if necessary, to manufacture preparations in the forms of tablets, coating tablets, granules, powders, capsules and the like by ordinary procedures. Examples of the excipient include lactose, sucrose, saccharose, glucose, corn starch, starch, talc, sorbit, crystal cellulose, dextrin, kaolin, calcium carbonate, and silicon dioxide. Examples of the binder include, for example, polyvinyl alcohol, polyvinyl ether, ethyl cellulose, methyl cellulose, gum Arabic, tragacanth, gelatine, shellac, hydroxypropyl cellulose, hydroxypropyl methyl cellulose, calcium citrate, dextrin, and pectin. Examples of the lubricant include, for example, magnesium stearate, talc, polyethylene glycol, silica, and hydrogenated vegetable oil. As the coloring agent, any material can be used which are approved to be added to ordinary pharmaceuticals. As the corrigent, cocoa powder, menthol, aromatic acid, peppermint oil, d-borneol, cinnamon powder and the like can be used. These tables and granules may be applied with sugarcoating, gelatin coating, or an appropriate coating, if necessary. Preservatives, antioxidant and the like may be added, if required.

For liquid preparations for oral administration such as emulsions, syrups, suspensions, and solutions, ordinary used inactive diluents, for example, water or vegetable oil may be used. For these preparations, besides inactive diluents, adjuvants such as wetting agents, suspending aids, sweating agents, flavoring agents, coloring agents or preservatives may be blended. After a liquid preparation is manufactured, the preparation may be filled in capsules made of a absorbable substance such as gelatin. Examples of solvents or suspending agents used for the preparations of parenteral administration such as injections or suppositories include, for example, water, propylene glycol, polyethylene glycol, benzyl alcohol, ethyl oleate, and lecithin. Examples of base materials used for preparation of suppositories include, for example, cacao butter, emulsified cacao butter, lauric fat, and witepsol. Methods for preparation of the aforementioned preparations are not limited, and any method ordinarily used in the art may be used.

When the composition are prepared in the form of injections, carriers such as, for example, diluents including water, ethanol, macrogol, propylene glycol, citric acid, acetic acid, phosphoric acid, lactic acid, sodium lactate, sulfuric acid and sodium hydroxide, pH modifiers and buffer solutions including sodium citrate, sodium acetate and sodium phosphate, stabilizers such as sodium pyrosulfite, ethylenediaminetetraacetic acid, thioglycolic acid and thiolactate may be used. For the preparation, a sufficient amount of a salt, glucose, mannitol or glycerin may be blended in the preparation to manufacture an isotonic solution, and an ordinary solubilizer, a soothing agent, or a topical anesthetic may be used.

When the preparation in the form of an ointment such as a paste, a cream, and a gel is manufactured, an ordinarily used base material, a stabilizer, a wetting agent, and a preservative may be blended, if necessary, and may be prepared by mixing the components by a common method. As the base material, for example, white petrolatum, polyethylene, paraffin, glycerin, cellulose derivatives, polyethylene glycol, silicon, and bentonite may be used. As the preservative, paraoxy methyl benzoate, paraoxy ethyl benzoate, paraoxy propyl benzoate and the like may be used. When the preparation in the form of a patch is manufactured, the aforementioned ointment, cream gel, or paste and the like may be applied by a common method to an ordinary support. As the support, fabric made of cotton, span rayon, and synthetic fibersor or nonwoven fabric, and a film or a foam sheet such as made of soft vinyl chloride, polyethylene, and polyurethane and the like may be preferably used.

A dose of the medicament of the present invention is not particularly limited. For oral administration, a dose may generally be 0.01 to 5,000 mg per day for an adult as the weight of the compound of the present invention. It is preferred to increase or decrease the above dose appropriately depending on the age, pathological conditions, and symptoms of a patient. The above dose may be administered once a day or 2 to 3 times a day as divided portions with appropriate intervals, or intermittent administration for every several days may be applied. When the medicament is used as an injection, the dose may be 0.001 to 100 mg per day for an adult as the weight of the compound of the present invention.

### Examples

The present invention will be explained more specifically with reference to the following examples. However the scope of the present invention is not limited to the following examples. The compound number in the following examples correspond to those in the table shown above. And the commercially available compounds, which were purchased and used for the examinations, are contained in these examples. As for such compounds, the suppliers of the reagents and the catalog code numbers are shown.

### Example 1: Preparation of the compound of Compound No. 1.

3,5-Bis(trifluoromethyl)aniline(500mg, 2.2mmol) and pyridine(0.5mL) were added to a solution of O-acetylsalicyloyl chloride(345mg, 1.7mmol) in benzene(10mL) under argon atmosphere, and the mixture was stirred at room temperature for 1 hour. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=3:1) to give the title compound(570mg, 84.2%) as a white solid.
mp 124-125°C.
¹H-NMR(DMSO-d₆): δ 2.36(3H, s), 7.19(1H, dd, J=8.0, 1.2Hz), 7.39(1H, td, J=7.6, 1.2Hz), 7.57(1H, ddd, J=8.0, 7.6, 1.6Hz), 7.65(1H, s), 7.83(1H, dd, J=8.0, 1.6Hz), 8.11(2H, s), 8.31(1H, s).

### Example 2: Preparation of the compound of Compound No. 2.

2N Aqueous sodium hydroxide(0.5mL, 1mmol) was added to a solution of 2-acetoxy-N-[3,5-bis(trifluoromethyl)phenyl]benzamide(Compound No. 1; 100mg, 0.25mmol) in ethanol(5mL), and the mixture was stirred at room temperature for 1 hour. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was recrystallized from n-hexane/ethyl acetate to give the title compound(40mg, 45.1%) as a white solid.
mp 179-180°C.
¹H-NMR(DMSO-d₆): δ 6.96-7.02(2H, m), 7.45(1H, ddd, J=8.0, 7.2, 1.6Hz), 7.81(1H, s), 7.87(1H, dd, J=8.0, 1.6Hz), 8.46(2H, s), 10.80(1H, s), 11.26(1H, s).

### Example 3: Preparation of the compound of Compound No. 3.

A mixture of 5-fluorosalicylic acid(156mg, 1mmol), 3,5-bis(trifluoromethyl)aniline(229mg, 1mmol), phosphorus trichloride(44 µ L, 0.5mmol) and monochlorobenzene(5mL) was refluxed for 3 hours under argon atmosphere. After the reaction mixture was cooled to room temperature, it was diluted with ethyl acetate(50mL). After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=6:1) to give the title compound(215mg, 58.7%) as a white solid.
¹H-NMR(DMSO-d₆): δ 7.04(1H, ddd, J=9.0, 4.5,1.2Hz), 7.30-7.37(1H, m), 7.66(1H, ddd, J=9.0, 3.3, 1.2Hz), 7.84(1H, s), 8.46(2H, s), 10.85(1H, s), 11.21(1H, brs).

When the method described in Example 3 is referred in the following examples, phosphorus trichloride was used as the acid halogenating agent. As the reaction solvent, solvents such as monochlorobenzene, toluene or the like were used.

### Example 4: Preparation of the compound of Compound No. 4.

Using 5-chlorosalicylic acid and 3,5-bis(trifluoromethyl)aniline as the raw materials, the same operation as the Example 3 gave the title compound.
Yield: 85.5%.
¹H-NMR(DMSO-d₆): δ 7.05(1H, d, J=8.7Hz), 7.49(1H, dd, J=8.7, 2.7Hz), 7.85(1H, s), 7.87(1H, d, J=2.7Hz), 8.45(2H, s), 10.85(1H, s), 11.39(1H, s).

### Example 5: Preparation of the compound of Compound No. 5.

Acetyl chloride(234mg, 3.3mmol)was added to a solution of N-[3,5-bis(trifluoromethylphenyl)]-5-chloro-2-hydroxybenzamide(Compound No. 4; 1.51g, 3mmol) and pyridine(285mg, 3.6mmol) in tetrahydrofuran(6mL) under ice cooling, and the mixture was stirred at room temperature for 1 hour. 2N Hydrochloric acid was added to the residue obtained by evaporation of the solvent under reduced pressure and the mixture was extracted with ethyl acetate. After the ethyl acetate layer was washed successively with water and brine, dried over anhydrous magnesium sulfate, the residue obtained by evaporation of the solvent under reduced pressure was recrystallized from n-hexane/ethyl acetate to give the title compound(1.06g, 83.0%) as a white solid.
¹H-NMR(DMSO-d₆): δ 2.22(3H, s), 7.35(1H, d, J=9.0Hz), 7.71(1H, dd, J=8.7, 2.7Hz), 7.85(1H, s), 7.88(1H, d, J=2.7Hz), 8.37(2H, s), 11.05(1H, brs).

When the method described in Example 5 is referred in the following examples, organic bases such as pyridine, triethylamine or the like were used as the base. As the reaction solvent, solvents such as dichloromethane, tetrahydrofuran, benzene or the like were used.

### Example 6: Preparation of the compound of Compound No. 6.

Using 5-bromosalicylic acid and 3,5-bis(trifluoromethyl)aniline as the raw materials, the same operation as the Example 3 gave the title compound.
Yield: 88.5%.
¹H-NMR(DMSO-d₆): δ 6.98(1H, d, J=8.8Hz), 7.59(1H, dd, J=8.8, 2.8Hz), 7.83(1H, s), 7.98(1H, d, J=2.8Hz), 8.43(2H, s), 10.82(1H, s), 11.37(1H, s).

This compound was obtained also by the following preparation method.

Iron powder(30mg, 0.54mmol) and bromine(0.02mL, 0.39mmol) were added to a solution of 2-acetoxy-N-[3,5-bis(trifluoromethyl)]benzamide(Compound No. 1; 100mg, 0.25mmol) in carbon tetrachloride(8mL), and the mixture was stirred at 50°C for 4 hours. After the reaction mixture was cooled to room temperature, it was poured into aqueous NaHSO₄ and extracted with ethyl acetate. The ethyl acetate layer was washed with water and brine, and dried over anhydrous sodium sulfate. The residue obtained by evaporation of the solvent under reduced pressure was purified by column chromatography on silica gel(n-hexane:ethyl acetate=4:1) to give the title compound(600mg, 54.9%) as a white solid.

### Example 7: Preparation of the compound of Compound No. 7.

Using 5-iodosalicylic acid and 3,5-bis(trifluoromethyl)aniline as the raw materials, the same operation as the Example 3 gave the title compound.
Yield: 62.2%.
¹H-NMR(DMSO-d₆): δ 6.86(1H, d, J=8.4Hz), 7.74(1H, dd, J=8.7, 2.4Hz), 7.84(1H, s), 8.13(1H, d, J=2.1Hz), 8.84(2H, s), 10.82(1H, s), 11.41(1H, s).

### Example 8: Preparation of the compound of Compound No. 8.

Using 5-nitrosalicylic acid and 3,5-bis(trifluoromethyl)aniline as the raw materials, the same operation as the Example 3 gave the title compound.
Yield: 57.2%.
¹H-NMR(DMSO-d₆): δ 7.18(1H, d, J=9.0Hz), 7.86(1H, s), 8.31(1H, dd, J=9.0, 3.0Hz), 8.45(2H, s), 8.70(1H, d, J=3.0Hz), 11.12(1H, s).

### Example 9: Preparation of the compound of Compound No. 9.

### (1) 2-Benzyloxy-5-formylbenzoic acid benzyl ester.

A mixture of 5-formylsalicylic acid(4.98g, 30mmol), benzyl bromide(15.39g, 90mmol), potassium carbonate(16.59g, 120mmol), and methyl ethyl ketone(350mL) was refluxed for 8 hours. After cooling, the solvent was evaporated under reduced pressure. 2N Hydrochloric acid was added to the residue, and the mixture was extracted with ethyl acetate. The layer was washed with water and brine, and dried over anhydrous magnesium sulfate. The residue obtained by evaporation of the solvent under reduced pressure was purified by column chromatography on silica gel(n-hexane:ethyl acetate=3:1), suspended and washed with isopropyl ether under heating at reflux to give the title compound(5.98g, 57.5%) as a white solid.
¹H-NMR(CDCl₃): δ 5.27(2H, s), 5.37(2H, s), 7.15(1H, d, J=9.0Hz), 7.26-7.46(10H, m), 7.99(1H, dd, J=9.0, 2.4Hz), 8.36(1H, d, J=2.4Hz), 9.91(1H, s).

### (2) 2-Benzyloxy-5-cyanobenzoic acid benzyl ester.

A mixture of 2-benzyloxy-5-formylbenzoic acid benzyl ester(693mg, 2mmol), hydroxylamine hydrochloride(167mg, 2.4mmol), and N-methylpyrrolidone(3mL) was stirred at 115 □ for 4 hours. After the reaction mixture was cooled, 2N hydrochloric acid(5mL) and water(30mL) were added and the mixture was extracted with ethyl acetate. The organic layer was washed with 2N aqueous sodium hydroxide, water, and brine, and dried over anhydrous magnesium sulfate. The residue obtained by evaporation of the solvent under reduced pressure was suspended and washed with isopropyl ether under heating at reflux to give the title compound(527mg, 76.7%) as a white solid.
¹H-NMR(CDCl₃): δ 5.23(2H, s), 5.35(2H, s), 7.08(1H, d, J=8.7Hz), 7.33-7, 43(10H, m), 7.70(1H, dd, J=8.7, 2.4Hz), 8.13(1H, d, J=2.4Hz).

### (3) 5-Cyanosalicylic acid.

Ethanol(10mL) and tetrahydrofuran(10mL) were added to 2-benzyloxy-5-cyanobenzoic acid benzyl ester(446mg, 1.3mmol) and 5% palladium on carbon(45mg), and the mixture was hydrogenated at room temperature for 2 hours. After the insoluble matter was filtered off, the solvent was evaporated under reduced pressure to give the title compound(212mg, 100.0%) as a white solid.
¹H-NMR(DMSO-d₆): δ 7.02(1H, d, J=8.7Hz), 7.82(1H, dd, J=8.7, 2.4Hz), 8.12(1H, d, J=2.1Hz).

### (4) N-[3,5-Bis(trifluoromethyl)phenyl]-5-cyano-2-hydroxybenzamide(Compound No. 9).

Using 5-cyanosalicylic acid and 3,5-bis(trifluoromethyl)aniline as the raw materials, the same operation as the Example 3 gave the title compound.
Yield: 16.6%.
¹H-NMR(DMSO-d₆): δ 7.15(1H, d, J=8.7Hz), 7.85(1H, s), 7.86(1H, dd, J=8.7, 2.1Hz), 8.22(1H, d, J=2.4Hz), 8.43(2H, s), 10.93(1H, s), 12.00(1H, brs).

### Example 10: Preparation of the compound of Compound No. 10.

Using 5-methylsalicylic acid and 3,5-bis(trifluoromethyl)aniline as the raw materials, the same operation as the Example 3 gave the title compound.
Yield: 54.9%.
¹H-NMR(DMSO-d₆): δ 6.92(1H, d, J=8.7Hz), 7.28(1H, dd, J=8.7, 1.8Hz), 7.71(1H, d, J=1.8Hz), 7.82(1H, s), 8.47(2H, s), 10.80(1H, s), 11.14(1H, s).

### Example 11: Preparation of the compound of Compound No. 11.

### (1) 5-[(1,1-Dimethyl)ethyl]salicylic acid.

Sulfamic acid(1.76g, 18.1mmol) and sodium dihydrogenphosphate(7.33g, 47mmol) were added to a solution of
5-[(1,1-dimethyl)ethyl]-2-hydroxybenzaldehyde(2.15g, 12-1mmol) in 1,4-dioxane(100mL) and water(40mL). A solution of sodium chlorite(1.76g, 15.5mmol) in water(10mL) was added to the mixture under ice cooling, and it was stirred for 1 hour. Then, sodium sulfite(1.80g, 14.3mmol) was added to the mixture, and it was stirred for 30 minutes. Concentrated hydrochloric acid was added to the reaction mixture, and pH was adjusted to 1. The residue obtained by evaporation of 1,4-dioxane under reduced pressure was extracted with ethyl acetate. The organic layer was washed with water and brine, and dried over anhydrous magnesium sulfate. The residue obtained by evaporation of the solvent under reduced pressure was washed with n-hexane under suspension to give the title compound(1.81g, 77.4%) as a white powder.
¹H-NMR(DMSO-d₆): δ 1.26(9H, s), 6.90(1H, d, J=9.0Hz), 7.58(1H, dd, J=8.7, 2.4Hz), 7.75(1H, d, J=2.4Hz), 11.07(1H, brs).

### (2) N-[3,5-Bis(trifluoromethyl)phenyl]-5-[(1,1-dimethyl)ethyl]-2-hydroxybenzamide (Compound No. 11).

Using 5-[(1,1-dimethyl)ethyl]salicylic acid and 3,5-bis(trifluoromethyl)aniline as the raw materials, the same operation as the Example 3 gave the title compound.
Yield: 53.8%.
¹H-NMR(DMSO-d₆): δ 1.30(9H, s), 6.96(1H, d, J=8.7Hz), 7.50(1H, dd, J=8.7, 2.4Hz), 7.82(1H, d, J=2.4Hz), 7.83(1H, s), 8.46(2H, s), 10.80(1H, s)11.12(1H, s).

### Example 12: Preparation of the compound of Compound No. 12.

### (1) 5-Acetyl-2-benzyloxybenzoic acid methyl ester.

A mixture of 5-acetylsalicylic acid methyl ester(13.59g, 70mmol), benzyl bromide(17.96g, 105mmol), potassium carbonate(19.35g, 140mmol) and methyl ethyl ketone(350mL) was refluxed for 8 hours. After cooling, the solvent was evaporated under reduced pressure. 2N Hydrochloric acid was added to the residue, and it was extracted with ethyl acetate. After the ethyl acetate layer was washed with water and brine, dried over anhydrous magnesium sulfate and concentrated, the residue was recrystallized from isopropyl ether to give the title compound(14.20g, 71.4%) as a white solid.
¹H-NMR(CDCl₃): δ 2.58(3H, s), 3.93(3H, s), 5.27(2H, s), 7.07(1H, d, J=8.7Hz), 7.26-7.43(3H, m), 7.47-7.50(2H, m), 8.07(1H, dd, J=8.7, 2.4Hz), 8.44(1H, d, J=2.4Hz).

### (2) 5-Acetyl-2-benzyloxybenzoic acid.

2N Sodium hydroxide(11mL) was added to a solution of 5-acetyl-2-benzyloxybenzoic acid methyl ester(5.69g, 20mmol) in a mixed solvent of methanol/tetrahydrofuran(20mL+20mL), and the mixture was stirred for 8 hours. 2N Hydrochloric acid was added to the residue obtained by evaporation of the solvent under reduced pressure and the mixture was extracted with dichloromethane. After the dichloromethane layer was washed successively with water and brine, dried over anhydrous magnesium sulfate, the residue obtained by evaporation of the solvent under reduced pressure was washed with isopropyl ether to give the title compound(4.92g, 91.0%) as a white solid.
¹H-NMR(DMSO-d₆): δ 2.55(3H, s), 5.32(2H, s), 7.30-7.43(4H, m), 7.49-7.52(2H, m), 8.09(1H, dd, J=9.0, 2.7Hz), 8.22(1H, d, J=2.4Hz).

### (3) 5-Acetyl-2-benzyloxy-N-[3,5-bis(trifluoromethyl)phenyl]benzamide.

Phosphorus oxychloride(1.85mL, 19.8mmol) was added to a solution of 5-acetyl-2-benzyloxybenzoic acid(4.87g, 18mmol), 3,5-bis(trifluoromethyl)aniline(4.54g, 19.8mmol) and pyridine(5.70g, 72mmol) in a mixed solvent of tetrahydrofuran/dichloromethane(72mL+36mL) under ice cooling, and the mixture was stirred at room temperature for 12 hours. 1N Hydrochloric acid(100mL) was added to the residue obtained by evaporation of the solvent under reduced pressure and the mixture was extracted with ethyl acetate. After the ethyl acetate layer was washed successively with water and brine, dried over anhydrous magnesium sulfate, the residue obtained by evaporation of the solvent under reduced pressure was purified by column chromatography on silica gel(n-hexane:ethyl acetate= 3:1→2:1) to give the title compound(5.47g, 63.1%) as a slightly yellowish green crystal.
¹H-NMR(DMSO-d₆): δ 2.57(3H, s), 7.11(1H, d, J=8.7Hz), 7.86(1H, s), 8.05(1H, dd, J=8.4, 2.1Hz), 8.44(1H, d, J=2.1Hz), 8.47(2H, s), 10.96(1H, s), 11.97(1H, brs).

When the preparation method described in Example 12(3) is referred in the following examples, phosphorus oxychloride was used as the acid halogenating agent. Pyridine was used as the base. As the reaction solvent, solvents such as dichloromethane, tetrahydrofuran or the like were used alone or as a mixture.

### (4) 5-Acetyl-N-[3,5-bis(trifluoromethyl)phenyl]-2-hydroxybenzamide(Compound No. 12).

Ethanol(6mL) and tetrahydrofuran(72mL) were added to 5-acetyl-2-benzyloxy-N-[3,5-bis(trifluoromethyl)phenyl]benzamide(602mg, 1.25mmol) and 5% palladium on carbon(60mg), and the mixture was stirrred at room temperature for 30 minutes under hydrogen atmosphere. After the insoluble matter was filtered off, the residue obtained by evaporation of the solvent under reduced pressure was recrystallized from n-hexane/ethyl acetate to give the title compound(230mg, 47.0%) as a white solid.
¹H-NMR(DMSO-d₆): δ 2.59(3H, s), 5.35(2H, s), 7.32-7.36(3H, m), 7.43(1H, d, J=8.7Hz), 7.52-7.55(2H, m), 7.82(1H, s), 8.16(1H, dd, J=8.7, 2.4Hz), 8.25(1H, d, J=2.4Hz), 8.31(2H, s), 10.89(1H, s).

### Example 13: Preparation of the compound of Compound No. 13.

Sodium borohydride(23.6mg, 0.62mmol) was added to a suspension of 5-acetyl-N-[3,5-bis(trifluoromethyl)phenyl]-2-hydroxybenzamide(Compound No. 12; 50.5mg, 0.13mmol) in ethanol(2mL), and the mixture was stirred at room temperature for 12 hours. The reaction mixture was poured into diluted hydrochloric acid and extracted with ethyl acetate. After the ethyl acetate layer was washed with water and brine, dried over anhydrous sodium sulfate, the residue obtained by evaporation of the solvent under reduced pressure was washed with isopropyl ether/n-hexane under suspension to give the title compound(39.7mg, 78.3%) as a white powder.
¹H-NMR(DMSO-d₆): δ 1.34(3H, d, J=6.3Hz), 4.71(1H, q, J=6.3Hz), 5.18(1H, brs), 6.97(1H, d, J=8.4Hz), 7.44(1H, dd, J=8.4, 2.1Hz), 7.84(1H, s), 7.86(1H, d, J=2.1Hz), 8.48(2H, s), 10.85(1H, s), 11.32(1H, s).

### Example 14: Preparation of the compound of Compound No. 14.

Pyridine(45 *µ*L, 0.56mmol) and O-methylhydroxylamine hydrochloride(25.8mg, 0.31mmol) were added to a solution of 5-acetyl-N-[3,5-bis(trifluoromethyl)phenyl]-2-hydroxybenzamide(Compound No. 12; 100.0mg, 0.26mmol) in ethanol(3mL), and the mixture was refluxed for 1 hour. After the reaction mixture was cooled to room temperature, it was poured into diluted hydrochloric acid and extracted with ethyl acetate. After the ethyl acetate layer was washed with water and brine, dried over anhydrous sodium sulfate, the residue obtained by evaporation of the solvent under reduced pressure was purified by column chromatography on silica gel(n-hexane:ethyl acetate=4:1) to give the title compound(102.1mg, 95.3%) as a white crystal.
¹H-NMR(DMSO-d₆): δ 2.19(3H, s), 3.91(3H, s), 7.05(1H, d, J=8.7Hz),7.77(1H, dd, J=8.7, 2.4Hz), 7.85(1H, s), 8.09(1H, d, J=2.4Hz), 8.47(2H, s), 10.87(1H, s), 11.48(1H, s).

### Example 15: Preparation of the compound of Compound No. 15.

Using 5-acetyl-N-[3,5-bis(trifluoromethyl)phenyl]-2-hydroxybenzamide (Compound No. 12) and O-benzylhydroxylamine hydrochloride as the raw materials, the same operation as the Example 14 gave the title compound.
Yield: 79.9%.
¹H-NMR(DMSO-d₆): δ 2.24(3H, s), 5.20(2H, s), 7.04(1H, d, J=8.7Hz), 7.29-7.47(5H, m), 7.76(1H, dd, J=8.7, 2.4Hz), 7.85(1H, s), 8.07(1H, d, J=2.1Hz), 8.46(2H, s), 10.87(1H, s), 11.47(1H, s).

### Example 16: Preparation of the compound of Compound No. 16.

### (1) 5-(2,2-Dicyanoethen-1-yl)-2-hydroxybenzoic acid.

5-Formylsalicylic acid (332mg, 2mmol) was added to a solution of malononitrile(132mg, 2mmol) in ethanol(6mL). Benzylamine(0.1mL) was added under ice cooling and the mixture was stirred at room temperature for 2 hours. The separated yellow crystal was filtered and recrystallized from ethanol to give the title compound(139.9mg, 32.7%) as a light yellow solid.
¹H-NMR(DMSO-d₆): δ 7.12(1H, d, J=8.7Hz), 8.09(1H, dd, J=8.7, 2.4Hz), 8.41(1H, s), 8.50(1H, d, J=2.4Hz).

### (2) N-[3,5-Bis(trifluoromethyl)phenyl]-5-(2,2-dicyanoethen-1-yl)-2-hydroxybenzamide (Compound No. 16).

Using 5-(2,2-dicyanoethen-1-yl)-2-hydroxybenzoic acid and 3,5-bis(trifluoromethyl)aniline as the raw materials, the same operation as the Example 3 gave the title compound.
Yield: 9.1%.
¹H-NMR(DMSO-d₆): δ 7.13(1H, d, J=9.0Hz), 7.83(1H, s), 8.04(1H, dd, J=9.0, 2.4Hz), 8.36(1H, s), 8.38(1H, d, J=2.4Hz), 8.43(2H, s), 11.43(1H, s).

### Example 17: Preparation of the compound of Compound No. 17.

### (1) 5-[(2-Cyano-2-methoxycarbonyl)ethen-1-yl]-2-hydroxybenzoic acid.

A mixture of 5-formylsalicylic acid(332mg, 2mmol), Cyanoacetic acid methyl ester(198mg, 2mmol), acetic acid(6mL) and triethylamine(0.2ml) was refluxed for 5 hours. After the reaction mixture was cooled to room temperature, it was poured into water, and the separated crystal was filtered and recrystallized from n-hexane to give the title compound(327.7mg, 66.3%) as a light yellow solid.
¹H-NMR(DMSO-d₆): δ 3.85(3H, s), 7.15(1H, d, J=8.7Hz), 8.20(1H, dd, J=8.7, 2.4Hz), 8.37(1H, s), 8.66(1H, d, J=2.4Hz).

### (2) 3-({N-[3,5-Bis(trifluoromethyl)phenyl]carbamoyl}-4-hydroxyphenyl)-2-cyanoacrylic acid methyl ester(Compound No. 17).

Using 5-[(2-cyano-2-methoxycarbonyl)ethen-1-yl]-2-hydroxybenzoic acid and 3,5-bis(trifluoromethyl)aniline as the raw materials, the same operation as the Example 3 gave the title compound.
Yield: 66.3%.
¹H-NMR(DMSO-d₆): δ 3.85(3H, s), 7.19(1H, d, J=9.0Hz), 7.85(1H, s), 8.20(1H, dd, J=8.7, 2.1Hz), 8.33(1H, s), 8.45(2H, s), 8.50(1H, d, J=2.1Hz), 11.00(1H, s), 11.03(1H, s).

### Example 18: Preparation of the compound of Compound No. 18.

2N Sodium hydroxide(0.11ml, 0.22mmol) was added to a solution of 3-({N-[3,5-bis(trifluoromethyl)phenyl]carbamoyl}-4-hydroxyphenyl)-2-cyanoacrylic acid methyl ester(Compound No. 17; 50mg, 0.11mmol) in ethanol(5mL), and the mixture was stirred at room temperature for 3 hours. The reaction mixture was poured into diluted hydrochloric acid and extracted with ethyl acetate. After the organic layer was washed with brine, dried over anhydrous magnesium sulfate, the residue obtained by evaporation of the solvent under reduced pressure was recrystallized from ethyl acetate to give the title compound(13.5mg, 30.4%) as a light yellow solid.
¹H-NMR(DMSO-d₆): δ 7.12(1H, d, J=8.4Hz), 7.84(1H, s), 7.94(1H, dd, J=8.4, 2.1Hz), 8.38(1H, d, J=2.1Hz), 8.45(2H, s), 9.87(1H, s), 11.41(1H, s).

### Example 19: Preparation of the compound of Compound No. 19.

A mixture of N-[3,5-bis(trifluoromethyl)phenyl]-2-hydroxy-5-iodobenzamide (Compound No. 7; 475mg, 1mmol), styrene(130mg, 1.25mmol), palladium acetate(4.5mg, 0.02mmol), tris(ortho-tolyl)phosphine(12.2mg, 0.04mmol), diisopropylamine(388mg, 3mmol) and N,N-dimethylformamide(2mL) was refluxed for 8 hours. After the reaction mixture was cooled to room temperature, water was added and the mixture was extracted with ethyl acetate. After the ethyl acetate layer was washed successively with water and brine, dried over anhydrous magnesium sulfate, the residue obtained by evaporation of the solvent under reduced pressure was purified by column chromatography on silica gel(n-hexane:isopropyl ether=2:1→1:1) to give the title compound(173mg, 38.3%) as a pale yellow solid.
¹H-NMR(DMSO-d₆): δ 7.04(1H, d, J=8.4Hz), 7.20-7.29(3H, m), 7.38(2H, t, J=7.5Hz), 7.59(2H, d, J=7.5Hz), 7.72(1H, dd, J=8.4, 2.1Hz), 7.86(1H, s), 8.07(1H, d, J=2.1Hz), 8.49(2H, s), 10.89(1H, s), 11.33(1H, brs).

### Example 20: Preparation of the compound of Compound No. 20.

Tetrakis(triphenylphosphine)palladium(23mg, 0.02mmol) and cuprous iodide(4mg, 0.02mmol) were added to a solution of N-[3,5-bis(trifluoromethyl)phenyl]-2-hydroxy-5-iodobenzamide(Compound No. 7; 950mg, 2mmol), trimethylsilylacetylene(246mg, 2.5mmol) and triethylamine(2mL) in N,N-dimethylformamide(4mL) under argon atmosphere, and the mixture was stirred at 40°C for 2 hours. After the reaction mixture was cooled to room temperature, it was poured into ethyl acetate(100mL) and 1N citric acid(100mL), stirred, and filtered through celite. After the ethyl acetate layer was washed successively with water and brine, dried over anhydrous magnesium sulfate, the residue obtained by evaporation of the solvent under reduced pressure was purified by column chromatography on silica gel(n-hexane:ethyl acetate=19:1) and crystallized by n-hexane to give the title compound(286mg, 32.1%) as a white crystal.
¹H-NMR(DMSO-d₆): δ 0.23(9H, s), 7.00(1H, d, J=8.7Hz), 7.54(1H, dd, J=8.7, 2.4Hz), 7.85(1H, s), 7.98(1H, d, J=2.1Hz), 8.46(2H, s), 10.86(1H, s), 11.69(1H, s).

### Example 21: Preparation of the compound of Compound No. 21.

2N Sodium hydroxide(1mL) was added to a solution of N-[3,5-bis(trifluoromethyl)phenyl]-2-hydroxy-5-[(trimethylsilyl)ethynyl]benzamide (Compound No. 20; 233mg, 0.5mmol) in methanol(1mL), and the mixture was stirred at room temperature for 1 hour. The reaction mixture was poured into 2N hydrochloric acid and extracted with ethyl acetate. After the ethyl acetate layer was washed successively with water and brine, dried over anhydrous magnesium sulfate, the residue obtained by evaporation of the solvent under reduced pressure was recrystallized from ethanol/water to give the title compound(67mg, 35.9%) as a light gray crystal.
¹H-NMR(DMSO-d₆): δ 4.11(1H, s), 7.02(1H, d, J=8.4Hz), 7.55(1H, dd, J=8.4, 2.1Hz), 7.85(1H, s), 7.98(1H, d, J=2.1Hz), 8.46(2H, s), 8.46(2H, s), 10.86(1H, s), 11.62(1H, s).

### Example 22: Preparation of the compound of Compound No. 22.

Using N-[3,5-bis(trifluoromethyl)phenyl]-2-hydroxy-5-iodobenzamide (Compound No. 7) and phenylacetylene as the raw materials, the same operation as the Example 20 gave the title compound.
Yield: 40.8%.
¹H-NMR(DMSO-d₆): δ 7.06(1H, d, J=8.4Hz), 7.42-7.46(3H, m), 7.53-7.57(2H, m), 7.64(1H, dd, J=8.7, 2.1Hz), 7.86(1H, s), 8.06(1H, d, J=2.1Hz), 8.48(2H, s), 10.94(1H, s), 11.64(1H, brs).

### Example 23: Preparation of the compound of Compound No. 23.

Tetrakis(triphenylphosphine)palladium(16mg, 0.0014mmol) was added to a solution of N-[3,5-bis(trifluoromethyl)phenyl]-2-hydroxy-5-iodobenzamide(Compound No. 7; 200mg, 0.42mmol) in 1,2-dimethoxyethane(3mL) under argon atmosphere, and the mixture was stirred at room temperature for 5 minutes. Then dihydroxyphenylborane(57mg, 0.47mmol) and 1mol/L aqueous sodium carbonate(1.3mL) were added and the mixture was refluxed for 2 hours. After the reaction mixture was cooled to room temperature, it was poured into diluted hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=6:1→3:1) to give the title compound(109mg, 61.1%) as a white crystal.
¹H-NMR(DMSO-d₆): δ 7.12(1H, d, J=8.7Hz), 7.33-7.38(1H, m), 7.48(2H, t, J=7.5Hz), 7.67-7.70(2H, m), 7.79(1H, dd, J=8.4, 2.4Hz), 7.87(1H, s), 8.17(1H, d, J=2.4Hz), 8.49(2H, s), 10.92(1H, s), 11.41(1H, s).

### Example 24: Preparation of the compound of Compound No. 24.

Using N-[3,5-bis(trifluoromethyl)phenyl]-2-hydroxy-5-(phenylethynyl)-benzamide(Compound No. 22) as the raw material, the same operation as the Example 12(4) gave the title compound.
Yield: 86.2%.
¹H-NMR(DMSO-d₆): δ 2.88(4H, s), 6.93(1H, d, J=8.1Hz), 7.15-7.34(6H, m), 7.76(1H, d, J=2.4Hz), 7.84(1H, s), 8.47(2H, s), 10.79(1H, s), 11.15(1H, s).

### Example 25: Preparation of the compound of Compound No. 25.

Using 2-hydroxy-5-(trifluoromethyl)benzoic acid and 3,5-bis(trifluoromethyl)aniline as the raw materials, the same operation as the Example 3 gave the title compound.
Yield: 44.7%.
¹H-NMR(CDCl₃): δ 7.17(1H, d, J=9.0Hz) 7.72-7.75(2H, m), 7.86(1H, s), 8.17(2H, s), 8.35(1H, s) 11.88(1H, s).
[2-Hydroxy-5-(trifluoromethyl)benzoic acid: Refer to "Chemical and Pharmaceutical Bulletin", 1996, Vol.44, No.4, p.734-745.]

### Example 26: Preparation of the compound of Compound No. 26.

Using 2-hydroxy-5-(pentafluoroethyl)benzoic acid and 3,5-bis(trifluoromethyl)aniline as the raw materials, the same operation as the Example 3 gave the title compound.
Yield: 65.7%.
¹H-NMR(CDCl₃): δ 7.19(1H, d, J=9.0Hz) 7.70(1H, dd, J=8.7, 2.1Hz), 7.81(1H, d, J=2.1Hz), 8.17(2H, s), 8.37(1H, s), 11.92(1H, s).
[2-Hydroxy-5-(pentafluoroethyl)benzoic acid: Refer to "Chemical and Pharmaceutical Bulletin", 1996, Vol.44, No.4, p.734-745.]

### Example 27: Preparation of the compound of Compound No. 27.

Using 2-hydroxy-5-(pyrrol-1-yl)benzoic acid and 3,5-bis(trifluoromethyl)aniline as the raw materials, the same operation as the Example 3 gave the title compound.
Yield: 57.8%.
¹H-NMR(DMSO-d₆): δ 6.27(2H, dd, J=2.4, 1.8Hz), 7.10(1H, d, J=9.0Hz), 7.29(2H, dd, J=2.4, 1.8Hz), 7.66(1H, dd, J=9.0, 2.7Hz), 7.86(1H, s), 7.98(1H, d, J=2.4Hz), 8.47(2H, s), 10.89(1H, s), 11.24(1H, s).

### Example 28: Preparation of the compound of Compound No. 28.

Using N-[3,5-bis(trifluoromethyl)phenyl]-2-hydroxy-5-iodobenzamide (Compound No. 7) and 2-thiopheneboronic acid as the raw materials, the same operation as the Example 23 gave the title compound.
Yield: 44.4%.
¹H-NMR(DMSO-d₆): δ 7.08(1H, d, J=8.4Hz), 7.14(1H, dd, J=5.4, 3.6Hz), 7.45(1H, dd, J=3.6, 1.2Hz), 7.51(1H, dd, J=5.1, 0.9Hz), 7.75(1H, dd, J=8.4, 2.4Hz), 7.59(1H, s), 8.08(1H, d, J=2.4Hz), 8.48(2H, s), 10.91(1H, s), 11.38(1H, s).

### Example 29: Preparation of the compound of Compound No. 29.

Using N-[3,5-bis(trifluoromethyl)phenyl]-2-hydroxy-5-iodobenzamide (Compound No. 7) and 3-thiopheneboronic acid as the raw materials, the same operation as the Example 23 gave the title compound.
Yield: 38.7%.
¹H-NMR(DMSO-d₆): δ 7.06(1H, d, J=8.7Hz), 7.57(1H, dd, J=4.8, 1.5Hz), 7.66(1H, dd, J=4.8, 3.0Hz), 7.81-7.84(2H, m), 7.86(1H, s), 8.18(1H, d, J=2.1Hz), 8.49(2H, s), 10.90(1H, s), 11.33(1H, s).

### Example 30: Preparation of the compound of Compound No. 30.

### (1) 2-Benzyloxy-5-(2-bromoacetyl)-N-[3,5-bis(trifluoromethyl)phenyl]benzamide.

Phenyltrimethylammonium tribromide(3.75g, 10mmol) was added to a solution of 5-acetyl-2-benzyloxy-N-[3,5-bis(trifluoromethyl)phenyl]benzamide (compound of Example 12(3); 4.81g, 10mmol) in tetrahydrofuran(30ml), and the mixture was stirred at room temperature for 12 hours. The reaction mixture was poured into water and extracted with ethyl acetate. After the ethyl acetate layer was washed successively with aqueous sodium hydrogen sulfite, water and brine, dried over anhydrous magnesium sulfate, the residue obtained by evaporation of the solvent under reduced pressure was purified by column chromatography on silica gel(n-hexane:ethyl acetate=4:1), and recrystallized from ethyl acetate/n-hexane to give the title compound(2.39g, 42.7%) as a white solid.
¹H-NMR(DMSO-d₆): δ 4.91(2H, s), 5.36(2H, s), 7.32-7.35(3H, m), 7.47(1H, d, J=9.0Hz), 7.52-7.56(2H, m), 7.82(1H, s), 8.21(1H, dd, J=8.7, 2.4Hz), 8.29(1H, d, J=2.4Hz), 8.31(2H, s), 10.91(1H, s).

### (2) 2-Benzyloxy-N-[3,5-bis(trifluoromethyl)phenyl]-5-(2-methylthiazol-4-yl)benzamide.

A mixture of 2-benzyloxy-5-(2-bromoacetyl)-N-[3,5-bis(trifluoromethyl)-phenyl]benzamide(280mg, 0.5mmol), thioacetamide(41mg, 0.55mmol), sodium hydrogen carbonate(50mg, 0.6mmol) and ethanol(15mL) was refluxed for 1 hour. After the reaction mixture was cooled to room temperature, it was poured into water and extracted with ethyl acetate. After the ethyl acetate layer was washed successively with water and brine, dried over anhydrous magnesium sulfate, the residue obtained by evaporation of the solvent under reduced pressure was purified by column chromatography on silica gel(hexane:ethyl acetate=4:1) to give the title compound(181mg, 67.5%) as a white solid.
¹H-NMR(DMSO-d₆): δ 2.72(3H, s), 5.29(2H, s), 7.33-7.36(3H, m), 7.40(1H, d, J=9.0Hz), 7.54-7.57(2H, m), 7.81(1H, s), 7.94(1H, s), 8.12(1H, dd, J=8.7, 2.1Hz), 8.27(1H, d, J=2.1Hz), 8.31(2H, s), 10.86(1H, s).

### (3) N-[3,5-Bis(trifluoromethyl)phenyl]-2-hydroxy-5-(2-methylthiazol-4-yl)benzamide (Compound No. 30).

Ethanol(10ml) was added to 2-benzyloxy-N-[3,5-bis(trifluoromethyl)-phenyl]-5-(2-methylthiazol-4-yl)benzamide(160mg, 0.3mmol) and 10% palladium on carbon(240mg), and the mixture was stirred for 3.5 hours under hydrogen atmosphere. The reaction mixture was filtered and the solvent was evaporated under reduced pressure to give the title compound(103.4mg, 79.2%) as a white solid.
¹H-NMR(DMSO-d₆): δ 2.72(3H, s), 7.08(1H, d, J=8.7Hz), 7.83(1H, s), 7.85(1H, s), 8.01(1H, dd, J=8.7, 2.4Hz), 8.42(1H, d, J=2.1Hz), 8.50(2H, s), 10.96(1H, s), 11.40(1H, s).

### Example 31: Preparation of the compound of Compound No. 31.

A mixture of 2-benzyloxy-5-(2-bromoacetyl)-N-[3,5-bis(trifluoromethyl)-phenyl]benzamide (compound of Example 12(3); 280mg, 0.5mmol), 2-aminopyridine(51.8mg, 0.55mmol), sodium hydrogen carbonate(50mg, 0.6mmol) and ethanol(10mL) was refluxed for 2 hours. After the reaction mixture was cooled to room temperature, it was poured into aqueous sodium hydrogen carbonate and extracted with ethyl acetate. After the ethyl acetate layer was washed successively with water and brine, dried over anhydrous magnesium sulfate, the residue obtained by evaporation of the solvent under reduced pressure was purified by column chromatography on silica gel(hexane:ethyl acetate=1:2) to give a white solid(130.3mg, 45.9%). Then, a mixture of this solid(108mg, 0.19mmol), 10% palladium on carbon(11mg), ethanol(8mL) and ethyl acetate(8mL) was stirred for 7 hours under hydrogen atmosphere. The reaction mixture was filtered and the residue obtained by evaporation of the solvent under reduced pressur was purified by column chromatography on silica gel(n-hexane:ethyl acetate=1:3) to give the title compound(18.3mg, 20.2%) as a white solid.
¹H-NMR(DMSO-d₆): δ 6.90(1H, dt, J=6.6, 0.9Hz), 7.10(1H, d, J=8.7Hz), 7.25(1H, m), 7.57(1H, d, J=9.0Hz), 7.86(1H, s), 8.04(1H, dd, J=8.7, 2.1Hz), 8.35(1H, s), 8.48-8.56(4H, m), 11.00(1H, s), 11.41(1H, s).

### Example 32: Preparation of the compound of Compound No. 32.

### (1) N-[3,5-Bis(trifluoromethyl)phenyl]-5-iodo-2-methoxymethoxybenzamide.

A mixture of N-[3,5-bis(trifluoromethyl)phenyl]-2-hydroxy-5-iodobenzamide (Compound No. 7; 4.75g, 10mmol), chloromethyl methyl ether(1.14ml, 15mmol), potassium carbonate(2.76g, 20mmol) and acetone(50mL) was refluxed for 8 hours. After the reaction mixture was cooled to room temperature, it was poured into diluted hydrochloric acid and extracted with ethyl acetate. After the ethyl acetate layer was washed successively with water and brine, dried over anhydrous magnesium sulfate, the residue obtained by evaporation of the solvent under reduced pressure was purified by column chromatography on silica gel(hexane:ethyl acetate=3:1) and recrystallized from n-hexane/ethyl acetate to give the title compound(3.96g, 76.3%) as a white solid.
¹H-NMR(DMSO-d₆): δ 3.38(3H, s), 5.28(2H, s), 7.12(1H, d, J=9.0Hz), 7.81(1H, s), 7.82(1H, dd, J=8.7, 2.4Hz), 7.88(1H, d, J=2.4Hz), 8.40(2H, s), 10.87(1H, s).

### (2) N-[3,5-Bis(trifluoromethyl)phenyl]-2-methoxymethoxy-5-(pyridin-2-yl)benzamide.

Tri-n-butyl(2-pyridyl)tin (0.13ml, 0.41mmol) and dichlorobis-(triphenylphosphine)palladium(32.1mg, 0.05mmol) were added to a solution of N-[3,5-bis(trifluoromethyl)phenyl]-5-iodo-2-methoxymethoxybenzamide(0.20g, 0.39mmol) in N,N-dimethylformamide(8ml), and the mixture was stirred at 100°C for 1.5 hours. After the reaction mixture was cooled to room temperature, it was poured into water and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=2:1→1:1) to give the title compound(37.9mg, 20.8%) as a white powder.
¹H-NMR(CDCl₃): δ 3.64(3H, s), 5.53(2H, s), 7.23-7.28(1H, m),7.36(1H, d, J=8.7Hz), 7.65(1H, s), 7.77-7.84(2H, m), 8.20(2H, s), 8.31(1H, dd, J=8.7, 2.4Hz), 8.68-8.70(1H, m), 8.83(1H, d, J=2.4Hz), 10.12(1H, s).

### (3) N-[3,5-Bis(trifluoromethyl)phenyl]-2-hydroxy-5-(pyridin-2-yl)benzamide (Compound No. 32).

Methanol(3ml) and concentrated hydrochloric acid(0.5ml) were added to N-[3,5-bis(trifluoromethyl)phenyl]-2-methoxymethoxy-5-(pyridin-2-yl)benzamide(37.9 mg, 0.08mmol), and the mixture was refluxed for 2 hours. After the reaction mixture was cooled to room temperature, it was poured into saturated aqueous sodium hydrogen carbonate and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=2:1) to give the title compound(16.2mg, 47.2%) as a white powder.
¹H-NMR(DMSO-d₆): δ 7.13(1H, d, J=8.4Hz), 7.33(1H, ddd, J=7.5, 6.3, 1.2Hz), 7.86-7.91(2H, m), 7.97(1H, d, J=7.8Hz), 8.20(1H, dd, J=8.7, 2.1Hz), 8.50(2H, s), 8.59(1H, d, J=2.4Hz), 8.64-8.66(1H, m), 10.97(1H, s), 11.53(1H, s).

### Example 33: Preparation of the compound of Compound No. 33.

Using 5-methoxysalicylic acid and 3,5-bis(trifluoromethyl)aniline as the raw materials, the same operation as the Example 3 gave the title compound.
Yield: 56.8%.
¹H-NMR(DMSO-d₆): δ 3.77(3H, s), 6.97(1H, d, J=9.0Hz), 7.10(1H, dd, J=9.0, 3.0Hz), 7.43(1H, d, J=3.0Hz), 7.84(1H, s), 8.47(2H, s), 10.84(1H, s), 10.91(1H, s).

### Example 34: Preparation of the compound of Compound No. 34.

### (1) 5-Acetyl-2-methoxybenzoic acid methyl ester.

Methyl iodide(2.5mL, 40.1mmol) was added to a mixture of 5-acetylsalicylic acid methyl ester(5.00g, 25.7mmol), sodium carbonate(7.10g, 51.4mmol) and N,N-dimethylformamide(25mL) under ice cooling, and the mixture was stirred at room temperature for 3 hours. The reaction mixture was poured into water, neutralized by hydrochloric acid, and extracted with ethyl acetate. After the ethyl acetate layer was washed successively with water and brine, dried over anhydrous sodium sulfate, the residue obtained by evaporation of the solvent under reduced pressure was washed under suspension(isopropyl ether/n-hexane) to give the title compound(5.17g, 96.5%) as a white crystal.
¹H-NMR(CDCl₃): δ 2.59(3H, s), 3.92(3H, s), 3.99(3H, s), 7.04(1H, d, J=8.7Hz), 8.12(1H, dd, J=8.7, 2.4Hz), 8.41(1H, d, J=2.4Hz).

### (2) 5-Isobutyryl-2-methoxybenzoic acid methyl ester.

Methyl iodide(0.5mL, 8.03mmol) was added to a mixture of 5-acetyl-2-methoxybenzoic acid methyl ester(0.50g, 2.40mmol), potassium tert-butoxide(0.81g, 7.22mmol) and tetrahydrofuran(10mL), and the mixture was stirred at room temperature for 1 hour. The reaction mixture was poured into water, neutralized by hydrochloric acid, and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate= 3:1→2:1) to give the title compound(143.1mg, 25.2%) as a light yellow oil.
¹H-NMR(CDCl₃): δ 1.22(6H, d, J=6.9Hz), 3.52(1H, m), 3.92(3H, s), 3.98(3H, s), 7.05(1H, d, J=8.7Hz), 8.13(1H, dd, J=8.7, 2.4Hz), 8.42(1H, d, J=2.4Hz).

### (3) 5-Isobutyryl-2-methoxybenzoic acid.

2N Aqueous sodium hydroxide(1mL) was added to a solution of 5-isobutyryl-2-methoxybenzoic acid methyl ester(143.1mg, 0.60mmol) in methanol(5mL), and the mixture was refluxed for 1 hour. After the reaction mixture was cooled to room temperature, it was poured into 2N hydrochloric acid and extracted with ethyl acetate. After the ethyl acetate layer was washed successively with water and brine, dried over anhydrous sodium sulfate, the solvent was evaporated under reduced pressure to give the title compound(134mg, quantitative) as a white crystal.
¹H-NMR(CDCl₃): δ 1.22(6H, d, J=6.9Hz), 3.59(1H, m), 4.15(3H, s), 7.16(1H, d, J=8.7Hz), 8.24(1H, dd, J=8.7, 2.4Hz), 8.73(1H, d, J=2.1Hz).

### (4) 5-Isobutyryl-N-[3,5-bis(trifluoromethyl)phenyl]-2-methoxybenzamide.

Using 5-isobutyryl-2-methoxybenzoic acid and 3,5-bis(trifluoromethyl)aniline as the raw materials, the same operation as the Example 3 gave the title compound.
Yield: 61.4%.
¹H-NMR(CDCl₃): δ 1.23(6H, d, J=6.9Hz), 3.64(1H, m), 4.20(3H, s), 7.18(1H, d, J=8.7Hz), 7.65(1H, s), 8.19(2H, s), 8.22(1H, dd, J=8.7, 2.1Hz), 8.88(1H, d, J=2.1Hz), 9.98(1H, s).

### (5) N-[3,5-Bis(trifluoromethyl)phenyl]-2-hydroxy-5-isobutyrylbenzamide(Compound No. 34).

A mixture of 5-isobutyryl-N-[3,5-bis(trifluoromethyl)phenyl]-2-methoxybenzamide(143.4mg, 0.33mmol), 2,4,6-collidine(3ml) and lithium iodide(53.1mg, 0.40mmol) was refluxed for 1 hour. After the reaction mixture was cooled to room temperature, it was poured into 2N hydrochloric acid and extracted with ethyl acetate. After the ethyl acetate layer was washed with brine, dried over anhydrous sodium sulfate, the residue obtained by evaporation of the solvent under reduced pressure was purified by column chromatography on silica gel(n-hexane:ethyl acetate=3:1) and crystallized by ethyl acetate/isopropyl ether to give the title compound(90.3mg, 65.3%) as a white crystal.
¹H-NMR(DMSO-d₆): δ 1.12(6H, d, J=6.9Hz), 3.66(1H, m), 7.12(1H, d, J=8.4Hz), 7.85(1H, s), 8.07(1H, dd, J=8.4, 2.4Hz), 8.45(1H, d, J=2.4Hz), 8.47(2H, s), 10.93(1H, s), 11.95(1H, brs).

### Example 35: Preparation of the compound of Compound No. 35.

Using 4-hydroxyisophthalic acid 1-methyl ester and 3,5-bis(trifluoromethyl)aniline as the raw materials, the same operation as the Example 3 gave the title compound.
Yield: 91.5%.
¹H-NMR(DMSO-d₆): δ 3.85(3H, s), 7.12(1H, d, J=8.4Hz), 7.86(1H, s), 8.02(1H, dd, J=8.7, 2.4Hz), 8.46-8.47(3H, m), 10.96(1H, s), 12.03(1H, brs).
[4-Hydroxyisophthalic acid 1-methyl ester: Refer to "Journal of the Chemical Society", (England), 1956, p.3099-3107.]

### Example 36: Preparation of the compound of Compound No. 36.

2N Aqueous sodium hydroxide(14mL) was added to a suspension of N-[3,5-bis(trifluoromethyl)phenyl]-4-hydroxyisophthalamic acid methyl ester(Comound No. 35; 2.85g, 7mmol) in a mixed solvent of methanol/tetrahydrofuran(14mL+14mL), and the mixture was refluxed for 2 hours. After the reaction mixture was cooled to room temperature, 2N hydrochloric acid(20ml) was added and the separated solid was filtered, washed with water, dried to give the title compound(2.68g, 97.4%) as a white crystal.
¹H-NMR(DMSO-d₆): δ 7.10(1H, d, J=8.7Hz), 7.82(1H, s), 7.86(1H, s), 8.01(1H, dd, J=8.7, 2.4Hz), 8.47(2H, s), 8.48(1H, d, J=2.4Hz), 10.97(1H, s), 11.98(1H, brs).

When the method described in Example 36 is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### Example 37: Preparation of the compound of Compound No. 37.

Using 4-hydroxyisophthalic acid(182mg, 1mmol), 3,5-bis(trifluoromethyl)-aniline(687mg, 3mmol), phosphorus trichloride(87 µL; 1mmol) and toluene(10mL), the same operation as the Example 3 gave the title compound(151mg, 25.0%) as a white crystal.
¹H-NMR(DMSO-d₆): δ 7.18(1H, d, J=8.7Hz), 7.82(1H, s), 7.86(1H, s), 8.11(1H, dd, J=8.7, 2.4Hz), 8.50(2H, s), 8.54(2H, s), 8.56(1H, d, J=2.4Hz), 10.79(1H, s), 10.99(1H, s), 11.84(1H, brs).

### Example 38: Preparation of the compound of Compound No. 38.

### (1) 4-Benzyloxy-N-[3,5-bis(trifluoromethyl)phenyl]isophthalamic acid methyl ester.

A solution of N-[3,5-bis(trifluoromethyl)phenyl]-4-hydroxyisophthalamic acid methyl ester(Compound No. 35; 8.15g, 20mmol) in N,N-dimethylformamide(100mL) was added to a suspension of sodium hydride(60%; 1.04g, 26mmol) in N,N-dimethylformamide(100mL) under ice cooling, and the mixture was stirred at room temperature for 1 hour. A solution of benzyl bromide(4.45g, 26mmol) in N,N-dimethylformamide(10mL) was added and the mixture was stirred at 60°C for 3 hours. After the reaction mixture was cooled to room temperature, it was poured into ice and water, and extracted with ethyl acetate. After the ethyl acetate layer was washed successively with water and brine, dried over anhydrous magnesium sulfate, the residue obtained by evaporation of the solvent under reduced pressure was recrystallized from ethyl acetate/n-hexane to give the title compound(5.38g, 54.1%) as a white solid.
¹H-NMR(DMSO-d₆): δ 3.87(3H, s), 5.33(2H, s), 7.33-7.36(3H, m), 7.46(1H, d, J=8.7Hz), 7.53-7.56(2H, m), 7.82(1H, s), 8.15(1H, dd, J=8.7, 2.1Hz), 8.25(1H, d, J=2.1Hz)8.28(2H, s), 10.87(1H, s).

### (2) 4-Benzyloxy-N-[3,5-bis(trifluoromethyl)phenyl]isophthalamic acid.

Using 4-benzyloxy-N-[3,5-bis(trifluoromethyl)phenyl]isophthalamic acid methyl ester as the raw material, the same operation as the Example 36 gave the title compound.
Yield: 79.7%.
¹H-NMR(DMSO-d₆): δ 5.32(2H, s), 7.32-7.34(3H, m), 7.43(1H, d, J=8.7Hz), 7.52-7.56(2H, m), 7.81(1H, s), 8.12(1H, dd, J=8.7, 2.1Hz), 8.22(1H, d, J=2.1Hz), 8.28(2H, s), 10.85(1H, s), 13.81(1H, brs).

### (3) 4-Benzyloxy-N³-[3,5-bis(trifluoromethyl)phenyl]-N¹,N¹-dimethylisophthalamide.

1-(3-Dimethylaminopropyl)-3-ethylcarbodiimide hydrochloride (hereinafter abbreviated as WSC · HCl; 95mg, 0.50mmol) was added to a solution of 4-benzyloxy-N-[3,5-bis(trifluoromethyl)phenyl]isophthalamic acid(242mg, 0.50mmol), dimethylamine hydrochloride(41mg, 0.50mmol) and triethylamine(51mg, 0.50mmol) in tetrahydrofuran(5mL) under ice cooling, and the mixture was stirred at room temperature for 3 hours. The reaction mixture was poured into water and extracted with ethyl acetate. After the ethyl acetate layer was washed successively with diluted hydrochloric acid, water and brine, dried over anhydrous magnesium sulfate, the residue obtained by evaporation of the solvent under reduced pressure was purified by column chromatography on silica gel(hexane:ethyl acetate=1:4) to give the title compound(165mg, 64.9%) as a white solid.
¹H-NMR(DMSO-d₆): δ 2.99(6H, s)5.29(2H, s), 7.32-7.38(4H, m), 7.52-7.56(2H, m), 7.64(1H, dd, J=8.7, 2.1Hz), 7.73(1H, d, J=2.1Hz), 7.80(1H, s), 8.28(2H, s), 10.83(1H, s).

When the method described in Example 38(3) is referred in the following examples, organic bases such as pyridine, triethylamine or the like were used as the base. As the reaction solvent, solvents such as dichloromethane, tetrahydrofuran or the like were used alone or as a mixture.

### (4) N³-[3,5-bis(trifluoromethyl)phenyl]-4-hydroxy-N¹,N¹-dimethylisophthalamide (Compound No. 38).

A mixture of 4-benzyloxy-N³-[3,5-bis(trifluoromethyl)phenyl]-N¹,N¹-dimethylisophthalamide(141mg, 0.28mmol), 5% palladium on carbon(14mg), ethanol(5ml) and ethyl acetate(5ml) was stirred at room temperature for 1 hour under hydrogen atmosphere. The reaction mixture was filtered and the filtrate was evaporated under reduced pressure to give the title compound(106mg, 91.2%) as a white solid.
¹H-NMR(DMSO-d₆): δ 2.98(6H, s), 7.02(1H, d, J=8.7Hz), 7.52(1H, dd, J=8.7, 2.1Hz), 7.84(1H, s), 7.95(1H, d, J=2.1Hz), 8.46(2H, s), 11.10(1H, brs), 11.63(1H, brs).

### Example 39: Preparation of the compound of Compound No. 39.

### (1) 2-Benzyloxy-N-[3,5-bis(trifluoromethyl)phenyl]-5-(piperidine-1-carbonyl)-benzamide.

Using 4-benzyloxy-N-[3,5-bis(trifluoromethyl)phenyl]isophthalamic acid(compound of Example 38(2)) and piperidine as the raw materials, the same operation as the Example 38(3) gave the title compound.
Yield: 56.4%.
¹H-NMR(CDCl₃): δ 1.53-1.70(6H, m), 3.44(2H, brs), 3.70(2H, brs), 5.26(2H, s), 7.24(1H, d, J=8.7Hz), 7.26(1H, s), 7.52-7.58(5H, m), 7.66(2H, s), 7.74(1H, dd, J=8.7, 2.4Hz), 8.37(1H, d, J=2.1Hz), 10.27(1H, s).

### (2) N-[3,5-Bis(trifluoromethyl)phenyl]-2-hydroxy-5-(piperidine-1-carbonyl)benzamide (Compound No. 39).

Using 2-benzyloxy-N-[3,5-bis(trifluoromethyl)phenyl]-5-(piperidine-1-carbonyl)benzamide as the raw material, the same operation as the Example 38(4) gave the title compound.
Yield: 96.3%, white solid.
¹H-NMR(DMSO-d₆): δ 1.51(4H, brs), 1.60-1.65(2H, m), 3.47(4H, brs), 7.04(1H, d, J=8.4Hz), 7.48(1H, dd, J=8.4, 2.1Hz), 7.85(1H, s), 7.92(1H, d, J=2.1Hz), 8.46(2H, s), 10.99(1H, s), 11.64(1H, brs).

### Example 40: Preparation of the compound of Compound No. 40.

### (1) 2-Benzyloxy-5-(4-benzylpiperidine-1-carbonyl)-N-[3,5-bis(trifluoromethyl)phenyl]-benzamide.

Using 4-benzyloxy-N-[3,5-bis(trifluoromethyl)phenyl]isophthalamic acid(compound of Example 38(2)) and 4-benzylpiperidine as the raw materials, the same operation as the Example 38(3) gave the title compound.
Yield: 76.7%.
¹H-NMR(CD₃OD): δ 1.18-1.38(2H, m), 1.67(1H, brs), 1.74(1H, brs), 1.84-1.93(1H, m), 2.60(2H, d, J=7.2Hz), 2.83(1H, brs), 3.10(1H, brs), 3.78(1H, brs), 4.59(1H, brs), 5.34(2H, s), 7.15-7.18(3H, m), 7.24-7.28(2H, m), 7.40-7.46(4H, m), 7.57-7.63(3H, m), 7.65(1H, dd, J=8.7, 2.4Hz), 7.96(2H, s), 8.05(1H, d, J=2.1Hz).

### (2) N-[3,5-Bis(trifluoromethyl)phenyl]-2-hydroxy-5-(4-benzylpiperidine-1-carbonyl)-benzamide(Compound No. 40).

Using 2-benzyloxy-5-(4-benzylpiperidine-1-carbonyl)-N-[3,5-bis-(trifluoromethyl)phenyl]-benzamide as the raw material, the same operation as the Example 38(4) gave the title compound.
Yield: 54.3%, white solid.
¹H-NMR(DMSO-d₆): δ 1.08-1.22(2H, m), 1.59-1.62(2H, m), 1.77-1.80(1H, m), 2.50-2.55(2H, m), 2.87(2H, brs), 3.75(1H, br), 4.39(1H, br), 7.06(1H, d, J=8.4Hz), 7.17-7.20(3H, m), 7.28(2H, t, J=7.2Hz), 7.49(1H, dd, J=8.4, 2.1Hz), 7.84(1H, s), 7.93(1H, d, J=2.1Hz), 8.47(2H, s), 10.89(1H, s), 11.65(1H, s).

### Example 41: Preparation of the compound of Compound No. 41.

### (1) 2-Methoxy-5-sulfamoylbenzoic acid.

2N Aqueous sodium hydroxide(30mL, 60mmol) was added to a solution of methyl 2-methoxy-5-sulfamoylbenzoate(4.91g, 20mmol) in methanol(30mL), and the mixture was stirred at room temperature for 1 hour. The reaction mixture was poured into 2N hydrochloric acid, and the separated solid was filtered to give the title compound(4.55g, 98.3%) as a white solid.
¹H-NMR(DMSO-d₆): δ 3.89(3H, s), 7.30(1H, d, J=8.7Hz), 7.32(2H, s), 7.92(1H, dd, J=8.7, 2.7Hz), 8.09(1H, d, J=2.7Hz), 13.03(1H, br).

### (2) N-[3,5-Bis(trifluoromethyl)phenyl]-2-methoxy-5-sufamoylbenzamide.

Using 2-methoxy-5-sulfamoylbenzoic acid and 3,5-bis(trifluoromethyl)aniline as the raw materials, the same operation as the Example 12(3) gave the title compound.
Yield: 24.2%.
¹H-NMR(DMSO-d₆): δ 3.97(3H, s), 7.38(2H, s), 7.39(1H, d, J=8.7Hz), 7.85(1H, s), 7.96(1H, dd, J=8.7, 2.4Hz), 8.06(1H, d, J=2.4Hz), 8.43(2H, s), 10.87(1H, s).

### (3) N-[3,5-Bis(trifluoromethyl)phenyl]-5-dimethylsufamoyl-2-methoxybenzamide.

A suspension of N-[3,5-bis(trifluoromethyl)phenyl]-2-methoxy-5-sufamoylbenzamide(442mg, 1.0mmol), methyl iodide(710mg, 5.0mmol), sodium carbonate(415mg, 3.0mmol) and acetonitrile(10mL) was refluxed for 3 hours. After the reaction mixture was cooled to room temperature, it was poured into water and extracted with ethyl acetate. After the ethyl acetate layer was washed successively with water and brine, dried over anhydrous magnesium sulfate, the residue obtained by evaporation of the solvent under reduced pressure was recrystallized from n-hexane/ethyl acetate to give the title compound(207mg, 44.1%) as a white solid.
¹H-NMR(DMSO-d₆): δ 2.62(6H, s), 3.99(3H, s), 7.45(1H, d, J=9.0Hz), 7.85(1H, s), 7.91(1H, dd, J=8.7, 2.4Hz), 7.95(1H, d, J=2.4Hz)8.43(2H, s), 10.90(1H, s).

### (4) N-[3,5-Bis(trifluoromethyl)phenyl]-5-dimethylsufamoyl-2-hydroxybenzamide (Compound No. 41).

Using N-[3,5-bis(trifluoromethyl)phenyl]-5-dimethylsufamoyl-2-methoxybenzamide as the raw material, the same operation as the Example 34(5) gave the title compound.
Yield: 45.5%.
¹H-NMR(DMSO-d₆):d 2.61(6H, s), 7.20(1H, d, J=8.7Hz), 7.77(1H, dd, J=8.7, 2.1Hz), 7.86(1H, s), 8.14(1H, d, J=2.1Hz)8.45(2H, s), 11.16(1H, s), 12.15(1H, br).

### Example 42: Preparation of the compound of Compound No. 42.

### (1) N-[3,5-Bis(trifluoromethyl)phenyl]-2-methoxy-5-(pyrrole-1-sulfonyl)benzamide.

A mixture of N-[3,5-bis(trifluoromethyl)phenyl]-2-methoxy-5-sulfamoylbenzamide(compound of Example 41(2); 442mg, 1mmol), 2,5-dimethoxytetrahydrofuran(159mg, 1.2mmol) and acetic acid(5mL) was refluxed for 2 hours. After the reaction mixture was cooled to room temperature, it was poured into water and extracted with ethyl acetate. After the ethyl acetate layer was washed successively with water, saturated aqueous sodium hydrogen carbonate and brine, dried over anhydrous magnesium sulfate, the residue obtained by evaporation of the solvent under reduced pressure was purified by column chromatography on silica gel(n-hexane:ethyl acetate=3:2) to give the title compound(436.5mg, 88.6%) as a white solid.
¹H-NMR(DMSO-d₆): δ 3.96(3H, s), 6.36(2H, dd, J=2.4, 2.1Hz), 7.37(2H, dd, J=2.4, 2.1Hz), 7.42(1H, d, J=9.0Hz), 7.85(1H, s), 8.80(1H, dd, J=9.0, 2.4Hz)8.18(1H, d, J=2.7Hz), 8.38(2H, s), 10.92(1H, s).

### (2) N-[3,5-Bis(trifluoromethyl)phenyl]-2-hydroxy-5-(pyrrole-1-sulfonyl)benzamide (Compound No. 42).

Using N-[3,5-bis(trifluoromethyl)phenyl]-2-methoxy-5-(pyrrole-1-sulfonyl)benzamide as the raw material, the same operation as the Example 34(5) gave the title compound.
Yield: 79.4%.
¹H-NMR(DMSO-d₆) δ 6.36(2H, dd, J=2.4, 2.1Hz), 7.18(1H, d, J=9.0Hz), 7.34(2H, dd, J=2.4, 2.1Hz), 7.86(1H, s), 7.99(1H, dd, J=9.0, 2.7Hz)8.31(1H, d, J=2.7Hz), 8.42(2H, s), 10.98(1H, s).

### Example 43: Preparation of the compound of Compound No. 43.

Using N-[3,5-bis(trifluoromethyl)phenyl]-2-hydroxy-5-nitrobenzamide (Compound No. 8) as the raw material, the same operation as the Example 38(4) gave the title compound.
Yield: 98.0%.
¹H-NMR(DMSO-d₆): δ 4.79(2H, brs), 6.76(1H, d, J=2.1Hz), 6.76(1H, s), 7.09(1H, dd, J=2.1, 1.2Hz), 7.80(1H, s), 8.45(2H, s), 10.30(1H, br), 10.84(1H, s).

### Example 44: Preparation of the compound of Compound No. 44.

Using 5-dimethylaminosalicylic acid and 3,5-bis(trifluoromethyl)aniline as the raw materials, the same operation as the Example 3 gave the title compound.
Yield: 28.8%.
¹H-NMR(DMSO-d₆): δ 2.85(6H, s), 6.92(1H, d, J=9.0Hz), 7.01(1H, dd, J=8.7, 3.0Hz), 7.22(1H, d, J=3.0Hz), 7.84(1H, s), 8.47(2H, s), 10.62(1H, s), 10.83(1H, s).

### Example 45: Preparation of the compound of Compound No. 45.

Benzoyl chloride(155mg, 1.1mmol) was added to a mixture of 5-amino-N-[3,5-bis(trifluoromethyl)phenyl]-2-hydroxybenzamide(Compound No. 43; 364mg, 1mmol), pyridine(95mg, 1.2mmol) and tetrahydrofuran(10mL) under ice cooling, and the mixture was stirred for 1 hour. The reaction mixture was poured into water and extracted with ethyl acetate. After the ethyl acetate layer was washed successively with water and brine, dried over anhydrous magnesium sulfate, the residue obtained by evaporation of the solvent under reduced pressure was purified by column chromatography on silica gel(n-hexane:ethyl acetate=4:1) to give the title compound(121mg, 25.7%) as a white solid.
¹H-NMR(DMSO-d₆): δ 7.04(1H, d, J=8.7Hz), 7.51-7.62(3H, m), 7.81(1H, dd, J=8.7, 2.4Hz), 7.83(1H, s), 7.98(2H, d, J=7.2Hz), 8.22(1H, d, J=2.4Hz), 8.49(2H, s), 10.27(1H, s), 10.89(1H, s), 11.07(1H, s).

### Example 46: Preparation of the compound of Compound No. 46.

4-Dimethylaminopyridine(3mg) and phenylisocyanate(30 µ L, 0.28mmol) were added to a solution of 5-amino-N-[3,5-bis(trifluoromethyl)phenyl]-2-hydroxybenzamide(Compound No. 43; 100.2mg, 0.28mmol) in acetonitrile(4ml), and the mixture was stirred at 60°C for 5 minutes. After the reaction mixture was cooled to room temperature, the residue obtained by evaporation of the solvent under reduced pressure was purified by column chromatography on silica gel(n-hexane:ethyl acetate=1:1) to give the title compound(54.8mg, 41.2%) as a light brown solid.
¹H-NMR(DMSO-d₆): δ 6.93-6.98(1H, m), 6.97(1H, d, J=9.3Hz),7.27(2H, t, J=7.8Hz), 7.34-7.46(2H, m), 7.50(1H, dd, J=9.0, 2.4Hz), 7.83(1H, s), 7.88(1H, s), 8.47(2H, s), 8.56(1H, s), 8.63(1H, s), 10.87(1H, s), 10.89(1H, s).

### Example 47: Preparation of the compound of Compound No. 47.

Using 5-amino-N-[3,5-bis(trifluoromethyl)phenyl]-2-hydroxybenzamide (Compound No. 43) and phenylisothiocyanate as the raw materials, the same operation as the Example 46 gave the title compound.
Yield: 66.3%.
¹H-NMR(DMSO-d₆): δ 7.00(1H, d, J=8.4Hz), 7.13(1H, tt, J=7.5, 1.2Hz),7.34(2H, t, J=7.8Hz), 7.45-7.51(3H, m), 7.84(1H, s), 7.87(1H, d, J=2.7Hz), 8.47(2H, s), 9.65(1H, s), 9.74(1H, s), 10.84(1H, s), 11.32(1H, s).

### Example 48: Preparation of the compound of Compound No. 48.

Using 5-[(4-nitrophenyl)diazenyl]salicylic acid and 3,5-bis(trifluoromethyl)aniline as the raw materials, the same operation as the Example 3 gave the title compound.
Yield: 11.3%.
¹H-NMR(DMSO-d₆): δ 7.23(1H, d, J=9.0Hz), 7.87(1H, s),8.06(2H, d, J=9.0Hz), 8.10(1H, dd, J=9.0, 2.4Hz), 8.44(2H, d, J=9.0Hz), 8.50(2H, s), 8.53(1H, d, J=2.4Hz), 11.13(1H, s), 12.14(1H, br).

### Example 49: Preparation of the compound of Compound No. 49.

Using 5-({[(4-pyridin-2-yl)sulfamoyl]phenyl}diazenyl)salicylic acid and 3,5-bis(trifluoromethyl)aniline as the raw materials, the same operation as the Example 3 gave the title compound.
Yield: 7.9%.
¹H-NMR(DMSO-d₆): δ 6.87(1H, t, J=6.0Hz), 7.22(1H, d, J=8.7Hz), 7.21-7.23(1H, m), 7.77(1H, t, J=8.4Hz), 7.87(1H, s), 7.95-7.98(3H, m), 8.03-8.07(4H, m), 8.47(1H, d, J=2.4Hz), 8.49(2H, s), 11.14(1H, s), 12.03(1H, br).

### Example 50: Preparation of the compound of Compound No. 50.

### (1) 4-Acetylamino-5-chloro-2-methoxybenzoic acid.

Using 4-acetylamino-5-chloro-2-methoxybenzoic acid methyl ester as the raw material, the same operation as the Example 36 gave the title compound.
Yield: 88.0%.
¹H-NMR(DMSO-d₆): δ 2.16(3H, s), 3.78(3H, s), 7.72(1H, s), 7.77(1H, s), 9.57(1H, s), 12.74(1H, s).

### (2) 4-Acetylamino-N-[3,5-bis(trifluoromethyl)phenyl]-5-chloro-2-methoxybenzamide.

Using 4-acetylamino-5-chloro-2-methoxybenzoic acid and 3,5-bis(trifluoromethyl)aniline as the raw materials, the same operation as the Example 12(3) gave the title compound.
Yield: 23.8%.
¹H-NMR(DMSO-d₆): δ 2.17(3H, s), 3.89(3H, s), 7.77-7.82(3H, m), 8.45-8.49(2H, m), 9.66(1H, s), 10.68(1H, s).

### (3) 4-Acetylamino-N-[3,5-bis(trifluoromethyl)phenyl]-5-chloro-2-hydoxybenzamide (Compound No. 50).

Using 4-acetylamino-N-[3,5-bis(trifluoromethyl)phenyl]-5-chloro-2-methoxybenzamide as the raw material, the same operation as the Example 34(5) gave the title compound.
Yield: 72.8%.
¹H-NMR(DMSO-d₆): δ 2.17(3H, s), 7.75(1H, s), 7.82(1H, s), 7.95(1H, s), 8.44(2H, s), 9.45(1H, s), 11.16(1H, brs), 11.63(1H, brs).

### Example 51: Preparation of the compound of Compound No. 51.

Using 4-chlorosalicylic acid and 3,5-bis(trifluoromethyl)aniline as the raw materials, the same operation as the Example 3 gave the title compound.
Yield: 55.8%.
¹H-NMR(DMSO-d₆): δ 7.05-7.08(2H, m), 7.84-7.87(2H, m), 8.45(2H, s), 10.84(1H, s)11.64(1H, brs).

### Example 52: Preparation of the compound of Compound No. 52.

Using 6-hydroxysalicylic acid and 3,5-bis(trifluoromethyl)aniline as the raw materials, the same operation as the Example 3 gave the title compound.
Yield: 86.9%.
¹H-NMR(DMSO-d₆): δ 6.36(2H,d,J=8.4Hz), 7.13(1H,t,J=8.4Hz),7.79(1H, s),8.38(2H, s),11.40(2H,brs),11.96(1H, brs).

### Example 53: Preparation of the compound of Compound No. 53.

Using 4-methylsalicylic acid and 3,5-bis(trifluoromethyl)aniline as the raw materials, the same operation as the Example 3 gave the title compound.
Yield: 42.9%.
¹H-NMR(DMSO-d₆): δ 2.32(3H, s)6.82(1H, d, J=6.6Hz)6.84(1H, s)7.83(1H, s)7.84(1H, d, J=8.5Hz)8.47(2H, s)10.76(1H, s)11.44(1H, s).

### Example 54: Preparation of the compound of Compound No. 54.

Using 5-bromo-4-hydroxysalicylic acid and 3,5-bis(trifluoromethyl)aniline as the raw materials, the same operation as the Example 3 gave the title compound.
Yield: 82.4%.
¹H-NMR(CDCl₃): δ 5.89(1H, s)6.70(1H, s)7.69(2H, s)7.95(1H, s)8.12(2H, s)11.62(1H, s).

### Example 55: Preparation of the compound of Compound No. 55.

Using 4-hydroxysalicylic acid and 3,5-bis(trifluoromethyl)aniline as the raw materials, the same operation as the Example 3 gave the title compound.
Yield: 29.9%.
¹H-NMR(DMSO-d₆): δ 6.37(1H, d, J=2.5Hz), 6.42(1H, dd, J=8.8, 2.5Hz), 7.81(1H, s), 7.86(1H, d, J=8.5Hz), 8.44(2H, s), 10.31(1H, s), 10.60(1H, s), 11.77(1H, s).

### Example 56: Preparation of the compound of Compound No. 56.

Using 3,5-dichlorosalicylic acid and 3,5-bis(trifluoromethyl)aniline as the raw materials, the same operation as the Example 3 gave the title compound.
Yield: 44.8%.
¹H-NMR(DMSO-d₆): δ 7.85(1H, d, J=2.5Hz), 7.91(1H, s), 8.01(1H, d, J=2.5Hz), 8.42(2H, s), 11.10(1H, s).

### Example 57: Preparation of the compound of Compound No. 57.

Using 3-hydroxysalicylic acid and 3,5-bis(trifluoromethyl)aniline as the raw materials, the same operation as the Example 3 gave the title compound.
Yield: 22.7%.
¹H-NMR(DMSO-d₆): δ 6.81(1H, t, J=8.0Hz), 7.01(1H, dd, J=8.0, 1.5Hz), 7.35(1H, dd, J=8.0, 1.5Hz), 7.84(1H, s), 8.46(2H, s), 9.56(1H, s), 10.79(1H, s), 10.90(1H, brs).

### Example 58: Preparation of the compound of Compound No. 58.

Using 3-methylsalicylic acid and 3,5-bis(trifluoromethyl)aniline as the raw materials, the same operation as the Example 3 gave the title compound.
Yield: 54.9%.
¹H-NMR(DMSO-d₆): δ 2.22(3H, s), 6.94(1H, t, J=7.4Hz), 7.42(1H, d, J=7.4Hz), 7.84-7.85(2H, m), 8.47(2H, s), 10.87(1H, s), 11.87(1H, s).

### Example 59: Preparation of the compound of Compound No. 59.

Using 3-methoxysalicylic acid and 3,5-bis(trifluoromethyl)aniline as the raw materials, the same operation as the Example 3 gave the title compound.
Yield: 34.6%.
¹H-NMR(DMSO-ds): δ 3.85(3H, s), 6.94(1H, t, J=8.0Hz), 7.20(1H, dd, J=8.0, 1.4Hz), 7.44(1H, dd, J=8.0, 1.4Hz), 7.84(1H, s), 8.45(2H, s), 10.82(1H, s), 10.94(1H, brs).

### Example 60: Preparation of the compound of Compound No. 60.

Using 5-[(1,1,3,3-tetramethyl)butyl]salicylic acid and 3,5-bis(trifluoromethyl)aniline as the raw materials, the same operation as the Example 3 gave the title compound.
Yield: 64.2%.
¹H-NMR(DMSO-d₆): δ 0.70(9H, s), 1.35(6H, s), 1.72(2H, s), 6.95(1H, d, J=8.4Hz), 7.50(1H, dd, J=8.0, 2.1Hz), 7.83(1H, s), 7.84(1H, d, J=2.1Hz), 8.46(1H, s), 10.77(1H, s), 11.20(1H, s).

### Example 61: Preparation of the compound of Compound No. 61.

Using 3,5,6-trichlorosalicylic acid and 3,5-bis(trifluoromethyl)aniline as the raw materials, the same operation as the Example 3 gave the title compound.
Yield: 26.2%.
¹H-NMR(DMSO-d₆): δ 7.88(1H, s), 7.93(1H, s), 8.33(2H, s), 10.88(1H, s), 11.36(1H, s).

### Example 62: Preparation of the compound of Compound No. 62.

Using 3,5-bis[(1,1-dimethyl)ethyl]salicylic acid and 3,5-bis(trifluoromethyl)aniline as the raw materials, the same operation as the Example 3 gave the title compound.
Yield: 65.0%.
¹H-NMR(DMSO-d₆): δ 1.34(9H, s), 1.40(9H, s), 7.49(1H, d, J=2.2Hz), 7.82(1H, d, J=2.2Hz), 7.91(1H, s), 8.40(2H, s), 10.82(1H, s), 12.44(1H, s).

### Example 63: Preparation of the compound of Compound No. 63.

Using 6-fluorosalicylic acid and 3,5-bis(trifluoromethyl)aniline as the raw materials, the same operation as the Example 3 gave the title compound.
Yield: 35.9%.
¹H-NMR(DMSO-d₆): δ 6.73-6.82(2H, m), 7.32(1H, ddd, J=1.4, 8.5, 15.3Hz), 7.83(1H, s), 8.39(2H, s), 10.50(1H, d, J=1.4Hz), 11.11(1H, s).

### Example 64: Preparation of the compound of Compound No. 64.

Using 3-chlorosalicylic acid and 3,5-bis(trifluoromethyl)aniline as the raw materials, the same operation as the Example 3 gave the title compound.
Yield: 61.3%.
¹H-NMR(DMSO-d₃): δ 7.05(1H, dd, J=7.6, 8.0Hz), 7.69(1H, dd, J=1.4, 13.3Hz), 7.90(1H, s), 7.93(1H, dd, J=1.4, 8.0Hz), 8.44(2H, s), 11.01(1H, s), 11.92(1H, br.s).

### Example 65: Preparation of the compound of Compound No. 65.

Using 4-methoxysalicylic acid and 3,5-bis(trifluoromethyl)aniline as the raw materials, the same operation as the Example 3 gave the title compound.
Yield: 14.2%.
¹H-NMR(DMSO-d₆): δ 3.81(3H, s), 6.54(1H, d, J=2.5Hz), 6.61(1H, dd, J=2.5, 8.8Hz), 7.83(1H, s), 7.95(1H, d, J=8.8Hz), 8.45(2H, s), 10.69(1H, s), 11.89(1H, s).

### Example 66: Preparation of the compound of Compound No. 66.

Using 6-methoxysalicylic acid and 3,5-bis(trifluoromethyl)aniline as the raw materials, the same operation as the Example 3 gave the title compound.
Yield: 63.1%.
¹H-NMR(DMSO-d₆): δ 3.24(3H, s), 6.03(1H, d, J=8.0Hz), 6.05(1H, d, J=8.5Hz), 6.71(1H, dd, J=8.2, 8.5Hz), 7.25(1H, s), 7.88(2H, s), 9.67(1H, s), 10.31(1H, s)

### Example 67: Preparation of the compound of Compound No. 67.

Using 5-amino-N-[3,5-bis(trifluoromethyl)phenyl]-2-hydroxybenzamide (Compound No. 43) and methanesulfonyl chloride as the raw materials, the same operation as the Example 45 gave the title compound.
Yield: 22.6%.
¹H-NMR(DMSO-d₆): δ 2.93(3H, s), 7.02(1H, d, J=8.4Hz), 7.31(1H, dd, J=8.4, 2.7Hz), 7.68(1H, d, J=2.7Hz), 7.83(1H, s), 8.46(2H, s), 9.48(1H, s), 10.85(1H, s), 11.15(1H, s).

### Example 68: Preparation of the compound of Compound No. 68.

Using 5-amino-N-[3,5-bis(trifluoromethyl)phenyl]-2-hydroxybenzamide (Compound No. 43) and benzenesulfonyl chloride as the raw materials, the same operation as the Example 45 gave the title compound.
Yield: 45.3%.
¹H-NMR(DMSO-d₆): δ 6.89(1H, d, J=8.7Hz), 7.10(1H, dd, J=8.7, 2.7Hz), 7.51-7.64(4H, m), 7.68-7.71(2H, m), 7.81(1H, s), 8.42(2H, s), 10.03(1H, s), 10.87(1H, s), 11.13(1H, brs).

### Example 69: Preparation of the compound of Compound No. 69.

Using 5-amino-N-[3,5-bis(trifluoromethyl)phenyl]-2-hydroxybenzamide (Compound No. 43) and acetyl chloride as the raw materials, the same operation as the Example 45 gave the title compound.
Yield: 44.8%.
¹H-NMR(DMSO-d₆): δ 2.02(3H, s), 6.97(1H, d, J=8.7Hz), 7.61(1H, dd, J=8.7, 2.7Hz), 7.82(1H, s), 7.99(1H, d, J=2.7Hz), 8.46(2H, s), 9.90(1H, s), 10.85(1H, s), 10.94(1H, s).

### Example 70: Preparation of the compound of Compound No. 70.

Using N-[3,5-bis(trifluoromethyl)phenyl]-2-methoxy-5-sulfamoylbenzamide (compound of Example 41(2)) as the raw material, the same operation as the Example 34(5) gave the title compound.
Yield: 59.9%.
¹H-NMR(DMSO-d₆): δ 7.17(1H, d, J=8.7Hz), 7.31(2H, s), 7.85(1H, s), 7.86(1H, dd, J=8.4, 2.4Hz), 8.26(1H, d, J=2.7Hz), 8.47(2H, s), 10.95(1H, s), 11.90(1H, s).

### Example 71: Preparation of the compound of Compound No. 71.

Using 1-hydroxynaphthalene-2-carboxylic acid and 3,5-bis(trifluoromethyl)aniline as the raw materials, the same operation as the Example 3 gave the title compound.
Yield: 65.5%.
¹H-NMR(DMSO-d₆): δ 7.51(1H, d, J=9.0Hz), 7.60(1H, td, J=7.8, 0.9Hz), 7.70(1H, td, J=7.8, 0.9Hz), 7.89(1H, s), 7.93(1H, d, J=8.4Hz), 8.09(1H, d, J=9.0Hz), 8.33(1H, d, J=8.7Hz), 8.51(2H, s), 10.92(1H, s), 13.36(1H, s).

### Example 72: Preparation of the compound of Compound No. 72.

Using 3-hydroxynaphthalene-2-carboxylic acid and 3,5-bis(trifluoromethyl)aniline as the raw materials, the same operation as the Example 3 gave the title compound.
Yield: 46.9%.
¹H-NMR(DMSO-d₆): δ 7.36-7.41(2H, m), 7.50-7.55(1H, m), 7.79(1H, d, J=8.2Hz), 7.85(1H, d, J=0.6Hz), 7.96(1H, d, J=8.0Hz), 8.51(2H, s), 10.98(1H, s), 11.05(1H, s).

### Example 73: Preparation of the compound of Compound No. 73.

Using 2-hydroxynaphthalene-1-carboxylic acid and 3,5-bis(trifluoromethyl)aniline as the raw materials, the same operation as the Example 3 gave the title compound.
Yield: 30.2%.
¹H-NMR(DMSO-d₆): δ 7.27(1H, d, J=8.8Hz), 7.32-7.38(1H, m), 7.45-7.50(1H, m), 7.72(1H, d, J=8.5Hz), 7.82-7.93(3H, m), 8.50(1H, s), 10.28(1H, s), 11.07(1H, brs).

### Example 74: Preparation of the compound of Compound No. 74.

### (1) 4-Bromo-3-hydroxythiophene-2-carboxylic acid.

A solution of 4-bromothiophene-2-carboxylic acid methyl ester(500mg, 2.1mmol), sodium hydroxide(261mg, 6.3mmol) in a mixed solvent of methanol/water(2.5mL+2.5mL) was refluxed for 2 hours. After the reaction mixture was cooled to room temperature, 2N hydrochloric acid was added to adjust pH to 1, and it was diluted with ethyl acetate. The ethyl acetate layer was washed successively with water and brine, and dried over anhydrous sodium sulfate. The solvent was evaporated under reduced pressure to give the title compound(326mg, 69.4%) as a red brown powder.
¹H-NMR(CDCl₃): δ 4.05(1H, brs), 7.40(1H, s).

### (2) 4-Bromo-3-hydroxy-N-[3,5-bis(trifluoromethyl)phenyl]thiophene-2-carboxamide (Compound No. 74).

Using 4-bromo-3-hydroxythiophene-2-carboxylic acid and 3,5-bis(trifluoromethyl)aniline as the raw materials, the same operation as the Example 3 gave the title compound.
Yield: 82.4%.
¹H-NMR(CDCl₃): δ 7.42(1H, s), 7.67(1H, brs), 7.78(1H, brs), 8.11(2H, s), 9.91(1H, brs).

### Example 75: Preparation of the compound of Compound No. 75.

Phosphorus oxychloride(0.112ml, 1.2mmol) was added to a solution of 5-chloro-2-hydroxynicotinic acid(174mg, 1mmol), 3,5-bis(trifluoromethyl)aniline(275mg, 1.2mmol), pyridine(316mg, 4mmol) in tetrahydrofuran/dichloromethane(20mL+10mL), and the mixture was stirred at room temperature for 2 hours. The reaction mixture was poured into ethyl acetate(100mL) and 0.2N hydrochloric acid(100mL), filtered through celite after stirring for 30 minutes, and the water layer was extracted with ethyl acetate. After the combined ethyl acetate layer was washed successively with water and brine, dried over anhydrous magnesium sulfate, the residue obtained by evaporation of the solvent under reduced pressure was purified by column chromatography on silica gel(n-hexane:ethyl acetate=2:1-1:1), washed with ethanol under suspension to give the title compound(183mg, 47.6%) as a white crystal.
mp >270°C.
¹H-NMR(DMSO-d₆): δ 7.83(1H, s), 8.15(1H, d, J=3.3Hz), 8.36(1H, d, J=3.0Hz), 8.40(2H, s), 12.43(1H, s).

When the preparation method described in Example 75 is referred in the following examples, phosphorus oxychloride was used as the condensating agent(acid halogenating agent). Pyridine was used as the base. As the reaction solvent, solvents such as dichloromethane, tetrahydrofuran or the like were used alone or as a mixture.

### Example 76: Preparation of the compound of Compound No. 76.

Using 3-hydroxypyridine-2-carboxylic acid and 3,5-bis(trifluoromethyl)aniline as the raw materials, the same operation as the Example 75 gave the title compound.
Yield: 45.0%.
¹H-NMR(CDCl₃): δ 7.40(1H, dd, J=8.4, 1.8Hz), 7.46(1H, dd, J=8.4, 4.2Hz), 7.68(1H, s), 8.16(1H, dd, J=4.2, 1.2Hz), 8.25(2H, s), 10.24(1H, s), 11.42(1H, s).

### Example 77: Preparation of the compound of Compound No. 77.

A solution of 6-chloro-oxindole(184mg, 1.1mmol) in tetrahydrofuran(5ml) and triethylamine(0.3mL) were added to a solution of 3,5-bis(trifluoromethyl)phenylisocyanate(255mg, 1.0mmol) in tetrahydrofuran(5mL) under argon atmosphere, and the mixture was stirred at room temperature for 4 hours. The reaction mixture was poured into diluted hydrochloric acid and extracted with ethyl acetate. The ethyl acetate layer was washed successively with water and brine, and dried over anhydrous magnesium sulfate. The residue obtained by evaporation of the solvent under reduced pressure was purified by column chromatography on silica gel(n-hexane:ethyl acetate=4:1) to give the title compound(172.2mg, 40.7%) as a pink solid.
¹H-NMR(DMSO-d₆): δ 3.97(2H, s), 7.29(1H, dd, J=8.1, 2.1Hz), 7.41(1H, d, J=8.1Hz), 7.88(1H, s), 8.04(1H, d, J=2.1Hz), 8.38(2H, s), 10.93(1H, s).

### Example 78: Preparation of the compound of Compound No. 78.

Using 3,5-bis(trifluoromethyl)phenylisocyanate and oxindole as the raw materials, the same operation as the Example 77 gave the title compound.
Yield: 44.8%.
¹H-NMR(DMSO-d₆): δ 3.98(2H, s), 7.22(1H, td, J=7.8, 1.2Hz), 7.33-7.40(2H, m), 7.87(1H, s), 8.02(1H, d, J=7.8Hz), 8.38(2H, s), 11.00(1H, s).

### Example 79: Preparation of the compound of Compound No. 79.

Using 3,5-bis(trifluoromethyl)phenylisocyanate and 5-chlorooxindole as the raw materials, the same operation as the Example 77 gave the title compound.
Yield: 31.1%.
¹H-NMR(DMSO-d₆): δ 3.99(2H, s), 7.41(1H, dd, J=8.7, 2.4Hz), 7.47(1H, d, J=2.1Hz), 7.87(1H, s), 8.01(1H, d, J=8.4Hz), 8.38(2H, s), 10.93(1H, s).

### Example 80: Preparation of the compound of Compound No. 80.

Using 3-hydroxyquinoxaline-2-carboxylic acid and 3,5-bis(trifluoromethyl)aniline as the raw materials, the same operation as the Example 3 gave the title compound.
Yield: 2.7%.
¹H-NMR(DMSO-d₆): δ 7.40-7.45(2H, m), 7.69(1H, td, J=8.4, 1.5Hz), 7.90-7.93(2H, m), 8.41(2H, s), 11.64(1H, s), 13.02(1H, s).

### Example 81: Preparation of the compound of Compound No. 81.

Using 5-chlorosalicylic acid and 2,5-bis(trifluoromethyl)aniline as the raw materials, the same operation as the Example 3 gave the title compound.
Yield: 3.6%.
¹H-NMR(CDCl₃): δ 7.03(1H, d, J=8.7Hz), 7.43-7.48(2H, m), 6.61(1H, d, J=8.1Hz), 7.85(1H, d, J=8.4Hz), 8.36(1H, brs), 8.60(1H, s), 11.31(1H, s).

### Example 82: Preparation of the compound of Compound No. 82.

Using N-[2,5-bis(trifluoromethyl)phenyl]-5-chloro-2-hydroxybenzamide (Compound No. 81) and acetyl chloride as the raw materials, the same operation as the Example 5 gave the title compound.
Yield: 6.6%.
¹H-NMR(CDCl₃): δ 2.35(3H, s), 7.17(1H, d, J=8.7Hz),7.54(1H, dd, J=8.7, 2.4Hz), 7.55(1H, d, J=8.1Hz), 7.80(1H, d, J=8.1Hz), 7.95(1H, d, J=2.4Hz), 8.60(1H, s), 8.73(1H, s).

### Example 83: Preparation of the compound of Compound No. 83.

Using 5-bromosalicylic acid and 2,5-bis(trifluoromethyl)aniline as the raw materials, the same operation as the Example 3 gave the title compound.
Yield: 24.0%.
¹H-NMR(DMSO-d₆): δ 7.03(1H, d, J=8.7Hz), 7.65(1H, dd, J=8.7, 2.7Hz), 7.76(1H, d, J=8.4Hz), 8.03(1H, d, J=8.1Hz)8.11(1H, d, J=2.7Hz), 8.74(1H, s), 11.02(1H, s), 12.34(1H, s).

### Example 84: Preparation of the compound of Compound No. 84.

Using 5-methylsalicylic acid and 2,5-bis(trifluoromethyl)aniline as the raw materials, the same operation as the Example 3 gave the title compound.
Yield: 1.5%.
¹H-NMR(CDCl₃): δ 2.36(3H, s), 6.97(1H, d, J=8.4Hz), 7.23(1H, s), 7.32(1H, dd, J=8.4, 1.5Hz), 7.57(1H, d, J=8.4Hz), 7.83(1H, d, J=8.4Hz), 8.46(1H, s), 8.69(1H, s), 11.19(1H, s).

### Example 85: Preparation of the compound of Compound No. 85.

Using 5-chlorosalicylic acid and 3-fluoro-5-(trifluoromethyl)aniline as the raw materials, the same operation as the Example 3 gave the title compound.
Yield: 62.0%.
¹H-NMR(DMSO-d₆): δ 7.04(1H, d, J=8.7Hz), 7.42(1H, d, J=8.4Hz), 7.48(1H, dd, J=9.0, 3.0Hz), 7.85(1H, d, J=2.4Hz), 7.94(1H, dd, J=11.4, 2.1Hz), 7.99(1H, s), 10.73(1H, s), 11.46(1H, s).

### Example 86: Preparation of the compound of Compound No. 86.

Using 5-bromosalicylic acid and 3-bromo-5-(trifluoromethyl)aniline as the raw materials, the same operation as the Example 3 gave the title compound.
Yield: 73.3%.
¹H-NMR(DMSO-d₆): δ 6.99(1H, d, J=9.0Hz), 7.60(1H, dd, J=9.0, 2.4Hz), 7.72(1H, s), 7.97(1H, d, J=2.7Hz), 8.16(1H, s), 8.28(1H, s), 10.69(1H, s), 11.45(1H, s).

### Example 87: Preparation of the compound of Compound No. 87.

Using 5-chlorosalicylic acid and 2-fluoro-5-(trifluoromethyl)aniline as the raw materials, the same operation as the Example 3 gave the title compound.
Yield: 77.9%.
¹H-NMR(DMSO-d₆): δ 7.07(1H, d, J=9.0Hz), 7.52(1H, dd, J=9.0, 2.7Hz), 7.58-7.61(2H, m), 7.95(1H, d, J=2.7Hz), 8.71(1H, d, J=7.5Hz), 10.90(1H, s), 12.23(1H, s).

### Example 88: Preparation of the compound of Compound No. 88.

Using 5-chlorosalicylic acid and 2-chloro-5-(trifluoromethyl)aniline as the raw materials, the same operation as the Example 3 gave the title compound.
Yield: 49.1%.
¹H-NMR(DMSO-d₆): δ 7.09(1H, d, J=9.0Hz), 7.53(1H, dd, J=9.0, 3.0Hz), 7.55(1H, dd, J=8.4, 2.7Hz), 7.83(1H, d, J=8.4Hz), 7.98(1H, d, J=3.0Hz), 8.88(1H, d, J=2.7Hz), 11.14(1H, s), 12.39(1H, s).

### Example 89: Preparation of the compound of Compound No. 89.

Using 5-chloro-N-[2-chloro-5-(trifluoromethyl)phenyl]-2-hydroxybenzamide (Compound No. 88) and acetyl chloride as the raw materials, the same operation as the Example 5 gave the title compound.
Yield: 34.0%.
¹H-NMR(CDCl₃): δ 2.39(3H, s), 7.16(1H, d, J=8.7Hz),7.37(1H, ddd, J=8.7, 2.4, 0.6Hz), 7.51-7.56(2H, m), 7.97(1H, d, J=3.0Hz), 8.85(1H, s), 8.94(1H, d, J=1.8Hz).

### Example 90: Preparation of the compound of Compound No. 90.

Using 5-bromosalicylic acid and 2-chloro-5-(trifluoromethyl)aniline as the raw materials, the same operation as the Example 3 gave the title compound.
Yield: 34.2%.
¹H-NMR(DMSO-d₆): δ 7.04(1H, d, J=8.7Hz), 7.56(1H, ddd, J=8.1, 2.4, 1.2Hz), 7.64(1H, dd, J=8.7, 2.7Hz), 7.83(1H, dd, J=8.1, 1.2Hz), 8.11(1H, d, J=2.7Hz), 8.87(1H, d, J=2.4Hz), 11.12(1H, s), 12.42(1H, s).

### Example 91: Preparation of the compound of Compound No. 91.

Using 5-chlorosalicylic acid and 2-nitro-5-(trifluoromethyl)aniline as the raw materials, the same operation as the Example 3 gave the title compound.
Yield: 8.1%.
¹H-NMR(DMSO-d₆): δ 7.08(1H, d, J=9.0Hz), 7.53(1H, dd, J=8.7, 2.7Hz), 7.73(1H, dd, J=8.4, 1.8Hz), 7.95(1H, d, J=3.0Hz), 8.36(1H, d, J=8.7Hz), 9.01(1H, d, J=1.8Hz), 12.04(1H, s), 12.20(1H, s).

### Example 93: Preparation of the compound of Compound No. 93.

Using 5-chlorosalicylic acid and 2-methyl-5-(trifluoromethyl)aniline as the raw materials, the same operation as the Example 3 gave the title compound.
Yield: 73.3%.
¹H-NMR(DMSO-d₆): δ 2.39(3H, s), 7.07(1H, d, J=8.7Hz), 7.44-7.54(3H, m), 7.99(1H, d, J=3.0Hz), 8.43(1H, s), 10.52(1H, s), 12.17(1H, brs).

### Example 93: Preparation of the compound of Compound No. 93.

Using 5-bromosalicylic acid and 3-methoxy-5-(trifluoromethyl)aniline as the raw materials, the same operation as the Example 3 gave the title compound.
Yield: 58.8%.
¹H-NMR(DMSO-d₆): δ 3.85(3H, s), 6.98(1H, d, J=8.7Hz), 7.03(1H, s), 7.57-7.61(2H, m), 7.77(1H, s), 8.00(1H, d, J=2.4Hz), 10.57(1H, s), 11.56(1H, s).

### Example 94: Preparation of the compound of Compound No. 94.

Using 5-bromosalicylic acid and 2-methoxy-5-(trifluoromethyl)aniline as the raw materials, the same operation as the Example 3 gave the title compound.
Yield: 71.3%.
¹H-NMR(DMSO-d₆): δ 3.99(3H, s), 7.03(1H, d, J=9.0Hz), 7.30(1H, d, J=8.7Hz), 7.47-7.51(1H, m), 7.61(1H, dd, J=9.0, 2.4Hz), 8.10(1H, d, J=2.4Hz), 8.82(1H, d, J=2.1Hz)11.03(1H, s), 12.19(1H, s).

### Example 95: Preparation of the compound of Compound No. 95.

Using 5-chlorosalicylic acid and 2-methoxy-5-(trifluoromethyl)aniline as the raw materials, the same operation as the Example 3 gave the title compound.
Yield: 83.4%.
¹H-NMR(DMSO-d₆): δ 4.00(3H, s), 7.08(1H, d, J=9.0Hz), 7.30(1H, d, J=8.7Hz), 7.47-7.52(2H, m), 7.97(1H, d, J=2.7Hz), 8.83(1H, d, J=2.4Hz), 11.05(1H, s), 12.17(1H, s).

### Example 96: Preparation of the compound of Compound No. 96.

Using 5-chlorosalicylic acid and 2-methylsulfanyl-5-(trifluoromethyl)aniline as the raw materials, the same operation as the Example 3 gave the title compound.
Yield: 79.2%.
¹H-NMR(DMSO-d₆): δ 2.57(3H, s), 7.07(1H, d, J=8.7Hz), 7.52(1H, dd, J=8.7, 2.4Hz), 7.55(1H, dd, J=8.4, 1.5Hz), 7.63(1H, d, J=8.1Hz), 8.00(1H, d, J=2.4Hz), 8.48(1H, d, J=1.5Hz), 10.79(1H, s), 12.26(1H, s).

### Example 97: Preparation of the compound of Compound No. 97.

Using 5-bromosalicylic acid and 2-(1-pyrrolidinyl)-5-(trifluoromethyl)aniline as the raw materials, the same operation as the Example 3 gave the title compound.
Yield: 44.5%.
¹H-NMR(DMSO-d₆): δ 1.86-1.91(4H, m), 3.20-3.26(4H, m), 6.99(1H, d, J=8.7Hz), 7.07(1H, d, J=8.7Hz), 7.43(1H, dd, J=8.7, 2.1Hz), 7.62(1H, dd, J=8.7, 2.4Hz), 7.94(1H, d, J=2.1Hz), 8.17(1H, d, J=2.4Hz), 10.54(1H, s), 12.21(1H, s).

### Example 98: Preparation of the compound of Compound No. 98.

Using 5-bromosalicylic acid and 2-morpholino-5-(trifluoromethyl)aniline as the raw materials, the same operation as the Example 3 gave the title compound.
Yield: 65.9%.
¹H-NMR(DMSO-d₆): δ 2.90(4H, dd, J=4.5, 4.2Hz), 3.84(4H, dd, J=4.8, 4.2Hz), 7.09(1H, d, J=8.4Hz), 7.48(2H, s), 7.61(1H, dd, J=8.4, 2.7Hz), 8.13(1H, d, J=2.7Hz), 8.90(1H, s), 11.21(1H, s), 12.04(1H, s).

### Example 99: Preparation of the compound of Compound No. 99.

Using 5-nitrosalicylic acid and 2-chloro-5-(trifluoromethyl)aniline as the raw materials, the same operation as the Example 3 gave the title compound.
Yield: 31.1%.
¹H-NMR(DMSO-d₆): δ 6.98(1H, d, J=9.3Hz), 7.52(1H, dd, J=8.4, 2.1Hz), 7.81(1H, d, J=8.4Hz), 8.21(1H, dd, J=9.0, 3.3Hz), 8.82(1H, d, J=3.0Hz), 8.93(1H, d, J=2.4Hz), 12.18(1H, s).

### Example 100: Preparation of the compound of Compound No. 100.

Using 5-methylsalicylic acid and 2-chloro-5-(trifluoromethyl)aniline as the raw materials, the same operation as the Example 3 gave the title compound.
Yield: 15.8%.
¹H-NMR(CDCl₃): δ 2.36(3H, s), 6.95(1H, d, J=8.1Hz), 7.26-7.31(2H, m), 7.37(1H, dd, J=8.4, 1.8Hz), 7.56(1H, d, J=8.4Hz), 8.65(1H, brs), 8.80(1H, d, J=1.8Hz), 11.33(1H, brs).

### Example 101: Preparation of the compound of Compound No. 101.

Using 5-methoxysalicylic acid and 2-chloro-5-(trifluoromethyl)aniline as the raw materials, the same operation as the Example 3 gave the title compound.
Yield: 56.4%.
¹H-NMR(DMSO-d₆): δ 3.77(3H, s), 6.91(1H, d, J=9.0Hz), 7.07(1H, dd, J=8.7, 3.0Hz), 7.20(1H, t, J=1.8Hz), 7.52-7.54(3H, m), 10.33(1H, s), 11.44(1H, s).

### Example 102: Preparation of the compound of Compound No. 102.

Using 5-methylsalicylic acid and 2-methyl-5-(trifluoromethyl)aniline as the raw materials, the same operation as the Example 3 gave the title compound.
Yield: 14.2%, white solid.
¹H-NMR(DMSO-d₆): δ 2.29(3H, s), 2.38(3H, s), 6.94(1H, d, J=8.4Hz), 7.27(1H, ddd, J=8.4, 2.4, 0.6Hz), 7.44(1H, dd, J=8.1, 1.5Hz), 7.52(1H, d, J=7.8Hz), 7.84(1H, d, J=2.4Hz), 8.46(1H, d, J=1.5Hz), 10.55(1H, s), 11.72(1H, s).

### Example 103: Preparation of the compound of Compound No. 103.

Using 5-methylsalicylic acid and 2-methoxy-5-(trifluoromethyl)aniline as the raw materials, the same operation as the Example 3 gave the title compound.
Yield: 77.9%.
¹H-NMR(CDCl₃): δ 2.35(3H, s), 4.02(3H, s), 6.93(1H, d, J=9.0Hz), 6.98(1H, d, J=8.4Hz), 7.25-7.28(2H, m), 7.36(1H, ddd, J=8.4, 2.1, 0.9Hz), 8.65(1H, brs), 8.73(1H, d, J=2.1Hz), 11.69(1H, s).

### Example 104: Preparation of the compound of Compound No. 104.

Using 5-chlorosalicylic acid and 3-bromo-5-(trifluoromethyl)aniline as the raw materials, the same operation as the Example 3 gave the title compound.
Yield: 37.1%.
¹H-NMR(DMSO-d₆): δ 7.03(1H, d, J=9.3Hz), 7.48(1H, dd, J=8.7, 2.4Hz), 7.72(1H, s), 7.84(1H, d, J=2.7Hz), 8.16(1H, s), 8.28(1H, s), 10.69(1H, s), 11.42(1H, s).

### Example 105: Preparation of the compound of Compound No. 105.

Using 5-chlorosalicylic acid and 3-methoxy-5-(trifluoromethyl)aniline as the raw materials, the same operation as the Example 3 gave the title compound.
Yield: 68.0%.
¹H-NMR(DMSO-d₆): δ 3.85(3H, s), 7.02(1H, s), 7.03(1H, d, J=8.7Hz), 7.48(1H, dd, J=8.7, 2.7Hz), 7.61(1H, s), 7.77(1H, s), 7.88(1H, d, J=2.7Hz), 10.57(1H, s), 11.53(1H, s).

### Example 106: Preparation of the compound of Compound No. 106.

Using 5-chlorosalicylic acid and 2-morpholino-5-(trifluoromethyl)aniline as the raw materials, the same operation as the Example 3 gave the title compound.
Yield: 64.8%.
¹H-NMR(DMSO-d₆): δ 2.90(4H, m), 3.84(4H, m), 7.15(1H, d, J=9.0Hz), 7.48(2H, s), 7.50(1H, dd, J=9.0, 2.7Hz), 8.00(1H, d, J=2.7Hz), 8.91(1H, s), 11.24(1H, s), 12.05(1H, s).

### Example 107: Preparation of the compound of Compound No. 107.

Using 5-chlorosalicylic acid and 2-bromo-5-(trifluoromethyl)aniline as the raw material, the same operation as the Example 3 gave the title compound.
Yield: 59.2%.
¹H-NMR(DMSO-d₆): δ 7.10(1H, d, J=8.7Hz), 7.48(1H, dd, J=8.4, 2.1Hz), 7.53(1H, dd, J=8.7, 3.0Hz), 7.97-7.99(2H, m), 8.81(1H, d, J=2.1Hz), 11.03(1H, s), 12.38(1H, s).

### Example 108: Preparation of the compound of Compound No. 108.

Using 5-chlorosalicylic acid and 3-amino-5-(trifluoromethyl)benzoic acid methyl ester as the raw materials, the same operation as the Example 3 gave the title compound.
Yield: 67.0%.
¹H-NMR(DMSO-d₆): δ 3.91(3H, s), 7.02(1H, d, J=9.3Hz), 7.43(1H, dd, J=9.0, 2.4Hz), 7.57(1H, d, J=2.4Hz), 8.13(1H, s), 8.23(1H, s), 8.29(1H, s), 8.36(1H, s), 11.52(1H, s).

### Example 109: Preparation of the compound of Compound No. 109.

2N Aqueous sodium hydroxide(0.6mL) was added to a suspension of 5-chloro-2-hydroxy-N-[3-methoxycarbonyl-5-(trifluoromethyl)phenyl]benzamide (Compound No. 108; 105mg, 0.281mmol) in methanol(2.5mL), and the mixture was stirred at room temperature for 3 hours. Water was added to the reaction mixture and it was washed with ethyl acetate. After the water layer was acidified by addition of diluted hydrochloric acid, it was extracted with ethyl acetate. After the ethyl acetate layer was washed successively with water and brine, dried over anhydrous sodium sulfate, the residue obtained by evaporation of the solvent under reduced pressure was crystallized by isopropyl ether to give the title compound(100mg, 99.0%) as a white solid.
¹H-NMR(DMSO-d₆): δ 7.04(1H, d, J=9.0Hz), 7.49(1H, dd, J=8.7, 2.7Hz), 7.91(1H, d, J=2.7Hz), 7.93(1H, s), 8.43(1H, s), 8.59(1H, s), 10.78(1H, s), 11.48(1H, s).

### Example 110: Preparation of the compound of Compound No. 110.

Using 5-chlorosalicylic acid and 2-(2-naphthyloxy)-5-(trifluoromethyl)aniline as the raw materials, the same operation as the Example 3 gave the title compound.
Yield: 89.6%.
¹H-NMR(CDCl₃): δ 6.94(1H, d, J=9.6Hz), 6.98(1H, d, J=9.2Hz), 7.25-7.41(4H, m), 7.48-7.57(3H, m), 7.81(1H, d, J=6.9Hz), 7.88(1H, d, J=6.9Hz), 7.95(1H, d, J=8.9Hz), 8.72(1H, s), 8.83(1H, d, J=2.0Hz), 11.70(1H, s).

### Example 111: Preparation of the compound of Compound No. 111.

Using 5-chlorosalicylic acid and 2-(2,4-dichlorophenoxy)-5-(trifluoromethyl)aniline as the raw materials, the same operation as the Example 3 gave the title compound.
Yield: 4.7%.
¹H-NMR(CDCl₃): δ 6.78(1H, d, J=8.9Hz), 7.02(1H, d, J=8.6Hz), 7.16(1H, d, J=8.6Hz), 7.33-7.38(3H, m), 7.42(1H, dd, J=8.6, 2.6Hz), 7.49(1H, d, J=2.6Hz)7.58(1H, d, J=2.3Hz), 8.66(1H, brs,), 8.82(1H, d, J=2.0Hz), 11.65(1H, s).

### Example 112: Preparation of the compound of Compound No. 112.

Using 5-chlorosalicylic acid and 2-[(4-trifluoromethyl)piperidino]-5-(trifluoromethyl)aniline as the raw materials, the same operation as the Example 3 gave the title compound.
Yield: 60.5%.
¹H-NMR(CDCl₃): δ 1.85-2.05(2H, m), 2.15(2H, d, J=10.9Hz), 2.28(1H, m), 2.82(2H, t, J=11.0Hz), 3.16(2H, d, J=12.2Hz), 7.02(1H, d, J=8.9Hz), 7.31(1H, d, J=8.3Hz), 7.42(2H, m), 7.50(1H, d, J=2.6Hz), 8.75(1H, s), 9.60(1H, s), 11.94(1H, s)

### Example 113: Preparation of the compound of Compound No. 113.

Using 5-chlorosalicylic acid and 2-(2,2,2-trifluoroethoxy)-5-(trifluoromethyl)aniline as the raw materials, the same operation as the Example 3 gave the title compound.
Yield: 94.5%.
¹H-NMR(CDCl₃): δ 4.58(2H, q, J=7.9Hz), 6.99-7.05(2H, m), 7.41-7.50(3H, m), 8.63(1H, brs), 8.79(1H, d, J=2.0Hz), 11.59(1H, s).

### Example 114: Preparation of the compound of Compound No. 114.

Using 5-chlorosalicylic acid and 2-(2-methoxyphenoxy)-5-(trifluoromethyl)aniline as the raw materials, the same operation as the Example 3 gave the title compound.
Yield: 80.6%.
¹H-NMR(DMSO-d₆): δ 3.74(3H, s), 6.70(1H, d, J=8.4Hz), 7.02(1H, d, J=8.7Hz), 7.07(1H, dd, J=1.5, 7.8Hz), 7.24-7.39(4H, m), 7.49(1H, dd, J=3.0, 8.7Hz), 8.00(1H, d, J=3.0Hz), 8.92(1H, d, J=2.1Hz), 11.36(1H, s), 12.18(1H, s).

### Example 115: Preparation of the compound of Compound No. 115.

Using 5-chlorosalicylic acid and 2-(4-chloro-3,5-dimethylphenoxy)-5-(trifluoromethyl)aniline as the raw materials, the same operation as the Example 3 gave the title compound.
Yield: 91.5%.
¹H-NMR(DMSO-d₆): δ 2.34(6H, s), 7.03(1H, d, J=8.8Hz), 7.05(1H, d, J=8.1Hz), 7.11(2H, s), 7.43-7.47(1H, m), 7.48(1H, dd, J=2.9, 8.8Hz), 7.97(1H, d, J=2.6Hz), 8.94(1H, d, J=2.2Hz), 11.25(1H, s), 12.12(1H, s).

### Example 116: Preparation of the compound of Compound No. 116.

Using 5-chlorosalicylic acid and 2-piperidino-5-(trifluoromethyl)aniline as the raw materials, the same operation as the Example 3 gave the title compound.
Yield: 73.7%.
¹H-NMR(CDCl₃): δ 1.68-1.72(2H, m), 1.80-1.88(4H, m), 2.89(4H, t, J=5.2Hz), 7.01(1H, d, J=8.7Hz), 7.31(1H, d, J=8.4Hz), 7.39-7.43(2H, m), 7.55(1H, d, J=2.4Hz), 8.73(1H, d, J=1.8Hz), 9.71(1H, s), 12.05(1H, s)

### Example 117: Preparation of the compound of Compound No. 117.

Using 5-chlorosalicylic acid and 2-(4-methylphenoxy)-5-(trifluoromethyl)-aniline as the raw materials, the same operation as the Example 3 gave the title compound.
Yield: 67.3%.
¹H-NMR(DMSO-d₆): δ 2.33(3H, s), 6.93(1H, d, J=8.8Hz), 7.03(1H, dd, J=0.5, 8.8Hz), 7.12(2H, d, J=8.2Hz), 7.29(2H, d, J=8.5Hz), 7.43(1H, dd, J=2.0, 8.6Hz), 7.48(1H, ddd, J=0.8, 2.7, 8.8Hz), 7.98(1H, dd, J=0.8, 2.7Hz), 8.94(1H, d, J=2.2Hz), 11.29(1H, s), 12.15(1H, s).

### Example 118: Preparation of the compound of Compound No. 118.

Using 5-chlorosalicylic acid and 2-(4-chlorophenoxy)-5-(trifluoromethyl)-aniline as the raw materials, the same operation as the Example 3 gave the title compound.
Yield: 74.5%.
¹H-NMR(DMSO-d₆): δ 7.01(1H, d, J=8.8Hz), 7.06(1H, d, J=8.5Hz), 7.22(1H, d, J=8.5Hz), 7.43-7.48(2H, m), 7.50(2H, d, J=8.2Hz), 7.94(1H, dd, J=0.5, 2.7Hz), 8.92(1H, d, J=2.2Hz), 11.20(1H, s), 12.10(1H, s).

### Example 119: Preparation of the compound of Compound No. 119.

Using 5-chloro-2-hydroxynicotinic acid and 2-chloro-5-(trifluoromethyl)aniline as the raw materials, the same operation as the Example 75 gave the title compound.
Yield: 42.9%.
¹H-NMR(DMSO-d₆): δ 7.52(1H, dd, J=8.4, 2.1Hz), 7.81(1H, d, J=8.4Hz), 8.16(1H, s), 8.39(1H, d, J=2.7Hz), 8.96(1H, d, J=2.1Hz), 12.76(1H, s), 13.23(1H, s).

### Example 120: Preparation of the compound of Compound No. 120.

Using O-acetylsalicyloyl chloride and 3,5-dichloroaniline as the raw materials, the same operation as the Example 1 gave the title compound.
Yield: 73.5%.
mp 167-168°C.
¹H-NMR(CDCl₃): δ 2.35(3H, s), 7.14-7.18(2H, m), 7.35-7.40(1H, m), 7.52-7.57(3H, m), 7.81(1H, dd, J=7.8, 1.8Hz), 8.05(1H, brs).

### Example 121: Preparation of the compound of Compound No. 121.

Using 2-acetoxy-N-(3,5-dichlorophenyl)benzamide(Compound No. 121) as the raw material, the same operation as the Example 2 gave the title compound.
Yield: 60.3%.
mp 218-219°C.
¹H-NMR(DMSO-d₆): δ 6.95-7.02(2H, m), 7.35-7.36(1H, m), 7.42-7.47(1H, m), 7.83-7.87(3H, m), 10.54(1H, s), 11.35(1H, s).

### Example 122: Preparation of the compound of Compound No. 122.

Using 5-chlorosalicylic acid and 2,5-dichloroaniline as the raw materials, the same operation as the Example 3 gave the title compound.
Yield: 10.8%.
¹H-NMR(DMSO-d₆): δ 7.08(1H, d, J=9.0Hz), 7.24-7.28(1H, m), 7.50-7.54(1H, m), 7.61(1H, dd, J=9.0, 3.0Hz), 7.97(1H, d, J=2.7Hz), 8.58(1H, d, J=2.4Hz), 11.02(1H, s), 12.35(1H, brs).

### Example 123: Preparation of the compound of Compound No. 123.

Using 5-bromosalicylic acid and 3,5-difluoroaniline as the raw materials, the same operation as the Example 3 gave the title compound.
Yield: 36.3%.
mp 259-261°C.
¹H-NMR(DMSO-d₆): δ 6.96-7.04(2H, m), 7.45-7.54(2H, m), 7.58(1H, dd, J=8.7, 2.7Hz), 7.94(1H, d, J=2.7Hz), 10.60(1H, s) 11.48(1H, s).

### Example 124: Preparation of the compound of Compound No. 124.

Using 5-fluorosalicylic acid and 3,5-dichloroaniline as the raw materials, the same operation as the Example 3 gave the title compound.
Yield: 33.3%.
mp 258-260°C.
¹H-NMR(DMSO-d₆): δ 7.00-7.05(1H, m), 7.28-7.37(2H, m), 7.63(1H, dd, J=9.3, 3.3Hz), 7.84(2H, d, J=2.1Hz), 10.56(1H, s), 11.23(1H, s).

### Example 125: Preparation of the compound of Compound No. 125.

Using 5-chlorosalicylic acid and 3,5-dichloroaniline as the raw materials, the same operation as the Example 3 gave the title compound.
Yield: 41.2%.
¹H-NMR(DMSO-d₆): δ 7.03(1H, d, J=9.0Hz), 7.36-7.37(1H, m), 7.48(1H, dd, J=8.7, 2.7Hz), 7.83-7.84(3H, m), 10.56(1H, s), 11.44(1H, s).

### Example 126: Preparation of the compound of Compound No. 126.

Using 5-bromosalicylic acid and 3,5-dichloroaniline as the raw materials, the same operation as the Example 3 gave the title compound.
Yield: 61.6%.
mp 243-244°C.
¹H-NMR(DMSO-d₆): δ 6.98(1H, d, J=8.7Hz), 7.36-7.37(1H, m), 7.59(1H, dd, J=9.0, 2.4Hz), 7.83(2H, d, J=1.8Hz), 7.95(1H, d, J=2.4Hz), 10.56(1H, s), 11.46(1H, s).

### Example 127: Preparation of the compound of Compound No. 127.

Using 5-iodosalicylic acid and 3,5-dichloroaniline as the raw materials, the same operation as the Example 3 gave the title compound.
Yield: 65.4%.
mp 244-245°C.
¹H-NMR(DMSO-d₆): δ 6.84(1H, d, J=9.0Hz), 7.35-7.37(1H, m), 7.72(1H, dd, J=9.0, 2.1Hz), 7.83(2H, d, J=1.8Hz), 8.09(1H, d, J=2.1Hz), 10.55(1H, s), 11.45(1H, s).

### Example 128: Preparation of the compound of Compound No. 128.

Using 3,5-dibromosalicylic acid and 3,5-dichloroaniline as the raw materials, the same operation as the Example 3 gave the title compound.
Yield: 44.2%.
mp 181-182°C.
¹H-NMR(DMSO-d₆): δ 7.42-7.43(1H, m), 7.80(2H, d, J=1.8Hz), 8.03(1H, d, J=2.1Hz), 8.17(1H, d, J=2.1Hz), 10.82(1H, s).

### Example 129: Preparation of the compound of Compound No. 129.

Using 4-chlorosalicylic acid and 3,5-dichloroaniline as the raw materials, the same operation as the Example 3 gave the title compound.
Yield: 57.2%.
mp 255-256°C.
¹H-NMR(DMSO-d₆): δ 7.03-7.06(2H, m), 7.34-7.36(1H, m), 7.82-7.85(3H,m), 10.51(1H, s), 11.70(1H, brs).

### Example 130: Preparation of the compound of Compound No. 130.

Using 5-nitrosalicylic acid and 3,5-dichloroaniline as the raw materials, the same operation as the Example 3 gave the title compound.
Yield: 83.1%.
mp 232-233°C.
¹H-NMR(DMSO-d₆): δ 7.16(1H, d, J=9.6Hz), 7.37-7.39(1H, m), 7.84(1H, d, J=2.1Hz), 8.29(1H, dd, J=9.0, 3.0Hz), 8.65(1H, d, J=3.0Hz), 10.83(1H, s).

### Example 131: Preparation of the compound of Compound No. 131.

Using 5-methylsalicylic acid and 3,5-dichloroaniline as the raw materials, the same operation as the Example 3 gave the title compound.
Yield: 71.0%.
mp 216-217°C.
¹H-NMR(DMSO-_{d}): δ 2.28(3H, s), 6.90(1H, d, J=8.4Hz), 7.26(1H, dd, J=8.7, 1.8Hz), 7.34-7.36(1H, m), 7.67(1H, d, J=1.5Hz), 7.85(2H, d, J=1.8Hz), 10.52(1H, s), 11.15(1H, s).

### Example 132: Preparation of the compound of Compound No. 132.

Using 5-methoxysalicylic acid and 3,5-dichloroaniline as the raw materials, the same operation as the Example 3 gave the title compound.
Yield: 29.8%.
mp 230-232°C.
¹H-NMR(DMSO-d₆): δ 3.76(3H, s), 6.95(1H, d, J=8.7Hz), 7.08(1H, dd, J=9.0, 3.0Hz), 7.35-7.36(1H, m), 7.40(1H, d, J=3.0Hz), 7.85(2H, d, J=1.5Hz), 10.55(1H, s), 10.95(1H, s).

### Example 133: Preparation of the compound of Compound No. 133.

Using 5-bromosalicylic acid and 3,5-dinitroaniline as the raw materials, the same operation as the Example 3 gave the title compound.
Yield: 32.2%.
mp 258-260°C.
¹H-NMR(DMSO-d₆): δ 6.98-7.02(1H, m), 7.59-7.63(1H, m), 7.96-7.97(1H, m), 8.56-8.58(1H, m), 9.03-9.05(2H, m), 11.04(1H, s), 11.39(1H, brs).

### Example 134: Preparation of the compound of Compound No. 134.

Using 5-chlorosalicylic acid and 2,5-bis[(1,1-dimethyl)ethyl]aniline as the raw materials, the same operation as the Example 3 gave the title compound.
Yield: 75.7%.
¹H-NMR(DMSO-d₆): δ 1.27(9H, s), 1.33(9H, s), 7.04(1H, d, J=9.0Hz), 7.26(1H, dd, J=8.4, 2.1Hz), 7.35-7.38(2H, m), 7.49(1H, dd, J=8.7, 2.7Hz), 8.07(1H, d, J=2.4Hz), 10.22(1H, s), 12.38(1H, brs).

### Example 135: Preparation of the compound of Compound No. 135.

Using 5-chlorosalicylic acid and 5-[(1,1-dimethyl)ethyl]-2-methoxyaniline as the raw materials, the same operation as the Example 3 gave the title compound.
Yield: 89.5%.
¹H-NMR(DMSO-d₆): δ 1.28(9H, s), 3.33(3H, s), 7.01(1H, d, J=8.7Hz), 7.05(1H, d, J=9.0Hz), 7.11(1H, dd, J=8.7, 2.4Hz), 7.47(1H, dd, J=9.0, 3.0Hz), 7.99(1H, d, J=3.0Hz), 8.49(1H, d, J=2.4Hz), 10.78(1H, s), 12.03(1H, s).

### Example 136: Preparation of the compound of Compound No. 136.

Using 5-chloro-N-{5-[(1,1-dimethyl)ethyl]-2-methoxyphenyl}-2-hydroxybenzamide(Compound No. 135) and acetyl chloride as the raw materials, the same operation as the Example 5 gave the title compound.
Yield: 87.5%.
¹H-NMR(CDCl₃): δ 1.35(9H, s), 2.37(3H, s), 3.91(3H, s), 6.86(1H, d, J=8.7Hz),7.12(1H, dd, J=8.7, 2.4Hz), 7.13(1H, d, J=9.0Hz), 7.47(1H, dd, J=9.0, 2.4Hz), 8.02(1H, d, J=2.7Hz), 8.66(1H, d, J=2.4Hz), 8.93(1H, s).

### Example 137: Preparation of the compound of Compound No. 137.

Using 5-bromosalicylic acid and 3,5-dimethylaniline as the raw materials, the same operation as the Example 3 gave the title compound.
Yield: 58.1%.
mp 188-190°C.
¹H-NMR(DMSO-d₆): δ 2.28(6H, s), 6.80(1H, s), 6.96(1H, d, J=8.7Hz), 7.33(2H, s), 7.58(1H, dd, J=9.0, 2.4Hz), 8.10(1H, d, J=2.4Hz), 10.29(1H, s), 11.93(1H, brs).

### Example 138: Preparation of the compound of Compound No. 138.

Using 5-chlorosalicylic acid and 3,5-bis[(1,1-dimethyl)ethyl]aniline as the raw materials, the same operation as the Example 3 gave the title compound.
Yield: 34.1%.
¹H-NMR(CDCl₃): δ 1.26(18H, s), 6.99(1H, d, J=8.7Hz), 7.29(1H, t, J=1.8Hz), 7.39(1, dd, J=9.0, 2.4Hz), 7.41(2H, d, J=1.5Hz), 7.51(1H, d, J=2.1Hz), 7.81(1H, brs), 12.01(1H, s).

### Example 139: Preparation of the compound of Compound No. 139.

Using N-{3,5-bis[(1,1-dimethyl)ethyl]phenyl}-5-chloro-2-hydroxybenzamide (Compound No. 138) and acetyl chloride as the raw materials, the same operation as the Example 5 gave the title compound.
Yield: 66.1%.
¹H-NMR(CDCl₃): δ 1.34(18H, s), 2.36(3H, s), 7.12(1H, d, J=8.4Hz),7.25(1H, d, J=1.5Hz), 7.44(2H, d, J=1.2Hz), 7.47(1H; dd, J=8.7, 2.7Hz), 7.87(1H, d, J=2.4Hz), 7.98(1H, s).

### Example 140: Preparation of the compound of Compound No. 140.

Using 5-bromosalicylic acid and 3,5-bis[(1,1-dimethyl)ethyl]aniline as the raw materials, the same operation as the Example 3 gave the title compound.
Yield: 45.2%.
¹H-NMR(DMSO-d₆): δ 1.30(18H, s), 6.95(1H, d, J=8.7Hz), 7.20(1H, t, J=1.5Hz), 7.56(2H, d, J=1.5Hz), 7.58(1H, dd, J=8.7, 2.4Hz), 8.12(1H, d, J=2.7Hz), 10.39(1H, s), 11.98(1H, s).

### Example 141: Preparation of the compound of Compound No. 141.

Using 5-chlorosalicylic acid and 3-amino-4-methoxybiphenyl as the raw materials, the same operation as the Example 3 gave the title compound.
Yield: 37.0%.
¹H-NMR(DMSO-d₆): δ 3.95(3H, s), 7.08(1H, d, J=8.7Hz), 7.20(1H, d, J=8.4Hz), 7.34(1H, t, J=7.2Hz), 7.40-7.50(4H, m), 7.62(1H, d, J=8.7Hz), 8.00(1H, d, J=3.0Hz), 8.77(1H, d, J=2.1Hz), 10.92(1H, s), 12.09(1H, s).

### Example 142: Preparation of the compound of Compound No. 142.

Using 5-bromosalicylic acid and 2,5-dimethoxyaniline as the raw materials, the same operation as the Example 3 gave the title compound.
Yield: 39.7%.
¹H-NMR(DMSO-d₆): δ 3.72(3H, s), 3.84(3H, s), 6.66(1H, ddd, J=9.0, 3.0, 0.6Hz), 6.99-7.03(2H, m), 7.58(1H, ddd, J=9.0, 2.7, 0.6Hz), 8.10(1H, dd, J=2.4, 0.6Hz), 8.12(1H, d, J=3.0Hz), 10.87(1H, s), 12.08(1H, s).

### Example 143: Preparation of the compound of Compound No. 143.

Using 5-bromosalicylic acid and 3,5-dimethoxyaniline as the raw materials, the same operation as the Example 3 gave the title compound.
Yield: 40.3%.
mp 207-209°C.
¹H-NMR(DMSO-d₆): δ 3.75(6H, s), 6.30-6.32(1H, m), 6.94-6.97(3H, m), 7.57(1H, dd, J=8.7, 2.4Hz), 8.04(1H, d, J=2.4Hz), 10.32(1H, s), 11.78(1H, s).

### Example 144: Preparation of the compound of Compound No. 144.

Using 5-bromosalicylic acid and 5-aminoisophthalic acid dimethyl ester as the raw materials, the same operation as the Example 3 gave the title compound.
Yield: 74.1%.
mp 254-256°C.
¹H-NMR(DMSO-d₆): δ 3.92(6H, s), 6.97(1H, d, J=9.0Hz), 7.60(1H, dd, J=9.0, 2.4Hz), 8.06(1H, d, J=2.4Hz), 8.24-8.25(1H, m), 8.62(2H, m), 10.71(1H, s), 11.57(1H, s).

### Example 145: Preparation of the compound of Compound No. 145.

Using 5-methylsalicylic acid and 2,5-bis[(1,1-dimethyl)ethyl]aniline as the raw materials, the same operation as the Example 3 gave the title compound.
Yield: 61.1%.
¹H-NMR(DMSO-d₆): δ 1.27(9H, s), 1.33(9H, s), 2.28(3H, s), 6.89(1H, d, J=8.1Hz), 7.24(1H, d, J=2.1Hz), 7.27(1H, d, J=2.1Hz), 7.32(1H, d, J=2.4Hz), 7.37(1H, d, J=8.4Hz), 7.88(1H, d, J=1.5Hz), 10.15(1H, s), 11.98(1H, brs).

### Example 146: Preparation of the compound of Compound No. 146.

Using 5-nitrosalicylic acid and 3,5-bis[(1,1-dimethyl)ethyl]aniline as the raw materials, the same operation as the Example 3 gave the title compound.
Yield: 46.7%.
¹H-NMR(CDCl₃): δ 1.37(18H, s), 7.13(1H, d, J=9.3Hz), 7.32(1H, t, J=1.8Hz), 7.46(2H, d, J=1.8Hz), 8.07(1H, s), 8.33(1H, dd, J=9.3, 2.1Hz), 8.59(1H, d, J=2.4Hz), 13.14(1H, s).

### Example 147: Preparation of the compound of Compound No. 147.

Using 5-methylsalicylic acid and 3,5-bis[(1,1-dimethyl)ethyl]aniline as the raw materials, the same operation as the Example 3 gave the title compound.
Yield: 16.3%.
¹H-NMR(CDCl₃): δ 1.35(18H, s), 2.35(3H, s), 6.94(1H, d, H=8.4Hz), 7.23-7.28(2H, m), 7.31(1H, s), 7.42(1H, d, J=1.8Hz), 7.88(1H, s), 11.86(1H, s).

### Example 148: Preparation of the compound of Compound No. 148.

Using 5-methoxysalicylic acid and 3,5-bis[(1,1-dimethyl)ethyl]aniline as the raw materials, the same operation as the Example 3 gave the title compound.
Yield: 12.7%.
¹H-NMR(DMSO-d₆): δ 1.30(18H, s), 3.77(3H, s), 6.91(1H, d, J=9.0Hz), 7.07(1H, dd, J=8.7, 3.0Hz), 7.19-7.20(1H, m), 7.52-7.54(3H, m), 10.33(1H, s), 11.44(1H, s).

### Example 149: Preparation of the compound of Compound No. 149.

Using 5-methylsalicylic acid and 5-[(1,1-dimethyl)ethyl]-2-methoxyaniline as the raw materials, the same operation as the Example 3 gave the title compound.
Yield: 84.7%.
¹H-NMR(CDCl₃): δ 1.35(9H, s), 2.34(3H, s), 3.93(3H, s), 6.86(1H, d, J=8.7Hz), 6.93(1H, d, J=8.4Hz), 7.12(1H, dd, J=8.7, 2.4Hz), 7.24(1H, dd, J=8.4, 1.8Hz), 7.27(1H, brs), 8.48(1H, d, J=2.4Hz), 8.61(1H, brs), 11.95(1H, s).

### Example 150: Preparation of the compound of Compound No. 150.

Using 5-bromo-2-hydroxy-N-[3,5-bis(methoxycarbonyl)phenyl]benzamide (Compound No. 144) as the raw material, the same operation as the Example 109 gave the title compound.
Yield: 89.0%.
¹H-NMR(DMSO-d₆): δ 6.98(1H, d, J=8.7Hz), 7.60(1H, dd, J=8.7, 2.4Hz), 7.24(1H, dd, J=8.7, 2.7Hz), 8.08(1H, d, J=2.7Hz), 8.24(1H, t, J=1.5Hz), 8.57(2H, d, J=1.2Hz), 10.67(1H, s), 11.64(1H, s).

### Example 151: Preparation of the compound of Compound No. 151.

Using 5-chlorosalicylic acid and 2-methyl-5-[(1-methyl)ethyl]aniline as the raw materials, the same operation as the Example 3 gave the title compound.
Yield: 19.1%.
¹H-NMR(CDCl₃): δ 1.26(6H, d, J=6.9Hz), 2.30(3H, s), 2.87-2.96(1H, m), 7.00(1H, d, J=8.7Hz), 7.08(1H, dd, J=7.8, 1.8Hz), 7.20(1H, d, J=7.8Hz), 7.40(1H, dd, J=8.7, 2.4Hz), 7.49(1H, d, J=2.7Hz), 7.50(1H, s), 7.71(1H, s), 11.99(1H, s).

### Example 152: Preparation of the compound of Compound No. 152.

Using 5-chlorosalicylic acid and 2,5-diethoxyaniline as the raw materials, the same operation as the Example 3 gave the title compound.
Yield: 59.2%.
¹H-NMR(DMSO-d₆): δ 1.32(3H, t, J=6.9Hz), 1.41(3H, t, J=6.9Hz), 3.97(2H, q, J=6.9Hz), 4.06(2H, q, J=6.9Hz), 6.61(1H, dd, J=9.0, 3.0Hz), 6.98(1H, d, J=8.7Hz), 7.10(1H, d, J=8.7Hz), 7.48(1H, dd, J=8.7, 2.7Hz), 7.97(1H, d, J=2.7Hz), 8.16(1H, d, J=3.0Hz), 10.96(1H, s), 11.91(1H, s).

### Example 153: Preparation of the compound of Compound No. 153.

Using 5-chlorosalicylic acid and 2,5-dimethylaniline as the raw materials, the same operation as the Example 3 gave the title compound.
Yield: 90.5%.
¹H-NMR(CDCl₃): δ 2.28(3H, s), 2.35(3H, s), 6.99(1H, d, J=8.8Hz), 7.02(1H, brs), 7.15(1H, d, J=7.7Hz), 7.40(1H, dd, J=8.8, 2.5Hz), 7.45(1H, brs), 7.49(1H, d, J=2.5Hz)7.70(1H, br), 11.96(1H, brs).

### Example 154: Preparation of the compound of Compound No. 154.

Using 5-chlorosalicylic acid and 5-chloro-2-cyanoaniline as the raw materials, the same operation as the Example 3 gave the title compound.
Yield: 90.0%.
¹H-NMR(DMSO-d₆): δ 7.09(1H, d, J=9.0Hz), 7.53(1H, dd, J=8.7, 3.0Hz), 7.82(1H, dd, J=8.7, 2.4Hz), 7.95(1H, d, J=3.0Hz), 8.07(1H, d, J=2.4Hz), 8.36(1H, d, J=9.0Hz), 11.11(1H, s), 12.36(1H, s).

### Example 155: Preparation of the compound of Compound No. 155.

Using 5-chlorosalicylic acid and 5-(N,N-diethylsulfamoyl)-2-methoxyaniline as the raw materials, the same operation as the Example 3 gave the title compound.
Yield: 44.8%.
¹H-NMR(CDCl₃): δ 1.17(6H, t, J=7.3Hz), 3.29(4H, q, J=7.3Hz), 4.05(3H, s), 7.00(2H, dd, J=2.3, 8.9Hz), 7.41(1H, dd, J=2.3, 8.9Hz), 7.48(1H, d, J=2.6Hz), 7.65(1H, dd, J=2.3, 8.6Hz), 8.56(1H, br.s), 8.84(1H, d, J=2.3Hz), 11.82(1H, s).

### Example 156: Preparation of the compound of Compound No. 156.

Using 5-chlorosalicylic acid and 2-chloro-5-nitroaniline as the raw materials, the same operation as the Example 3 gave the title compound.
Yield: 73.3%.
¹H-NMR(CD₃OD): δ 6.98(1H, d, J=8.6Hz), 7.43(1H, dd, J=2.6, 8.6Hz), 7.74(1H, d, J=8.9Hz), 7.99(1H, dd, J=3.0, 8.9Hz), 8.08(1H, d, J=2.6Hz), 9.51(1H, d, J=2.6Hz)

### Example 157: Preparation of the compound of Compound No. 157.

Using 5-chlorosalicylic acid and 5-(N-phenylcarbamoyl)-2-methoxyaniline as the raw material, the same operation as the Example 3 gave the title compound.
Yield: 40.3%.
¹H-NMR(DMSO-d₆): δ 3.99(3H, s), 7.09(2H, dd, J=6.6, 6.9Hz), 7.24(1H, d, J=8.6Hz), 7.35(2H, dd, 6.9, 7.3Hz), 7.49(1H, d, J=2.3, 8.9Hz), 7.77(3H, d, J=8.6Hz), 8.00(1H, s), 8.97(1H, s), 10.17(1H, s), 10.91(1H, s), 12.11(1H, s).

### Example 158: Preparation of the compound of Compound No. 158.

Using 5-chlorosalicylic acid and 2,5-dimethoxyaniline as the raw materials, the same operation as the Example 3 gave the title compound.
Yield: 73.9%.
¹H-NMR(CDCl₃): δ 3.82(3H, s), 3.93(3H, s), 6.66(1H, dd, J=3.0, 8.9Hz), 6.86(1H, d, J=8.9Hz), 6.98(1H, d, J=8.9Hz), 7.39(1H, dd, J=2.6, 8.9Hz), 7.47(1H, d, J=2.6Hz), 8.08(1H, d, J=3.0Hz), 8.60(1H, br.s), 12.03(1H, s).

### Example 159: Preparation of the compound of Compound No. 159.

Using 5-chlorosalicylic acid and 5-acetylamino-2-methoxyaniline as the raw materials, the same operation as the Example 3 gave the title compound.
Yield: 16.9%.
¹H-NMR(DMSO-d₆): δ 2.01(3H, s), 3.85(3H, s), 7.03(2H, t, J=9.6Hz), 7.49(2H, dd, J=8.9, 9.2Hz), 7.96(1H, s), 8.51(1H, s), 9.87(1H, s), 10.82(1H, s), 12.03(1H, d, J=4.0Hz).

### Example 160: Preparation of the compound of Compound No. 160.

Using 5-chlorosalicylic acid and 5-methoxy-2-methylaniline as the raw materials, the same operation as the Example 3 gave the title compound.
Yield: 100%.
¹HNMR(CDCl₃): δ 2.29(3H, s), 3.82(3H, s), 6.75(1H, dd, J=2.6, 8.2Hz), 7.00(1H, d, J=8.9Hz), 7.16(1H, d, J=8.6Hz), 7.38(1H, d, 2.3Hz), 7.41(1H, dd, J=2.3, 8.9Hz), 7.48(1H, d, J=2.3Hz), 7.70(1H, br.s), 11.92(1H, s).

### Example 161: Preparation of the compound of Compound No. 161.

Using 5-chlorosalicylic acid and 2,5-dibutoxyaniline as the raw materials, the same operation as the Example 3 gave the title compound.
Yield: 73.9%.
¹H-NMR(CDCl₃): δ 0.98(3H, t, J=7.2Hz), 1.05(3H, t, J=7.2Hz), 1.44-1.65(4H, m), 1.72-1.79(2H, m), 1.81-1.91(2H, m), 3.97(2H, t, J=6.3Hz), 4.07(2H, t, J=6.3Hz), 6.64(1H, dd, J=9.0, 3.0Hz), 6.85(1H, d, J=9.3Hz), 6.99(1H, d, J=9.0Hz), 7.39(1H, dd, J=8.7, 2.4Hz), 7.44(1H, d, J=2.7Hz), 8.08(1H, d, J=3.0Hz), 8.76(1H, s), 12.08(1H, s).

### Example 162: Preparation of the compound of Compound No. 162.

Using 5-chlorosalicylic acid and 2,5-diisopentyloxyaniline as the raw materials, the same operation as the Example 3 gave the title compound.
Yield: 59.7%.
¹H-NMR(CDCl₃): δ 0.97(6H, d, J=6.6Hz), 1.03(6H, d, 6.6Hz), 1.64-1.98(6H, m), 3.99(2H, t, J=6.6Hz), 4.09(2H, t, J=6.3Hz), 6.63(1H, dd, J=8.7, 3.0Hz), 6.85(1H, d, J=8.7Hz), 6.98(1H, d, J=8.7Hz), 7.38(1H, dd, J=9.0, 2.4Hz), 7.43(1H, d, J=2.7Hz), 8.09(1H, d, J=3.0Hz), 8.75(1H, s), 12.08(1H, s).

### Example 163: Preparation of the compound of Compound No. 163.

Using 5-chlorosalicylic acid and 5-carbamoyl-2-methoxyaniline as the raw materials, the same operation as the Example 3 gave the title compound.
Yield: 31.2%.
¹H-NMR(CD₃OD): δ 4.86(3H, s), 6.93(1H, d, J=7.6Hz), 7.18(1H, d, J=8.6Hz), 7.35(1H, dd, J=3.0, 7.6Hz), 7.47(1H, dd, J=2.0, 8.6Hz), 8.00(1H, d, J=3.0Hz), 8.80(1H, d, J=2.0Hz).

### Example 164: Preparation of the compound of Compound No. 164.

Using 5-chlorosalicylic acid and 5-[(1,1-dimethyl)propyl]-2-phenoxyaniline as the raw materials, the same operation as the Example 3 gave the title compound.
Yield: 65.2%.
¹H-NMR(CDCl₃): δ 0.69(3H, t, J=7.6Hz), 1.29(6H, s), 1.64(2H, q, J=7.6Hz), 6.91(1H, dd, J=1.7, 7.6Hz), 6.96(1H, d, J=8.9Hz), 7.03(2H, d, J=8.9Hz), 7.10(1H, dt, J=1.7, 7.6Hz), 7.16(1H, dt, J=1.7, 7.6Hz), 7.31-7.40(4H, m), 8.42(1H, dd, J=2.0, 7.9Hz), 8.53(1H, br.s)11.94(1H, s).

### Example 165: Preparation of the compound of Compound No. 165.

Using 5-chlorosalicylic acid and 2-hexyloxy-5-(methylsulfonyl)aniline as the raw materials, the same operation as the Example 3 gave the title compound.
Yield: 33.0%.
¹H-NMR(CDCl₃): δ 0.92(3H, t, J=6.9Hz), 1.40-1.59(6H, m), 1.90-2.01(2H, m), 3.09(3H, s), 4.22(2H, t, J=6.3Hz), 7.01(1H, d, J=8.9Hz), 7.06(1H, d, J=8.6Hz), 7.40-7.43(2H, m), 7.73(1H, dd, J=8.6, 2.3Hz), 8.74(1H, brs), 8.99(1H, d, J=2.3Hz), 11.76(1H, s).

### Example 166: Preparation of the compound of Compound No. 163.

Using 5-chlorosalicylic acid and 3'-amino-2,2,4'-trimethylpropiophenone as the raw materials, the same operation as the Example 3 gave the title compound.
Yield: 44.8%.
¹H-NMR(CDCl₃): δ 1.38(9H, s), 2.38(3H, s), 7.01(1H, d, J=8.9Hz), 7.31(1H, d, J=7.9Hz), 7.42(1H, dd, J=8.9, 2.6Hz), 7.53(1H, d, J=2.6Hz), 7.57(1H, dd, J=7.9, 2.0Hz), 7.83(1H, brs), 8.11(1H, d, J=2.0Hz), 11.82(1H, s).

### Example 167: Preparation of the compound of Compound No. 167.

Using 5-chlorosalicylic acid and 5-methoxy-2-(1-pyrrolyl)aniline as the raw materials, the same operation as the Example 3 gave the title compound.
Yield: 53.4%.
¹H-NMR(CDCl₃): δ 2.46(3H, s), 6.51-6.52(2H, m), 6.82-6.85(3H, m), 6.93(1H, d, J=8.9Hz), 7.06(1H, d, J=7.9Hz), 7.30(1H, d, J=7.9Hz), 7.32(1H, dd, J=2.3, 8.9Hz), 7.61(1H, s), 8.29(1H, s), 11.86(1H, br.s).

### Example 168: Preparation of the compound of Compound No. 168.

Using 5-chlorosalicylic acid and 5-chloro-2-tosylaniline as the raw materials, the same operation as the Example 3 gave the title compound.
Yield: 8.0%.
¹H-NMR(CDCl₃): δ 2.38(3H, s), 7.02(1H, d, J=8.9Hz), 7.25-7.31(3H, m), 7.46(1H, dd, J=2.6, 8.9Hz), 7.68(2H, d, J=8.6Hz), 7.74(1H, d, J=2.3Hz), 7.96(1H, d, J=8.6Hz), 8.56(1H, d, J=2.0Hz), 10.75(1H, s), 11.70(1H, s).

### Example 169: Preparation of the compound of Compound No. 169.

Using 5-chlorosalicylic acid and 2-chloro-5-tosylaniline as the raw materials, the same operation as the Example 3 gave the title compound.
Yield: 43.5%.
¹H-NMR(CDCl₃): δ 2.38(3H, s), 7.02(1H, d, J=8.9Hz), 7.27(1H, d, J=7.9Hz), 7.29(1H, dd, J=2.0, 6.6Hz), 7.46(1H, dd, J=2.3, 8.9Hz), 7.68(2H, d, J=8.6Hz), 7.73(2H, d, J=2.3Hz), 7.97(1H, d, J=8.6Hz), 8.56(1H, d, J=2.0Hz), 10.73(1H, s), 11.71(1H, s).

### Example 170: Preparation of the compound of Compound No. 170.

Using 5-chlorosalicylic acid and 2-fluoro-5-(methylsulfonyl)aniline as the raw materials, the same operation as the Example 3 gave the title compound.
Yield: 28.8%.
¹H-NMR(CDCl₃): δ 3.12(3H, s), 7.03(1H, d, J=8.9Hz), 7.38(1H, dd, J=8.6, 10.2Hz), 7.45(1H, dd, J=2.3, 8.9Hz), 7.53(1H, d, J=2.3Hz), 7.80(1H, ddd, J=2.3, 4.6, 8.6Hz), 8.25(1H, s), 8.98(1H, dd, J=2.3, 7.7Hz), 11.33(1H, br.s).

### Example 171: Preparation of the compound of Compound No. 171.

Using 5-chlorosalicylic acid and 2-methoxy-5-phenoxyaniline as the raw materials, the same operation as the Example 3 gave the title compound.
Yield: 77.0%.
¹H-NMR(CDCl₃): δ 3.98(3H, s), 6.80(1H, d, J=8.8Hz), 6.90(1H, d, J=8.8Hz), 6.95-7.00(3H, m), 7.04-7.09(1H, m), 7.29-7.35(2H, m), 7.38(1H, dd, J=8.8, 2.6Hz), 7.47(1H, d, J=2.6Hz), 8.19(1H, d, J=2.9Hz), 8.61(1H, brs), 11.92(1H, s).

### Example 172: Preparation of the compound of Compound No. 172.

Using 5-chlorosalicylic acid and 3-amino-4-methylbiphenyl as the raw materials, the same operation as the Example 3 gave the title compound.
Yield: 47.7%.
¹H-NMR(DMSO-d₆): δ 2.33(3H, s), 7.06(1H, d, J=8.7Hz), 7.43-7.52(4H, m), 7.64-7.67(2H, m), 8.04(1H, d, J=2.7Hz), 8.19(1H, d, J=1.5Hz), 10.40(1H, s), 12.22(1H, s).

### Example 173: Preparation of the compound of Compound No. 173.

Using 5-chlorosalicylic acid and 5-(α,α-dimethylbenzyl)-2-methoxyaniline as the raw materials, the same operation as the Example 3 gave the title compound.
Yield: 89.0%.
¹H-NMR(CDCl₃): δ 1.72(6H, s), 3.93(3H, s), 6.83(1H, d, J=8.8Hz), 6.93(1H, dd, J=2.6, 8.8Hz), 6.96(1H, d, J=9.2Hz), 7.15-7.20(1H, m), 7.25-7.28(4H, m), 7.36(1H, dd, J=2.6, 8.8Hz), 7.46(1H, d, J=2.6Hz), 8.35(1H, d, J=2.6Hz), 8.51(1H, s), 12.04(1H, s).

### Example 174: Preparation of the compound of Compound No. 174.

Using 5-chlorosalicylic acid and 5-morpholino-2-nitroaniline as the raw materials, the same operation as the Example 3 gave the title compound.
Yield: 4.1%.
¹H-NMR(DMSO-d₆): δ 3.46-3.52(4H, m), 3.85-3.94(4H, m), 7.03(1H, d, J=8.8Hz), 7.47(1H, dd, J=2.9, 8.8Hz), 7.80(1H, dd, J=2.6, 8.8Hz), 7.82(1H, d, J=2.6Hz), 7.88(1H, d, J=8.8Hz), 8.20(1H, d, J=2.2Hz), 10.70(1H, s), 11.43(1H, s)

### Example 175: Preparation of the compound of Compound No. 175.

Using 5-chlorosalicylic acid and 5-fluoro-2-(1-imidazolyl)aniline as the raw materials, the same operation as the Example 3 gave the title compound.
Yield: 33.8%.
¹H-NMR(DMSO-d₆): δ 6.99(1H, d, J=8.8Hz), 7.12-7.19(2H, m), 7.42-7.51(3H, m), 7.89(1H, d, J=2.8Hz), 7.93(1H, d, J=1.1Hz), 8.34(1H, dd, J=11.4, 2.8Hz), 10.39(1H, s), 11.76(1H, brs).

### Example 176: Preparation of the compound of Compound No. 176.

Using 5-chlorosalicylic acid and 2-butyl-5-nitroaniline as the raw materials, the same operation as the Example 3 gave the title compound.
Yield: 15.3%.
¹H-NMR(CDCl₃): δ 0.99(3H, t, J=7.3Hz), 1.39-1.51(2H, m), 1.59-1.73(2H, m), 2.71-2.79(2H, m), 7.03(1H, d, J=8.9Hz), 7.41-7.49(3H, m), 7.92(1H, s), 8.07(1H, dd, J=2.3, 8.4Hz), 8.75(1H, d, J=2.4Hz), 11.51(1H, s).

### Example 177: Preparation of the compound of Compound No. 177.

Using 5-chlorosalicylic acid and 5-[(1,1-dimethyl)propyl]-2-hydroxyaniline as the raw materials, the same operation as the Example 3 gave the title compound.
Yield: 36.0%.
¹H-NMR(CDCl₃): δ 0.70(3H, t, J=7.4Hz), 1.28(6H, s), 1.63(2H, q, J=7.4Hz), 6.97(1H, d, J=6.3Hz), 7.00(1H, d, J=6.6Hz), 7.08(1H, s), 7.14(1H, dd, J=2.5, 8.6Hz), 7.36(1H, d, J=2.2Hz), 7.42(1H, dd, J=2.5, 8.8Hz), 7.57(1H, d, J=2.5Hz), 8.28(1H, s), 11.44(1H, s).

### Example 178: Preparation of the compound of Compound No. 178.

Using 5-chlorosalicylic acid and 2-methoxy-5-methylaniline as the raw materials, the same operation as the Example 3 gave the title compound.
Yield: 74.2%.
¹H-NMR(DMSO-d₆): δ 2.27(3H, s), 3.85(3H, s), 6.90(1H, dd, J=9.0, 2.4Hz), 6.98(1H, d, J=9.0Hz), 7.05(1H, d, J=9.0Hz), 7.47(1H, dd, J=9.0, 3.0Hz), 7.97(1H, d, J=3.0Hz), 8.24(1H, d, J=2.4Hz), 10.79(1H, s), 12.03(1H, s).

### Example 179: Preparation of the compound of Compound No. 179.

Using 5-chlorosalicylic acid and 2,5-difluoroaniline as the raw materials, the same operation as the Example 3 gave the title compound.
Yield: 81.5%.
¹H-NMR(DMSO-d₆): δ 6.98-7.07(1H, m), 7.07(1H, d, J=9.0Hz), 7.37-7.49(1H, m), 7.52(1H, dd, J=8.7, 3.0Hz), 7.95(1H, d, J=2.7Hz), 8.15-8.22(1H, m), 10.83(1H, s), 12.25(1H, s).

### Example 180: Preparation of the compound of Compound No. 180.

Using 5-chlorosalicylic acid and 3,5-difluoroaniline as the raw materials, the same operation as the Example 3 gave the title compound.
Yield: 82.0%.
¹H-NMR(DMSO-d₆): δ 7.00(1H, tt, J=9.3, 2.1), 7.03(1H, d, J=9.0Hz), 7.47(1H, dd, J=7.5, 2.7Hz), 7.49(1H, d, J=2.7Hz), 7.51(1H, d, J=2.1Hz), 7.82(1H, d, J=3.0Hz), 10.63(1H, s), 11.43(1H, brs).

### Example 181: Preparation of the compound of Compound No. 181.

Using 3-hydroxynaphthalene-2-carboxylic acid and 3,5-dichloroaniline as the raw materials, the same operation as the Example 3 gave the title compound.
Yield: 44.3%.
mp 254-255°C.
¹H-NMR(DMSO-d₆): δ 7.34-7.39(3H, m), 7.49-7.54(1H, m), 7.76-7.79(1H, m), 7.89(2H, d, J=1.8Hz), 7.92(1H, m), 8.39(1H, s), 10.75(1H, s), 11.01(1H, s).

### Example 182: Preparation of the compound of Compound No. 182.

Using 2-hydroxynaphthalene-1-carboxylic acid and 3,5-dichloroaniline as the raw materials, the same operation as the Example 3 gave the title compound.
Yield: 51.2%.
mp 246-248°C.
¹H-NMR(DMSO-d₆): δ 7.26(1H, d, J=9.3Hz), 7.31-7.37(2H, m), 7.44-7.50(1H, m), 7.65-7.68(1H, m), 7.85-7.90(4H, m), 10.23(1H, s), 10.74(1H, s).

### Example 183: The compound of Compound No. 183.

This compound is a commercially available compound.
Supplier: Sigma-Aldrich.
Catalog code number: S01361-8.

### Example 184: Preparation of the compound of Compound No. 184.

Using 5-chloro-2-hydroxynicotinic acid and 3,5-bis[(1,1-dimethyl)ethyl]aniline as the raw materials, the same operation as the Example 75 gave the title compound. Yield: 59.1%.
¹H-NMR(DMSO-d₆): δ 1.29(18H, s), 7.18(1H, t, J=1.8Hz), 7.52(2H.d, J=1.8Hz), 8.07(1H, d, J=2.4Hz), 8.35(1H, d, J=3.3Hz), 11.92(1H, s), 13.10(1H, s).

### Example 185: Preparation of the compound of Compound No. 185.

### (1) 2-Amino-4-[(1,1-dimethyl)ethyl]thiazole.

A mixture of 1-bromo-3,3-dimethyl-2-butanone(5.03g, 28.1mmol), thiourea(2.35g, 30.9mmol) and ethanol(30mL) was refluxed for 1.5 hours. After the reaction mixture was cooled to room temperature, it was poured into saturated aqueous sodium hydrogen carbonate and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=2:1→1:1) to give the title compound(3.99g, 90.9%) as an yellowish white powder.
¹H-NMR(CDCl₃):d 1.26(9H, s), 4.96(2H, brs), 6.09(1H, s).

When the method described in Example 185(1) is referred in the following examples, solvents such as ethanol or the like were used as the reaction solvent.

### (2) 2-Acetoxy-5-bromo-N-{4-[(1,1-dimethyl)ethyl]thiazol-2-yl}benzamide.

Using 2-acetoxy-5-bromobenzoic acid and 2-amino-4-[(1,1-dimethyl)ethyl]thiazole as the raw materials, the same operation as the Example 75 gave the title compound.
Yield: 59.4%.
¹H-NMR(CDCl₃):d 1.31(9H, s), 2.44(3H, s), 6.60(1H, s), 7.13(1H, d, J=8.4Hz), 7.68(1H, dd, J=8.7, 2.4Hz), 8.17(1H, d, J=2.4Hz), 9.72(1H, brs). [2-Acetoxy-5-bromosalicylic acid: It was obtained, using 5-bromosalicylic acid and acetic anhydride as the raw materials, by the same operation as the Example 34(1) with reference to "European Journal of Medicinal Chemistry", (France), 1996, Vol.31, p.861-874. It was obtained by the same operation as the following Example 244(1).]

### (3) 5-Bromo-N-{4-[(1,1-dimethyl)ethyl]thiazol-2-yl}-2-hydroxybenzamide(Compound No. 185).

2N Sodium hydroxide(0.2mL) was added to a solution of 2-acetoxy-5-bromo-N-{4-[(1,1-dimethyl)ethyl]thiazol-2-yl}benzamide(100. 1mg, 0.25mmol) in tetrahydrofuran(3mL), and the mixture was stirred at room temperature for 20 minutes. The reaction mixture was poured into diluted hydrochloric acid and extracted with ethyl acetate. After the ethyl acetate layer was washed with brine, dried over anhydrous sodium sulfate, the residue obtained by evaporation of the solvent under reduced pressure was crystallized by isopropyl ether/n-hexane to give the title compound(70.1mg, 78.9%) as a white powder.
¹H-NMR(DMSO-d₆): δ 1.30(9H, s), 6.80(1H, brs), 6.95(1H, brs), 7.57(1H, brs), 8.06(1H, d, J=2.4Hz), 11.82(1H, brs), 13.27(1H, brs).

### Example 186: Preparation of the compound of Compound No. 186.

### (1) 2-Acetoxy-5-bromo-N-{5-bromo-4-[(1,1-dimethyl)ethyl]thiazol-2-yl}benzamide.

N-Bromosuccinimide(97.9mg, 0.55mmol) was added to a solution of 2-acetoxy-5-bromo-N-{4-[(1,1-dimethyl)ethyl]thiazol-2-yl}benzamide(compound of Example 185(2); 0.20g, 0.50mmol) in acetonitrile(10mL), and the mixture was stirred at room temperature for 1 hour. The residue obtained by evaporation of the solvent under reduced pressure was purified by column chromatography on silica gel(n-hexane:ethyl acetate=3:1) to give the title compound as a crude product.

### (2) 5-Bromo-N-{5-bromo-4-[(1,1-dimethyl)ethyl]thiazol-2-yl}-2-hydroxybenzamide (Compound No. 186).

Using 2-acetoxy-5-bromo-N-{5-bromo-4-[(1,1-dimethyl)ethyl]thiazol-2-yl}-benzamide as the raw material, the same operation as the Example 2 gave the title compound.
Yield: 90.9%(2 steps).
¹H-NMR(DMSO-d₆): δ 1.42(9H, s), 6.99(1H, d, J=8.7Hz), 7.61(1H, dd, J=8.7, 2.7Hz), 8.02(1H, d, J=2.4Hz), 11.79(1H, brs), 12.00(1H, brs).

### Example 187: Preparation of the compound of Compound No. 187.

Using 5-bromosalicylic acid and 2-amino-5-bromo-4-(trifluoromethyl)thiazole as the raw materials, the same operation as the Example 3 gave the title compound.
Yield: 22.4%.
mp 215°C(dec.).
¹H-NMR(DMSO-d₆): δ 7.00(1H, d, J=8.8Hz), 7.61(1H, dd, J=8.8, 2.8Hz), 7.97(1H, d, J=2.4Hz).
[2-Amino-5-bromo-4-(trifluoromethyl)thiazole: Refer to "Journal of Heterocyclic Chemistry", (USA), 1991, Vol.28, p.1017.]

### Example 188: Preparation of the compound of Compound No. 188.

### (1) α-Bromo-pivaloylacetonitrile.

N-Bromosuccinimide(1.42g, 7.99mmol) was added to a solution of pivaloylacetonitrile(1.00g, 7.99mmol) in carbon tetrachloride(15mL), and the mixture was refluxed for 15 minutes. After the reaction mixture was cooled to room temperature, the insoluble matter was filtered off, and the residue obtained by evaporation of the filtrate under reduced pressure was purified by column chromatography on silica gel(n-hexane:ethyl acetate=4:1) to give the title compound(1.43g, 87.9%) as an yellowish brown oil.
¹H-NMR(CDCl₃): δ 1.33(9H, s), 5.10(1H, s).

When the method described in Example 188(1) is referred in the following examples, N-bromosuccinimide was used as the brominating agent. As the reaction solvent, solvents such as carbon tetrachloride or the like were used.

### (2) 2-Amino-5-cyano-4-[(1,1-dimethyl)ethyl]thiazole.

Using α-bromo-pivaloylacetonitrile and thiourea as the raw materials, the same operation as the Example 185(1) gave the title compound.
Yield: 66.3%.
¹H-NMR(CDCl₃): δ 1.41(9H, s), 5.32(2H, s).

### (3) 5-Chloro-N-{5-cyano-4-[(1,1-dimethyl)ethyl]thiazol-2-yl}-2-hydroxybenzamide (Compound No. 188).

Using 5-chlorosalicylic acid and 2-amino-5-cyano-4-[(1,1-dimethyl)-ethyl]thiazole as the raw materials, the same operation as the Example 3 gave the title compound.
Yield: 63.4%.
¹H-NMR(DMSO-d₆): δ 1.43(9H, s), 7.06(1H, d, J=8.7Hz), 7.51(1H, dd, J=8.7, 3.0Hz), 7.85(1H, d, J=2.7Hz), 12.31(2H, br).

### Example 189: Preparation of the compound of Compound No. 189.

Using 5-bromosalicylic acid and 2-amino-5-cyano-4-[(1,1-dimethyl)ethyl]-thiazole(compound of Example 188(2)) as the raw materials, the same operation as the Example 3 gave the title compound.
Yield: 61.3%.
¹H-NMR(DMSO-d₆): δ 1.43(9H, s), 7.00(1H, d, J=8.7Hz), 7.62(1H, dd, J=8.7, 2.7Hz), 7.97(1H, d, J=2.7Hz), 11.75(1H, br), 12.43(1H, br).

### Example 190: Preparation of the compound of Compound No. 190.

Using 5-bromosalicylic acid and 2-amino-5-methylthiazole as the raw materials, the same operation as the Example 3 gave the title compound.
Yield: 12.9%.
¹H-NMR(DMSO-d₆): δ 2.33(3H, s), 6.91(1H, d, J=7.6Hz), 7.26(1H, s), 7.54(1H, d, J=9.6Hz), 8.03(1H, d, J=2.8Hz).

### Example 191: Preparation of the compound of Compound No. 191.

Using 5-bromosalicylic acid and 2-amino-4,5-dimethylthiazole as the raw materials, the same operation as the Example 3 gave the title compound.
Yield: 14.4%.
¹H-NMR(DMSO-d₆): δ 2.18(3H, s), 2.22(3H, s), 6.89(1H, d, J=8.8Hz), 7.51(1H, d, J=6.8Hz), 8.02(1H, d, J=2.8Hz), 13.23(1H, brs).

### Example 192: Preparation of the compound of Compound No. 192.

Using 5-bromosalicylic acid and 2-amino-5-methyl-4-phenylthiazole as the raw materials, the same operation as the Example 3 gave the title compound.
Yield: 27.7%.
mp 243-244°C.
¹H-NMR(CD₃OD): δ 2.47(3H, s), 6.92(1H, d, J=8.7Hz), 7.36-7.41(1H, m), 7.44-7.50(2H, m), 7.53(1H, dd, J=9.0, 2.7Hz), 7.57-7.61(2H, m), 8.16(1H, d, J=2.7Hz).
[2-Amino-5-methyl-4-phenylthiazole: Refer to "Yakugaku Zasshi: Journal of The Pharmaceutical Society of Japan", 1961, Vol.81, p.1456.]

### Example 193: Preparation of the compound of Compound No. 193.

Using (4-fluorophenyl)acetone as the raw material, the same operation as the Examples 188(1)-(3) gave the title compound.
Yield: 28.8%(3 steps).

### (1) α-Bromo-(4-fluorophenyl)acetone.

¹H-NMR(CDCl₃): δ 2.33(3H, s), 5.41(1H, s), 7.07(2H, t, J=8.7Hz), 7.43(2H, dd, J=8.7, 5.1Hz).

### (2) 2-Amino-4-methyl-5-(4-fluorophenyl)thiazole.

¹H-NMR(CDCl₃): δ 2.27(3H, s), 4.88(2H, s), 7.07(2H, t, J=8.7Hz), 7.32(2H, dd, J=8.7, 5.4Hz).

### (3) 5-Bromo-N-[4-methyl-5-(4-fluorophenyl)thiazol-2-yl]-2-hydroxybenzamide (Compound No. 193).

¹H-NMR(DMSO-d₆): δ 2.36(3H, s), 6.95(1H, d, J=8.4Hz), 7.33(2H, t, J=8.7Hz), 7.52-7.59(3H, m), 8.06(1H, d, J=3.0Hz), 12.01-13.65(2H, br).

### Example 194: Preparation of the compound of Compound No. 194.

Using 3-(trifluoromethyl)phenylacetone as the raw material, the same operation as the Examples 188(1)-(3) gave the title compound.
Yield: 39.8%(3 steps).

### (1) α-Bromo-3-(trifluoromethyl)phenylacetone.

¹H-NMR(CDCl₃): δ 2.38(3H, s), 5.43(1H, s), 7.52(1H, t, J=7.8Hz), 7.61-7.66(2H, m), 7.69-7.70(1H, m).

### (2) 2-Amino-4-methyl-5-[3-(trifluoromethyl)phenyl]thiazole.

¹H-NMR(CDCl₃): δ 2.32(3H, s), 4.95(2H, s), 7.46-7.56(3H, m), 7.59-7.61(1H, m).

### (3) 5-Bromo-N-{4-methyl-5-[3-(trifluoromethyl)phenyl]thiazol-2-yl}-2-hydroxybenzamide(Compound No. 194).

¹H-NMR(DMSO-d₆): δ 2.40(3H, s), 6.97(1H, d, J=8.7Hz), 7.59(1H, dd, J=8.7, 2.4Hz), 7.71-7.84(4H, m), 8.06(1H, d, J=2.4Hz), 12.09(1H, br), 12.91-13.63(1H, br).

### Example 195: Preparation of the compound of Compound No. 195.

Using 2,2-dimethyl-3-hexanone as the raw material, the same operation as the Examples 188(1)-(3) gave the title compound.
Yield: 17.0%(3 steps).

### (2) 2-Amino-4-[(1,1-dimethyl)ethyl]-5-ethylthiazole.

¹H-NMR(CDCl₃): δ 1.21(3H, t, J=7.5Hz), 1.32(9H, s), 2.79(2H, q, J=7.5Hz), 4.63(2H, brs).

### (3) 5-Bromo-N-{4-[(1,1-dimethyl)ethyl]-5-ethylthiazol-2-yl}-2-hydroxybenzamide (Compound No. 195).

¹H-NMR(CDCl₃): δ 1.32(3H, t, J=7.5Hz), 1.41(9H, s), 2.88(2H, q, J=7.5Hz), 6.84(1H, d, J=9.0Hz), 7.44(1H, dd, J=8.7, 2.4Hz), 8.05(1H, d, J=2.7Hz), 11.46(2H, br).

### Example 196: Preparation of the compound of Compound No. 196.

Using 5-bromosalicylic acid and 2-amino-4-ethyl-5-phenylthiazole as the raw materials, the same operation as the Example 3 gave the title compound.
Yield: 17.4%.
mp 224-225°C.
¹H-NMR(DMSO-d₆): δ 1.24(3H, t, J=7.6Hz), 2.70(2H, q, J=7.6Hz), 6.95(1H, brd, J=7.6Hz), 7.39-7.42(1H, m), 7.45-7.51(4H, m), 7.56(1H, brd, J=8.0Hz), 8.06(1H, d, J=2.8Hz), 11.98(1H, brs).

### Example 197: Preparation of the compound of Compound No. 197.

Using benzyl isopropyl ketone as the raw material, the same operation as the Examples 188(1)-(3) gave the title compound.
Yield: 4.4%(3 steps).

### (2) 2-Amino-4-isopropyl-5-phenylthiazole.

¹H-NMR(CDCl₃): δ 1.23(6H, d, J=6.6Hz), 3.05(1H, m), 4.94(2H, s), 7.28-7.41(5H, m).

### (3) 5-Bromo-N-(4-isopropyl-5-phenylthiazol-2-yl)-2-hydroxybenzamide(Compound No. 197).

¹H-NMR(DMSO-d₆): δ 1.26(6H, d, J=6.0Hz), 3.15(1H, m), 6.98(1H, brs), 7.43-7.53(5H, m), 7.59(1H, brs), 8.08(1H, d, J=2.7Hz), 11.90(1H, brd), 13.33(1H, brd).

### Example 198: Preparation of the compound of Compound No. 198.

Using 1-phenyl-2-hexanone as the raw material, the same operation as the Examples 188(1)-(3) gave the title compound.
Yield: 52.6%(3 steps).

### (1) α-Bromo-1-phenyl-2-hexanone.

¹H-NMR(CDCl₃): δ 0.85(3H, t, J=7.2Hz), 1.19-1.32(2H, m), 1, 50-1.60(2H, m), 2.59(2H, td, J=7.5, 3.9Hz), 5.44(1H, s), 7.34-7.45(5H, m).

### (2) 2-Amino-4-butyl-5-phenylthiazole.

¹H-NMR(CDCl₃): δ 0.89(3H, t, J=7.5Hz), 1.28-1.41(2H, m), 1.61-1.71(2H, m), 2.56-2.61(2H, m), 4.87(2H, s), 7.25-7.40(5H, m).

### (3) 5-Bromo-N-(4-butyl-5-phenylthiazol-2-yl)-2-hydroxybenzamide(Compound No. 198).

¹H-NMR(DMSO-d₆): δ 0.85(3H, t, J=7.2Hz), 1.23-1.35(2H, m), 1.59-1.69(2H, m), 2.70(2H, t, J=7.2Hz), 6.96(1H, d, J=6.9Hz), 7.39-7.59(6H, m), 8.07(1H, d, J=2.4Hz), 11.93(1H, br), 13.18-13.59(1H, br).

### Example 199: Preparation of the compound of Compound No. 199.

### (1) 4-Bromo-2,2,6,6-tetramethyl-3,5-heptanedione [α-Bromo-dipivaloylmethane].

N-Bromosuccinimide(965.8mg, 5.42mmol) was added to a solution of 2,2,6,6-tetramethyl-3,5-heptanedione(dipivaloylmethane; 1.00g, 5.42mmol) in carbon tetrachloride(10mL), and the mixture was refluxed for 2 hours. After the reaction mixture was cooled to room temperature, the insoluble matter was filtered off, and the filtrate was evaporated under reduced pressure to give the title compound(1.42g, quant.) as a white crystal.
¹H-NMR(CDCl₃): δ 1.27(18H, s), 5.67(1H, s).

When the method described in Example 199(1) is referred in the following examples, N-bromosuccinimide was used as the brominating agent. As the reaction solvent, solvents such as carbon tetrachloride or the like were used.

### (2) 2-Amino-4-[(1,1-dimethyl)ethyl]-5-[(2,2-dimethyl)propionyl]thiazole.

A mixture of 4-bromo-2,2,6,6-tetramethyl-3,5-heptanedione(α-bromo-dipivaloylmethane; 1.42g, 5.40mmol), thiourea(451.8mg, 5.94mmol) and ethanol(15mL) was refluxed for 2 hours. After the reaction mixture was cooled to room temperature, it was poured into saturated aqueous sodium hydrogen carbonate and extracted with ethyl acetate. After the ethyl acetate layer was washed successively with water and brine, dried over anhydrous sodium sulfate, the residue obtained by evaporation of the solvent under reduced pressure was crystallized by dichloromethane/n-hexane to give the title compound (1.23g, 94.5%) as a white crystal.
¹H-NMR(CDCl₃): δ 1.26(9H, s), 1.29(9H, s), 5.03(2H, s).

### (3) 5-Chloro-N-{4-[(1,1-dimethyl)ethyl]-5-[(2,2-dimethyl)propionyl]thiazol-2-yl}-2-hydroxybenzamide(Compound No. 199).

A mixture of 5-chlorosalicylic acid(143.6mg, 0.83mmol), 2-amino-4-[(1,1-dimethyl)ethyl]ethyl-5-[(2,2-dimethyl)propionyl]thiazole(200.0mg, 0.83mmol), phosphorus trichloride(40 µL, 0.46mmol) and chlorobenzene(4mL) was refluxed for 3 hours. After the reaction mixture was cooled to room temperature, the residue obtained by concentration of the solvent under reduced pressure was purified by column chromatography on silica gel(n-hexane:ethyl acetate=3:1) to give the title compound(159.1mg, 48.4%) as a white powder.
¹H-NMR(CDCl₃): δ 1.33(9H, s), 1.35(9H, s), 6.99(1H, d, J=8.7Hz), 7.43(1H, dd, J=9.0, 2.7Hz), 7.70(1H, d, J=2.7Hz), 10.52(2H, br).

When the method described in Example 199(3) is referred in the following examples, phophorus trichloride was used as the acid halogenating agent. As the reaction solvent, solvents such as monochlorobenzene, toluene or the like were used.

### Example 200: Preparation of the compound of Compound No. 200.

Using 5-chloro-N-{4-[(1,1-dimethyl)ethyl]-5-[(2,2-dimethyl)propionyl]thiazol-2-yl}-2-hydroxybenzamide(compound No. 199) and acetyl chloride as the raw materials, the same operation as the Example 5 gave the title compound.
Yield: 65.3%.
¹H-NMR(CDCl₃): δ 1.32(9H, s), 1.33(9H,s),2.46(3H, s), 7.22(1H, d, J=8.4Hz), 7.56(1H, dd, J=8.7, 2.4Hz), 8.05(1H, d, J=2.7Hz), 9.82(1H, brs).

### Example 201: Preparation of the compound of Compound No. 201.

Using 5-bromosalicylic acid and 2-amino-4-[(1,1-dimethyl)ethyl]-5-[(2,2-dimethyl)propionyl]thiazole(compound of Example 199(2)) as the raw materials, the same operation as the Example 199(3) gave the title compound.
Yield: 23.8%.
¹H-NMR(CDCl₃): δ 1.33(9H, s), 1.35(9H, s), 6.94(1H, d, J=8, 7Hz), 7.55(1H, dd, J=8.7, 2.1Hz), 7.85(1H, d, J=2.1Hz), 10.51(2H, br).

### Example 202: Preparation of the compound of Compound No. 202.

Using pivaloylacetic acid ethyl ester as the raw material, the same operation as the Examples 199(1)-(3) gave the title compound.
Yield: 45.7%(3 steps).

### (1) α-Bromo-pivaloylacetic acid ethyl ester.

¹H-NMR(CDCl₃): δ 1.28(9H, s), 1.29(3H, t, J=7.2Hz), 4.26(2H, q, J=7.2Hz), 5.24(1H, s).

### (2) 2-Amino-4-[(1,1-dimethyl)ethyl]thiazole-5-carboxylic acid ethyl ester.

¹H-NMR(CDCl₃): δ 1.32(3H, t, J=7.2Hz), 1.43(9H, s), 4.24(2H, q, J=7.2Hz), 5.18(2H, s).

### (3) 2-(5-Bromo-2-hydroxybenzoyl)amino-4-[(1,1-dimethyl)ethyl]thiazole-5-carboxylic acid ethyl ester(Compound No. 202).

¹H-NMR(DMSO-d₆): δ 1.30(3H, t, J=7.2Hz), 1.44(9H, s), 4.27(2H, q, J=6.9Hz), 7.00(1H, d, J=8.7Hz), 7.63(1H, dd, J=8.7, 2.7Hz), 8.02(1H, d, J=2.4Hz), 11.80(1H, br), 12.12(1H, br).

### Example 203: Preparation of the compound of Compound No. 203.

Using 2-(5-bromo-2-hydroxybenzoyl)amino-4-[(1,1-dimethyl)-ethyl]thiazole-5-carboxylic acid ethyl ester(Compound No. 202) as the raw material, the same operation as the Example 36 gave the title compound.
Yield: 85.5%.
¹H-NMR(DMSO-d₆): δ 1.44(9H, s), 7.00(1H, d, J=9.0Hz), 7.62(1H, dd, J=9.0, 2.7Hz), 8.02(1H, d, J=2.4Hz), 11.83(1H, brs), 12.04(1H, brs), 12.98(1H, brs).

### Example 204: Preparation of the compound of Compound No. 204.

### (1) 2-Amino-5-bromo-4-[(1,1-dimethyl)ethyl]thiazole.

N-Bromosuccinimide(1.00g, 5.6mmol) was added to a solution of 2-amino-4-[(1,1-dimethyl)ethyl]thiazole(compound of Example 185(1); 0.87g, 5.6mmol) in carbon tetrachloride(9mL), and the mixture was stirred at room temperature for 1 hour. Hexane was added to the reaction mixture. The insoluble matter was filtered off, and the residue obtained by evaporation of the filtrate under reduced pressure was purified by column chromatography on silica gel(n-hexane: ethyl acetate=2:1) to give the title compound(1.23g, 93.7%) as an yellowish gray powder.
¹H-NMR(CDCl₃): δ 1.39(9H, s), 4.81(2H, brs).

### (2) 2-Amino-4-[(1,1-dimethyl)ethyl]-5-piperidinothiazole.

A mixture of 2-amino-5-bromo-4-[(1,1-dimethyl)ethyl]thiazole(0.10g, 0.42mmol), piperidine(0.1mL), potassium carbonate(0.20g) and acetonitrile(4mL) was refluxed for 3 hours. After the reaction mixture was cooled to room temperature, it was poured into water and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=2:1) to give the title compound(80.7mg, 79.3%) as an yellow crystal.
¹H-NMR(CDCl₃): δ 1.32(9H, s), 1.64(4H, t, J=5.7Hz), 1.71-1.77(2H, m), 2.35(2H, brs), 2.99(2H, brs), 4.68(2H, s).

When the preparation method described in Example 204(2) is referred in the following examples, bases such as potassium carbonate or the like were used as the base. As the reaction solvent, solvents such as acetonitrile or the like were used.

### (3) 2-Acetoxy-5-bromo-N-{4-[(1,1-dimethyl)ethyl]-5-piperidinothiazol-2-yl}benzamide.

Phosphorus oxychloride(46 µ L, 0.50mmol) was added to a mixture of 2-acetoxy-5-bromobenzoic acid(90.3mg, 0.35mmol), 2-amino-4-[(1,1-dimethyl)ethyl]-5-piperidinothiazole(80.7mg, 0.34mmol), pyridine(0.1mL) and tetrahydrofuran(3mL) under argon atmosphere, and the mixture was stirred at room temperature for 2 hours. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=3:1) to give the title compound(84.3mg) as a crude product.

When the preparation method described in Example 204(3) is referred in the following examples, phosphorus oxychloride was used as the acid halogenating agent. As the reaction base, pyridine was used. As the reaction solvent, solvents such as dichloromethane, tetrahydrofuran or the like were used.

### (4) 5-Bromo-N-{4-[(1,1-dimethyl)ethyl]-5-piperidinothiazol-2-yl}-2-hydroxybenzamide (Compound No. 204).

2N Aqueous sodium hydroxide(0.1mL) was added to a solution of 2-acetoxy-5-bromo-N-{4-[(1,1-dimethyl)ethyl]-5-piperidinothiazol-2-yl}benzamide (crude product, 84.3mg) in ethanol(3mL), and the mixture was stirred at room temperature for 1 hour. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=4:1) to give the title compound(54.1mg, 36.3%; 2 steps) as a white powder.
¹H-NMR(CDCl₃): δ 1.41(9H, s), 1.56(2H, brs), 1.67-1.74(4H, m), 2.79(4H, brs), 6.85(1H, d, J=9.0Hz), 7.45(1H, dd, J=9.0, 2.4Hz), 8.06(1H, d, J=2.4Hz), 11.70(2H, br).

When the preparation method described in Example 204(4) is referred in the following examples, inorganic bases such as sodium hydroxide, potassium carbonate or the like were used as the base. As the reaction solvent, solvents such as water, methanol, ethanol, tetrahydrofuran or the like were used alone or as a mixture.

### Example 205: Preparation of the compound of Compound No. 205.

Using 2-amino-5-bromo-4-[(1,1-dimethyl)ethyl]thiazole(compound of Example 204(1)) and morpholine as the raw materials, the same operation as the Examples 204(2)-(4) gave the title compound.
Yield: 17.1%.

### (2) 2-Amino-4-[(1,1-dimethyl)ethyl]-5-morpholinothiazole.

¹H-NMR(CDCl₃): δ 1.33(9H, s), 2.76(4H, brs), 3.79(4H, brs), 4.66(2H, s).

### (3) 2-Acetoxy-5-bromo-N-{4-[(1,1-dimethyl)ethyl]-5-morpholinothiazol-2-yl}benzamide.

The product was used for the next reaction as a crude product.

### (4) 5-Bromo-N-{4-[(1,1-dimethyl)ethyl]-5-morpholinothiazol-2-yl}-2-hydroxybenzamide (Compound No. 205).

¹H-NMR(CDCl₃): δ 1.24(9H, s), 2.89(4H, dd, J=4.8, 4.2Hz), 3.83(4H, dd, J=4.5, 4.2Hz), 6.89(1H, d, J=9.0Hz), 7.49(1H, dd, J=9.0, 2.4Hz), 7.98(1H, d, J=2.1Hz), 11.20(2H, br).

### Example 206: Preparation of the compound of Compound No. 206.

Using 2-amino-5-bromo-4-[(1,1-dimethyl)ethyl]thiazole(compound of Example 204(1)) and 4-methylpiperazine as the raw materials, the same operation as the Examples 204(2)-(4) gave the title compound.
Yield: 6.9%.

### (2) 2-Amino-4-[(1,1-dimethyl)ethyl]-5-(4-methylpiperazin-1-yl)thiazole.

¹H-NMR(DMSO-d₆): δ 1.25(9H, s), 2.12(2H, brs), 2.19(3H, s), 2.57(2H, brs), 2.72(4H, brs), 6.51(2H, s).

### (3) 2-Acetoxy-N-{4-[(1,1-dimethyl)ethyl]-5-(4-methylpiperazin-1-yl)thiazol-2-yl}benzamide.

The product was used for the next reaction as a crude product.

### (4) 5-Bromo-N-{4-[(1,1-dimethyl)ethyl]-5-(4-methylpiperazin-1-yl)thiazol-2-yl}-2-hydroxybenzamide(Compound No. 206).

¹H-NMR(CD₃OD): δ 1.41(9H, s), 2.55(3H, s), 2.87(4H, brs), 3.03(4H, brs), 6.88(1H, d, J=8.7Hz), 7.49(1H, dd, J=8.7, 2.7Hz), 8.11(1H, d, J=2.7Hz).

### Example 207: Preparation of the compound of Compound No. 207.

Using 2-amino-5-bromo-4-[(1,1-dimethyl)ethyl]thiazole(compound of Example 204(1)) and 4-phenylpiperazine as the raw materials, the same operation as the Examples 204(2)-(4) gave the title compound.
Yield: 6.9%.

### (2) 2-Amino-4-[(1,1-dimethyl)ethyl]-5-(4-phenylpiperazin-1-yl)thiazole.

¹H-NMR(CDCl₃): δ 1.34(9H, s), 2.80(2H, brs), 3.03(4H, brs), 3.55(2H, brs), 4.69(2H, s), 6.88(1H, tt, J=7.2, 1.2Hz), 6.95(2H, dd, J=9.0, 1.2Hz), 7.28(2H, dd, J=8.7, 7.2Hz).

### (3) 2-Acetoxy-5-bromo-N-{4-[(1,1-dimethyl)ethyl]-5-(4-phenylpiperazin-1-yl)thiazol-2-yl}benzamide.

The product was used for the next reaction as a crude product.

### (4) 5-Bromo-N-{4-[(1,1-dimethyl)ethyl]-5-(4-phenylpiperazin-1-yl)thiazol-2-yl}-2-hydroxybenzamide(Compound No. 207).

¹H-NMR(DMSO-d₆): δ 1.39(9H, s), 2.97(4H, s), 3.30(4H, s), 6.82(1H, t, J=7.5Hz), 6.97(2H, brs), 6.99(2H, t, J=7.5Hz), 7.58(1H, brs), 8.05(1H, d, J=2.4Hz), 11.69(1H, brs), 11.82(1H, brs).

### Example 208: Preparation of the compound of Compound No. 208.

Using 5-bromosalicylic acid and 2-amino-4-phenylthiazole as the raw materials, the same operation as the Example 199(3) gave the title compound.
Yield: 16.0%.
mp 239°C(dec.).
¹H-NMR(DMSO-d₆): δ 7.02(1H, d, J=8.4Hz), 7.34(1H, t, J=7.6Hz), 7.44(2H, t, J=7.6Hz), 7.62(1H, dd, J=8.4, 2.8Hz), 7.67(1H, s), 7.92(2H, d, J=7.2Hz), 8.08(1H, d, J=2.8Hz), 11.88(1H, brs), 12.05(1H, brs).

### Example 209: Preparation of the compound of Compound No. 209.

Using 5-bromosalicylic acid and 2-amino-4-phenylthiazole-5-acetic acid methyl ester as the raw materials, the same operation as the Example 199(3) gave the title compound.
Yield: 32.1%.
mp 288.5-229.5°C.
¹H-NMR(DMSO-d₆): δ 3.66(3H, s), 3.95(2H, s), 6.99(1H, d, J=8.0Hz), 7.42(1H, d, J=6.0Hz), 7.48(2H, brt, J=7.6Hz), 7.56-7.61(3H, m), 8.07(1H, d, J=2.4Hz), 11.85(1H, brs), 11.98(1H, brs).

### Example 210: Preparation of the compound of Compound No. 210.

2N Sodium hydroxide(0.5mL, 1mmol) was added to a solution of {2-[(5-bromo-2-hydroxybenzoyl)amino]-4-phenylthiazol-5-yl}acetic acid methyl ester(Compound No. 209; 75mg, 0.17mmol) in methanol(5mL), and the mixture was stirred at room temperature for 12 hours. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was washed with n-hexane-ethyl acetate under suspension to give the title compound(56mg, 77.3%) as a light yellow white crystal.
mp 284-286°C.
¹H-NMR(DMSO-d₆): δ 3.84(2H, s), 6.98(1H, d, J=8.8Hz), 7.42(1H, d, J=6.8Hz), 7.49(2H, t, J=7.6Hz), 7.58-7.61(3H, m), 8.07(1H, d, J=2.8Hz), 12.25(1H, brs).

### Example 211: Preparation of the compound of Compound No. 211.

Using 5-bromosalicylic acid and 2-amino-4,5-diphenylthiazole as the raw materials, the same operation as the Example 199(3) gave the title compound.
Yield: 25.9%.
mp 262-263°C.
¹H-NMR(DMSO-d₆): δ 7.02(1H, d, J=8.1Hz), 7.34-7.47(10H, m), 7.63(1H, d, J=6.9Hz), 8.08(1H, d, J=2.4Hz), 11.88(1H, brs), 12.08(1H, brs).
[2-Amino-4,5-diphenylthiazole: Refer to "Nihon Kagaku Zasshi", 1962, Vol.83, p.209.]

### Example 212: Preparation of the compound of Compound No. 212.

Using 5-bromosalicylic acid and 2-amino-4-benzyl-5-phenylthiazole as the raw materials, the same operation as the Example 199(3) gave the title compound.
Yield: 28.1%.
mp 198-200°C.
¹H-NMR(DMSO-d₆): δ 4.08(2H, s), 6.95(1H, d, J=8.8Hz), 7.15-7.22(3H, m), 7.30(2H, t, J=7.6Hz), 7.38-7.43(1H, m), 7.47(4H, d, J=4.4Hz), 7.57(1H, brd, J=8.8Hz), 8.05(1H, d, J=2.4Hz), 11.98(1H, brs).
[2-Amino-4-benzyl-5-phenylthiazole: Refer to "Chemical and Pharmaceutical Bulletin", 1962, Vol.10, p.376.]

### Example 213: Preparation of the compound of Compound No. 213.

Using 5-bromosalicylic acid and 2-amino-5-phenyl-4-(trifluoromethyl)thiazole as the raw materials, the same operation as the Example 199(3) gave the title compound.
Yield: 33.2%.
mp 250°C(dec.). ¹H-NMR(DMSO-d₆): δ 7.02(1H, d, J=8.8Hz), 7.51(5H, s), 7.63(1H, dd, J=8.8, 2.4Hz), 8.02(1H, d, J=2.8Hz), 12.38(1H, brs).

### Example 214: Preparation of the compound of Compound No. 214.

Using 1-phenyl-1,3-butanedione as the raw material, the same operation as the Examples 199(1)-(3) gave the title compound.
Yield: 8.9%(3 steps).

### (1) α-Bromo-1-phenyl-1,3-butanedione.

¹H-NMR(CDCl₃): δ 2.46(3H, s), 5.62(1H, s), 7.48-7.54(2H, m), 7.64(1H, tt, J=7.5, 2.1Hz), 7.97-8.01(2H, m).

### (2) 2-Amino-5-acetyl-4-phenylthiazole.

¹H-NMR(DMSO-d₆): δ 2.18(3H, s), 7.50-7.55(2H, m), 7.59-7.68(3H, m), 8.69(2H, brs).

### (3) 5-Bromo-N-(5-acetyl-4-phenylthiazol-2-yl)-2-hydroxybenzamide(Compound No. 214).

¹H-NMR(DMSO-d₆): δ 2.44(3H, s), 6.99(1H, d, J=9.0Hz), 7.55-7.71(4H, m), 7.76-7.80(2H, m), 8.01(1H, d, J=2.4Hz), 12.36(2H, br).

### Example 215: Preparation of the compound of Compound No. 215.

Using 1,3-diphenyl-1,3-propanedione as the raw material, the same operation as the Examples 199(1)-(3) gave the title compound.
Yield: 49.7%.

### (1) α-Bromo-1,3-diphenyl-1,3-propanedione.

¹H-NMR(CDCl₃): δ 6.55(1H, s), 7.45-7.50(4H, m), 7.61(2H, tt, J=7.2, 2.1Hz), 7.98-8.01(4H, m).

### (2) 2-Amino-5-benzoyl-4-phenylthiazole.

¹H-NMR(DMSO-d₆): δ 7.04-7.18(5H, m), 7.22-7.32(3H, m), 7.35-7.38(2H, m), 8.02(2H, s).

### (3) 5-Bromo-N-(5-benzoyl-4-phenylthiazol-2-yl)-2-hydroxybenzamide(Compound No. 215).

¹H-NMR(DMSO-d₆): δ 7.03(1H, d, J=8.7Hz), 7.17-7.30(5H, m), 7.39-7.47(3H, m), 7.57-7.60(2H, m), 7.64(1H, dd, J=8.7, 2.7Hz), 8.05(1H, d, J=2.4Hz), 11.82(1H, brs), 12.35(1H, brs).

### Example 216: Preparation of the compound of Compound No. 216.

Using 5-bromosalicylic acid and 2-amino-4-phenylthiazole-5-carboxylic acid ethyl ester as the raw materials, the same operation as the Example 199(3) gave the title compound.
Yield: 28.6%.
mp 197-199°C.
¹H-NMR(DMSO-d₆): δ 1.21(3H, t, J=6.8Hz), 4.20(2H, q, J=6.8Hz), 7.01(1H, d, J=8.8Hz), 7.43-7.48(3H, m), 7.63(1H, dd, J=8.8, 2.4Hz), 7.70-7.72(2H, m), 8.04(1H, d, J=2.4Hz), 12.33(1H, brs).

### Example 217: Preparation of the compound of Compound No. 217.

Using 2-(5-bromo-2-hydroxybenzoyl)amino-4-phenylthiazole-5-carboxylic acid ethyl ester(compound No. 216) as the raw material, the same operation as the Example 36 gave the title compound.
Yield: 67.0%.
¹H-NMR(DMSO-d₆): δ 7.00(1H, d, J=8.8Hz), 7.42-7.44(3H, m), 7.62(1H, dd, J=8.8, 2.4Hz), 7.70-7.72(2H, m), 8.04(1H, d, J=2.4Hz), 12.31(1H, brs), 12.99(1H, brs).

### Example 218: Preparation of the compound of Compound No. 218.

Using 5-chlorosalicylic acid and 2-amino-4-phenylthiazole-5-carboxylic acid ethyl ester as the raw materials, the same operation as the Example 199(3) gave the title compound.
Yield: 69.4%.
¹H-NMR(DMSO-d₆): δ 1.22(3H, t, J=7.5Hz), 4.21(2H, q, J=7.5Hz), 7.07(1H, d, J=8.7Hz), 7.43-7.47(3H, m), 7.53(1H, dd, J=8.7, 2.4Hz), 7.70-7.74(2H, m), 7.92(1H, d, J=3.0Hz), 11.88(1H, br), 12.29(1H, brs).

### Example 219: Preparation of the compound of Compound No. 219.

Using pentafluorobenzoylacetic acid ethyl ester as the raw material, the same operation as the Examples 199(1)-(3) gave the title compound.
Yield: 40.0%(3 steps).

### (1) α-Bromo-pentafluorobenzoylacetic acid ethyl ester.

It was used for the next reaction as a crude product.

### (2) 2-Amino-4-(pentafluorophenyl)thiazole-5-carboxylic acid ethyl ester.

¹H-NMR(CDCl₃): δ 1.23(3H, t, J=7.2Hz), 4.21(2H, q, J=7.2Hz), 5.41(2H, s).

### (3) Ethyl 2-(5-bromo-2-hydroxybenzoyl)amino-4-(pentafluorophenyl)thiazole-5-carboxylate(Compound No. 219).

¹H-NMR(DMSO-d₆): δ 1.20(3H, t, J=7.2Hz), 2.51(2H, q, J=7.2Hz), 7.02(1H, d, J=8.7Hz), 7.64(1H, dd, J=8.7, 2.7Hz), 7.90(1H, d, J=3.0Hz), 11.92(1H, br), 12.58(1H, br).

### Example 220: Preparation of the compound of Compound No. 220.

A mixure of 2-(5-bromo-2-hydroxybenzoyl)amino-4-phenylthiazole-5-carboxylic acid(Compound No. 217; 0.20g, 0.48mmol), methylamine 40% methanol solution(0.2ml), 1-hydroxybenzotriazole hydrate(96.7mg, 0.72mmol), WSC · HCl(137.2mg, 0.72mmol) and tetrahydrofuran(15mL) was stirred at room temperature for 18 hours. The reaction mixture was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=1:2), and crystallized by dichloromethane/n-hexane to give the title compound(87.9mg, 42.6%) as a white powder.
¹H-NMR(DMSO-d₆): δ 2.70(3H, d, J=4.5Hz), 7.02(1H, d, J=9.0Hz), 7.40-7.48(3H, m), 7.63(1H, dd, J=9.0, 2.4Hz), 7.68-7.71(2H, m), 8.06(1H, d, J=2.4Hz), 8.16(1H, t, J=4.5Hz), 11.88(1H, br), 12.15(1H, brs).

When the method described in Example 220 is referred in the following examples, WSC · HCl and 1-hydroxybenzotriazole hydrate were used as the dehydrocondensating agent. As the reaction solvent, solvents such as tetrahydrofuran or the like were used.

### Example 221: Preparation of the compound of Compound No. 221.

Using 2-(5-bromo-2-hydroxybenzoyl)amino-4-phenylthiazole-5-carboxylic acid (Compound No. 217) and 70% aqueous ethylamine solution as the raw materials, the same operation as the Example 220 gave the title compound.
Yield: 62.5%.
¹H-NMR(DMSO-d₆): δ 1.05(3H, t, J=6.9Hz), 3.15-3.24(2H, m), 7.02(1H, d, J=8.7Hz), 7.40-7.47(3H, m), 7.63(1H, dd, J=8.7, 3.0Hz), 7.69-7.72(2H, m), 8.06(1H, d, J=2.4Hz), 8.20(1H, t, J=5.4Hz), 11.84(1H, br), 12.14(1H, brs).

### Example 222: Preparation of the compound of Compound No. 222.

Using 2-(5-bromo-2-hydroxybenzoyl)amino-4-phenylthiazole-5-carboxylic acid (Compound No. 217) and isopropylamine as the raw materials, the same operation as the Example 220 gave the title compound.
Yield: 23.9%.
¹H-NMR(DMSO-d₆): δ 1.07(6H, d, J=6.3Hz), 4.02(1H, m), 7.02(1H, d, J=9.0Hz), 7.40-7.52(3H, m), 7.64(1H, dd, J=8.7, 2.7Hz), 7.69-7.73(2H, m), 8.06(1H, d, J=2.7Hz), 11.89(1H, br), 12.14(1H, brs).

### Example 223: Preparation of the compound of Compound No. 223.

Using 2-(5-bromo-2-hydroxybenzoyl)amino-4-phenylthiazole-5-carboxylic acid (Compound No. 217) and 2-phenethylamine as the raw materials, the same operation as the Example 220 gave the title compound.
Yield: 62.2%.
¹H-NMR(DMSO-d₆): δ 2.78(2H, t, J=7.5Hz), 3.43(2H, q, J=7.5Hz), 7.02(1H, d, J=9.0Hz), 7.19-7.24(3H, m), 7.27-7.33(2H, m), 7.39-7.41(3H, m), 7.61-7.65(3H, m), 8.06(1H, d, J=2.4Hz), 8.25(1H, t, J=6.0Hz), 11.85(1H, brs), 12.15(1H, brs).

### Example 224: Preparation of the compound of Compound No. 224.

Using 5-bromosalicylic acid and 2-amino-4-(trifluoromethyl)thiazole-5-carboxylic acid ethyl ester as the raw materials, the same operation as the Example 199(3) gave the title compound.
Yield: 88.7%.
¹H-NMR(DMSO-d₆): δ 1.32(3H, t, J=7.2Hz), 4.33(2H, q, J=7.2Hz), 7.01(1H, d, J=8.7Hz), 7.63(1H, dd, J=8.7, 2.7Hz), 7.98(1H, d, J=2.4Hz), 12.64(1H, br).

### Example 225: Preparation of the compound of Compound No. 225.

Using 4-hydroxybiphenyl-3-carboxylic acid and 2-amino-4-phenylthiazole-5-carboxylic acid ethyl ester as the raw materials, the same operation as the Example 199(3) gave the title compound.
Yield: 61.7%.
mp 207-208°C.
¹H-NMR(DMSO-d₆): δ 1.23(3H, t, J=7.2Hz), 4.22(2H, q, J=7.2Hz), 7.16(1H, d, J=8.7Hz), 7.36(1H, t, J=7.5Hz), 7.45-7.50(5H, m), 7.69-7.76(4H, m), 7.85(1H, dd, J=8.7, 2.4Hz), 8.31(1H, d, J=2.4Hz), 11.73(1H, brs), 12.60(1H, brs).
[4-Hydroxybiphenyl-3-carboxylic acid: Refer to "Tetrahedron", (USA), 1997, Vol.53, p.11437.]

### Example 226: Preparation of the compound of Compound No. 226.

Using (4'-fluoro-4-hydroxybiphenyl)-3-carboxylic acid and 2-amino-4-phenylthiazole-5-carboxylic acid ethyl ester as the raw materials, the same operation as the Example 199(3) gave the title compound.
Yield: 62.7%.
mp 237-238°C.
¹H-NMR(DMSO-d₆): δ 1.22(3H, t, J=7.2Hz), 4.21(2H, q, J=7.2Hz), 7.13(1H, d, J=8.4Hz), 7.28(2H, t, J=8.8Hz), 7.44-7.45(3H, m), 7.71-7.75(4H, m), 7.81(1H, dd, J=8.8, 2.4Hz), 8.27(1H, d, J=2.4Hz), 11.67(1H, brs), 12.58(1H, brs).
[(4'-Fluoro-4-hydroxybiphenyl)-3-carboxylic acid: Refer to "Tetrahedron", 1997, Vol.53, p.11437.]

### Example 227: Preparation of the compound of Compound No. 227.

Using (2',4'-difluoro-4-hydroxybiphenyl)-3-carboxylic acid and 2-amino-4-phenylthiazole-5-carboxylic acid ethyl ester as the raw materials, the same operation as the Example 199(3) gave the title compound.
Yield: 45.6%.
mp 206-207°C.
¹H-NMR(DMSO-d₆): δ 1.22(3H, t, J=7.2Hz), 4.22(2H, q, J=7, 2Hz), 7.17(1H, d, J=9.0Hz), 7.21(1H, td, J=8.7, 2.4Hz), 7.38(1H, ddd, J=11.7, 9.3, 2.4Hz), 7.44-7.46(3H, m), 7.60-7.75(4H, m), 8.13-8.14(1H, m), 11.86(1H, brs), 12.46(1H, brs).

### Example 228: Preparation of the compound of Compound No. 228.

### (1) [4-Hydroxy-4'-(trifluoromethyl)biphenyl]-3-carboxylic acid.

A mixture of 5-bromosalicylic acid(500mg, 2.30mmol), dihydroxy-4-(trifluoromethyl)phenylborane(488mg, 2.57mmol), palladium acetate(10mg, 0.040mmol) and 1mol/L aqueous sodium carbonate(7mL) was stirred at 80°C for 1 hour. After the reaction mixture was cooled to room temperature, it was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was methyl-esterified by trimethylsilyldiazomethane and methanol according to the fixed procedure, and purified by column chromatography on silica gel(n-hexane:ethyl acetate=5:1) to give a colourless liquid(563mg). 2N Sodium hydroxide(3mL) was added to a solution of this liquid in methanol(10mL), and the mixture was stirred at 60°C for 1 hour. After the reaction mixture was cooled to room temperature, it was poured into 2N hydrochloric acid and extracted with ethyl acetate. After the ethyl acetate layer was washed successively with water and brine, dried over anhydrous magnesium sulfate, the residue obtained by evaporation of the solvent under reduced pressure was washed with n-hexane/dichloromethane under suspension to give the title compound(458mg, 70.4%) as a white crystal.
mp 185°C(dec.).
¹H-NMR(DMSO-d₆): δ 7.09(1H, d, J=8.8Hz), 7.77(2H, d, J=8.0Hz), 7.85(2H, d, J=8.0Hz), 7.90(1H, dd, J=8.8, 2.0Hz), 8.10(1H, d, J=2.4Hz), 11.80(1H, brs).

### (2) 2-{[4-Hydroxy-4'-(trifluoromethyl)biphenyl]-3-carbonyl}amino-4-phenylthiazole-5-carboxylic acid ethyl ester(Compound No. 228).

Using [4-hydroxy-4'-(trifluoromethyl)biphenyl]-3-carboxylic acid and 2-amino-4-phenylthiazole-5-carboxylic acid ethyl ester as the raw materials, the same operation as the Example 199(3) gave the title compound.
Yield: 41.7%.
mp 236-237°C.
¹H-NMR(DMSO-d₆): δ 1.22(3H, t, J=7.2Hz), 4.21(2H, q, J=7.2Hz), 7.18(1H, d, J=8.8Hz), 7.44-7.45(3H, m), 7.72-7.74(2H, m), 7.81(2H, d, J=8.4Hz), 7.91(1H, dd, J=8.8, 2.4Hz), 7.93(2H, d, J=8.4Hz), 8.36(1H, d, J=2.4Hz), 11.78(1H, brs), 12.62(1H, brs).

### Example 229: Preparation of the compound of Compound No. 229.

Using 2-hydroxy-5-(1-pyrrolyl)benzoic acid and 2-amino-4-phenylthiazole-5-carboxylic acid ethyl ester as the raw materials, the same operation as the Example 199(3) gave the title compound.
Yield: 55.0%.
¹H-NMR(DMSO-d₆): δ 1.22(3H, t, J=7.2Hz), 4.22(2H, q, J=7.2Hz), 6.26(2H, t, J=2.1Hz), 7.13(1H, d, J=8.7Hz), 7.32(2H, t, J=2.1Hz), 7.43-7.47(3H, m), 7.70-7.75(3H, m), 8.09(1H, d, J=2.7Hz), 11.58(1H, brs), 12.55(1H, brs).

### Example 230: Preparation of the compound of Compound No. 230.

### (1) 2-Hydroxy-5-(2-thienyl)benzoic acid.

Tetrakis(triphenylphosphine)palladium(80mg, 0.07mmol) was added to a solution of 5-bromosalicylic acid(500mg, 2.30mmol) in 1,2-dimethoxyethane(5mL) under argon atmosphere, and the mixture was stirred at room temperature for 10 minutes. Then dihydroxy-2-thienylborane(324mg, 2.53mmol) and 1M sodium carbonate(7mL) were added to the mixture, and it was refluxed for 2 hours. After the reaction mixture was cooled to room temperature, it was poured into 2N hydrochloric acid and extracted with ethyl acetate. After the ethyl acetate layer was washed successively with water and brine, dried over anhydrous sodium sulfate, the residue obtained by evaporation of the solvent under reduced pressure was methyl-esterified by trimethylsilyldiazomethane and methanol according to the fixed procedure, and purified by column chromatography on silica gel(n-hexane:ethyl acetate=5:1) to give an yellow liquid(277mg). 2N Sodium hydroxide(1.5mL) was added to a solution of this liquid in methanol(5mL), and the mixture was stirred at 60°C for 1 hour. After the reaction mixture was cooled to room temperature, it was poured into 2N hydrochloric acid and extracted with ethyl acetate. After the ethyl acetate layer was washed successively with water and brine, dried over anhydrous magnesium sulfate, the residue obtained by evaporation of the solvent under reduced pressure was crystallized from n-hexane/dichloromethane to give the title compound(58mg, 11.5%) as a white crystal.
¹H-NMR(DMSO-d₆): δ 6.95(1H, d, J=8.8Hz), 7.09(1H, dd, J=4.8, 3.6Hz), 7.37(1H, dd, J=4.0, 1.2Hz), 7.45(1H, dd, J=5.2, 1.2Hz), 7.74(1H, dd, J=8.8, 2.8Hz), 7.96(1H, d, J=2.8Hz).

### (2) 2-[2-Hydroxy-5-(2-thienyl)benzoyl]amino-4-phenylthiazole-5-carboxylic acid ethyl ester(Compound No. 230).

Using 2-hydroxy-5-(2-thienyl)benzoic acid and 2-amino-4-phenylthiazole-5-carboxylic acid ethyl ester as the raw materials, the same operation as the Example 199(3) gave the title compound.
Yield: 58.2%.
mp 213-214°C.
¹H-NMR(DMSO-d₆): δ 1.22(3H, t, J=7.2Hz9, 4.21(2H, q, J=7.2Hz), 7.10(1H, d, J=9.2Hz), 7.12(1H, dd, J=4.8, 3.6Hz), 7.44-7.46(4H, m), 7.50(1H, dd, J=4.8, 1.2Hz), 7.71-7.74(2H, m), 7.79(1H, dd, J=8.8, 2.4Hz), 8.21(1H, d, J=2.4Hz), 11.78(1H, brs), 12.44(1H, brs).

### Example 231: Preparation of the compound of Compound No. 231.

### (1) 2-Amino-4-[3,5-bis(trifluoromethyl)phenyl]thiazole.

Phenyltrimethylammonium tribromide(753mg, 2mmol) was added to a solution of 3',5'-bis(trifluoromethyl)acetophenone(0.51g, 2.0mmol) in tetrahydrofuran(5mL) and the mixture was stirred at room temperature for 5 hours. The reaction mixture was poured into water and extracted with ethyl acetate. After the ethyl acetate layer was washed with brine, dried over anhydrous sodium sulfate, ethanol(5mL) and thiourea(152mg, 2mmol) were added to the residue obtained by evaporation of the solvent under reduced pressure, and the mixture was refluxed for 30 minutes. After the reaction mixture was cooled to room temperature, it was poured into saturated aqueous sodium hydrogen carbonate and extracted with ethyl acetate. After the ethyl acetate layer was washed with brine and dried over anhydrous sodium sulfate, the residue obtained by evaporation of the solvent under reduced pressure was purified by column chromatography on silica gel(n-hexane:ethyl acetate=2:1) and washed with n-hexane under suspension to give the title compound(520.1mg, 83.3%) as a light yellow white crystal.
¹H-NMR(CDCl₃): δ 5.03(2H, s), 6.93(1H, s), 7.77(1H, s), 8.23(2H, s).

### (2) 5-Chloro-2-hydroxy-N-{4-[3,5-bis(trifluoromethyl)phenyl]thiazol-2-yl}benzamide (Compound No. 231).

A mixture of 5-chlorosalicylic acid(172.6mg, 1mmol), 2-amino-4-[3,5-bis(trifluoromethyl)phenyl]thiazole(312.2mg, 1mmol), phosphorus trichloride(44 µL, 0.5mmol) and monochlorobenzene(5mL) was refluxed for 4 hours. After the reaction mixture was cooled to room temperature, it was poured into water and extracted with ethyl acetate. After the ethyl acetate layer was washed with brine, dried over anhydrous sodium sulfate, the residue obtained by evaporation of the solvent under reduced pressure was purified by column chromatography on silica gel(n-hexane:ethyl acetate =3:1→2:1) to give the title compound(109.8mg, 23.5%) as a pale yellow white powder.
¹H-NMR(DMSO-d₆): δ 7.08(1H, d, J=8.7Hz), 7.53(1H, dd, J=9.0, 3.0Hz), 7.94(1H, d, J=3.0Hz), 8.07(1H, s), 8.29(1H, s), 8.60(2H, s), 11.77(1H, s), 12.23(1H, s).

### Example 232: Preparation of the compound of Compound No. 232.

Using 5-chlorosalicylic acid and 2-amino-4,5,6,7-tetrahydrobenzo[b]thiophene-3-carboxylic acid ethyl ester as the raw materials, the same operation as the Example 3 gave the title compound.
Yield: 49.6%.
¹H-NMR(DMSO-d₆): δ 1.32(3H, t, J=7.2Hz), 1.74(4H, br), 2.63(2H, br), 2.75(2H, br), 4.30(2H, q, J=7.2Hz), 7.05(1H, d, J=9.0Hz), 7.50(1H, dd, J=8.7, 3.0Hz), 7.92(1H, d, J=3.0Hz), 12.23(1H, s), 13.07(1H, s).

### Example 233: Preparation of the compound of Compound No. 233.

Using 5-bromosalicylic acid and 3-amino-5-phenylpyrazole as the raw materials, the same operation as the Example 3 gave the title compound.
Yield: 9.2%.
¹H-NMR(DMSO-d₆): δ 6.98(1H, d, J=8.8Hz), 7.01(1H, s),7.35(1H, t, J=7.6Hz), 7.46(2H, t, J=7.6Hz), 7.58(1H, dd, J=8.8, 2.8Hz), 7.74-7.76(2H, m), 8.19(1H, s), 10.86(1H, s), 12.09(1H, s), 13.00(1H, brs).

### Example 234: Preparation of the compound of Compound No. 234.

### (1) 2-Amino-4,5-diethyloxazole.

Cyanamide(0.75g, 17.7mmol) and sodium ethoxide(1.21g, 17.7mmol) were added to a solution of propioin(1.03g, 8.87mmol) in ethanol(15mL), and the mixture was stirred at room temperature for 3.5 hours. The reaction mixture was poured into water and extracted with ethyl acetate. After the ethyl acetate layer was washed successively with water and brine, dried over anhydrous sodium sulfate, the residue obtained by evaporation of the solvent under reduced pressure was purified by column chromatography on silica gel(dichloromethane:methanol=9:1) to give the title compound(369.2mg, 29.7%) as an yellow amorphous.
¹H-NMR(DMSO-d₆): δ 1.04(3H, t, J=7.5Hz), 1.06(3H, t, J=7.5Hz), 2.20(2H, q, J=7.5Hz), 2.43(2H, q, J=7.5Hz), 6.15(2H, s).

### (2) 2-Acetoxy-5-bromo-N-(4,5-diethyloxazol-2-yl)benzamide.

Using 2-acetoxy-5-bromobenzoic acid and 2-amino-4,5-diethyloxazole as the raw materials, the same operation as the Example 5 gave the title compound.
Yield: 22.0%.
¹H-NMR(CDCml₃): δ 1.22(3H, t, J=7.5Hz), 1.23(3H, t, J=7.5Hz), 2.38(3H, s), 2.48(2H, q, J=7.5Hz), 2.57(2H, q, J=7.5Hz), 6.96(1H, d, J=8.7Hz), 7.58(1H, dd, J=8.7, 2.7Hz), 8.32(1H, s), 11.40(1H, br).

### (3) 5-Bromo-N-(4,5-diethyloxazol-2-yl)-2-hydroxybenzamide(Compound No. 234).

Using 2-acetoxy-5-bromo-N-(4,5-diethyloxazol-2-yl)benzamide as the raw material, the same operation as the Example 2 gave the title compound.
Yield: 70.2%.
¹H-NMR(CDCl₃) δ :1.25(3H, t, J=7.5Hz), 1.26(3H, t, J=7.5Hz), 2.52(2H, q, J=7.5Hz), 2.60(2H, q, J=7.5Hz), 6.84(1H, d, J=8.7Hz), 7.43(1H, dd, J=8.7, 3.0Hz), 8.17(1H, d, J=3.0Hz), 11.35(1H, br), 12.83(1H, br).

### Example 235: Preparation of the compound of Compound No. 235.

Using 5-bromosalicylic acid and 2-amino-4,5-diphenyloxazole as the raw materials, the same operation as the Example 3 gave the title compound.
Yield: 32.6%.
mp 188-189°C.
¹H-NMR(DMSO-d₆): δ 6.98(1H, d, J=8.7Hz), 7.40-7.49(6H, m), 7.53-7.56(2H, m), 7.59-7.63(3H, m), 8.01(1H, d, J=2.4Hz), 11.80(2H, brs).
[2-Amino-4,5-diphenyloxazole: Refer to "Zhournal Organicheskoi Khimii: Russian Journal of Organic Chemistry", (Russia), 1980, Vol.16, p.2185.]

### Example 236: Preparation of the compound of Compound No. 236.

### (1) 2-Amino-4,5-bis(furan-2-yl)oxazole.

Cyanamide(218.8mg, 5.20mmol) and sodium ethoxide(530.8mg, 7.80mmol) were added to a solution of furoin(0.50g, 2.60mmol) in ethanol(15mL), and the mixture was stirred at room temperature for 2 hours. The reaction mixture was poured into water and extracted with ethyl acetate. After the ethyl acetate layer was washed successively with water and brine, dried over anhydrous sodium sulfate, the residue obtained by evaporation of the solvent under reduced pressure was purified by column chromatography on silica gel(n-hexane:ethyl acetate=1:1→1:2) to give the title compound(175.0mg, 31.1%) as a dark brown crystal.
¹H-NMR(DMSO-d₆): δ 6.59(1H, dd, J=3.3, 2.1Hz), 6.62(1H, dd, J=3.3, 2.1Hz), 6.73(1H, dd, J=3.3, 0.6Hz), 6.80(1H, dd, J=3.3, 0.9Hz), 7.05(2H, s), 7.75-7.76(2H, m).

### (2) 5-Bromo-N-[4,5-bis(furan-2-yl)oxazol-2-yl]-2-hydroxybenzamide(Compound No. 236).

Using 5-bromosalicylic acid and 2-amino-4,5-bis(furan-2-yl)oxazole as the raw materials, the same operation as the Example 3 gave the title compound.
Yield: 12.9%.
¹H-NMR(DMSO-d₆): δ 6.65(1H, dd, J=3.6, 1.8Hz), 6.68(1H, dd, J=3.6, 1.BHz), 6.75(1H, d, J=8, 7Hz), 6.92(1H, dd, J=3.6, 0.9Hz), 6.93(1H, d, J=3.3Hz), 7.37(1H, dd, J=8.7, 2.7Hz), 7.80(1H, dd, J=1.8, 0.9Hz), 7.84(1H, dd, J=1.8, 0.9Hz), 7.92(1H, d, J=3.0Hz), 14.88(2H, br).

### Example 237: Preparation of the compound of Compound No. 237.

### (1) 2-Acetoxy-N-(5-trifluoromethyl-1,3,4-thiadiazol-2-yl)benzamide.

Using O-acetylsalicyloyl chloride and 2-amino-5-trifluoromethyl-1,3,4-thiadiazole as the raw materials, the same operation as the Example 1 gave the title compound.
Yield: 51.1%.
¹H-NMR(DMSO-d₆): δ 2.23(3H, s), 7.32(1H, dd, J=8.0, 1.2Hz),7.45(1H, td, J=7.6, 1.2Hz), 7.69(1H, td, J=8.0, 2.0Hz), 7.87(1H, dd, J=8.0, 2.0Hz), 13.75(1H, brs).

### (2) 2-Hydroxy-N-(5-trifluoromethyl-1,3,4-thiadiazol-2-yl)benzamide(Compound No. 237).

Using 2-acetoxy-N-(5-trifluoromethyl-1,3,4-thiadiazol-2-yl)benzamide as the raw material, the same operation as the Example 2 gave the title compound.
Yield: 92.9%.
¹H-NMR(DMSO-d₆): δ 7.00(1H, td, J=8.0, 0.8Hz),7.06(1H, d, J=8.4Hz), 7.51(1H, ddd, J=8.4, 7.6, 2.0Hz), 7.92(1H, dd, J=8.0, 1.6Hz), 12.16(1H, br).

### Example 238: Preparation of the compound of Compound No. 238.

Using 5-bromosalicylic acid and 2-amino-5-trifluoromethyl-1,3,4-thiadiazole as the raw materials, the same operation as the Example 3 gave the title compound.
Yield: 80.2%.
¹H-NMR(DMSO-d₆): δ 7.01(1H, d, J=9.0Hz), 7.63(1H, dd, J=8.7, 2.7Hz), 7.97(1H, d, J=2.4Hz).

### Example 239: Preparation of the compound of Compound No. 239.

Using 5-chlorosalicylic acid and 3-aminopyridine as the raw materials, the same operation as the Example 3 gave the title compound.
Yield: 23.2%.
¹H-NMR(DMSO-d₆): δ 7.02(1H, d, J=9.3Hz), 7.42(1H, ddd, J=9.0, 4.8, 0.6Hz), 7.47(1H, dd, J=8.7, 5.7Hz), 7.92(1H, d, J=2.7Hz), 8.15(1H, ddd, J=8.4, 2.4, 1.5Hz), 8.35(1H, dd, J=7.8, 1.5Hz), 8.86(1H, d, J=2.4Hz), 10.70(1H, s).

### Example 240: Preparation of the compound of Compound No 240.

Using 5-chlorosalicylic acid and 5-amino-2-chloropyridine as the raw materials, the same operation as the Example 3 gave the title compound.
Yield: 12.2%.
¹H-NMR(DMSO-d₆): δ 7.04(1H, d, J=9.0Hz), 7.49(1H, dd, J=9.0, 3.0Hz), 7.54(1H, d, J=8.4Hz), 7.88(1H, d, J=2.7Hz), 8.21(1H, dd, J=8.7, 2.7Hz), 8.74(1H, d, J=2.7Hz), 10.62(1H, s), 11.57(1H, s).

### Example 241: Preparation of the compound of Compound No. 241.

Using 5-chlorosalicylic acid and 2-amino-6-chloro-4-methoxypyrimidine as the raw materials, the same operation as the Example 3 gave the title compound.
Yield: 2.2%, white solid.
¹H-NMR(DMSO-d₆): δ 3.86(3H, s), 6.85(1H, s), 7.01(1H, d, J=9.0Hz), 7.47(1H, dd, J=9.0, 3.0Hz), 7.81(1H, d, J=3.0Hz), 11.08(1H, s), 11.65(1H, s).

### Example 242: Preparation of the compound of Compound No. 242.

Using 5-chlorosalicylic acid and 3-aminoquinoline as the raw materials, the same operation as the Example 3 gave the title compound.
Yield: 4.3%.
¹H-NMR(DMSO-d₆): δ 7.07(1H, d, J=8.7Hz), 7.51(1H, dd, J=9.0, 3.0Hz), 7.61(1H, dt, J=7.8, 1.2Hz), 7.70(1H, dt, J=7.8, 1.5Hz), 7.98(2H, d, J=3.0Hz), 8.01(1H, s), 8.82(1H, d, J=2.4Hz), 10.80(1H, s), 11.74(1H, s).

### Example 243: Preparation of the compound of Compound No. 243.

Using 5-chlorosalicylic acid and 2-amino-6-bromopyridine as the raw materials, the same operation as the Example 3 gave the title compound.
Yield: 12.3%.
¹H-NMR(DMSO-d₆): δ 7.07(1H, d, J=8.7Hz), 7.42(1H, d, J=7.8Hz), 7.51(1H, dd, J=8.7, 2.7Hz), 7.82(1H, t, J=7.5Hz), 7.94(1H, d, J=3.0Hz), 8.24(1H, d, J=7.8Hz), 10.95(1H, s), 11.97(1H, s).

### Example 244: Preparation of the compound of Compound No. 244.

### (1) 2-Acetoxy-5-chlorobenzoic acid.

Concentrated sulfuric acid(0.08mL) was added slowly to a mixture of 5-chlorosalicylic acid(13.35g, 77mmol) and acetic anhydride(20mL). After the reaction mixture was solidified, it was poured into ice water and extracted with ethyl acetate. The organic layer was washed with water and brine, and dried over anhydrous sodium sulfate. The residue obtained by evaporation of the solvent under reduced pressure was washed with n-hexane under suspension to give the title compound(15.44g, 93.0%) as a white crystal.
¹H-NMR(DMSO-d₆): δ 2.25(3H, s), 7.27(1H, d, J=8.7Hz), 7.72(1H, dd, J=8.7, 2.7Hz), 7.89(1H, d, J=2.7Hz), 13.47(1H, s).

### (2) 2-Acetoxy-5-chloro-N-(pyridazin-2-yl)benzamide.

Using 2-acetoxy-5-chlorobenzoic acid and 2-aminopyridazine as the raw materials, the same operation as the Example 204(3) gave the title compound.
Yield: 19.7%.
¹H-NMR(CDCl₃): δ 2.42(3H, s), 7.19(1H, d, J=8.7Hz), 7.54(1H, dd, J=8.7, 2.7Hz), 8.01(1H, d, J=2.4Hz), 8.28(1H, dd, J=2.4, 1.8Hz), 8.42(1H, d, J=2.4Hz), 9.09(1H, s), 9.66(1H, d, J=1.8Hz).

### (3) 5-Chloro-2-hydroxy-N-(pyridazin-2-yl)benzamide(Compound No. 244).

Using 2-acetoxy-5-chloro-N-(pyridazin-2-yl)benzamide as the raw material, the same operation as the Example 2 gave the title compound.
Yield: 72.6%.
¹H-NMR(DMSO-d₆): δ 7.09(1H, d, J=9.0Hz), 7.52(1H, dd, J=8.7, 2.7Hz), 7.96(1H, d, J=2.7Hz), 8.44-8.47(2H, m), 9.49(1H, s), 10.99(1H, s), 12.04(1H, s).

### Example 245: Preparation of the compound of Compound No. 245.

Using 5-bromosalicylic acid and 2-amino-5-bromopyrimidine as the raw materials, the same operation as the Example 3 gave the title compound.
Yield: 10.3%.
¹H-NMR(DMSO-d₆): δ 6.98(1H, d, J=8.8Hz), 7.59(1H, dd, J=8.8, 2.4Hz), 8.00(1H, d, J=2.8Hz), 8.86(2H, s), 11.09(1H, s), 11.79(1H, s).

### Example 246: Preparation of the compound of Compound No. 246.

Using 2-(5-bromo-2-hydroxybenzoyl)amino-4-phenylthiazole-5-carboxylic acid(Compound No. 217) and propylamine as the raw materials, the same operation as the Example 220 gave the title compound.
Yield: 23.1%.
¹H-NMR(DMSO-d₆): δ 0.82(3H, t, J=7.5Hz), 1.39-1.51(2H, m), 3.13(2H, q, J=6.6Hz), 7.02(1H, d, J=9.0Hz), 7.40-7.48(3H, m), 7.63(1H, dd, J=8.7, 2.7Hz), 7.68-7.72(2H, m), 8.06(1H, d, J=2.7Hz), 8.18(1H, t, J=5.7Hz), 11.87(1H, brs), 12.14(1H, brs).

### Example 247: Preparation of the compound of Compound No. 247.

A mixture of 5-sulfosalicylic acid(218mg, 1mmol), 3,5-bis(trifluoromethyl)aniline(229mg, 1mmol), phosphorus trichloride(88 µ L, 1mmol) and o-xylene(5mL) was refluxed for 3 hours. After the reaction mixture was cooled to room temperature, it was purified by column chromatography on silica gel(n-hexane:ethyl acetate=3:1) to give the title compound(29mg, 9.2%) as a white solid.
¹H-NMR(DMSO-d₆): δ 7.15(1H, d, J=8.8Hz), 7.65(2H, s), 7.73(1H, s), 7.81(1H, s), 7.82(1H, dd, J=8.7, 2.5Hz), 8.23(1H, d, J=2.5Hz), 8.38(2H, s), 10.87(1H, s), 11.15(1H, brs).

### Example 248: Preparation of the compound of Compound No. 248.

A mixture of 5-chlorosalicylic acid(87mg, 0.5mmol), 2,2-bis(3-amino-4-methylphenyl)-1,1,1,3,3,3-hexafluoropropane(363mg, 1mmol), phosphorus trichloride(44µL, 0.5mmol) and toluene(4mL) was refluxed for 4 hours. After the reaction mixture was cooled to room temperature, it was purified by column chromatography on silica gel(n-hexane:ethyl acetate=5:1) to give the white title compound(16mg, 4.9%). (The compound of Compound No. 251 described in the following Example 251 was obtained as a by-product.)
¹H-NMR(DMSO-d₆): δ 2.34(6H, s), 7.04(4H, d, J=8.8Hz), 7.39(2H, d, J=8.4Hz), 7.48(2H, dd, J=8.8, 2.9Hz), 7.96(2H, d, J=2.9Hz), 8.19(2H, s), 10.44(2H, s), 12.17(2H, s).

### Example 249: Preparation of the compound of Compound No. 249.

Using 3-phenylsalicylic acid and 3,5-bis(trifluoromethyl)aniline as the raw materials, the same operation as the Example 3 gave the title compound.
Yield: 64.6%.
¹H-NMR(DMSO-d₆): δ 7.12(1H, t, J=8.1Hz), 7.37(1H, tt, J=7.5, 1.5Hz), 7.43-7.48(2H, m), 7.56-7.60(3H, m), 7.91(1H, s), 8.07, (1H, dd, J=8.1, 1.5Hz), 8.48(2H, s), 11.00(1H, s), 12.16(1H, s).

### Example 250: Preparation of the compound of Compound No. 250.

Using 4-fluorosalicylic acid and 3,5-bis(trifluoromethyl)aniline as the raw materials, the same operation as the Example 3 gave the title compound.
Yield: 65.7%.
¹H-NMR(DMSO-d₆): δ 6.81-6.90(2H, m), 7.84(1H, s,), 7.93-7.98(1H, m,), 8.45(2H, s,), 10.78(1H, s), 11.81(1H, s,).

### Example 251: Preparation of the compound of Compound No. 251.

This compound was obtained by separation from the mixture with the compound of Compound No. 248 described in the aforementioned Example 248.
Yield: 9.4%.
¹H-NMR(CD₃OD): δ 2.16(3H, s), 2.34(3H, s), 6.69(1H, d, J=8.2Hz), 6.76(1H, brs)6.95(1H, d, J=8.8Hz), 7.02(1H, d, J=8.0Hz), 7.15(1H, d, J=8.2Hz), 7.29(1H, d, J=8.2Hz), 7.37(1H, dd, J=8.8, 2.6Hz), 7.97(1H, d, J=2.6Hz), 7.98(1H, s).

### Example 252: Preparation of the compound of Compound No. 252.

Using 5-chlorosalicylic acid and 4-[2-amino-4-(trifluromethyl)phenoxy]-benzonitrile as the raw materials, the same operation as the Example 3 gave the title compound.
Yield: 11.6%.
¹H-NMR(CD₃OD): δ 6.88(1H, d, J=8.6Hz), 7.19(2H, d, J=8.9Hz), 7.24(1H, d, J=8.6Hz), 7.33(1H, dd, J=8.8, 2.8Hz), 7.46(1H, dd, J=8.9, 1.9Hz), 7.76(2H, d, J=8.9Hz), 7.98(1H, d, J=2.7Hz), 8.96(1H, s).

### Example 253: Preparation of the compound of Compound No. 253.

Using 5-chlorosalicylic acid and 3-amino-4-(4-methoxyphenoxy)-benzotrifluoride as the raw materials, the same operation as the Example 3 gave the title compound.
Yield: 88.1%.
¹H-NMR(CDCl₃): δ 3.85(3H, s)6.81(1H, d, J=8.5Hz), 6.97-7.02(3H, m), 7.08(2H, d, J=8.8Hz), 7.30(1H, m), 7.40(1H, dd, J=8.8, 1.9Hz), 7.45(1H, d, J=2.2Hz), 8.70(1H, s), 8.78(1H, d, J=1.6Hz), 11.76(1H, s).

### Example 254: Preparation of the compound of Compound No. 254.

Using salicylic acid and 2,5-bis(trifluoromethyl)aniline as the raw materials, the same operation as the Example 3 gave the title compound.
Yield: 47.8%.
¹H-NMR(CD₃OD): δ 7.00-7.06(2H, m), 7.48(1H, dt, J=1.5, 7.5Hz), 7.74(1H, d, J=8.4Hz), 8.01-8.08(2H, m), 8.79(1H, s), 11.09(1H, s), 12.03(1H, s).

### Example 255: Preparation of the compound of Compound No. 255.

### (1) 2-Amino-4-(2,4-dichlorophenyl)thiazole.

Using 2',4'-dichloroacetophenone and thiourea as the raw materials, the same operation as the Example 231(1) gave the title compound.
Yield: 97.1%.
¹H-NMR(CDCl₃): δ 5.01(2H, s), 7.09(1H, s), 7.28(1H, dd, J=8.4, 2.1Hz), 7.45(1H, d, J=2.1Hz), 7.82(1H, d, J=8.4Hz).

### (2) 5-Chloro-2-hydroxy-N-[4-(2,4-dichlorophenyl)thiazol-2-yl]benzamide(Compound No. 255).

Using 5-chlorosalicylic acid and 2-amino-4-(2,4-dichlorophenyl)thiazole as the raw materials, the same operation as the Example 3 gave the title compound.
Yield: 8.0%.
¹H-NMR(DMSO-d₆): δ 7.08(1H, d, J=8.7Hz), 7.50-7.55(2H, m), 7.72-7.76(2H, m), 7.91(1H, d, J=8.4Hz), 7.95(1H, d, J=2.4Hz), 11.87(1H, brs), 12.09(1H, brs).

### Example 256: Preparation of the compound of Compound No. 256.

Using 3-isopropylsalicylic acid and 3,5-bis(trifluoromethyl)aniline as the raw materials, the same operation as the Example 3 gave the title compound.
Yield: 99.2%.
¹H-NMR(CDCl₃): δ 1.26(6H, d, J=6.9Hz), 3.44(1H, Hept, J=6.9Hz), 6.92(1H, t, J=7.8Hz), 7.38(1H, dd, J=8.1, 1.2Hz), 7.44(1H, d, J=7.5Hz), 7.69(1H, s), 8.13(3H, s), 11.88(1H, s).

### Example 257: Preparation of the compound of Compound No. 257.

Bromine(14.4 µL, 0.28mmol) and iron powder(1.7mg, 0.03mmol) were added to a solution of N-[3,5-bis(trifluoromethyl)phenyl]-2-hydroxy-3-isopropylbenzamide (Compound No. 256; 100mg, 0.26mmol) in carbon tetrachloride(5mL) under argon atmosphere, and the mixture was stirred at room temperature for 2 hours. The reaction mixture was diluted with ethyl acetate. The ethyl acetate layer was washed successively with water and brine, and dried over anhydrous magnesium sulfate. The residue obtained by evaporation of the solvent under reduced pressure was crystallized from n-hexane/ethyl acetate to give the title compound(110mg, 91.5%) as a white solid. ¹H-NMR(CDCl₃): δ 1.25(6H, d, J=6.9Hz), 3.39(1H, Hept, J=6.9Hz), 7.49-7.51(2H, m), 7.71(1H, brs), 8.11-8.14(3H, m), 11.81(1H, brs).

### Example 258: Preparation of the compound of Compound No. 258.

N-Bromosuccinimide(88.2mg, 0.50mmol) was added to a solution of N-[3,5-bis(trifluoromethyl)phenyl]-2-hydroxy-3-methylbenzamide(Compound No. 58; 150mg, 0.41mmol) in a mixed solvent of methanol/water(3:1; 5mL), and the mixture was stirred at room temperature for 10 minutes. The reaction mixture was diluted with ethyl acetate. The ethyl acetate layer was washed successively with 10% aqueous sodium thiosulfate, water and brine, and dried over anhydrous magnesium sulfate. The residue obtained by evaporation under reduced pressure was purified by column chromatography on silica gel(n-hexane:ethyl acetate=5:1) to give the title compound(167mg, 91.5%) as a white powder.
¹H-NMR(CDCl₃): δ 2.28(3H, s), 7.47(1H, s), 7.50(1H, d, J=2.4Hz), 7.71(1H, s), 8.08(1H, brs), 8.13(2H, s), 11.71(1H, s).

### Example 259: Preparation of the compound of Compound No. 259.

Using N-[3,5-bis(trifluoromethyl)phenyl]-2-hydroxy-3-phenylbenzamide (Compound No. 249), the same operation as the Example 258 gave the title compound.
Yield: 67.5%.
¹H-NMR(DMSO-d₆): δ 7.36-7.50(3H, m), 7.55-7.59(2H, m), 7.71(1H, d, J=2.1Hz), 7.93(1H, brs), 8.28(1H, d, J=2.1Hz), 8.45(2H, s), 11.06(1H, brs), 12.16(1H, brs).

### Example 260: Preparation of the compound of Compound No. 260.

### (1) 2-Amino-4-(3,4-dichlorophenyl)thiazole.

Using 3',4'-dichloroacetophenone and thiourea as the raw materials, the same operation as the Example 231(1) gave the title compound.
Yield: 77.8%.
¹H-NMR(DMSO-d₆): δ 7.17(2H, s), 7.24(1H, s), 7.62(1H, d, J=8.4Hz), 7.78(1H, dd, J=8.7, 2.7Hz), 8.22(1H, d, J=2.4Hz).

### (2) 5-Chloro-2-hydroxy-N-[4-(3,4-dichlorophenyl)thiazol-2-yl]benzamide(Compound No. 260).

Using 5-chlorosalicylic acid and 2-amino-4-(3,4-dichlorophenyl)thiazole as the raw materials, the same operation as the Example 3 gave the title compound.
Yield: 15.1%.
¹H-NMR(DMSO-d₆): δ 7.08(1H, d, J=8.7Hz), 7.52(1H, dd, J=8.7, 2.7Hz), 7.71(1H, d, J=8.4Hz), 7.91(1H, d, J=1.8Hz), 7.94(1H, s), 8.18(1H, d, J=1.5Hz), 12.09(2H, bs).

### Example 261: Preparation of the compound of Compound No. 261.

### (1) 2-Amino-4-[4-(trifluoromethyl)phenyl]thiazole.

Using 4'-(trifluoromethyl)acetophenone and thiourea as the raw materials, the same operation as the Example 231(1) gave the title compound.
Yield: 77.5%.
¹H-NMR(DMSO-d₆): δ 7.18(2H, s), 7.26(1H, s), 7.72(2H, d, J=8.4Hz), 8.00(2H, d, J=8.1Hz).

### (2) 5-Chloro-2-hydroxy-N-{4-[4-(trifluoromethyl)phenyl]thiazol-2-yl}benzamide (Compound No. 261).

Using 5-chlorosalicylic acid and 2-amino-4-[4-(trifluoromethyl)phenyl]thiazole as the raw materials, the same operation as the Example 3 gave the title compound.
Yield: 16.0%.
¹H-NMR(DMSO-d₆): δ 7.09(1H, d, J=9.0Hz), 7.53(1H, dd, J=8.7, 2.7Hz), 7.81(2H, d, J=8.4Hz), 7.96(1H, d, J=2.4Hz), 7.98(1H, s), 8.16(2H, d, J=8.1Hz), 11.91(1H, bs), 12.13(1H, bs).

### Example 262: Preparation of the compound of Compound No. 262.

### (1) Methyl 2-methoxy-4-phenylbenzoate.

Dichlorobis(triphenylphosphine)palladium(29mg, 0.04mmol) was added to a solution of methyl 4-chloro-2-methoxybenzoate(904mg, 4.5mmol), phenylboronic acid(500mg, 4.1mmol) and cesium carbonate(2.7g, 8.2mmol) in N,N-dimethylformamide(15mL) under argon atmosphere, and the mixture was stirred at 120°C for 8 hours. After the reaction mixture was cooled to room temperature, it was diluted with ethyl acetate. The ethyl acetate layer was washed successively with water and brine, and dried over anhydrous sodium sulfate. The residue obtained by evaporation of the solvent under reduced pressure was purified by column chromatography on silica gel(n-hexane:ethyl acetate=10:1) to give the title compound(410mg, 41.2%) as a colourless oil.
¹H-NMR(CDCl₃): δ 3.91(3H, s), 3.98(3H, s), 7.17(1H, d, J=1.5Hz), 7.20(1H, dd, J=8.1, 1.5Hz), 7.31-7.50(3H, m), 7.59-7.63(2H, m), 7.89(1H, d, J=8.1Hz).

### (2) 2-Methoxy-4-phenylbenzoic acid

2N Aqueous sodium hydroxide(5mL) was added to a solution of methyl 2-methoxy-4-phenylbenzoate(410mg, 1.69mmol) in methanol(5mL), and the mixture was refluxed for 1 hour. After the reaction mixture was cooled to room temperature, the solvent was evaporated under reduced pressure. 2N hydrochloric acid was added to the obtained residue and the separated crystal was filtered to give the title compound(371mg, 96.0%) as a crude product.
¹H-NMR(DMSO-d₆): δ 3.93(3H, s), 7.29(1H, dd, J=8.1, 1.5Hz), 7.34(1H, d, J=1.5Hz), 7.40-7.53(3H, m), 7.73-7.77(3H, m), 12.60(1H, s).

### (3) N-[3,5-Bis(trifluoromethyl)phenyl]-2-methoxy-4-phenylbenzamide.

Using 2-methoxy-4-phenylbenzoic acid and 3,5-bis(trifluoromethyl)aniline as the raw materials, the same operation as the Example 3 gave the title compound.
Yield: 97.5%.
¹H-NMR(CDCl₃): δ 4.19(3H, s), 7.25(1H, m), 7.38-7.53(4H, m), 7.62-7.65(3H, m), 8.12(2H, s), 8.35(1H, d, J=8.1Hz), 10.15(1H, brs).

### (4) N-[3,5-Bis(trifluoromethyl)phenyl]-2-hydroxy-4-phenylbenzamide(Compound No. 262).

1M Boron tribromide-dichloromethane solution(0.71mL, 0.71mmol) was added to a solution of N-[3,5-bis(trifluoromethyl)phenyl]-2-methoxy-4-phenylbenzamide(100mg, 0.24mmol) in dichloromethane(5mL), and the mixture was stirred at room temperature for 1 hour. The reaction mixture was diluted with ethyl acetate, washed successively with water and brine, and dried over anhydrous magnesium sulfate. The residue obtained by evaporation of the solvent under reduced pressure was purified by column chromatography on silica gel(n-hexane:ethyl acetate=5:1) to give the title compound(69.3mg, 71.6%) as a white powder.
¹H-NMR(DMSO-d₆): δ 7.20(1H, dd, J=8.4.1.8Hz), 7.30(1H, d, J=1.8Hz), 7.39-7.51(3H, m), 7.60-7.64(3H, m), 7.70(1H, brs), 8.15(2H, s), 8.19(1H, brs), 11.59(1H, s).

### Example 263: Preparation of the compound of Compound No. 263.

### (1) 2-Amino-4-(2,5-difluorophenyl)thiazole.

Using 2',5'-difluoroacetophenone and thiourea as the raw materials, the same operation as the Example 231(1) gave the title compound.
Yield: 77.8%.
¹H-NMR(DMSO-d₆): δ 7.45(1H, d, J=2.7Hz), 7.11-7.17(1H, m), 7.19(2H, s), 7.28-7.36(1H, m), 7.65-7.71(1H, m).

### (2) 5-Chloro-2-hydroxy-N-[4-(2,5-difluorophenyl)thiazol-2-yl]benzamide(Compound No. 263).

Using 5-chlorosalicylic acid and 2-amino-4-(2,5-difluorophenyl)thiazole as the raw materials, the same operation as the Example 3 gave the title compound.
Yield: 36.5%.
¹H-NMR(DMSO-d₆): δ 7.09(1H, d, J=8.7Hz), 7.22-7.30(1H, m), 7.37(1H, m), 7.53(1H, dd, J=8.7, 3.0Hz), 7.72(1H, d, J=2.4Hz), 7.77-7.84(1H, m), 7.94(1H, d, J=3.0Hz), 11.89(1H, bs), 12.12(1H, bs).

### Example 264: Preparation of the compound of Compound No. 264.

### (1) 2-Amino-4-(4-methoxyphenyl)thiazole.

Using 4'-methoxyacetophenone and thiourea as the raw materials, the same operation as the Example 231(1) gave the title compound.
Yield: 85.2%.
¹H-NMR(DMSO-d₆): δ 3.76(3H, s), 6.82(1H, s), 6.92(2H, d, J=9.0Hz), 7.01(2H, s), 7.72(2H, d, J=8.7Hz).

### (2) 5-Chloro-2-hydroxy-N-[4-(4-methoxyphenyl)thiazol-2-yl]benzamide(Compound No. 264).

Using 5-chlorosalicylic acid and 2-amino-4-(4-methoxyphenyl)thiazole as the raw materials, the same operation as the Example 3 gave the title compound.
Yield: 16.4%.
¹H-NMR(DMSO-d₆): δ 3.80(3H, s), 7.01(2H, d, J=9.0Hz), 7.07(1H, d, J=8.7Hz), 7.50-7.55(2H, m), 7.86(2H, d, J=9.0Hz), 7.96(1H, d, J=2.7Hz), 11.90(1H, bs), 12.04(1H, bs).

### Example 265: Preparation of the compound of Compound No. 265.

### (1) 2-Amino-4-[3-(trifluoromethyl)phenyl]thiazole.

Using 3'-(trifluoromethyl)acetophenone and thiourea as the raw materials, the same operation as the Example 231(1) gave the title compound.
Yield: 94.1%.
¹H-NMR(DMSO-d₆): δ 7.19(2H, s), 7.27(1H, s), 7.61(2H, dd, J=3.9, 1.5Hz), 8.07-8.13(2H, m).

### (2) 5-Chloro-2-hydroxy-N-{4-[3-(trifluoromethyl)phenyl]thiazol-2-yl}benzamide (Compound No. 265).

Using 5-chlorosalicylic acid and 2-amino-4-[3-(trifluoromethyl)phenyl]thiazole as the raw materials, the same operation as the Example 3 gave the title compound.
Yield: 31.0%.
¹H-NMR(DMSO-d₆): δ 7.13(1H, d, J=8.7Hz), 7.53(1H, dd, J=9.0, 2.7Hz), 7.70(1H, d, J=2.4Hz), 7.71(1H, d, J=1.2Hz), 7.95(1H, d, J=2.7Hz), 8.00(1H, s), 8.24-8.27(2H, m), 12.16(2H, bs).

### Example 266: Preparation of the compound of Compound No. 266.

### (1) 2-Amino-4-(2,3,4,5,6-pentafluorophenyl)thiazole.

Using 2',3',4',5',6'-pentafluoroacetophenone and thiourea as the raw materials, the same operation as the Example 231(1) gave the title compound.
Yield: 86.7%.
¹H-NMR(CDCl₃): δ 5.19(2H, s), 6.83(1H, s).

### (2) 5-Chloro-2-hydroxy-N-[4-(2,3,4,5,6-pentafluorophenyl)thiazol-2-yl]benzamide (Compound No. 266).

Using 5-chlorosalicylic acid and 2-amino-4-(2,3,4,5,6-pentafluorophenyl)-thiazole as the raw materials, the same operation as the Example 3 gave the title compound.
Yield: 23.8%.
¹H-NMR(DMSO-d₆): δ 7.08(1H, d, J=8.7Hz), 7.53(1H, dd, J=8.7, 2.7Hz), 7.73(1H, s), 7.93(1H, d, J=2.7Hz), 11.85(1H, bs), 12.15(1H, bs).

### Example 267: Preparation of the compound of Compound No. 267.

Using 5-chlorosalicylic acid and 2-amino-4-methylbenzophenone as the raw materials, the same operation as the Example 3 gave the title compound.
Yield: 8.7%.
¹H-NMR(CDCl₃): δ 2.50(3H, s), 6.98(1H, d, J=8.3Hz), 6.99(1H, d, J=7.3Hz), 7.39(1H, dd, J=2.0, 8.6Hz), 7.48-7.64(4H, m), 7.72(2H, d, J=7.6Hz), 7.83(1H, d, J=2.3Hz), 8.57(1H, s), 12.18(1H, s), 12.34(1H, br.s).

### Example 268: Preparation of the compound of Compound No. 268.

Iron(3mg, 0.05mmol) and bromine(129 µ l, 2.5mmol) were added to a solution of 2-hydroxy-N-[2,5-bis(trifluoromethyl)phenyl]benzamide(Compound No. 254; 175mg, 0.5mmol) in carbon tetrachloride(5mL), and the mixture was stirred at 50°C for 12 hours. After the reaction mixture was cooled to room temperature, it was washed with saturated aqueous sodium hydrogen carbonate, water and brine, and dried over anhydrous magnesium sulfate. The residue obtained by evaporation of the solvent under reduced pressure was purified by column chromatography on silica gel(n-hexane:ethyl acetate=2:1) to give the title compound(184.2mg, 72.7%) as a white crystal.
¹H-NMR(DMSO-d₆): δ 7.92-7.98(1H, m), 8.06(1H, d, J=2.1Hz), 8.09(1H, d, J=8.4Hz), 8.22(1H, d, J=2.1Hz), 8.27-8.32(1H, m), 11.31(1H, s).

### Test Example 1: Inhibitory test against cancer cell proliferation (1)

Cancer cells(Jurkat: Human T-cell leukemia, MIA PACA-2: Human pancreatic cancer, RD: Human rhabdomyoma, HepG2: Human liver cancer) were cultured for 3 days in the presence or absence of a test compound using RPMI1640 medium containing 10% bovine fetal serum or Dalbecco's Modified Eagle's Medium containing 10% FBS. The number of living cells was counted by MTS method, and amounts of cell proliferation were compared and inhibitory ratios were measured. In the table below, 50% inhibitory concentrations against the proliferation of each cancer cell are shown.

| Compound Number | IC₅₀(µM) | | | |
|---|---|---|---|---|
| | Jurkat | MIA PaCa-2 | RD | HepG2 |
| 4 | 0.74 | 0.65 | 1.03 | 0.69 |
| 6 | 0.38 | 0.60 | 0.74 | 0.61 |
| 11 | 1.21 | 0.78 | 1.96 | 1.82 |
| 19 | 2.06 | 1.75 | 2.84 | 2.63 |
| 23 | 1.99 | 1.53 | 2.01 | 1.96 |
| 27 | 1.20 | 1.19 | 1.26 | 1.96 |
| 29 | 1.64 | 1.55 | 2.20 | 1.84 |
| 51 | 1.28 | 1.03 | 1.31 | 1.88 |
| 90 | 0.48 | 0.51 | 0.49 | 1.97 |
| 93 | 1.43 | 0.81 | 1.87 | 1.99 |
| 140 | 2.43 | 1.42 | 3.19 | 2.57 |
| 199 | 0.44 | 0.46 | 0.57 | 1.26 |
| 201 | 0.57 | 0.49 | 0.59 | 1.37 |
| 205 | 1.89 | 1.45 | 1.94 | 3.50 |
| 207 | 1.64 | 1.26 | 1.52 | 1.76 |

### Test Example 2: Inhibitory test against cancer cell proliferation (2)

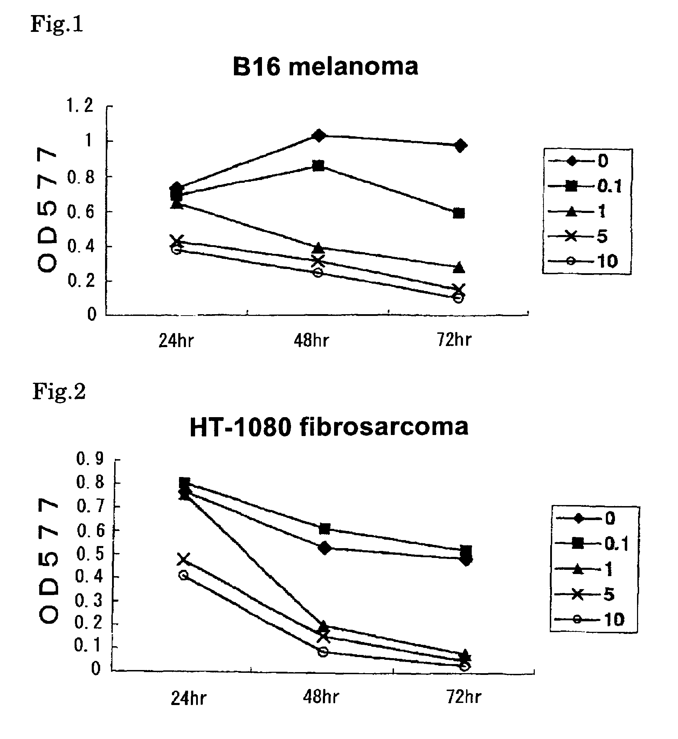

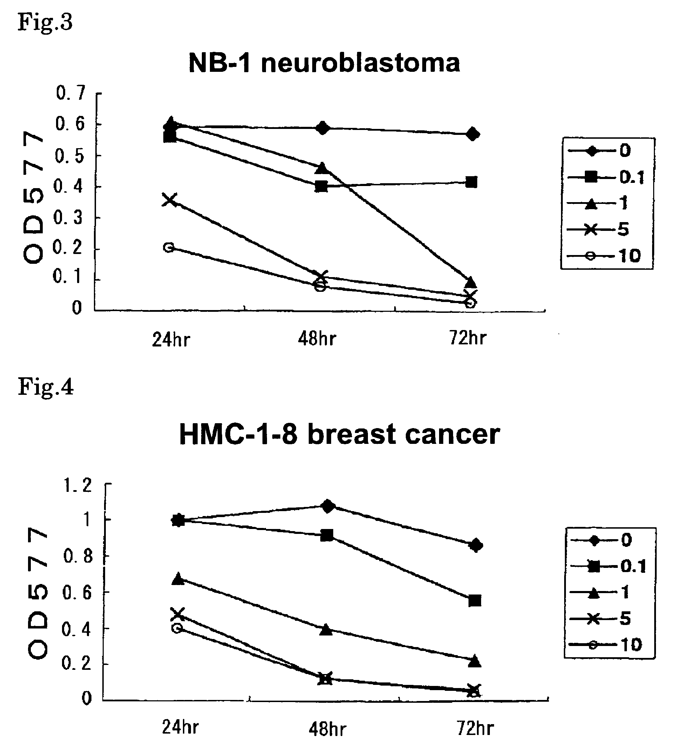

Cancer cells(B16 melanoma, HT-1080 fibrosarcoma, NB-1 neuroblastoma, HMC-1-8 breast cancer) were cultured in the presence of a test compound(0.1, 1.0, 5.0, 10 µM) or in the absence of the test compound using Modified Eagle's Medium containing 5% bovine fetal serum without phenol red or RPMI1640 medium containing 5% bovine fetal serum. After 24, 48 and 72 hours, the number of living cells was counted by the MTT method. The results of Compound No. 4 obtained by the above method are shown in Figs. 1 to 4.

### Test Example 3: Metastasis inhibitory test using B16 mouse bearing B16 melanoma

B16 melanoma cells (5 x 10⁵ cells/mouse) were inoculated from a tail vein of a B6 mouse of the same kind by intravenous injection, and a test substance was administered once a day for 5 weeks from the day of the inoculation by intraperitoneal injection. Then, the test animal was sacrificed and the lungs were removed. The number of colonies of melanoma in the lungs was compared with that in the control(test substance administered at 0 mg/kg). The results are shown below.

| | Compound Number | Dose(mg/kg) | Survival Ratio after 5 weeks(%) | Colony Formation | |
|---|---|---|---|---|---|
| | - | 0 | 50 | - | |
| | 4 | 30 | 100 | + + | |
| ±: Same as control; +: Inhibited; + + : Remarkably inhibited; + + +: No Formation | | | | | |

### Test Example 4: Toxicity test by continuous administration

A test compound(30mg/kg) was administered intraperitoneally to a 6-week-old male SD rat once a day for 4 weeks. After the administration was completed, urinalysis, hematoscopy, and biochemical examination of blood were carried out, and as a result, no findings that indicated toxicity were observed. These results indicate that, at an effective dose to exhibit anticancer action, the medicament of the present invention has no toxic action that relate to side effects observed with existing anticancer agents such as hepatic disorder, renal disorder, and myelosuppression.

### Test Example 5: Anticancer effects on tumors

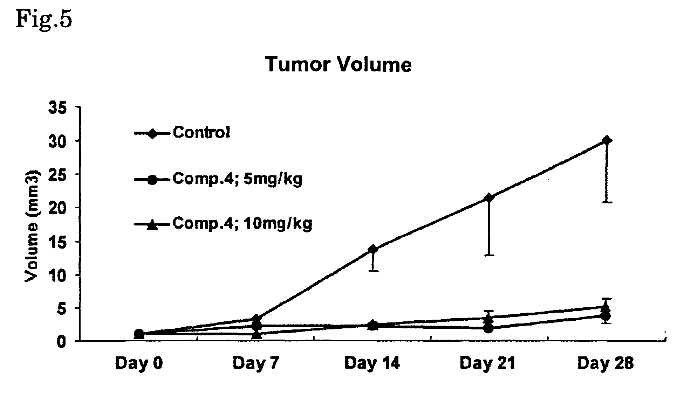

Human breast cancer cells engrafted in a nude mouse and sufficiently proliferated were isolated and cut in 5mm squares. The cells were transplanted in the back of 4-weel-old female nude mice under ether anesthesia. Two weeks after the transplantation of the tumor, a test compound was administered intraperitoneally once a day. The day before the start of the administration of the test compound was regarded as 0 days(day 0), and each volume of the tumor(tumor volume; unit: mm³) after 7days, 14 days, 21 days, and 28 days was measured. The results when 5mg/kg or 10mg/kg of Compound No.4 was administered as the test compound and those of the control(test compound dose: 0 mg/kg) are shown in Fig.5. In Fig.5, "control" shows the result when 0 mg/kg of the test compound was administered, and "Compound 4" shows the result when Compound No.4 was administered.

### Test Example 6: Inhibitory test against cancer cell proliferation (3)

Similar operations to Test Example 1 were carried out using cancer cells(HepG2: human liver cancer, A549: human lung cancer, MIA PACA-2: human pancreatic cancer). In the table below, 50% inhibitory concentrations against proliferation of each cancer cell are shown.

| Compound Number | IC₅₀(µM) | | |
|---|---|---|---|
| | HepG2 | A549 | MIA PaCa-2 |
| 4 | 0.72 | 4.03 | 0.82 |
| 75 | 0.79 | 2.06 | 0.95 |
| 189 | 1.30 | 6.47 | 2.15 |
| 192 | 11.02 | 23.91 | 9.42 |
| 199 | 0.59 | 5.15 | 0.56 |
| 205 | 4.23 | >10 | >10 |
| 213 | 3.41 | 7.43 | 4.69 |
| 215 | 4.98 | 8.31 | 2.76 |

### Industrial Applicability

The medicament of the present invention has superior anticancer actions and reduced side effects and toxicity. Therefore, the medicament is useful as an agent for preventive and/or therapeutic treatment of cancers.

## Claims

1. A medicament for the prevention and/or treatment of cancers which comprises as an active ingredient a substance selected from the group consisting of a compound represented by the following general formula (I) and a pharmacologically acceptable salt thereof, and a hydrate thereof and a solvate thereof: 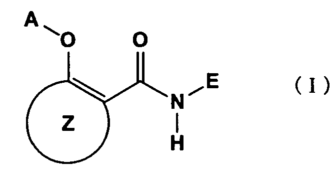 wherein A represents hydrogen atom or acetyl group,
E represents a 2,5-di-substituted or a 3,5-di-substituted phenyl group, or a monocyclic or a fused polycyclic heteroaryl group which may be substituted, provided that the compound wherein said heteroaryl group is ① a fused polycyclic heteroaryl group wherein the ring which binds directly to ―CONH― group in the formula (I) is a benzene ring, ② unsubstituted thiazol-2-yl group, or ③ unsubstituted benzothiazol-2-yl group is excluded,
ring Z represents an arene which may have one or more substituents in addition to the group represented by formula ―O―A wherein A has the same meaning as that defined above and the group represented by formula ―CONH―E wherein E has the same meaning as that defined above, or a heteroarene which may have one or more substituents in addition to the group represented by formula ―O―A wherein A has the same meaning as that defined above and the group represented by formula ― CONH―E wherein E has the same meaning as that defined above.

2. The medicament according to claim 1, wherein A is a hydrogen atom.

3. The medicament according to any one of claims 1 or 2, wherein ring Z is a C₆ to C₁₀ arene which may have one or more substituents in addition to the group represented by formula ―O―A wherein A has the same meaning as that defined in the general formula (I) and the group represented by formula ―CONH―E wherein E has the same meaning as that defined in the general formula (I), or a 5 to 10-membered heteroarene which may have one or more substituents in addition to the group represented by formula ―O―A wherein A has the same meaning as that defined in the general formula (I) and the group represented by formula ―CONH―E wherein E has the same meaning as that defined in the general formula (I).

4. The medicament according to claim 3, wherein ring Z is a benzene ring which may have one or more substituents in addition to the group represented by formula ―O―A wherein A has the same meaning as that defined in the general formula (I) and the group represented by formula ―CONH―E wherein E has the same meaning as that defined in the general formula (I), or a naphthalene ring which may have one or more substituents in addition to the group represented by formula ― O―A wherein A has the same meaning as that defined in the general formula (I) and the group represented by formula ―CONH―E wherein E has the same meaning as that defined in the general formula (I).

5. The medicament according to claim 4, wherein ring Z is a benzene ring which is substituted with halogen atom(s) in addition to the group represented by formula ―O―A wherein A has the same meaning as that defined in the general formula (I) and the group represented by formula ―CONH―E wherein E has the same meaning as that defined in the general formula (I).

6. The medicament according to claim 4, wherein ring Z is a naphthalene ring.

7. The medicament according to any one of claims 1 to 6, wherein E is a 2,5-di-substituted phenyl group or a 3,5-di-substituted phenyl group.

8. The medicament according to claim 7, wherein E is a 2,5-di-substituted phenyl group wherein at least one of the said substituents is trifluoromethyl group, or a 3,5-di-substituted phenyl group wherein at least one of the said substituents is trifluoromethyl group.

9. The medicament according to claim 8, wherein E is 3,5-bis(trifluoromethyl)phenyl group.

10. The medicament according to any one of claims 1 to 6, wherein E is a monocyclic or a fused polycyclic heteroaryl group which may be substituted, provided that the compound wherein said heteroaryl group is ① a fused polycyclic heteroaryl group wherein the ring which binds directly to ―CONH― group in the formula (I) is a benzene ring, ② unsubstituted thiazol-2-yl group, or ③ unsubstituted benzothiazol-2-yl group is excluded.

11. The medicament according to claim 10, wherein E is a 5-membered monocyclic heteroaryl group which may be substituted, provided that the compounds wherein said heteroaryl group is unsubstituted thiazol-2-yl group are excluded.
